(19) Europäisches Patentamt
European Patent Office
Office européen des brevets

(11) EP 2 423 182 A1

(12) **EUROPEAN PATENT APPLICATION**
published in accordance with Art. 153(4) EPC

(43) Date of publication:
**29.02.2012 Bulletin 2012/09**

(21) Application number: **10767025.9**

(22) Date of filing: **19.04.2010**

(51) Int Cl.:
***C07C 235/46*** *(2006.01)* ***A61K 31/343*** *(2006.01)*
***A61K 31/351*** *(2006.01)* ***A61K 31/357*** *(2006.01)*
***A61K 31/36*** *(2006.01)* ***A61K 31/381*** *(2006.01)*
***A61K 31/40*** *(2006.01)* ***A61K 31/402*** *(2006.01)*
***A61K 31/4035*** *(2006.01)* ***A61K 31/404*** *(2006.01)*
***A61K 31/41*** *(2006.01)* ***A61K 31/415*** *(2006.01)*
***A61K 31/421*** *(2006.01)*

(86) International application number:
**PCT/JP2010/056901**

(87) International publication number:
**WO 2010/122968 (28.10.2010 Gazette 2010/43)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **21.04.2009 JP 2009102832**
**27.10.2009 JP 2009246264**

(71) Applicant: **Astellas Pharma Inc.**
**Tokyo 103-8411 (JP)**

(72) Inventors:
- **KAWANO, Tomoaki**
**Tokyo 103-8411 (JP)**
- **YONETOKU, Yasuhiro**
**Tokyo 103-8411 (JP)**
- **HANAZAWA, Takeshi**
**Tokyo 103-8411 (JP)**
- **NIGAWARA, Takahiro**
**Tokyo 103-8411 (JP)**
- **FUKUDOME, Hiroki**
**Tokyo 103-8411 (JP)**
- **MORITANI, Hiroshi**
**Tokyo 103-8411 (JP)**

(74) Representative: **Bates, Philip Ian**
**Reddie & Grose**
**16 Theobalds Road**
**London**
**WC1X 8PL (GB)**

(54) **DIACYLETHYLENEDIAMINE COMPOUND**

(57) [Problem]

A compound which is useful as an anti-obesity agent is provided.

[Means for Solution]

The present inventors have investigated a compound having a DGAT1 inhibitory action, which is promising as an active ingredient of a pharmaceutical composition for treating obesity, type II diabetes mellitus, fatty liver, and diseases associated with these diseases, and as a result, they have found that the diacylethylenediamine compound of the present invention has an excellent DGAT1 inhibitory action, thereby completing the present invention. That is, the diacylethylenediamine compound of the present invention has a DGAT1 inhibitory action, and can be therefore used as an agent for preventing and/or treating obesity, type II diabetes mellitus, fatty liver, and diseases associated with these diseases.

EP 2 423 182 A1

## Description

Technical Field

The present invention relates to a diacylethylenediamine compound which is useful as an active ingredient of a pharmaceutical composition, for example, a pharmaceutical composition for treating obesity.

Background Art

Obesity is a state in which there is an imbalance between energy intake and energy consumption in a biological body, and in which excess energy is over-accumulated in the adipose tissues as neutral fats, mainly triglycerides, and is deeply related to the onset and progress of diseases such as insulin resistance, diabetes, arteriosclerosis, non-alcoholic steatohepatitis, or hypertension. Further, it is known that accumulation of excess triglycerides in the liver, muscles, and the like, as well as the adipose tissues, causes dysfunction in these tissues. Recently, the number of patients suffering from obesity is increasing as lifestyles have changed, but the methods for treating obesity are limited. Therefore, there is a demand for the development of a new drug for treating obesity.

DGAT is an enzyme involved in a final step of a triglyceride biosynthesis pathway, that is, a reaction for producing triglyceride from diacylglycerol and fatty acyl-CoA, and the subtypes, DGAT1 and DGAT2, have been reported. It has been clarified that the amino acid sequence of DGAT1 has a low homology with DGAT2 and has a high homology with ACAT (Proc. Nat. Acad. Sci. 95:13018-13023, 1998; J. Biol. Chem. 276:38870-38876,2001). As a phenotype of a DGAT1 knockout mice, resistance to high-fat diet-induced obesity, improved insulin resistance, increased leptin sensitivity, decrease in the amount of fat in the liver, and increased energy consumption, and the like have been reported (Nature Genetics 25: 87-90, 2000; J. Clin. Invest. 109:1049-1055, 2002). In addition, DGAT1 hetero-knockout mice show an intermediate phenotype between a wild type and homo-deficient mice (Arterioscler. Thromb. Vasc. Biol. 25; 482-486, 2005), and accordingly, DGAT1 inhibition is considered to be promising as a target for drug therapy for obesity, type II diabetes mellitus, fatty liver, and other related diseases derived from these diseases.

For example, it has been reported that a compound represented by the following formula has a DGAT1 inhibitory action (Patent Document 1).

[Chem. 1]

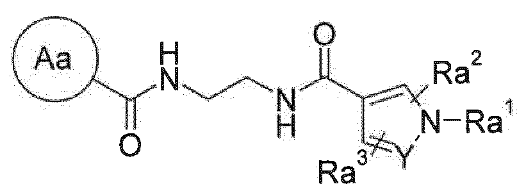

[refer to the patent publication for the symbols in the formula].

Furthermore, it has been reported that a compound represented by the following formula has a DGAT1 inhibitory action (Patent Document 2).

[Chem. 2]

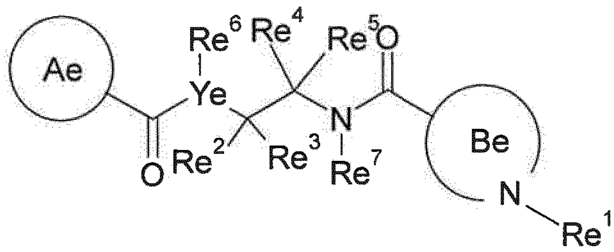

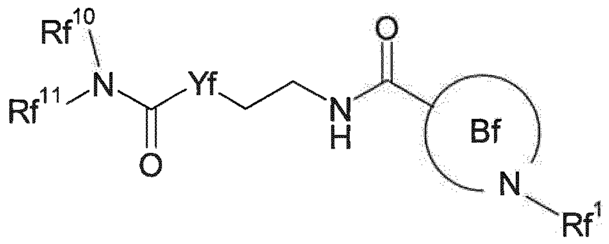

[refer to the patent publication for the symbols in the formula].

Furthermore, it has been reported that a compound represented by the following formula has a DGAT1 inhibitory action (Patent Document 3).

[Chem. 3]

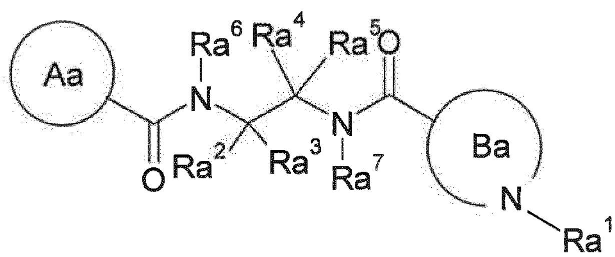

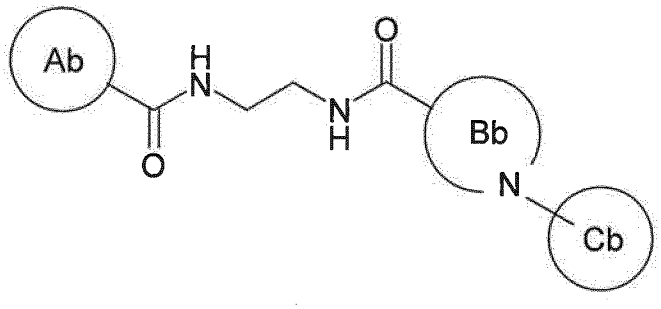

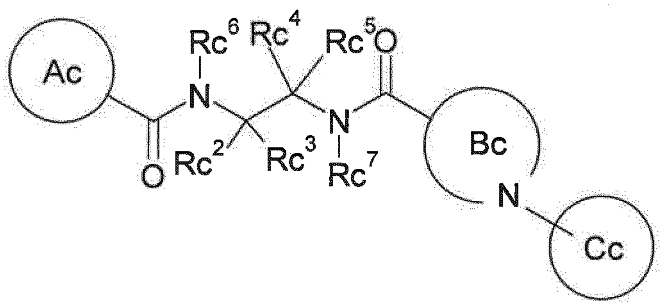

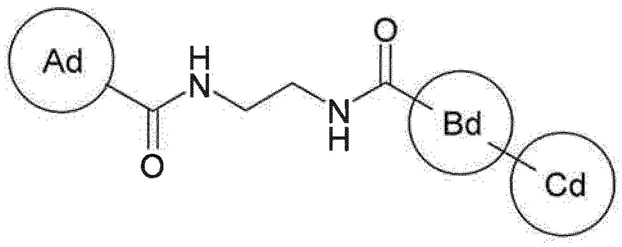

[refer to the patent publication for the symbols in the formula].

Moreover, in addition to the above, a compound having a DGAT1 inhibitory action has been reported (Patent Document 4, Patent Document 5, Patent Document 6, and Patent Document 7).

Furthermore, there have been reports on several diacylethylenediamines (Patent Document 8 and Non-Patent Document 1).

However, there is no disclosure or suggestion of the compound of the formula (I) or a salt thereof according to the present invention in any of the above literature.

Related Art

Patent Document

[Patent Document 1] Pamphlet of International Publication WO 2006/082952
[Patent Document 2] Pamphlet of International Publication WO 2008/011130
[Patent Document 3] Pamphlet of International Publication WO 2008/011131
[Patent Document 4] Pamphlet of International Publication WO 2007/141517
[Patent Document 5] Pamphlet of International Publication WO 2007/138304
[Patent Document 6] Pamphlet of International Publication WO 2007/138311
[Patent Document 7] Pamphlet of International Publication WO 2006/064189
[Patent Document 8] Pamphlet of International Publication WO 2009/076618

Non-Patent Document

[Non-Patent Document 1] chemical Research in Chinese Universities, 2009, Vol. 25, No. 2, pp. 178 to 182

Summary of the Invention

Problems to Be Solved by the Invention

A diacylethylenediamine compound which is useful as an active ingredient of a pharmaceutical composition, for example, a pharmaceutical composition for treating obesity is provided.

Means for solving the Problems

The present inventors have extensively studied a compound having a DGAT1 inhibitory action, and as a result, they have found that the diacylethylenediamine compound of the present invention has a DGAT1 inhibitory action, thereby completing the present invention.

That is, the present invention relates to a compound of the formula (I) or a salt thereof, and a pharmaceutical composition comprising a compound of the formula (I) or a salt thereof, and a pharmaceutically acceptable excipient.

[Chem. 4]

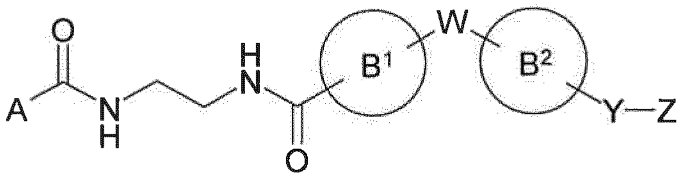

(I)

(wherein A represents aryl which may be substituted, cycloalkyl which may be substituted, an aromatic heterocycle which may be substituted, a non-aromatic heterocycle which may be substituted, or a group represented by the formula (II):

[Chem. 5]

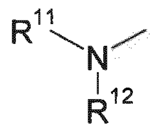

(II)

in which $R^{11}$ and $R^{12}$ are the same as or different from each other, and represent- H, $C_{1-6}$ alkyl, aryl which may be substituted, or $C_{3-8}$ cycloalkyl which may be substituted, provided that $R^{11}$ and $R^{12}$ are not -H at the same time, and $R^{11}$ and $R^{12}$ may be combined with the nitrogen atom to which they bind to form cyclic amino which may be substituted,

Ring $B^1$ represents phenylene, pyridinediyl, naphthalenediyl, or 1,2,3,4-tetrahydronaphthalenediyl, each of which may be substituted with at least one group selected from the group consisting of -OH, $C_{1-6}$ alkyl which may be substituted with at least one halogen atom, -O-$C_{1-6}$ alkyl which may be substituted with at least one halogen atom, $C_{3-8}$ cycloalkyl, and halogen,

W represents -O-, a bond, -O-$C_{1-6}$ alkylene, -NH-, or $C_{1-6}$ alkylene,

Ring $B^2$ represents cyclohexanediyl, cyclopentanediyl, or a bridged ring, each of which may be substituted with $C_{1-6}$ alkyl, and in the case where W is a bond, it may represent piperidinediyl or 8-azabicyclo[3.2.1]octanediyl,

Y represents a bond, $C_{1-6}$ alkylene, or -O-$C_{1-6}$ alkylene, and

Z represents -$CO_2$H or a biological equivalent thereof; carbamoyl which may be substituted with one or two groups selected from $C_{1-6}$ alkyl (in which the $C_{1-6}$ alkyl may be substituted with amino or carboxyl), phenyl and benzyl; -CO-(cyclic amino which may be substituted with one or two $C_{1-6}$ alkyl groups); -OH; amino which may be substituted with one or two $C_{1-6}$ alkyl groups; -NH-C(=O)-$C_{1-6}$ alkyl; or -NH-C(=O)-$C_{3-8}$ cycloalkyl).

In this regard, when the symbols in any of the chemical formulae in the present specification are also used in other formulae, the same symbols denote the same meanings, unless there is no specific instruction.

Furthermore, the present invention relates to a pharmaceutical composition for preventing or treating obesity, including a compound or a salt thereof of the formula (I). Further, the pharmaceutical composition includes an agent for preventing or treating obesity, including the compound of the formula (I) or a salt thereof.

In addition, the present invention relates to use of the compound of the formula (I) or a salt thereof for the manufacture of a pharmaceutical composition for preventing or treating obesity, the compound of the formula (I) or a salt thereof for use in the prevention or treating of obesity and a method for preventing or treating obesity, comprising administering to a subject an effective amount of the compound of the formula (I) or a salt thereof Here, the "subject" refers to humans or other animals in need of the prevention or treatment thereof, and in a certain embodiment, humans in need of the prevention or treatment thereof.

Effects of the Invention

The compound of the formula (I) or a salt thereof has a DGAT1 inhibitory action, and can be used as an agent for preventing and/or treating obesity.

Embodiments for Carrying Out the Invention

Hereinafter, the present invention will be described in detail.

In the present specification, the "alkyl" includes a straight alkyl and a branched alkyl. Accordingly, the "$C_{1-6}$ alkyl" is a straight or a branched alkyl having 1 to 6 carbon atoms, for example, methyl, ethyl, propyl, isopropyl, butyl, tert-butyl, pentyl, hexyl, or the like, in another embodiment, methyl, ethyl, propyl, or isopropyl, in a further embodiment, methyl or ethyl, and in a further embodiment, methyl.

The "alkylene" is a divalent group formed by the removal of any one hydrogen atom of the "alkyl" above. Accordingly, the "$C_{1-6}$ alkylene" is a straight or a branched alkylene having 1 to 6 carbon atoms, for example, methylene, ethylene, trimethylene, tetramethylene, pentamethylene, hexamethylene, methylmethylene, dimethylmethylene, ethylmethylene, methylethylene, dimethylethylene, ethylethylene, or the like, in another embodiment, methylene or ethylene, and in a further embodiment, methylene.

The "aryl" is a monocyclic to tricyclic aromatic hydrocarbon ring group having 6 to 14 carbon atoms. Specific examples thereof include phenyl and naphthyl, in another embodiment, phenyl, and in a further embodiment, naphthyl.

The "cycloalkyl" is a saturated hydrocarbon ring group having 3 to 8 ring members, the cycloalkyl may have a bridge and may be fused with a benzene ring, and a proportion of the bonds may be unsaturated. Specific examples thereof include cyclopropyl, cyclobutyl, cyclopentyl, cyclohexyl, cyclohexenyl, cycloheptyl, cyclooctyl, cyclooctadienyl, norbornyl, bicyclo[2.2.2]octyl, bicyclo[3.1.1]heptyl, bicyclo[4.1.0]heptyl, bicyclo[3.2.1]octyl, adamantyl, indanyl, indenyl, 1,2-dihydronaphthyl, 1,2,3,4-tetrahydronaphthyl, and the like.

The "bridged ring" is a divalent group of a saturated hydrocarbon ring having 6 to 10 ring members containing a bridge. Specific examples thereof include divalent groups such as bicyclo[3.1.0]hexane, norbomane, bicyclo[2.2.2]octane, bicyclo[3.1.1]heptane, bicyclo[4.1.0]heptane, bicyclo[3.2.1]octane, adamantane, and the like.

The "aromatic heterocycle" is an aromatic heterocycle group having 5 to 6 ring members, containing at least one hetero atom selected from O, N, and S as a ring-constituting atom, and the aromatic heterocycle may be fused with a benzene ring or a thiophene ring. Specific examples thereof include pyrrolyl, furyl, thienyl, pyrazolyl, imidazolyl, oxazolyl, thiazolyl, oxadiazolyl, thiadiazolyl, pyridyl, pyridazinyl, pyrimidinyl, pyradyl, indolyl, isoindolyl, benzofuryl, benzothienyl, indazolyl, benzoimidazolyl, benzooxazolyl, benzothiazolyl, quinolyl, isoquinolyl, cinnolinyl, phthalazinyl, quinazolinyl, quinoxalinyl, thienopyridyl, thienopyrimidinyl, thienopyrazyl, and the like.

The "non-aromatic heterocycle" is a non-aromatic heterocycle group having 3 to 7 ring members, containing at least one hetero atom selected from O, N, and S as a ring-constituting atom, the non-aromatic heterocycle may be fused with a benzene ring, a thiophene ring, or a cyclohexane ring, and a proportion of the bonds may be unsaturated. Further, the sulfur atom that is a ring-constituting atom may be oxidized. Specific examples of the non-aromatic heterocycle include aziridinyl, azetidinyl, pyrrolidinyl, piperidinyl, azepanyl, oxetanyl, tetrahydrofuryl, tetrahydropyranyl, thietanyl, tetrahydrothienyl, tetrahydrothiopyranyl, 1,1-dioxidetetrahydrothiopyranyl, oxazolidinyl, thiazolidinyl, 1,1-dioxidethiazolidinyl, isoxazolidinyl, isothiazolidinyl, 1,1-dioxideisothiazolidinyl, piperazinyl, morpholinyl, thiomorpholinyl, 1,1-dioxidethiomorpholinyl, dioxanyl, indolinyl, isoindolinyl, dihydroquinolyl, tetrahydroquinolyl, dihydroisoquinolyl, tetrahydroisoquinolyl, decahydroisoquinolyl, tetrahydrothienopyridyl, tetrahydrobenzoazepine, tetrahydrobenzodiazepine, dihydrobenzofuryl, dihydrobenzothienyl, dihydrobenzopyranyl, dihydrobenzothiopyranyl, dihydrobenzodioxynyl, benzodioxolyl, and the like.

The "cyclic amino" is a non-aromatic heterocycle group having a nitrogen atom among the above "non-aromatic heterocycles", which has a bonding arm on the nitrogen atom, and specific examples thereof include pyrrolidin-1-yl, piperidin-1-yl, azepan-1-yl, oxazolidin-3-yl, thiazolidin-3-yl, 1,1-dioxidethiazolidin-3-yl, isoxazolidin-2-yl, isothiazolidin-2-yl, 1,1-dioxideisothiazolidin-2-yl, piperazin-1-yl, morpholin-4-yl, thiomorpholin-4-yl, 1,1-dioxidethiomorpholin-4-yl, indolin-1-yl, isoindolin-2-yl, 1,2,3,4-tetrahydroquinolin-1-yl, 1,2,3,4-tetrahydroisoquinolin-2-yl, decahydroquinolin-1-yl, decahydroquinolin-2-yl, 4,5,6,7-tetrahydrothieno[3,2-c]pyridin-5-yl, and the like.

The "halogen" means -F, -Cl, -Br, or -I, in another embodiment, -F, -Cl, or -Br, in a further embodiment, -F or -Cl, and in a further embodiment, -F.

The "$-CO_2H$ or a biological equivalent thereof" means $-CO_2H$, or another atom or an atom group, which is electronically or sterically configuration equivalent to $-CO_2H$, is capable of releasing acidic protons, and has common biological properties. Example thereof include $-CO_2H$, -CO-NH-OH, -CO-NH-O-$C_{1-6}$ alkyl, -CO-NH-CN, -CO-NH-$SO_2$-$C_{1-6}$ alkyl, -CO-NH-$SO_2$-N($C_{1-6}$ alkyl)$_2$, or tetrazolyl, oxadiazolonyl, oxadiazolethionyl, oxathiadiazolyl, thiadiazolonyl, triazolethionyl, hydroxyisoxazole, and the like, in another embodiment, $-CO_2H$ -CO-NH-$SO_2$-$C_{1-6}$ alkyl, -CO-NH-$SO_2$-N($C_{1-6}$ alkyl)$_2$, and tetrazolyl, and in a further embodiment, $-CO_2H$.

An embodiment of "phenylene" is 1,4-phenylene, an embodiment of "pyridinediyl" is pyridine-2,5-diyl or pyridine-3,6-diyl, an embodiment of "naphthalenediyl" is naphthalene-2,6-diyl or naphthalene-3,7-diyl, an embodiment of "1,2,3,4-tetrahydronaphthalenediyl" is 1,2,3,4-tetrahydronaphthalene-2,6-diyl or 1,2,3,4-tetrahydronaphthalene-3,7-diyl, an embodiment of "cyclohexanediyl" is cyclohexane-1,4-diyl, an embodiment of "cyclopentanediyl" is cyclopente-1,3-diyl, an embodiment of "piperidinediyl" is piperidine-1,4-diyl, and an embodiment of "8-azabicyclo[3.2.1]octanediyl" is 8-azabicyclo[3.2.1]octane-3,8-diyl.

In the present specification, the expression "which may be substituted" represents "which is not substituted" or "which is substituted with 1 to 5 substituents". Further, if it has a plurality of substituents, the substituents may be the same as or different from each other.

Examples of the acceptable substituent in the "aryl which may be substituted", "cycloalkyl which may be substituted", "aromatic heterocycle which may be substituted", and "non-aromatic heterocycle which may be substituted" in A of the formula (I) include the following:

(1) halogen,
(2) $C_{1-6}$ alkyl which may be substituted with at least one group selected from the group consisting of halogen, aryl, -OH, -O-$C_{1-6}$ alkyl, -O-aryl, $C_{3-8}$ cycloalkyl, and oxo,
(3) -O-$C_{1-6}$ alkyl or -S-$C_{1-6}$ alkyl, each of which may be substituted with at least one group selected from the group consisting of halogen, $C_{3-8}$ cycloalkyl, -O-$C_{1-6}$ alkyl, aryl (in which the aryl may be substituted with at least one halogen atom), and a non-aromatic heterocycle,
(4) aryl or -O-aryl, each of which may be substituted with at least one group selected from the group consisting of halogen, $C_{1-6}$ alkyl (in which the $C_{1-6}$ alkyl may be substituted with at least one halogen atom), -O-$C_{1-6}$ alkyl (in which the $C_{1-6}$ alkyl may be substituted with at least one halogen atom), $C_{3-8}$ cycloalkyl, and cyano,
(5) $C_{3-8}$ cycloalkyl or -O-$C_{3-8}$ cycloalkyl, each of which may be substituted with at least one $C_{1-6}$ alkyl group,
(6) an aromatic heterocycle or -O-aromatic heterocycle, each of which may be substituted with at least one group selected from the group consisting of halogen, $C_{1-6}$ alkyl, and $C_{3-8}$ cycloalkyl,
(7) amino or cyclic amino, each of which may be substituted with at least one group selected from the group consisting of $C_{1-6}$ alkyl (in which the $C_{1-6}$ alkyl may be substituted with at least one aryl) and aryl,
(8) -CO-$C_{1-6}$ alkyl or -$SO_2$-$C_{1-6}$ alkyl, each of which may be substituted with at least one halogen atom, and
(9) -OH.

Examples of the acceptable substituent in the "aryl which may be substituted", "cycloalkyl which may be substituted", and "cyclic amino which may be substituted" in $R^{11}$ or $R^{12}$ of the formula (I) include:

(1) halogen,
(2) $C_{1-6}$ alkyl which may be substituted with at least one group selected from the group consisting of halogen, aryl, -OH, -O-$C_{1-6}$ alkyl, -O-aryl, $C_{3-8}$ cycloalkyl, and oxo,
(3) -O-$C_{1-6}$ alkyl or -S-$C_{1-6}$ alkyl, each of which may be substituted with at least one group selected from the group consisting of halogen, $C_{3-8}$ cycloalkyl, -O-$C_{1-6}$ alkyl, aryl (in which the aryl may be substituted with at least one halogen atom), and a non-aromatic heterocycle,
(4) aryl or -O-aryl, each of which may be substituted with at least one group selected from the group consisting of halogen, $C_{1-6}$ alkyl (in which the $C_{1-6}$ alkyl may be substituted with at least one halogen atom), -O-$C_{1-6}$ alkyl (in which the $C_{1-6}$ alkyl may be substituted with at least one halogen atom), $C_{3-8}$ cycloalkyl, and cyano, and
(5) $C_{3-8}$ cyloalkyl or -O-$C_{3-8}$ cycloalkyl, each of which may be substituted with at least one $C_{1-6}$ alkyl group.

Embodiments of the compound of the formula (I) or a salt thereof are shown below.

(1) The compound or a salt thereof, wherein A is aryl which may be substituted, cycloalkyl which may be substituted, an aromatic heterocycle which may be substituted, a non-aromatic heterocycle which may be substituted, or a group represented by the formula (II), $R^{11}$ and $R^{12}$ are the same as or different from each other and are -H, aryl which may be substituted, or $C_{3-8}$ cycloalkyl which may be substituted, provided that $R^{11}$ and $R^{12}$ are not -H at the same time, in which $R^{11}$ and $R^{12}$ may be combined with the nitrogen atom to which the bind to form cyclic amino which may be substituted; in another embodiment, the compound or a salt thereof, wherein A is phenyl, naphthyl, thienyl, or benzothienyl, each of which may be substituted with at least one group selected from the group consisting of fluoro , chloro, methyl, and cyclopropyl; in a further embodiment, the compound or a salt thereof, wherein A is naphthyl; in a further embodiment, the compound or a salt thereof, wherein A is thienyl which may be substituted with halogen; in the further embodiment, the compound or a salt thereof, wherein A is benzothienyl which may be substituted with halogen; and in a further embodiment, the compound or a salt thereof, wherein A is phenyl which may be substituted with at least one group selected from the group consisting of chloro, methyl, and cyclopropyl.

(2) The compound or a salt thereof, wherein Ring $B^1$ is a group represented by the formula (III):

[Chem. 6]

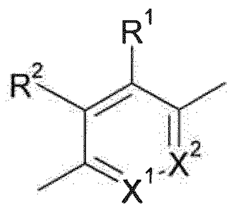

(III)

wherein $X^1$ represents N or $CR^3$, $X^2$ represents N or $CR^4$, and $R^1$, $R^2$, $R^3$ and $R^4$ are the same as or different from each other and represent -H, -OH, $C_{1-6}$ alkyl which may be substituted with at least one halogen atom, -O-$C_{1-6}$ alkyl which may be substituted with at least one halogen atom, or $C_{3-8}$ cycloalkyl or halogen; in another embodiment, the compound or a salt thereof, wherein Ring $B^1$ is 1,4-phenylene which may be substituted with at least one halogen atom; and in a further embodiment, the compound or a salt thereof, wherein Ring $B^1$ is 1,4-phenylene which may be substituted with one or two fluorine atoms.

(3) The compound or a salt thereof, wherein W is -O- or a bond; and in another embodiment, the compound or a salt thereof, wherein W is -O-.

(4) The compound or a salt thereof, wherein Ring $B^2$ is cyclohexane-1,4-diyl.

(5) The compound or a salt thereof, wherein Y is a bond or $C_{1-6}$ alkylene, in another embodiment, the compound or a salt thereof, wherein Y is a bond or methylene; in another embodiment, the compound or a salt thereof, wherein Y is a bond; and in a further embodiment, the compound or a salt thereof, wherein Y is methylene.

(6) The compound or a salt thereof, wherein Z is -$CO_2H$ or a biological equivalent thereof, or -$CONH_2$, and in another embodiment, the compound or a salt thereof, wherein Z is -$CO_2H$.

(7) The compound or a salt thereof, which is a combination of two or more of the groups described in (1) to (6) above.

The compound or a salt thereof as described above in (7), which is a combination of two or more of the groups described in (1) to (6) above, is included in the present invention, but the specific examples thereof also include the following embodiments.

(8) The compound or a salt thereof, wherein A is aryl which may be substituted, cycloalkyl which may be substituted, aromatic heterocycle which may be substituted, a non-aromatic heterocycle which may be substituted, or a group represented by the formula (II), and $R^{11}$ and $R^{12}$ are the same as or different from each other and are -H, aryl which may be substituted, or $C_{3-8}$ cycloalkyl which may be substituted, provided that $R^{11}$ and $R^{12}$ are not -H at the same time, in which $R^{11}$ and $R^{12}$ may be combined with nitrogen atom to which they bind form a cyclic amino which may be substituted, Ring $B^1$ is a group represented by the formula (III), $X^1$ is N or $CR^3$, $X^2$ is N or $CR^4$, and $R^1$, $R^2$, $R^3$ and $R^4$ are the same as or different from each other and are -H, -OH, $C_{1-6}$ alkyl which may be substituted with at least one halogen atom, -O-$C_{1-6}$ alkyl which may be substituted with at least one halogen atom, $C_{3-8}$ cycloalkyl or halogen, W is -O- or a bond, Ring $B^2$ is cyclohexane-1,4-diyl, Y is a bond or $C_{1-6}$ alkylene, and Z is -$CO_2H$ or a biological equivalent thereof, or -$CONH_2$.

(9) The compound or a salt thereof, wherein Ring $B^1$ is 1,4-phenylene which may be substituted with at least one halogen atom, W is -O-, Ring $B^2$ is cyclohexane-1,4-diyl, Y is a bond or methylene, and Z is -$CO_2H$.

(10) The compound or a salt thereof according to (9), wherein Ring $B^1$ is 1,4-phenylene which may be substituted with one or two fluorine atoms.

(11) The compound or a salt thereof according to (10), wherein Y is a bond.

Examples of the specific compounds encompassed by the compound of the formula (I) or a salt thereof include:

cis-4-[4-({2-[(4-cyclopropylbenzoyl)amino]ethyl}carboyl)phenoxy]cyclohexanecarboxylic acid,
cis-4-(4-{[2-(2-naphthoylamino)ethyl]carbamoyl}phenoxy)cyclohexanecarboxylic acid,
cis-4-[4-({2-[(4-chloro-3-methylbenzoyl)amino]ethyl} carbamoyl)phenoxy]cyclohexanecarboxylic acid,
cis-4-(3-fluoro-4-{[2-(2-naphthoylamino)ethyl]carbamoyl}phenoxy)cyclohexanecarboxylic acid,
cis-4-(3,5-difluoro-4-{[2-(2-naphthoylamino)ethyl]carbamoyl}phenoxy)cyclohexanecarboxylic acid,
cis-4-(2,3-difluoro-4-{[2-(2-naphthoylamino)ethyl]carbamoyl}phenoxy)cyclohexanecarboxylic acid,
cis-4-(2,5-difluoro-4-{[2-(2-naphthoylamino)ethyl]carbamoyl}phenoxy)cyclohexanecarboxylic acid,
cis-4-{4-[(2-{[(3-chloro-1-benzothiophen-2-yl)carbonyl]amino}ethyl)carbamoyl]phenoxy}cyclohexanecarboxylic acid,
cis-4-{4-[(2-{[{5-chlorothiophen-2-yl)carbonyl]amino}ethyl)carbamoyl]-2,3-difluorophenoxy}cyclohexanecarboxylic acid,

cis-4-{3-fluoro-4-[(2-{[(5-fluoro-1-benzothiophen-2-yl)carbonyl]amino}ethyl)carbamoyl]phenoxy}cyclohexanecarboxylic acid,
[cis-4-(2,5-difluoro-4-{[2-(2-naphthoylamino)ethyl]carbamoyl}phenoxy)cyclohexyl]acetic acid, and

salts thereof.

The compound of the formula (I) may exist in the form of tautomers or geometrical isomers depending on the kind of substituents. In the present specification, the compound of the formula (I) shall be described in only one form of isomers, yet the present invention includes other isomers, each of separatedisomers, or a mixture thereof. In addition, the compound of the formula (I) may have asymmetric carbon atoms or axial asymmetry in some cases, and correspondingly, it may exist in the form of optical isomers. The present invention includes both an separated form of the optical isomers of the compound of the formula (I) or a mixture thereof.

Moreover, the present invention also includes a pharmaceutically acceptable prodrug of the compound of the formula (I). The pharmaceutically acceptable prodrug is a compound having a group that can be converted into an amino group, a hydroxyl group, a carboxyl group, or the like through solvolysis or under physiological conditions. Examples of the group forming the prodrug include the groups described in Prog. Med., 5, 2157-2161 (1985) and Pharmaceutical Research and Development, Hirokawa Publishing Company (1990), Vol. 7, Drug Design 163-198.

Furthermore, the salt of the compound of the formula (I) is a pharmaceutically acceptable salt of the compound of the formula (I) and may form an acid addition salt or a salt with a base depending on the kind of substituents. Specific examples thereof include acid addition salts with inorganic acids such as hydrochloric acid, hydrobromic acid, hydroiodic acid, sulfuric acid, nitric acid, phosphoric acid, and the like, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, glutamic acid, and the like, and salts with inorganic bases such as sodium, potassium, magnesium, calcium, aluminum, and the like or organic bases such as methylamine, ethylamine, ethanolamine, lysine, ornithine, and the like, salts with various amino acids or amino acid derivatives such as acetylleucine and the like, ammonium salts, etc.

In addition, the present invention also includes various hydrates or solvates, and polymorphic crystalline forms of the compound of the formula (I) and a salt thereof. In addition, the present invention also includes compounds labeled with various radioactive or non-radioactive isotopes.

(Preparation Methods)

The compound of the formula (I) and a salt thereof can be prepared using the characteristics based on the basic structure or the type of substituents thereof and by applying various known synthesis methods. During the preparation, replacing the relevant functional group with a suitable protective group (a group that can be easily converted into the functional group) at the stage from starting material to an intermediate may be effective depending on the type of the functional group in the production technology in some cases. The protective group for such a functional group may include, for example, the protective groups described in "Greene's Protective Groups in Organic Synthesis (4th Ed., 2006)", P. G. M. Wuts and T. W. Greene, and one of these may be selected and used as necessary depending on the reaction conditions. In this kind of method, a desired compound can be obtained by introducing the protective group, by carrying out the reaction and by eliminating the protective group as necessary.
In addition, the prodrug of the compound of the formula (I) can be produced by introducing a specific group or by carrying out the reaction using the resulting compound of the formula (I) at the stage from a starting material to an intermediate, just as in the case of the above-mentioned protective group. The reaction can be carried out using methods known to those skilled in the art, such as ordinary esterification, amidation, dehydration, and the like.

Hereinbelow, the representative preparation methods for the compound of the formula (I) will be described. Each of the production processes may also be carried out with reference to the References appended in the present description. Further, the preparation methods of the compound of the formula (I) are not limited to the examples as shown below.

(Production Process 1)

[Chem. 7]

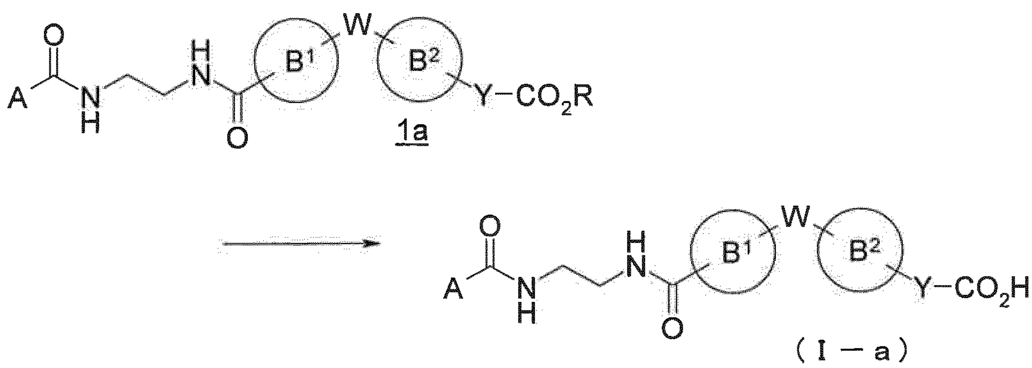

1a

(I－a)

(wherein $-CO_2R$ represents an ester group such as an alkyl ester, a benzyl ester, and the like (for example, methoxycarbonyl, ethoxycarbonyl, tert-butoxycarbonyl, benzyloxycarbonyl, and the like).

The present production process is a method for preparing a compound (I-a) that is the compound of the present invention by hydrolyzing the compound 1a.
The hydrolysis reaction can be carried out with reference to "Greene's Protective Groups in Organic Synthesis (4th Ed, 2006)" as described above.

(Production Process 2)

[Chem. 8]

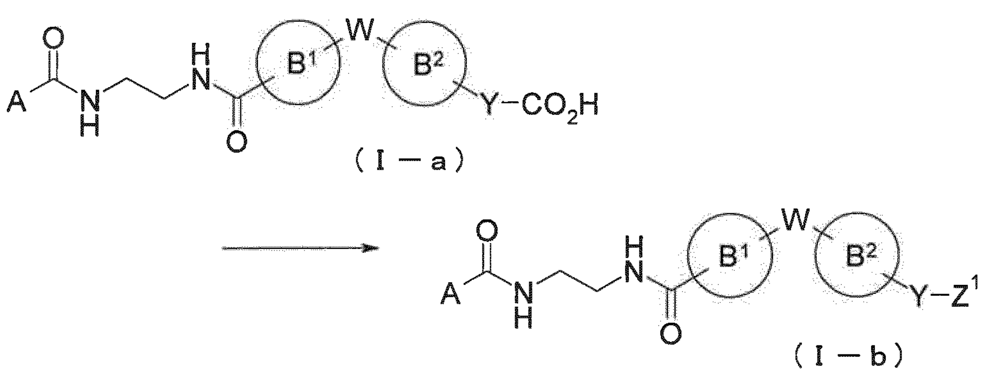

(I－a)

(I－b)

(wherein $Z^1$ represents $-CONH_2$ or a biological equivalent of-$CO_2H$).

The present production process is a step in which a carboxyl group of a compound (I-a) that is the compound of the present invention prepared by Production Process 1 is converted to a biological equivalent of a carboxamide or carboxylic group.
For example, in the case where $Z^1$ is $-CO-NH-SO_2-C_{1-6}$ alkyl or $-CO-NH-SO_2-N(C_{1-6}$ alkyl$)_2$, the compound can be prepared by a condensation reaction with a corresponding sulfonamide or the like, and for example, in the case where $Z^1$ is $-CONH_2$, the compound can be prepared by a condensation reaction using ammonium chloride, ammonium hydroxide, an aqueous ammonia solution, or the like. Further, for example, in the case where $Z^1$ is tetrazolyl, carboxamide is induced, followed by dehydration by an ordinary method to induce a nitrile, which is reacted with sodium azide, thereby preparing the compound. In addition, a carboxyl group can be converted to a biological equivalent thereof by a method apparent to a person skilled in the art.

(Starting Material Synthesis 1)

[Chem. 9]

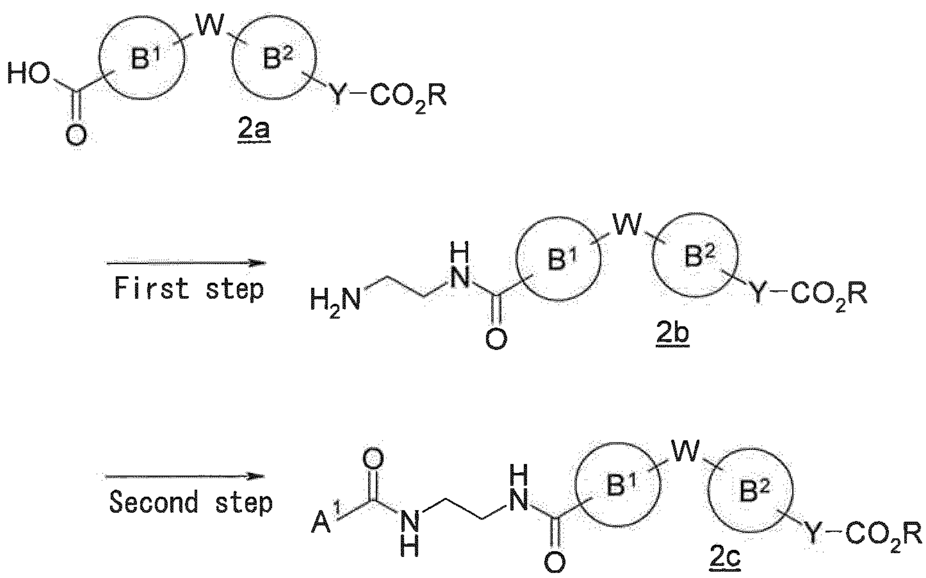


(wherein $A^1$ represents aryl, cycloalkyl, an aromatic heterocycle, or a non-aromatic heterocycle, each of which may be substituted).

The present production process is a method for preparing a compound 2c, in which A is aryl, cycloalkyl, an aromatic heterocycle, or a non-aromatic heterocycle, among the compounds 1a that are the starting compounds of Production Process 1.

(First Step)

The present step is a step in which ethylenediamine is added to the compound 2a prepared by the method described in Pamphlet of International Publication WO 2007/115935 or a method in accordance therewith. A compound 2b can be prepared by, for example, condensing ethylenediamine having one amino group protected with the compound 2a, and removing the protecting group of the amino group.

The condensation reaction can be carried out using the compound 2a and ethylenediamine having one amino group protected in equivalent amounts or with either thereof in an excess amount, and stirring them under any temperature condition from cooling to heating, preferably at -20˚C to 60˚C, usually for 0.1 hours to 5 days, in a solvent which is inert to the reaction, in the presence of a condensing agent. The solvent as used herein is not particularly limited, but examples thereof include aromatic hydrocarbons such as benzene, toluene, xylene, and the like, halogenated hydrocarbons such as dichloromethane, 1,2-dichloroethane, chloroform, and the like, ethers such as diethyl ether, tetrahydrofuran (THF), dioxane, dimethoxyethane, and the like, N,N-dimethylformamide (DMF), dimethylsulfoxide (DMSO), ethyl acetate, acetonitrile, water, or a mixture thereof. Examples of the condensing agent include 1-(3-dimethylaminopropyl)-3-ethylcarbodiimide, dicyclohexylcarbodiimide, 1,1'-carbonyldiimidazole, diphenylphosphoryl azide, phosphorus oxychloride, and the like, but are not limited thereto. It may be preferable in some cases for the reaction to use an additive such as 1-hydroxybenzotriazole and the like. It may be preferable in some cases for the progress of the reaction to use an organic base such as triethylamine, N,N-diisopropylethylamine, N-methylmorpholine, and the like, or an inorganic base such as potassium carbonate, sodium carbonate, potassium hydroxide, and the like.

The reaction for removing the protecting group of an amino group can be carried out with reference to "Greene's Protective Groups in Organic Synthesis ($4^{th}$ Ed., 2006)" as described above.

(Second Step)

The present step is a step for acylating the compound 2b.
Acylation can be carried out using the compound 2b and a corresponding carboxylic acid ($A_1$-$CO_2H$), by employing the method of the preceding paragraph of First Step in the present Production Process, or alternatively by converting the corresponding carboxylic acid to a reactive derivative, and then reacting the reactive derivative with the compound 2b. Examples of the reactive derivative of the carboxylic acid include acid halides obtained by the reaction with a halogenating agent such as phosphorus oxychloride, thionyl chloride, and the like, mixed acid anhydrides obtained by the reaction with isobutyl chloroformate or the like, active esters obtained by condensation with 1-hydroxybenzotriazole or the like, and others. The reaction of the reactive derivative and the compound 2b can be carried out by stirring them under any temperature condition from cooling to heating, preferably at -20˚C to 60˚C, usually for 0.1 hours to 5 days, in a solvent which is inert to the reaction. The solvent as used herein is not particularly limited, but examples thereof include halogenated hydrocarbons, ethers, aromatic hydrocarbons, or a mixture thereof.

(Starting Material Synthesis 2)

[Chem. 10]

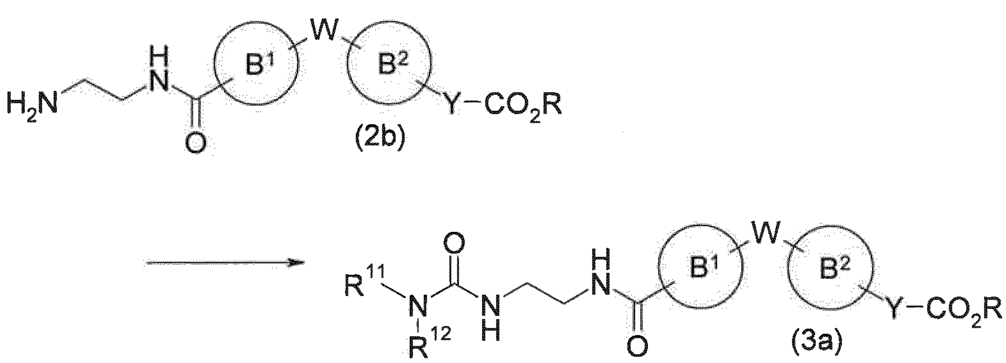

The present Production Process is a process for preparing a compound 3a in which A is a group represented by the formula (II), among the compounds 1a that are starting compounds of Production Process 1.
The present Production Process is a process for preparing a compound 3c by adding an aminocarbonyl group to the compound 2b.
In the case of using an amine (HN(-$R^{11}$)(-$R^{12}$)), 1,1'-carbonyldiimidazole, phosgene, triphosgene, or the like can be used as a carbonyl source. This reaction can be carried out using the compound 2b, the corresponding amine, and the carbonyl source in equivalent amounts or with any one thereof in an excess amount, and stirring the mixture under any temperature condition from cooling to heating, preferably under any temperature condition from room temperature to refluxing, usually for 0.1 hours to 5 days, in a solvent which is inert to the reaction. The solvent as used herein is not particularly limited, but examples thereof include ethers, halogenated hydrocarbons, N,N-dimethylformamide, N,N-dimethylacetamide, dimethylsulfoxide, or a mixture thereof. It may be preferable for the progress of the reaction to use a base such as triethylamine, N,N-diisopropylethylamine, N-methylmorpholine, pyridine, and the like.
Furthermore, in the case where one of-$R^{11}$ and -$R^{12}$ is -H (for example, a case where -$R^{12}$ is -H), a corresponding isocyanate ($R^{11}$-N=C=O) can be used, and in this case, the reaction can be carried out using the compound 2b and a corresponding isocyanate in equivalent amounts or with either thereof in an excess amount, and stirring the mixture under any temperature condition from cooling to heating, preferably under any temperature condition from heating to refluxing, usually for 0.1 hours to 5 days, in a solvent which is inert to the reaction. The solvent as used herein is not particularly limited, but examples thereof include ethers, halogenated hydrocarbons, N,N-dimethylformamide, N,N-dimethylacetamide, dimethylsulfoxide, or a mixture thereof. Further, the isocyanate can also be produced by, for example, by Curtius rearrangement using the corresponding carboxylic acid and diphenylphosphoryl azide, and then used.

(Starting Material Synthesis 3)

[Chem. 11]

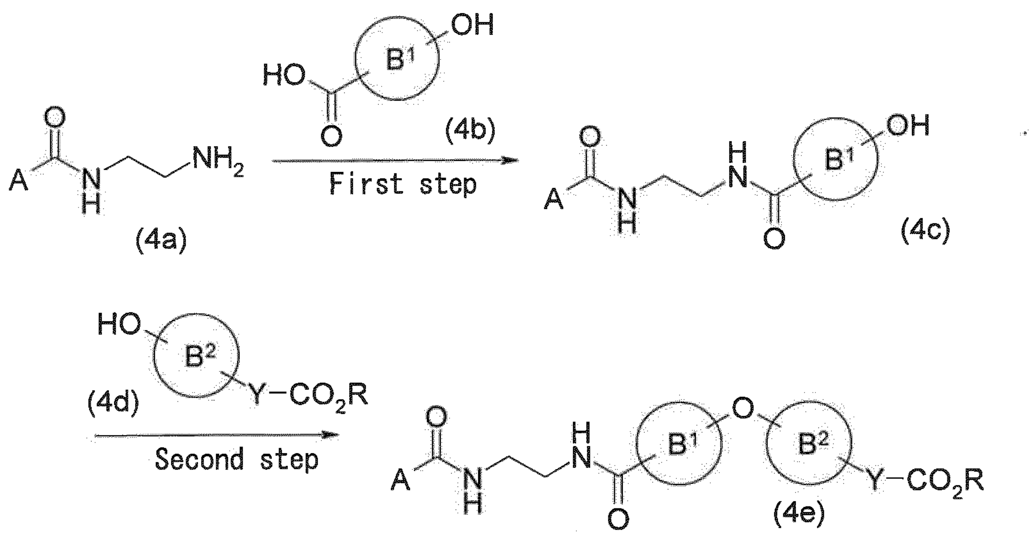


The present Production Process is a process for preparing compound 4e, in which W is -O-, among the compounds 1a that are the starting compounds of Production Process 1.

(First Step)

The present step is a step in which the compound 4a prepared in accordance with the method described in Journal of Medicinal Chemistry, 1993, Vol. 36, No. 24, pp. 3968-3970, or a method equivalent thereto is condensed with the compound 4b.
The condensation reaction can be carried out using the method described in First Step of the preceding paragraph or the method described in Second Step in Starting Material Synthesis 1.

(Second Step)

The present step is a step in which the compound 4c and the compound 4d are condensed.
For the condensation reaction, a Mitsunobu reaction that is usually employed by a person skilled in the art can be used. The Mitsunobu reaction can be carried out using the compound 4c and the compound 4d in equivalent amounts or with either thereof in an excess amount, using a mixture thereof and an activating agent prepared from a phosphorous compound such as tributylphosphine, triphenylphosphine, and the like and an azodicarbonyl compound such as ethyl azodicarboxylate, 1,1'-(azodicarbonyl)dipiperidine, and the like, or a reagent such as cyanomethylenebutylphosphorane and the like, under any temperature condition from cooling to heating, preferably under any temperature condition from heating to heating, usually for 0.1 hours to 5 days, in a solvent which is inert to the reaction. The solvent as used herein is not particularly limited, but examples thereof include halogenated hydrocarbons, ethers, aromatic hydrocarbons, or a mixture thereof.
Furthermore, when carrying out the present step, an alkylation reaction may be employed. When carrying out the alkylation reaction, the reaction can be carried out by converting a hydroxyl group of the compound 4c or the compound 4d to a leaving group, for example, halogen such as chlorine, bromine, iodine, and the like, or sulfonyloxy such as methanesulfonyloxy, ethanesulfonyloxy, benzenesulfonyloxy, 4-methylbenzenesulfonyloxy, trifluoromethanesulfonyloxy, and the like, and stirring them under any temperature condition from cooling to heating, preferably under any temperature condition from room temperature to heating, usually for 0.1 hours to 5 days, in the presence of a suitable base, in a solvent which is inert to the reaction.
The compounds of the formula (I) can be isolated and purified as their free compounds, salts, hydrates, solvates, or polymorphic crystalline forms thereof. The salts of the compound of the formula (I) can be prepared by carrying out the treatment of a conventional salt forming reaction.
Isolation and purification are carried out by employing ordinary chemical operations such as extraction, fractional crystallization, various types of fractional chromatography, and the like.
Various isomers can be prepared by selecting an appropriate starting compound or separated by using the difference

in the physicochemical properties between the isomers. For example, the optical isomers can be obtained by means of a general method for designing optical resolution of racemic products (for example, fractional crystallization for inducing diastereomer salts with optically active bases or acids, chromatography using a chiral column or the like, and others), and further, the isomers can also be prepared from an appropriate optically active starting compound.

The pharmacological activity of the compound of the formula (I) was confirmed by the tests shown below.

Test Example 1 1 DGAT1 Inhibitory Activity Test

(1) Production of Bacmid-hDGAT1

A base sequence encoding a human DGAT1 (hDGAT1) (1467 bases of CDS in Genbank Accession No. NM_012079) was cloned and ligated to pFastBac™1 (Invitrogen) to produce a pFastBac1-hDGAT1. From this plasmid, an engineered baculovirus liquid (Bacmid-hDGAT1) was prepared using a Bac-to-Bac (registered trademark) Baculovirus Expression System (Invitrogen).

(2) Preparation of Sf9 Cell-Derived DGAT1-Expressing Microsomal Fraction

Sf9 cells were seeded into a 225-$cm^2$ Flask Angled Neck (CORNING) to a confluency of 80%, and statically cultured in an incubator at 27°C using an EX-CELL$^{7M}$ 420 INSECT SERUM-FREE MEDIUM (SAFC Biosciences, Inc.). After 24 hours, the culture medium was taken out, and a liquid obtained by diluting 1.67 mL of an engineered baculovirus medium (Bacmid-hDGAT1) with 3.33 mL of an EX-CELL™ 420 INSECT SERUM-FREE MEDIUM was added thereto, and the resultant was cultured under shaking in an incubator at 27°C for 1 hour. Thereafter, 25 mL of an EX-CELL™ 420 INSECT SERUM-FREE MEDIUM was added thereto, followed by culturing in an incubator at 27°C for 72 hours. The infected cells were harvested, diluted with 1.5 mL of a buffer A (40 mM phosphate buffer (pH 7.2) including 100 mM sucrose and 50 mM KCl) containing a Complete Protease Inhibitor Cocktail (Roche Diagnostics K. K.), and then subjected to sonication using a SONIFER 250 (BRANSON Ultrasonic Corp.). This suspension was centrifuged at 10000xg for 5 minutes and the supernatant was recovered. This supernatant was centrifuged at 100000xg for 60 minutes and the resulting precipitate was dissolved in 600 μL of a buffer A and then subjected to sonication again, to prepare a suspension. This suspension was taken as a hDGAT1-expressing Sf9 cell microsome.

(3) DGAT1 Activity Measurement

For the DGAT1 activity, a test substance in DMSO (final DMSO concentration 2%) in which 100 mM Tris-HCl (pH 8.0),2 mM $MgCl_2$, 0.01% BSA, a hDGAT1-expressing Sf9 cell microsome, 200 μM dioleoyl glycerol or dipalmitoyl glycerol, 8.9 μM [$^{14}$C] oleoyl-CoA, and 1.68% acetone were added to a phospholipid FlashPlate (PerkinElmer Life Science). The reaction was carried out at 30°C for 60 minutes, and then twice the amount (100 μL), relative to the reaction liquid, of a mixture of isopropyl alcohol:0.1 M carbonate buffer (pH 9.0) at a ratio of 1:1 was added thereto to stop the reaction. The next day, the count was measured with a TopCount microplate scintillation counter (PerkinElmer Life Science). The count without addition of the test substance was taken as a control (TG with addition of DMSO). The count without addition of the test substance and the microsome was taken as a Blank TG. The DGAT1 inhibitory rate of the test substance was calculated by the following calculation equation:

$$\text{Inhibitory Rate (\%)}=(\text{TG with addition of test substance-Blank TG}) \div (\text{TG with addition of DMSO-Blank TG}) \times 100$$

The DGAT1 inhibitory rates were calculated when the concentrations of the test substance in the reaction liquid were 1,10, 100, and 1000 nM. Linear regression was performed using the inhibitory rates obtained, and a test substance concentration ($IC_{50}$) required to inhibit the DGAT1 activity by 50% was calculated by an SAS 8.2 software package (SAS Institute Japan, Ltd., Tokyo, Japan).

As a result, some of the compounds of the formula (I) showed a DGAT1 inhibitory action at an $IC_{50}$ value of 50 nM or less in the above test. The results of the present test of some of the compounds of the formula (I) are shown in Table 1.

[Table 1]

| Example No. | $IC_{50}$/nM | | Example No. | $IC_{50}$/nM |
|---|---|---|---|---|
| 6 | 20 | | 187 | 8.1 |
| 18 | 21 | | 245 | 14 |
| 20 | 2.1 | | 299 | 6.7 |
| 56 | 14 | | 300 | 5.6 |
| 63 | 0.4 | | 454 | 1.0 |
| 79 | 2.3 | | 469 | 1.7 |
| 82 | 9.9 | | 480 | 5.2 |
| 83 | 5.2 | | 490 | 3.8 |
| 106 | 5.3 | | 531 | 0.1 |
| 135 | 5.8 | | 571 | 1.6 |
| 148 | 4.2 | | 604 | 32 |
| 169 | 2.7 | | | |

Test Example 2

Inhibitory Action on Increase in Triglyceride (TG) by Fat Administration

8- to 15-week male C57BL/6J mice were fasted for four hours, and the test substances were administered to the mice. The test substance was administered after suspending it in a 0.5% methyl cellulose solution. After 20 hours, fat (intralipid 20%, Terumo (Fresenius), 10 mL/kg) was orally administered thereto. Immediately before and after 2 hours from administration of the fat, blood was collected from the tail vein to obtain plasma. For measurement of the TG in plasma, a TG increase in the plasma by fat administration was calculated using a Triglyceride E Test Wako (Wako Pure Chemical Industries Ltd.). Using the TG increase in the plasma in a group to which 0.5% methyl cellulose had been administered as a control, the TG increase inhibitory rate when the test substance had been administered was determined.
As a result, among the Example Compounds below, the compound of Example 79 showed a TG increase inhibitory rate of 79% with an administration amount of 0.3 mg/kg, the compound of Example 169 showed a TG increase inhibitory rate of 75% with an administration amount of 0.3 mg/kg, the compound of Example 454 showed a TG increase inhibitory rate of 106% with an administration amount of 0.3 mg/kg, the compound of Example 469 showed a TG increase inhibitory rate of 109% with an administration amount of 0.3 mg/kg, and the compound of Example 490 showed a TG increase inhibitory rate of 92% with an administration amount of 0.3 mg/kg.

Test Example 3 Anti-Obesity Action in DIO Mice

High-fat diet formula (Research Diet D12492, 60 kcal% fat) were given to 9-week male C57BL/6J mice. For acclimatization of the mice, the solvent used in the test from the 4$^{th}$ week was administered thereto. From the 5$^{th}$ week, the test substance was orally administered once or twice daily. The test substance was administered after suspending it in a 0.5% methyl cellulose solution. Administration was performed repeatedly for 2 or 4 weeks, and variation in the weights due to the test substance administration was observed. By taking the increase in the weight in the group to which the solvent had been administered within a test period as 100%, the weight increase inhibitory rate of the test substance was calculated.
As a result, among the Example Compounds below, the compound of Example 454 showed a weight increase inhibitory rate of 93% with an administration of once per day, an administration period of 2 weeks, and an administration amount of 1 mg/kg, and the compound of Example 490 showed a weight increase inhibitory rate of 98% with an administration of once per day, an administration period of 2 weeks, and an administration amount of 1 mg/kg.
As a result of the tests above, it was confirmed that the compound of the formula (I) has an anti-obesity action in a diet-induced obesity model, and can be therefore used for preventing and/or treating obesity, type II diabetes mellitus, fatty liver, or the like.
A pharmaceutical composition containing one or two or more kinds of the compound of the formula (I) or a salt thereof as an active ingredient can be prepared using excipients that are usually used in the art, that is, excipients for

pharmaceutical preparation, carriers for pharmaceutical preparation, and the like according to the methods usually used. Administration can be accomplished either by oral administration via tablets, pills, capsules, granules, powders, solutions, and the like, or parenteral administration injections, such as intraarticular, intravenous, or intramuscular injections, and the like, suppositories, ophthalmic solutions, eye ointments, transdermal liquid preparations, ointments, transdermal patches, transmucosal liquid preparations, transmucosal patches, inhalers, and the like.

The solid composition for use in the oral administration according to the present invention is used in the form of tablets, powders, granules, or the like. In such a solid composition, one or more active ingredient(s) are mixed with at least one inactive excipient, such as lactose, mannitol, glucose, hydroxypropyl cellulose, microcrystalline cellulose, starch, polyvinylpyrrolidone, and/or magnesium aluminometasilicate. In a conventional method, the composition may contain inactive additives, such as a lubricant such as magnesium stearate, a disintegrating agent such as sodium carboxymethyl starch and the like, a stabilizer, or a solubilization assisting agent. If necessary, tablets or pills may be coated with sugar or a film of a gastric or enteric coating substance.

The liquid composition for oral administration contains pharmaceutically acceptable emulsions, solutions, suspensions, syrups, elixirs, or the like, and also contains generally used inert diluents, for example, purified water or ethanol. In addition to the inert diluent, the liquid composition may also contain auxiliary agents, such as a solubilization assisting agent, a moistening agent, and a suspending agent, sweeteners, flavors, aromatics, and antiseptics.

The injections for parenteral administration include sterile aqueous or non-aqueous solution preparations, suspensions and emulsions. The aqueous solvent includes, for example, distilled water for injection and physiological saline. Examples of the non-aqueous solvent include palcohols such as ethanol. Such a composition may further contain a tonicity agent, an antiseptic, a moistening agent, an emulsifying agent, a dispersing agent, a stabilizing agent, or a solubilizing aid. These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

The agent for external use includes ointments, plasters, creams, jellies, patches, sprays, lotions, eye drops, eye ointments, and the like. The agents contain generally used ointment bases, lotion bases, aqueous or non-aqueous liquid preparations, suspensions, emulsions, and the like.

As the transmucosal agents such as an inhaler, a transnasal agent, and the like, those in the form of a solid, liquid, or semi-solid state are used, and can be prepared in accordance with a conventionally known method. For example, a known excipient, and also a pH adjusting agent, an antiseptic, a surfactant, a lubricant, a stabilizing agent, a thickening agent, or the like may be appropriately added thereto. For their administration, an appropriate device for inhalation or blowing can be used. For example, a compound may be administered alone or as a powder of formulated mixture, or as a solution or suspension in combination with a pharmaceutically acceptable carrier, using a conventionally known device or sprayer, such as a measured administration inhalation device, and the like. A dry powder inhaler or the like may be for single or multiple administration use, and a dry powder or a powder-containing capsule may be used. Alternatively, this may be in a form such as a pressurized aerosol spray which uses an appropriate ejection agent, for example, a suitable gas such as chlorofluoroalkane, hydrofluoroalkane, carbon dioxide, and the like, or other forms.

In oral administration, the daily dose is generally from about 0.001 to 100 mg/kg, preferably from 0.1 to 30 mg/kg, and more preferably 0.1 to 10 mg/kg, per body weight, administered in one portion or in 2 to 4 divided portions. In the case of intravenous administration, the daily dose is suitably administered from about 0.0001 to 10 mg/kg per body weight, once a day or two or more times a day. In addition, a transmucosal agent is administered at a dose from about 0.001 to 100 mg/kg per body weight, once a day or two or more times a day. The dose is appropriately decided in response to the individual case by taking the symptoms, the age, and the gender, and the like into consideration.

Although varying depending on administration routes, dosage forms, administration sites, or the types of excipients and additives, the pharmaceutical composition of the present invention contains 0.01 to 100% by weight, and in a certain embodiment, 0.01 to 50% by weight of one or more kinds of the compound of the formula (I) or a salt thereof, which is an active ingredient.

The compound of the formula (I) can be used in combination with various agents for treating or preventing the diseases, in which the compound of the formula (I) is considered effective, as described above. The combined preparation may be administered simultaneously or separately and continuously, or at a desired time interval. The preparations to be co-administered may be prepared individually or may be a pharmaceutical composition including various agents for treating or preventing the diseases, in which the compound of the formula (I) is considered effective, as described above, and the compound of the formula (I).

## Examples

Hereinbelow, the preparation methods for the compound of the formula (I) will be described in more detail with reference to Examples. Further, the present invention is not limited to the compounds described in the Examples as described below. Furthermore, the production processes for the starting compounds will be described in Preparation

Examples. Further, the preparation methods for the compound of the formula (I) are not limited to the preparation methods of the specific Examples as below, but the compound of the formula (I) can be prepared by any combination of the preparation methods or the methods that are apparent to a person skilled in the art.

Furthermore, the following abbreviations may be used in some cases in Examples, Preparation Examples, and Tables below.

Pr: Preparation Example No. (in which the Preparation Example Compound having "/Cl" described after the Preparation Example No. denotes that the Preparation Example Compound was isolated as hydrochloride, and the Preparation Example Compound having "/TF" described after the Preparation Example No. denotes that the Preparation Example Compound was isolated as trifluoroacetate. Further, the compound in which "*" is attached to the Preparation Example No. denotes that the compound is an optically active form), Ex: Example No. (in which the compound in which "*" is attached to the Example No. denotes that the compound is an optically active form), No: Compound No., Structure: Chemical Structural Formula (Me: methyl, Et: ethyl, iPr: isopropyl, tBu: tert-butyl, nPen: normal pentyl, Ph: phenyl, and Bn: benzyl), Syn: Preparation method (the numeral shows that this compound was prepared by the same preparation method as the compound having the Example No. described in this section), PSy: Preparation method (the numeral shows that this compound was prepared by the same preparation method as the compound having the Preparation Example No. described in this section), Data: Physical data (which show the data of the compound as follows. EI: EI-MS; ESP: ESI-MS (Pos); ESN: ESI-MS (Neg); FP: FAB-MS (Pos); FN: FAB-MS (Neg); NMR1: δ (ppm) of the characteristic peak in $^1$H-NMR in DMSO-$d_6$; NMR2: δ (ppm) of the characteristic peak in $^1$H-NMR in $CDCl_3$).

Preparation Example 1

To a mixture of ethyl trans-4-hydroxycyclohexanecarboxylate (8 g), benzyl 4-hydroxybenzoate (11.69 g), 1,1'-(azodicarbonyl)dipiperidine (15.24 g), and THF (150 ml) was added dropwise tributylphosphine (14.9 ml) under ice-cooling, followed by stirring at room temperature for 2 hours, and then stirring at 60˚C for 10 hours. The reaction mixture was returned to room temperature, then filtered, and washed with THF, and then the filtrate was concentrated under reduced pressure. The resulting residue was purified by silica gel column chromatography (hexane:ethyl acetate=85:15) to obtain 8.55 g of benzyl 4-{[cis-4-(ethoxycarbonyl)cyclohexyl]oxy}benzoate as a colorlessoil.

Preparation Example 2

To a mixture of benzyl 4-{[cis-4-(ethoxycarbonyl)cyclohexyl]oxy}benzoate (8.5 g) and EtOH (150 ml) was added 10% palladium on activated carbon (850 mg), followed by stirring at room temperature for 4 hours under a hydrogen atmosphere (balloon pressure). The reaction mixture was filtered through Celite and washed with EtOH, and then the resulting filtrate was concentrated under reduced pressure to obtain 5.8 g of 4-{[cis-4-(ethoxycarbonyl)cyclohexyl]oxy}benzoic acid as a colorless solid.

Preparation Example 3

To a mixture of 4-{[cis-4-(ethoxycarbonyl)cyclohexyl]oxy}benzoic acid (4 g), 1-ethyl-3-(3-dimethylaminopropyl) carbodiimide monohydrochloride (3.4 g), 1-hydroxybenzotriazole monohydrate (2.7 g), triethylamine (2.9 ml), and DMF (60 ml) was added tert-butyl (2-aminoethyl)carbamate (2.6 ml), followed by stirring at room temperature for 14 hours. To the reaction mixture was added water, and the precipitated solid was collected by filtration and then washed with diisopropyl ether to obtain 5.34 g of ethyl cis-4-[4-({2-[(tert-butoxycarbonyl)amino]ethyl}carbamoyl)phenoxy]cyclohexanecarboxylate as a colorless solid.

Preparation Example 4

To a mixture of ethyl cis-4-[4-({2-[(tert-butoxycarbonyl)amino]ethyl}carbamoyl)phenoxy]cyclohexanecarboxylate (5.34 g) and dioxane (80 ml) was added 4 M hydrogen chloride/dioxane (40 ml), followed by stirring at room temperature for 3 hours. The reaction mixture was concentrated under reduced pressure and the residue was washed with diisopropyl ether to obtain 4.55 g of ethyl cis-4-{4-[(2-aminoethyl)carbamoyl]phenoxy}cyclohexanecarboxylate hydrochloride as a colorless solid.

Preparation Example 5

To a mixture of ethyl 4-{4-[(2-aminoethyl)carbamoyl]phenoxy}cyclohexanecarboxylate hydrochloride (150 mg), chloroform (5 ml), and triethylamine (0.17 ml) was added phenyl isocyanate (0.053 ml), followed by stirring at room temperature for 1 hour. To the reaction mixture was added a saturated aqueous sodium hydrogen carbonate solution,

followed by carrying out a separation of the layers, ,and the organic layer was washed with a saturated aqueous sodium chloride solution, dried over anhydrous magnesium sulfate, and then concentrated under reduced pressure. The residue was purified by silica gel column chromatography (chloroform:methanol (MeOH)=90:10) to obtain 164 mg of ethyl 4-[4-({2-[(anilinocarbonyl)amino]ethyl}carbamoyl)phenoxy]cyclohexanecarboxylate as a colorless solid.

Preparation Example 6

To a mixture of ethyl cis-4-{4-[(2-aminoethyl)carbamoyl]phenoxy}cyclohexanecarboxylate hydrochloride (200 mg), triethylamine (0.08 ml), and methylene chloride (10 ml) was added carbonyldiimidazole (100 mg) at 0˚C, followed by stirring at 0˚C for 10 minutes, and then 4-phenylpiperidine (110 mg) was added thereto, followed by stirring at room temperature for 3 days. To the reaction mixture was added water, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (chloroform:MeOH=100:0 to 95:5) to obtain 288 mg of ethyl cis-4-{4-[(2-{[(4-phenylpiperidin-1-yl)carbonyl]amino}ethyl)carbamoyl]phenoxy}cyclohexanecarboxylate as a colorless solid.

Preparation Example 7

A mixture of ethyl 4-{4-[(2-aminoethyl)carbamoyl]phenoxy}cyclohexanecarboxylate hydrochloride (100 mg), 4-phenylcyclohexanecarboxylic acid (66 mg), 1-methyl-3-(3-dimethylaminopropyl)carbodiimide monohydrochloride (67 mg), 1-hydroxybenzotriazole monohydrate (54 mg), and THF (1.5 ml) was stirred at room temperature for 9 hours. To the reaction mixture was added a saturated aqueous sodium hydrogen carbonate solution, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (chloroform) to obtain 123 mg of ethyl 4-{4-[(2-{[(4-phenylcyclohexyl) carbonyl]amino}ethyl)carbamoyl]phenoxy}cyclohexanecarboxylate as a colorless solid.

Preparation Example 8

To a mixture of ethyl trans-4-{4-[(2-inoethyl)carbamoyl]phenoxy}cyclohexanecarboxylate hydrochloride (100 mg), chloroform (10 ml), and triethylamine (0.14 ml) was added benzoyl chloride (0.038 ml) at 0˚C, followed by stirring at 0˚C for 1 hour. The reaction mixture was added to a saturated aqueous sodium hydrogen carbonate solution, followed by carrying out a separation of the layers using chloroform, and the organic layer was washed with a saturated aqueous sodium chloride solution, dried over anhydrous magnesium sulfate, and then concentrated under reduced pressure. The residue was washed with diisopropylether to obtain 118 mg of ethyl trans-4-(4-{[2-(benzoylamino)ethyl]carbamoyl} phenoxy)cyclohexanecarboxylate as a colorless solid.

Preparation Example 9

A mixture of N-(2-aminoethyl)-4-chlorobenzamide hydrochloride (400 mg), 2-fluoro-4-hydroxybenzoic acid (320 mg), 1-ethyl-3-(3-dimethylaminopropyl)carbodiimide monohydrochloride (500 mg), 1-hydroxybenzotriazole monohydrate (400 mg), triethylamine (0.4 ml), and DMF (10 ml) was stirred at room temperature overnight. To the reaction mixture was added water, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (chloroform: MeOH=100:0 to 95:5) to obtain 410 mg of N-{2-[(4-chlorobenzoyl)amino]ethyl}-2-fluoro-4-hydroxybenzamide as a colorless solid.

Preparation Example 10

To a mixture of N-{2-[(4-chlorobenzoyl)amino]ethyl}-2-fluoro-4-hydroxybenzamide (400 mg), ethyl trans-4-hydroxycyclohexanecarboxylate (220 mg), triphenylphosphine (350 mg), and THF (4 ml) was added a 2.2 M diethyl azodicarboxylate solution in toluene (0.58 ml) at room temperature, followed by stirring at 60˚C overnight. To the reaction mixture was added water, followed by carrying out a separation of the layers using ethyl acetate, and the organic layer was washed with a saturated aqueous sodium chloride solution, dried over anhydrous magnesium sulfate, and then concentrated under reduced pressure. The residue was purified by silica gel column chromatography (chloroform: MeOH=100:0 to 95:5) to obtain 457 mg of ethyl cis-4-[4-({2-[(4-chlorobenzoyl)amino]ethyl}carbamoyl)-3-fluorophenoxy] cyclohexanecarboxylate as a colorless solid.

Preparation Example 11 I

To a mixture of ethyl cis-4-hydroxycyclohexanecarboxylate (800 mg), triphenylphosphine (1.34 g), THF (15 ml), and 4-fluorophenol (521 mg) was added dropwise a 2.2 M diethyl azodicarboxylate solution in toluene (2.3 ml) over 10 minutes under ice-cooling, followed by stirring at 60˚C for 4 hours. The reaction mixture was added to a saturated aqueous sodium hydrogen carbonate solution, followed by carrying out a separation of the layers using ethyl acetate. Thereafter, the organic layer was washed with a saturated aqueous sodium chloride solution, dried over anhydrous magnesium sulfate, and then concentrated under reduced pressure. The residue was crudely purified by silica gel column chromatography (hexane:ethyl acetate=85:15). To a mixture of the crude product (685 mg), THF (5 ml), and EtOH (5 ml) was added a 1 M aqueous sodium hydroxide solution, followed by stirring at room temperature for 14 hours. The solvent was evaporated under reduced pressure, and then to the residue was added water, followed by carrying out a separation of the layers using ethyl acetate. Then, the aqueous layer was adjusted to pH 3 by the addition of 1 M hydrochloric acid, followed by carrying out a separation of the layers using ethyl acetate. The organic layer was dried over anhydrous magnesium sulfate and then concentrated under reduced pressure to obtain 242 mg of trans-4-(4-fluorophenoxy)cyclohexanecarboxylic acid as a colorless solid.

Preparation Example 12

A mixture of 3-fluoro-4-hydroxybenzoic acid (5 g), benzyl bromide (4.19 ml), DMF (33 ml), and potassium carbonate (4.9 g) was stirred at 40˚C for 6 hours. The reaction mixture was left to stand for cooling, then diluted with ethyl acetate, washed with water, dried over anhydrous magnesium sulfate, and then concentrated under reduced pressure. The residue was purified by silica gel column chromatography (chloroform:ethyl acetate=100:0 to 50:50) to obtain 1.7 g of benzyl 3-fluoro-4-hydroxybenzoate as a colorless solid.

Preparation Example 13

To a mixture of 4-[(cis-4-carbamoylcyclohexyl)oxy]-N-{2-[(4-chlorobenzoyl)amino]ethyl}benzamide (330 mg) and THF (20 ml) was added anhydrous trifluoroacetic acid (0.29 ml), followed by stirring at room temperature for 30 minutes, and then anhydrous trifluoroacetic acid (0.07 ml) was added thereto, followed by stirring for 10 minutes. To the reaction mixture was added a saturated aqueous sodium hydrogen carbonate solution, followed by carrying out a separation of the layers using ethyl acetate. Thereafter, the organic layer was washed with a saturated aqueous sodium chloride solution, dried over anhydrous magnesium sulfate, and then concentrated under reduced pressure. To the resulting residue were added diisopropylether and EtOH, and then the solid was collected by filtration and washed with EtOH to obtain 168 mg of 4-chloro-N-[2-({4-[(cis-4-cyanocyclohexyl)oxy]benzoyl}amino)ethyl]benzamide as a colorless solid.

Preparation Example 14

To a mixture of methyl 4-(4-oxocyclohexyl)benzoate (1.3 g) and toluene (10 ml) was added (tert-butoxycarbonylmethylene)triphenylphosphorane (2.5 g), followed by stirring at 105˚C for 24 hours. To the reaction mixture were added ethyl acetate and water, followed by carrying out a separation of the layers, and the organic layer was washed with a saturated aqueous sodium chloride solution, dried over anhydrous magnesium sulfate, and then concentrated under reduced pressure. The residue was purified by silica gel column chromatography (hexane:ethyl acetate=100:0 to 90:10) to obtain 1.35 g of methyl 4-[4-(2-tert-butoxy-2-oxoethylidene)cyclohexyl]benzoate as a colorless solid.

Preparation Example 15

To a mixture of methyl 4-[4-(2-tert-butoxy-2-oxoethylidene)cyclohexyl]benzoate (1.3 g), MeOH (20 ml), and THF (6 ml) was added 10% palladium on activated carbon (130 mg), followed by stirring at room temperature for 4 hours under a hydrogen atmosphere (3 atm). The reaction mixture was filtered through Celite and washed with MeOH, and then the resulting filtrate was concentrated under reduced pressure to obtain 1.11 g of {4-[4-(methoxycarbonyl)phenyl]cyclohexyl}acetic acid as a colorless solid.

Preparation Example 16

To a mixture of {4-[4-(methoxycarbonyl)phenyl]cyclohexyl}acetic acid (1.1 g) and methylene chloride (15 ml) was added oxalic chloride (0.7 ml) at 0˚C, followed by stirring at room temperature for 3 hours. This reaction mixture was concentrated under reduced pressure, and then tert-butanol (15 ml) and diisopropylethylamine (1.4 ml) were added

thereto, followed by stirring at room temperature overnight. The reaction mixture was concentrated under reduced pressure and diluted with ethyl acetate, and then this mixture was washed sequentially with a saturated aqueous sodium hydrogen carbonate solution and a saturated aqueous sodium chloride solution, dried over anhydrous magnesium sulfate, and then concentrated under reduced pressure. The residue was purified by silica gel column chromatography (hexane:ethyl acetate=100:0 to 80:20) to obtain 1.14 g of methyl 4-[4-(2-tert-butoxy-2-oxoethyl)cyclohexyl]benzoate as a colorless solid.

Preparation Example 17

To a mixture of methyl 4-[4-(2-tert-butoxy-2-oxoethyl)cyclohexyl]benzoate (1.1 g), THF (11 ml), and MeOH (11 ml) was added a 1 M aqueous sodium hydroxide solution (5 ml) at room temperature, followed by stirring at room temperature for 5 hours and then at 50˚C for 1 hour. The reaction mixture was returned to room temperature, and then concentrated under reduced pressure. Water was added, and then a 10% aqueous citric acid solution was added thereto until the pH became 5. The precipitated solid was collected by filtration to obtain 835 mg of 4-[4-(2-tert-butoxy-2-oxoethyl) cyclohexyl]benzoic acid as a colorless solid.

Preparation Example 18

A mixture of ethyl 3-chloro-4-hydroxybenzoate (2 g), potassium iodide (165 mg), bromomethylcyclobutane (1.7 ml), potassium carbonate (2.8 g), and DMF (50 ml) was stirred at 60˚C for 5 hours. The reaction mixture was left to stand for cooling, then diluted with ethyl acetate, washed sequentially with water and a saturated aqueous sodium chloride solution, dried over anhydrous magnesium sulfate, and then concentrated under reduced pressure to obtain an oil. To a mixture of this oil, THF (20 ml), and MeOH (20 ml) was added a 1 M aqueous sodium hydroxide solution (20 ml), followed by stirring overnight. To the reaction mixture was added 1 M hydrochloric acid (20 ml), followed by concentrating under reduced pressure to remove the organic solvent. The precipitated solid was collected by filtration to obtain 1.31 g of 3-chloro-4-(cyclobutylmethoxy)benzoic acid as a colorless solid.

Preparation Example 19

A mixture of 4-fluoro-3-hydroxybenzoic acid (2 g), potassium iodide (250 mg), bromomethylcyclopropane (3.7 ml), potassium carbonate (5.3 g), and DMF (27 ml) was stirred at 60˚C overnight. To the reaction mixture was added ethyl acetate, then washed sequentially with water and a saturated aqueous sodium chloride solution, dried over anhydrous magnesium sulfate, and then concentrated under reduced pressure to obtain an oil. To a mixture of this oil, THF (30 ml), and MeOH (30 ml) was added a 1 M aqueous sodium hydroxide solution (30 ml), followed by stirring for 5 hours. To the reaction mixture was added 1 M hydrochloric acid (30 ml), and then concentrated under reduced pressure to remove the organic solvent. The precipitated solid was collected by filtration to obtain 2.55 g of 3-(cyclopropylmethoxy)-4-fluorobenzoic acid as a colorless solid.

Preparation Example 20

A mixture of ethyl 4-bromo-3-fluorobenzoate (1 g), cyclopropylboronic acid monohydrate (643 mg), tetrakistriphenylphosphinepalladium (235 mg), potassium phosphate (3.1 g), toluene (10 ml), and water (1 ml) was thoroughly stirred at 110˚C overnight. To the reaction mixture was added water, followed by carrying out a separation of the layers using ethyl acetate, and the organic layer was washed with a saturated aqueous sodium chloride solution, dried over anhydrous magnesium sulfate, and then concentrated under reduced pressure. The residue was crudely purified by silica gel column chromatography (hexane:ethyl acetate=85:15), and then to a mixture of the crude product, THF (20 ml), and MeOH (20 ml) was added a 1 M aqueous sodium hydroxide solution (20 ml), followed by stirring at room temperature overnight. To the reaction mixture was added 1 M hydrochloric acid (20 ml), followed by concentrating under reduced pressure to remove the organic solvent. The precipitated solid was collected by filtration to obtain 600 mg of 4-cyclopropyl-3-fluorobenzoic acid as a colorless solid.

Preparation Example 21

A mixture of ethyl 4-bromo-3-chlorobenzoate (1 g), cyclopropylboronic acid monohydrate (602 mg), tetrakistriphenylphosphinepalladium (220 mg), potassium phosphate (2.8 g), toluene (10 ml), and water (1 ml) was stirred under reflux overnight. To the reaction mixture was added water, followed by carrying out a separation of the layers using ethyl acetate, and the organic layer was washed with a saturated aqueous sodium chloride solution, dried over anhydrous magnesium sulfate, and then concentrated under reduced pressure. The residue was purified by silica gel column

chromatography (hexane:ethyl acetate=100:0 to 90:10) to obtain 770 mg of ethyl 3-chloro-4-cyclopropylbenzoate as a colorless oil.

Preparation Example 22

To a mixture of ethyl 3-chloro-4-cyclopropylbenzoate (760 mg), THF (10 ml), and EtOH (10 ml) was added a 1 M aqueous sodium hydroxide solution (10 ml), followed by stirring at room temperature overnight. The reaction mixture was concentrated under reduced pressure, diluted with water, and then adjusted to pH 2 by the addition of 1 M hydrochloric acid (20 ml). The precipitated solid was collected by filtration to obtain 666 mg of 3-chloro-4-cyclopropylbenzoic acid as a colorless solid.

Preparation Example 23

To a mixture of 3-fluorophenol (400 mg), ethyl cis-4-hydroxycyclohexanecarboxylate (770 mg), triphenylphosphine (1.4 g), and THF (5 ml) was added a 2.2 M diethyl azodicarboxylate solution in toluene (2 ml) under ice-cooling, followed by stirring at room temperature overnight. To the reaction mixture were added ethyl acetate (20 ml) and a saturated aqueous sodium chloride solution (20 ml), followed by carrying out a separation of the layers , and the organic layer was dried over anhydrous magnesium sulfate, and then concentrated under reduced pressure. To the residue were added hexane (16 ml) and ethyl acetate (4 ml), and the precipitate was separated by filtration. Then, the resulting filtrate was concentrated under reduced pressure. The residue was purified by silica gel column chromatography (hexane: ethyl acetate=100:0 to 93:7) to obtain 357 mg of ethyl trans-4-(3-fluorophenoxy)cyclohexanecarboxylate as a colorless oil.

Preparation Example 24

To a mixture of ethyl trans-4-(3-fluorophenoxy)cyclohexanecarboxylate (350 mg), THF (4 ml), and EtOH (4 ml) was added a 1 M aqueous sodium hydroxide solution (4 ml), followed by stirring at room temperature overnight. The reaction mixture was concentrated under reduced pressure to remove the organic solvent, and then 1 M hydrochloric acid (5 ml) was added thereto, followed by stirring for a while. The precipitated solid was collected by filtration to obtain 278 mg of trans 4-(3-fluorophenoxy)cyclohexanecarboxylic acid as a colorless solid.

Preparation Example 116

A mixture of 6-hydroxynicotinic acid (1.5 g), 1-ethyl-3-(3-dimethylaminopropyl)carbodiimide monohydrochloride (2.5 g), 1-hydroxybenzotriazole monohydrate (2.0 g), triethylamine (2.5 ml), tert-butyl (2-aminoethyl)carbamate (1.8 ml), and DMF (30 ml) was stirred at room temperature overnight. To the reaction mixture was added water, followed by carrying out a separation of the layers using a mixed solvent (3:1) of chloroform and isopropanol, and the organic layer was dried over anhydrous magnesium sulfate, and then concentrated under reduced pressure. The residue was purified by silica gel column chromatography (chloroform:MeOH=100:0 to 90:10) and the resulting product was solidified by the addition of hexane-ethyl acetate (=1:1), and then the solid was collected by filtration and washed to obtain 1.91 g of tert-butyl (2-{[(6-hydroxypyridin-3-yl)carbonyl]amino}ethyl)carbamate as a colorless solid.

Preparation Example 117

To a mixture of tert-butyl (2-{[(6-hydroxypyridin-3 - yl)carbonyl]amino}ethyl)carbamate (1.9 g), ethyl trans-4-hydroxycyclohexanecarboxylate (1.3 g), triphenylphosphine (2.7 g), and THF (50 ml) was added a 2.2 M diethyl azodicarboxylate solution in toluene (3.7 ml) at 0˚C, followed by stirring at room temperature overnight. To the reaction mixture was added water, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (chloroform:MeOH=100:0 to 95:5) to obtain 1.41 g of ethyl cis-4-{[5-({2-[(tert-butoxycarbonyl)amino]ethyl}carbamoyl)pyridin-2-yl]oxy} cyclohexanecarboxylate as a colorless solid.

Preparation Example 118 8

A mixture of benzyl 4-{[cis-4-(ethoxycarbonyl)cyclohexyl]oxy}-2-hydroxybenzoate (530 mg), iodomethane (0.4 ml), potassium carbonate (220 mg), and DMF (5 ml) was stirred at room temperature for 3.5 hours, and then stirred at 45˚C for 1 hour. Thereafter, potassium carbonate (175 mg) was added thereto, followed by stirring at room temperature overnight. To the reaction mixture was added water (20 ml), followed by carrying out a separation of the layers using

ethyl acetate, and the organic layer was washed with a saturated aqueous sodium chloride solution, dried over anhydrous magnesium sulfate, and then concentrated under reduced pressure to obtain 605 mg of benzyl 4-{[cis-4-(ethoxycarbonyl) cyclohexyl]oxy}-2-methoxybenzoate as a brownoil.

Preparation Example 119

A mixture of benzyl 2-chloro-4-{([cis-4-(ethoxycarbonyl)cydohexyl]oxy)benzoate (500 mg), cyclopropylboronic acid monohydrate (190 mg), tetrakistriphenylphosphinepalladium (70 mg), potassium phosphate (894 mg), toluene (5 ml), and water (0.5 ml) was stirred under reflux overnight. To the reaction mixture was added water, followed by carrying out a separation of the layers using ethyl acetate, and the organic layer was dried over anhydrous magnesium sulfate, and then concentrated under reduced pressure. The residue was purified by silica gel column chromatography (hexane: ethyl acetate=90:10) to obtain 320 mg of benzyl 2-cyclopropyl-4-([cis-4-(ethoxycarbonyl)cyclohexyl]oxy]benzoate as a colorless oil.

Preparation Example 120

To a mixture of benzyl 4-fluoro-2-(trifluoromethyl)benzoate (4.1 g), 2-(methylsulfonyl)ethanol (2.53 g), and DMF (50 ml) was added potassium tert-butoxide (4.67 g) under ice-cooling, followed by stirring at room temperature for 3 hours. To the reaction mixture were added 1 M hydrochloric acid and water, followed by carrying out a separation of the layers using ethyl acetate, and the organic layer was washed with water and a saturated aqueous sodium chloride solution, dried over anhydrous magnesium sulfate, and then concentrated under reduced pressure. The residue was purified by silica gel column chromatography (hexane:ethyl acetate=90:10 to 80:20) to obtain 1.19 g of benzyl 4-hydroxy-2-(trifluoromethyl)benzoate as a pale yellow oil.

Preparation Example 121

To a mixture of 2-chloro-4-{[cis-4-(ethoxycarbonyl)cyclohexyl]oxylbenzoic acid (1.8 g), 1-ethyl-3-(3-dimethylaminopropyl)carbodiimide monohydrochloride (1.37 g), 1-hydroxybenzotriazole monohydrate (1.10 g), triethylamine (1.15 ml), and DMF (50 ml) was added tert-butyl (2-aminoethyl)carbamate (0.96 ml), followed by stirring at room temperature overnight. To the reaction mixture was added water (200 ml), and the precipitated solid was collected by filtration. To a mixture of this solid and dioxane (30 ml) was added 4 M hydrogen chloride/dioxane (15 ml), followed by stirring at room temperature for 5 hours. The reaction mixture was concentrated under reduced pressure and the residue was washed with a mixed solvent of hexane-diisopropylether to obtain 2.09 g of ethyl cis-4-{4-[(2-aminoethyl)carbamoyl]-3-chlorophenoxy}cyclohexanecarboxylate hydrochloride as a colorless solid.

Preparation Example 122

To a mixture of methyl [trans-4-(4-hydroxyphenyl)cyclohexyl]acetate (1.25 g), methylene chloride (25 ml), and N-ethyl-N-isopropylpropan-2-amine (1.05 ml) was added trifluoromethanesulfonyl chloride (0.636 ml) at 0˚C, followed by stirring at room temperature overnight. To the reaction mixture was added a saturated aqueous sodium hydrogen carbonate solution, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (hexane:ethyl acetate=100:0 to 50:50) to obtain 1.38 g of methyl [trans-4-(4-{[(trifluoromethyl)sulfonyl]oxy}phenyl)cyclohexyl]acetate as a colorless solid.

Preparation Example 123

A mixture of methyl [trans-4-(4-{[(trifluoromethyl)sulfonyl]oxy}phenyl)cyclohexyl]acetate (200 mg), DMF (10 ml), N-(2-aminoethyl-4-chloro-3-methylbenzamide hydrochloride (200 mg), a 1,1'-bis(diphenylphosphino)ferrocene palladium(II) dichloride/dichloromethane complex (90 mg), and triethylamine (0.2 ml) was stirred at 90˚C for 18 hours under 1 atm of carbon monoxide. The reaction mixture was returned to room temperature, and then water (50 ml) was added thereto. The precipitated solid was collected by filtration. This solid was purified by silica gel column chromatography (chloroform:MeOH=100:0 to 95:5 to 90:10) to obtain 96 mg of methyl {trans-4-[4-({2-[(4-chloro-3-methylbenzoyl)amino] ethyl}carbamoyl)phenyl]cyclohexyl}acetate as a brown solid.

Preparation Example 124

To a mixture of cis-4-(hydroxymethyl)cyclohexanecarboxylic acid (5 g) and ethanol (50 ml) was added concentrated sulfuric acid (0.3 ml) at room temperature, followed by stirring under reflux for 8 hours. The reaction mixture was returned to room temperature and concentrated under reduced pressure. The residue was added to a saturated aqueous sodium hydrogen carbonate solution, followed by carrying out a separation of the layers using chloroform, and the organic layer was dried over anhydrous magnesium sulfate, and then concentrated under reduced pressure to obtain 5.8 g of ethyl cis-4-(hydroxymethyl)cyclohexanecarboxylate as a colorless loil.

Preparation Example 125

To a mixture of ethyl 1-methyl-4-oxocyclohexanecarboxylate (3 g) and ethanol (30 ml) was added sodium borohydride (616 mg) at 0˚C, followed by stirring for 2 hours. To the reaction mixture were added water and ethyl acetate, followed by carrying out a separation of the layers using ethyl acetate, and the organic layer was washed sequentially with a saturated aqueous sodium hydrogen carbonate solution and a saturated aqueous sodium chloride solution, dried over anhydrous magnesium sulfate, and then concentrated under reduced pressure. The residue was purified by silica gel column chromatography (hexane:ethyl acetate=100:0 to 90:10 to 80:20) to obtain 1.27 g of ethyl cis-4-hydroxy-1-methylcyclohexanecarboxylate as a colorless oil.

Preparation Example 126

A mixture of ethyl cis-4-[4-({2-[(4-hydroxybenzoyl)amino]ethyl}carbamoyl)phenoxy]cyclohexanecarboxylate (50 mg), DMF (2 ml), potassium carbonate (30 mg), and 2-chlorobenzyl bromide (0.019 ml) was stirred at 60˚C overnight. To the reaction mixture was added water, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (chloroform:MeOH=100:0 to 95:5), and then the precipitated solid was washed with diisopropylether to obtain 53 mg of ethyl cis-4-(4-{[2-({4-[(2-chlorobenzyl)oxy]benzoyl}amino)ethyl]carbamoyl}phenoxy)cyclohexanecarboxylate as a colorless solid.

Preparation Example 127

A mixture of benzyl 4-fiuorobenzoate (1.5 g), DMSO (10 ml)), methyl piperidin-4-ylacetate hydrochloride (1.26 g), and potassium carbonate (1.81 mg) was stirred at 100˚C for 20 hours. The reaction mixture was added to water, followed by carrying out a separation of the layers using ethyl acetate, and the organic layer was washed with a saturated aqueous sodium chloride solution, dried over anhydrous magnesium sulfate, and then concentrated under reduced pressure. The residue was purified by silica gel column chromatography (hexane:ethyl acetate=80:20) to obtain 1.475 g of benzyl 4-[4-(2-methoxy-2-oxoethyl)piperidin-1-yl]benzoate as a colorless solid.

Preparation Example 128

To a mixture of tert-butyl 4-{[cis-4-(ethoxycarbonyl)cyclohexyl]oxy}-2,5-difluorobenzoate (4 g) and dioxane (40 ml) was added a 4 M hydrogen chloride solution in dioxane (60 ml), followed by stirring at room temperature for 1 hour and then stirring at 60˚C for 5 hours. The reaction mixture was concentrated under reduced pressure and the residue was washed with diisopropyl ether to obtain 3.36 g of 4-{[cis-4-(ethoxycarbonyl)cyclohexyl]oxy}-2,5-difluorobenzoic acid as a colorless solid.

Preparation Example 129

To a mixture of tert-butyl 4-(benzyloxy)-2,5-difluorobenzoate (7.47 g) and THF (101 ml) was added 10% palladium on activated carbon (747 mg), followed by stirring at room temperature for 5 hours under a hydrogen atmosphere (balloon pressure). The reaction mixture was filtered through Celite and washed with ethanol, and then the resulting filtrate was concentrated under reduced pressure. The residue was purified by silica gel column chromatography (chloroform:ethyl acetate=98:2 to 50:50) to obtain 4 g of tert-butyl 2,5-difluoro-4-hydroxybenzoate as a colorless solid.

Preparation Example 130

To a mixture of tert-butyl (2-aminoethyl)carbamate (4.88 ml), triethylamine (4.49 ml), and DMF (100 ml) was

added 2-naphthoyl chloride (5.59 g), followed by stirring at room temperature for 2 hours. The reaction mixture was concentrated under reduced pressure, then water (200 ml) was added thereto, and the precipitated solid was collected by filtration. To a mixture of this solid and dioxane (200 ml) was added 4 M hydrogen chloride solution in dioxane (100 ml), followed by stirring at room temperature overnight. The reaction mixture was filtered, and the solid was collected by filtration and then washed with diisopropylether to obtain 7.7 g ofN-(2-aminoethyl)-2-naphthamide hydrochloride as a colorless solid.

Preparation Example 131

To a mixture of tert-butyl (2-aminoethyl)carbamate (4.34 ml), triethylamine (4.5 ml), and methylene chloride (50 ml) was added 2-chlorophenyl isocyanate (3.8 g), followed by stirring at room temperature for 2 hours. The reaction mixture was concentrated under reduced pressure, then water (200 ml) was added thereto, and the precipitated solid was collected by filtration. To a mixture of this solid and dioxane (20 was added a 4 M hydrogen chloride solution in dioxane (50 ml), followed by stirring at room temperature for 5 hours. The reaction mixture was concentrated under reduced pressure and the residue was washed with diisopropyl ether to obtain 5.89 g of 1-(2-aminoethyl)-3-(2-chlorophenyl)urea hydrochloride as a colorless solid.

Preparation Example 132

To a mixture of benzyl alcohol (2.23 ml) and THF (30 ml) was added potassium tert-butoxide (4.67 g) at 5˚C, followed by stirring for 0.5 hours. To this reaction mixture was added a mixture of tert-butyl 3,4,5-trifluorobenzoate (5 g) and THF (50 ml) at -65˚C, followed by stirring at -65˚C for 1 hour and then stirring at room temperature for 5 hours. To the reaction mixture was added water (150 ml), followed by carrying out a separation of the layers using ether, and the organic layer was washed sequentially with water and a saturated aqueous sodium chloride solution, dried over anhydrous magnesium sulfate, and then concentrated under reduced pressure. To a mixture of this residue and THF (80 ml) was added 10% palladium on activated carbon (500 mg), followed by stirring at room temperature for 2 hours under a hydrogen atmosphere (balloon pressure). The reaction mixture was filtered through Celite and washed with THF, and then the resulting filtrate was concentrated under reduced pressure. The residue was purified by silica gel column chromatography (chloroform:ethyl acetate=98:2 to 50:50) to obtain 3.22 g of tert-butyl 3,5-difluoro-4-hydroxybenzoate as a colorless solid.

Preparation Example 133

To a mixture of diisopropylamine (0.94 ml) and THF (10 ml) was added a 1.6 M n-butyllithium hexanesolution (4.4 ml) at -55˚C over 5 minutes, and then a solution of 2-chloro-3-cyclopropylthiophene (960 mg) in THF (5 ml) was added thereto at -68˚C over 10 minutes, followed by stirring for 50 minutes. Thereafter, to the reaction mixture was added dry ice, followed by returning to room temperature. To the reaction mixture were added water (30 ml) and a 1 M aqueous sodium hydroxide solution (10 ml), and then the aqueous layer was washed with hexane. To the aqueous layer was added 1 M hydrochloric acid (30 ml), followed by carrying out a separation of the layers using ethyl acetate, and the organic layer was washed with a saturated aqueous sodium chloride solution, dried over anhydrous magnesium sulfate, and then concentrated under reduced pressure to obtain 765 mg of 5-chloro-4-cyclopropylthiophene-2-carboxylic acid as a pale yellow solid.

Preparation Example 134

To a mixture of methyl 4-chloro-3-hydroxybenzoate (500 mg), 5-chloro-2,3-difluoropyridine (801 mg), and DMF (10 ml) was added potassium carbonate (1.48 g), followed by stirring at 80˚C for 16 hours. The reaction mixture was returned to room temperature, and water was added thereto, followed by carrying out a separation of the layers using ethyl acetate. The organic layer was washed sequentially with water and a saturated aqueous sodium chloride solution, dried over anhydrous magnesium sulfate, and then concentrated under reduced pressure. The residue was purified by silica gel column chromatography (hexane:ethyl acetate=100:0 to 90:10) to obtain 521 mg of methyl 4-chloro-3-[(5-chloro-3-fluoropyridin-2-yl)oxy]benzoate as a colorless solid.

Preparation Example 135

A mixture of ethyl 3-chloro-4-(trifluoromethyl)benzoate (1 g), potassium cyclopropyltrifluoroborate (644 mg), 2-dichlorohexylphosphino-2',4',6'-triisopropylbiphenyl (189 mg), potassium carbonate (1.64 g), palladium(II) acetate (44 mg), THF (12 ml), and water (1.2 ml) was stirred at 100˚C for 12 hours using a microwave reactor. To the reaction

mixture was added water, followed by carrying out a separation of the layers using ethyl acetate, and the organic layer was dried over anhydrous magnesium sulfate, and then concentrated under reduced pressure. The residue was crudely purified by silica gel column chromatography (hexane:ethyl acetate=95:5) to obtain an oil. Using this oil, the same operation was carried out, and then to the resulting oil were added MeOH (15 ml), EtOH (15 ml), and a 1 M aqueous sodium hydroxide solution (15 ml), followed by stirring at room temperature overnight. To the reaction mixture was added 1 M hydrochloric acid (15 ml), followed by concentrating under reduced pressure, and the organic solvent was evaporated. The precipitated solid was collected by filtration to obtain 120 mg of 3-cyclopropyl-4-(trifluoromethyl)benzoic acid as a colorless solid.

Preparation Example 136

To a mixture of N-{2-[(4-aminobenzoyl)amino]ethyl}-2-naphthamide hydrochloride (200 mg), ethyl 4-cyclohexanonecarboxylate (108 mg), sodium acetate (50 mg), and methylene chloride (5 ml) was added acetic acid (0.05 ml), followed by stirring at room temperature for 1 hour, and then sodium triacetoxyborohydride (180 mg) was added thereto, followed by stirring for 2 hours. Thereafter, DMF (2 ml) was added thereto, followed by stirring overnight. To the reaction mixture was added a saturated aqueous sodium hydrogen carbonate solution, followed by carrying out a separation of the layers using ethyl acetate, and the organic layer was washed with a saturated aqueous sodium chloride solution, dried over anhydrous magnesium sulfate, and then concentrated under reduced pressure. The residue was purified by silica gel column chromatography (chloroform:MeOH=100:0 to 95:5) to obtain 96 mg of ethyl 4-[(4-{[2-(2-naphthoylamino)ethyl]carbamoyl}phenyl)amino]cyclohexanecarboxylate as a colorless oil.

Preparation Example 137

To a mixture of benzyl 4-[(4-hydroxycyclohexyl)oxy]benzoate (800 mg), tert-butyl bromoacetate (726 mg), and THF (10 ml) was added 60% oily sodium hydride (110 mg), followed by stirring at room temperature for 16 hours. To the reaction mixture was added a saturated aqueous ammonium chloride solution, followed by carrying out a separation of the layers using ethyl acetate, and the organic layer was washed with a saturated aqueous sodium chloride solution, dried over anhydrous magnesium sulfate, and then concentrated under reduced pressure. The residue was purified by silica gel column chromatography (hexane:ethyl acetate=95:5 to 75:25) to obtain 272 mg of benzyl 4-{[4-(2-tert-butoxy-2-oxoethoxy)cyclohexyl]oxy}benzoate as a colorless oil.

Preparation Example 138

A mixture of benzyl 4-(1,4-dioxaspiro[4.5]decan-8-yloxy)benzoate (3.0 g), 1 M hydrochloric acid (20 ml), THF (20 ml), and EtOH (20 ml) was stirred at room temperature overnight, and then stirred at 45˚C for 40 minutes. The reaction mixture was left to stand to room temperature, and 1 M hydrochloric acid (7.5 ml) was added thereto, followed by stirring at room temperature for 4.5 hours. The reaction mixture was concentrated under reduced pressure to remove EtOH and THF, followed by carrying out a separation of the layers using ethyl acetate. The organic layer was washed with a saturated aqueous sodium chloride solution, dried over anhydrous magnesium sulfate, and then concentrated under reduced pressure. The residue was purified by silica gel column chromatography (hexane:ethyl acetate=95:5 to 65:35) to obtain 2.5 g of benzyl 4-[(4-oxocyclohexyl)oxy]benzoate as a colorless oil.

Preparation Example 139

To a mixture of benzyl 4-[(dimethoxyphosphoryl)methyl]benzoate (1.44 g) and THF (6.8 ml) was added potassium tert-butoxide (448 mg) under ice-cooling, followed by stirring at 0˚C for 1 hour, and then ethyl 4-oxocyclohexanecarboxylate (680 mg) was added thereto, followed by stirring at 0˚C for 3 hours. To the reaction mixture were added ethyl acetate and water, followed by carrying out a separation of the layers using ethyl acetate, and the organic layer was washed sequentially with water and a saturated aqueous sodium chloride solution, dried over anhydrous magnesium sulfate, and then concentrated under reduced pressure. The residue was purified by silica gel column chromatography (hexane:ethyl acetate=90: 10) to obtain an oil. To a mixture of this oil and THF (10 ml) was added 10% palladium on activated carbon (5 mg), followed by stirring at room temperature for 3 hours under a hydrogen atmosphere (balloon pressure). The reaction mixture was filtered through Celite and washed with THF, and then the resulting filtrate was concentrated under reduced pressure. To the resulting residue was added hexane (5 ml), and the precipitated solid was collected by filtration to obtain 10 mg of 4-{[4-(ethoxycarbonyl)cyclohexyl]methyl}benzoic acid as a colorless solid.

Preparation Example 140

A mixture of N-(2-{[4-(1,4-dioxaspiro[4.5]decan-8-yloxy)benzoyl]amino}ethyl)-2-naphthamide (2.0 g), water (20 ml), and acetic acid (100 ml) was stirring at 65˚C for 0.5 hours. The reaction mixture was concentrated under reduced pressure, then water was added thereto, and the precipitated solid was collected by filtration and washed with diisopropylether to obtain 1.8 g of N-[2-({4-[(4-oxocyclohexyl)oxyl]benzoyl}amino)ethyl]-2-naphthamide as a colorless solid.

Example 1

To a mixture of ethyl trans-4-(4-{[2-(benzoylamino)ethyl]carbamoyl}phenoxy)cyclohexanecarboxylate (102 mg), EtOH (3 ml), and THF (3 ml) was added a 1 M aqueous sodium hydroxide solution (0.9 ml) at room temperature, followed by stirring at room temperature for 5 hours. The reaction mixture was concentrated under reduced pressure, and water was added thereto, followed by adjusting to pH 3 by the addition of 1 M hydrochloric acid. The precipitated solid was collected by filtration to obtain 85 mg of trans-4-(4-{[2-(benzoylamino)ethyl]carbamoyl}phenoxy)cyclohexanecarboxylic acid as a colorless solid.

Example 2

To a mixture of ethyl cis-4-{4-[(2-aminoethyl)carbamoyl]phenoxy}cyclohexanecarboxylate hydrochloride (100 mg), chloroform (1 ml), and triethylamine (0.11 ml) was added trans-2-phenylcyclopropyl isocyanate (45 mg), followed by stirring at room temperature for 3 hours, and then the reaction mixture was concentrated under reduced pressure to obtain a residue. To this residue were added EtOH (3 ml) and THF (3 ml), and then a 1 M aqueous sodium hydroxide solution (0.5 ml) was added thereto, followed by stirring at room temperature overnight. To the reaction mixture was added 1 M hydrochloric acid (2.5 ml), followed by stirring, and the precipitated solid was collected by filtration to obtain 110 mg of rel-cis-4-(4-{[2-({[(1R, 2S)-2-phenylcyclopropyl]carbamoyl}amino)ethyl]carbamoyl}phenoxy)cyclohexanecarboxylic acid as a colorless solid.

Example 3

To a mixture of ethyl cis-4-{4-[(2-aminoethyl)carbamoyl]phenoxy}cyclohexanecarboxylate hydrochloride (100 mg), 1-benzofuran-5-carboxylic acid (53 mg), 1-ethyl-3-(3-dimethylaminopropyl)carbodiimide monohydrochloride (65 mg), 1-hydroxybenzotriazole monohydrate (45 mg), and DMF (2 ml) was added triethylamine (0.12 ml), followed by stirring at room temperature for 6 hours. To the reaction mixture was added water (8 ml), followed by stirring, and then the precipitated solid was collected by filtration and dried to obtain a colorless solid. To this solid were added EtOH (3 ml) and THF (3 ml), and then a 1 M aqueous sodium hydroxide solution (1 ml) was added thereto, followed by stirring at room temperature overnight. To the reaction mixture was added 1 M hydrochloric acid (1.1 ml), followed by stirring, then concentrating under reduced pressure, and removal of EtOH and THF by evaporation. The precipitated solid was collected by filtration and washed with water to obtain 105 mg of cis-4-[4-({2-[(1-benzofuran-5-ylcarbonyl)amino]ethyl} carbamoyl)phenoxy]cyclohexanecarboxylic acid as a colorless solid.

Example 4

To a mixture of ethyl cis-4-{4-[(2-aminoethyl)carbamoyl]-2-fluorophenoxy}cyclohexanecarboxylate hydrochloride (52 mg), methylene chloride (2 ml), and triethylamine (0.05 ml) was added 3-fluoro-4-(trifluoromethyl)benzoyl chloride (0.027 ml) at 0˚C, followed by stirring at room temperature overnight. The reaction mixture was concentrated under reduced pressure, then water was added thereto, and the precipitated solid was collected by filtration. To this solid were added MeOH (2 ml) and THF (2 ml), and then a 1 M aqueous sodium hydroxide solution (2 ml) was added thereto, followed by stirring at 50˚C for 2 hours. To the reaction mixture was added 1 M hydrochloric acid (2 ml), followed by removal of the solvent by concentrating under reduced pressure and evaporating the solvent, and the precipitated solid was collected by filtration to obtain 58 mg of cis-4-{2-fluoro-4-[(2-{[3-fluoro-4-(trifluoromethyl)benzoyl] amino}ethyl)carbamoyl]phenoxy]cyclohexanecarboxylic acid as a colorless solid.

Example 5

To a mixture of ethyl cis-4-{4-[(2-aminoethyl)carbamoylJphenoxy}cyclohexanecarboxylate hydrochloride (100 mg), methylene chloride (5 ml), and triethylamine (0.045 ml) was added carbonyldiimidazole (48 mg) at 0˚C, followed by stirring at 0˚C for 10 minutes, and then 1-phenylpiperazine (0.049 ml) was added thereto, followed by stirring at room temperature overnight. The reaction mixture was concentrated under reduced pressure, and the residue was purified

by silica gel column chromatography (chloroform:MeOH=97.5:2.5) to obtain a colorless solid. To this solid were added EtOH (3 ml) and THF (3 ml), and then a 1 M aqueous sodium hydroxide solution (3 ml) was added thereto, followed by stirring at room temperature for 6 hours. To the reaction mixture was added 1 M hydrochloric acid (3 ml), then removal of EtOH and THF by concentrating under reduced pressure, and the precipitated solid was collected by filtration to obtain 70 mg ofcis-4-{4-[(2-{[(4-phenylpiperazin-1-yl)carbonyl]amino}ethyl)carboyl]phenoxy}cyclohexanecarboxylic acid as a colorless solid.

Example 6

To a mixture of tert-butyl {4-[4-({2-[(4-chlorobenzoyl)amino]ethyl}carbamoyl)phcnyl]cyclohexyl}acetate (197 mg) and methylene chloride (3 ml) was added trifluoroacetic acid (1 ml) at 0˚C, followed by stirring at room temperature for 3 hours. The reaction mixture was concentrated under reduced pressure, then water was added thereto, and the precipitated solid was collected by filtration to obtain 140 mg of {4-[4-({2-[(4-chlorobenzoyl)amino]ethyl}carbamoyl)phenyl]cyclohexyl}acetic acid as a colorless solid.

Example 7

To a mixture of cis-4-[4-({2-[(4-chlorobenzoyl)amino]ethyl}carbamoyl)phenoxy]cyclohexanecarboxylic acid (100 mg), THF (4 ml), and DMF (1ml) was added carbonyldiimidazole (55 mg), followed by stirring at 60˚C for 40 minutes, and methanesulfonamide (40 mg) and 1,8-diazabicyclo[5.4.0]undeca-7-ene (0.045 ml) were added thereto under ice-cooling, followed by stirring at room temperature for 3 days. To the reaction mixture were added 1 M hydrochloric acid (10 ml) and ethyl acetate (10 ml), and the insoluble materials were collected by filtration to obtain 41 mg of 4-chloro-N-(2-{[4-({cis-4-[(methylsulfonyl)carbamoyl]cyclohexyl}oxy)benzoyl]amino}ethyl)benzarnide as a colorless solid.

Example 8

A mixture of cis-4-[4-({2-[(4-chlorobenzoyl)amino]ethyl}carbamoyl)phenoxy]cyclohexanecarboxylic acid (400 mg), ammonium chloride (60 mg), 1-ethyl-3-(3-dimethylaminopropyl)carbodiimide monohydrochloride (225 mg), 1-hydroxybenzotriazole monohydrate (160 mg), DMF (5 ml), and triethylamine (0.16 ml) was stirred at room temperature for 5 hours. To the reaction mixture was added water (10 ml), and the solid was collected by filtration and washed with water to obtain 372 mg of 4-[(cis-4-carbamoylcyclohexyl)oxy]-N-{2-[(4-chlorobenzoyl)amino]ethyl}benzamide as a colorless solid.

Example 9

A mixture of 4-chloro-N-[2-({4-[(cis-4-cyanocyclohexyl)oxy]benzoyl}amino)ethyl]benzamide (298 mg), sodium azide (220 mg), triethylamine hydrochloride (480 mg), and 1-methyl-2-pyrrolidinone (3 ml) was stirred at 140˚C for 11 hours. To the reaction mixture were added water and chloroform, and the insoluble materials were collected by filtration, and the solid was purified by silica gel column chromatography (chlorofonn:MeOH=95:5 to 80:20), and then the precipitated solid was washed with ethyl acetate to obtain 16 mg of 4-chloro-N-{2-[(4-{[cis-4-(1H-tetrazol-5-yl)cyclohexyl]oxy}benzoyl)amino]ethyl}benzamide as a brown solid.

Example 200

A mixture ofN-(2-aminoethyl)-4-chlorobenzamide hydrochloride (32 mg), 4-{[cis-4-(ethoxycarbonyl)cyclohexyl]oxy}-2-methoxybenzoic acid (58 mg), 1-methyl-3-(3-dimethylaminopropyl)carbodiimide monohydrochloride (35 mg), 1-hydroxybenzotriazole monohydrate (30 mg), THF (3 ml), and triethylamine (0.075 ml) was stirred at room temperature for 16 hours. To the reaction mixture was added water, followed by carrying out a separation of the layers using ethyl acetate, and the organic layer was washed sequentially with a saturated aqueous sodium hydrogen carbonate solution, 1 M hydrochloric acid, and asaturated aqueous sodium chloride solution, dried over anhydrous magnesium sulfate, and then concentrated under reduced pressure. To the residue were added EtOH (2 ml), THF (2 ml), and a 1 M aqueous sodium hydroxide solution (0.6 ml), followed by stirring at 45˚C for 3 hours. To the reaction mixture were added 1 M hydrochloric acid (0.6 ml) and water (5 ml), followed by stirring and then removal of EtOH and THF by concentrating under reduced pressure. The residue was subjected to extraction with ethyl acetate, and the organic layer was washed with saturated aqueous sodium chloride solution, dried over anhydrous magnesium sulfate, and then concentrated under reduced pressure. To the residue were added ethyl acetate and hexane, and the precipitated solid was collected by filtration and washed with water to obtain 46 mg of cis-4-[4-({2-[(4-chlorobenzoyl)amino] ethyl} carbamoyl)-3-methoxyphenoxy]cyclohexanecarboxylic acid as a colorless solid.

Example 201

To a mixture of N-{2-[(4-hydroxybenzoyl)amino]ethyl}-2-naphthamide (170 mg), methyl (trans-4-hydroxycyclohexyl)acetate (80 mg), triphenylphosphine (140 mg), and THF (2 ml) was added a 2.2 M diethyl azodicarboxylate solution in toluene (0.24 ml) at room temperature, followed by stirring at room temperature overnight. The reaction mixture was added to a saturated aqueous sodium hydrogen carbonate solution, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (chloroform:MeOH=100:0 to 95:5), and to the resulting product were added EtOH (5 ml), THF (5 ml), and a 1 M aqueous sodium hydroxide solution (2 ml), followed by stirring at room temperature overnight. The reaction mixture was concentrated under reduced pressure, and EtOH and THF was evaporated. To the residue was added water, followed by adjusting to pH 4 by the addition of a 10% aqueous citric acid solution, and the precipitated solid was collected by filtration. This solid was purified by silica gel column chromatography (chloroform:MeOH=100:0 to 90:10) to obtain 20 mg of [cis-4-(4-{[2-(2-naphthoylamino}ethyl]carbamoyl}phenoxy)cyclohexyl]acetic acid as a colorless solid.

Example 202

To a mixture of ethyl cis-4-(4-{[2-(2-naphthoylamino)ethyl]carbamoyl}phenoxy)cyclohexanecarboxylate (100 mg) and methylene chloride (3 ml) was added a 1.0 M diisobutylaluminum hydride solution in toluene (0.5 ml) under ice-cooling, followed by stirring at the same temperature for 45 minutes. Thereafter, a 1.0 M diisobutylaluminum hydride solution in toluene (0.5 ml) was added thereto, followed by stirring for 15 minutes, and a 1.0 M diisobutylaluminum hydride solution in toluene (0.5 ml) was added thereto again, followed by stirring for 1 hour. To the reaction mixture were added 1 M hydrochloric acid (5 ml) and a saturated aqueous potassium sodium tartrate solution (5 ml), followed by carrying out a separation of the layers using ethyl acetate, and the organic layer was washed with a saturated aqueous sodium chloride solution, dried over anhydrous magnesium sulfate, and then concentrated under reduced pressure. The residue was purified by silica gel column chromatography (chloroform:MeOH=98:2 to 90:10), to the resulting product were added ethyl acetate and hexane, and the precipitated solid was collected by filtration to obtain 20 mg of N-{2-[(4-{[cis-4-(hydroxymethyl) cyclohexyl]oxy}benzoyl)amino]ethyl}-2-naphthamide as a colorless solid.

Example 203

A mixture of cis-4-(4- {[2-(2-naphthoylamino)ethyl]carbamoyl}phenoxy)cyclohexanecarboxylic acid (50 mg), methaneamine hydrochloride (10 mg), 1-ethyl-3-(3"dimethylaminopropyl)carbodiimide monohydrochloride (30 mg), 1-hydroxybenzotriazole monohydrate (22 mg), DMF (1 ml), and triethylamine (0.05 ml) was stirred at room temperature overnight. To the reaction mixture was added water, and the precipitated solid was collected by filtration to obtain 48 mg of N-{2-[(4-{[cis-4-(methylcarbamoyl)cyclohexyl]oxy}benzoyl)arnino]ethyl}-2-naphthamide as a colorless solid.

Example 204

A mixture of cis-4-(4-{[2-(2-naphthoylamino)ethyl]carbamoyl}phenoxy)cyclohexanecarboxylic acid (50 mg), tert-butyl (2-aminoethyl)carbamate (20 mg), 1-ethyl-3-(3-dimethylaminopropyl)carbodiimide monohydrochloride (30 mg), 1-hydroxybenzotriazole monohydrate (22 mg), DMF (1 ml), and triethylamine (0.04 ml) was stirred at room temperature overnight. To the reaction mixture was added water, and the precipitated solid was collected by filtration. To this solid were added dioxane (1 ml) and 4 M hydrochloric acid/ethyl acetate (1 ml), followed by stirring at room temperature for 4 hours. The reaction mixture was concentrated under reduced pressure, and the residue was solidified with diisopropylether and washed to obtain 28 mg of N-(2-{[4-({cis-4-[(2-aminoethyl)carbamoyl]cyclohexyl}oxy)benzoyl]amino}ethyl)-2-naphthamide hydrochloride as a colorless solid.

Example 205

A mixture of cis-4-(4-{[2-(2-naphthoylamino)ethyl]carbamoyl}phenoxy)cyclohexanecarboxylic acid (50 mg), glycine ethyl ester hydrochloride (18 mg), 1-ethyl-3-(3-dimethylaminopropyl)carbodiimide monohydrochloride (30 mg), 1-hydroxybenzotriazole monohydrate (22 mg), DMF (1 ml), and triethylamine (0.05 ml) was stirred at room temperature overnight. To the reaction mixture was added water, and the precipitated solid was collected by filtration. To this solid were added EtOH (2 ml), THF (2 ml), and a 1 M aqueous sodium hydroxide solution (0.5 ml), followed by stirring at 50˚C for 4 hours. The reaction mixture was concentrated under reduced pressure to remove EtOH and THF. To the residue was added water, followed by adjusting to pH 4 by the addition of a 10% aqueous citric acid solution, and the precipitated

solid was collected by filtration to obtain 34 mg of N-{[cis-4-(4-{[2-(2-naphthoylamino)ethyl]carbamoyl}phenoxy)cyclohexyl]carbonyl}glycine as a colorless solid.

Example 206

A mixture of cis-4-(4-{[2-(2-naphthoylamino)ethyl]carbamoyl}phenoxy)cyclohexanecarboxylic acid (50 mg), 3-hydroxybutan-2-one (14 mg), 1-ethyl-3-(3-dimethylaminopropyl)carbodiimide monohydrochloride (30 mg), 1-hydroxybenzotriazole monohydrate (22 mg), DMF (1 ml), and triethylamine (0.04 ml) was stirred at room temperature overnight. To the reaction mixture was added water, and the precipitated solid was collected by filtration. To this solid were added acetic acid (2 ml) and ammonium acetate (50 mg), followed by refluxing overnight. To the reaction mixture were added water and a saturated aqueous sodium hydrogen carbonate solution, followed by carrying out a separation of the layers using chloroform, and the organic layer was washed with a saturated aqueous sodium chloride solution, dried over anhydrous magnesium sulfate, and then concentrated under reduced pressure. The residue was purified by silica gel column chromatography (chloroform:MeOH=100:0 to 90:10) to obtain 4 mg of N-{2-[(4-{[cis-4-(4,5-dimethyl-1,3-oxazol-2-yl)cyclohexyl]oxy}benzoyl)amino]ethyl}-2-naphthamide as a colorless solid.

Example 207

To a mixture of N-[2-({4-[(4-oxocyclohexyl)oxy]benzoyl}amino)ethyl]-2-naphthamide (100 mg) and methanol (10 ml) was added sodium borohydride (9 mg) at 0˚C, followed by stirring for 0.5 hours. The reaction mixture was concentrated under reduced pressure, and then 1 M hydrochloric acid was added thereto, followed by extracting with chloroform. The organic layer was dried over anhydrous magnesium sulfate and then concentrated under reduced pressure. The residue was washed with diisopropylether to obtain 48 mg of N-[2-({4-[(4-hydroxy cyclohexyl)oxy]benzoyl}amino)ethyl]-2-naphthamide as a colorless solid.

Example 208

To a mixture ofN-[2-({4-[(4-oxocyclohexyl)oxy]benzoyl}amino)ethyl]-2-naphthamide (100 mg) and ether (10 ml) was added a 3.0 M methylmagnesium bromide ether solution (0.4 ml) at 0˚C, followed by stirring for 0.5 hours. The reaction mixture was concentrated under reduced pressure, and then 1 M hydrochloric acid was added thereto, followed by carrying out a separation of the layers using chloroform. The organic layer was dried over anhydrous magnesium sulfate and then concentrated under reduced pressure. The residue was washed with diisopropyl ether to obtain 56 mg ofN-[2-({4-[(4-hydroxy-4-methyleyelohexyl)oxy]benzoyl}amino)ethyl]-2-naphthamide as a colorless solid.

Example 209

A mixture of tert-butyl [cis-4-(4-{[2-(2-naphthoylamino)ethyl]carbamoyl}phenoxy)cyclohexyl]carbamate (623 mg) and 4 M hydrogen chloride/ethyl acetate (7 ml) was stirred at room temperature overnight. The reaction mixture was concentrated under reduced pressure, and to the resulting residue were added ethyl acetate, ethanol, and water at 100˚C to make a solution, followed by being left to cool at room temperature. Thereafter, the precipitated solid was collected by filtration to obtain 368 mg of N-[2-({4-[(cis-4-aminocyclohexyl)oxy]benzoyl}amino)ethyl]-2-naphthamide hydrochloride as a colorless solid.

Example 210

To a mixture of N-[2-({4-[(cis-4-aminocyclohexyl)oxy]benzoyl}amino)ethyl]-2-naphthamide hydrochloride (70 mg) and methylene chloride (3 ml) were added triethylamine (0.065 ml) and acetyl chloride (0.016 ml), followed by stirring at room temperature for 3 hours. Thereafter, the solvent was evaporated, to the residue was added water, followed by stirring for a while, and the solid was collected by filtration to obtain 69 mg of N-[2-({4-[(cis-4-acetamidecyclohexyl)oxy]benzoyl}amino)ethyl]-2-naphthamide as a colorless solid.

The chemical structural formulae of Preparation Example Compounds are shown in Tables 2 to 42 below. Further, the chemical structural formulae of Example Compounds are shown in Tables 43 to 133 below.
Moreover, the preparation methods and the physical data of Preparation Example Compounds are shown in Tables 134 to 144 below. Further, the preparation methods and the physical data of Example Compounds are shown in Tables 145 to 169 below.

[Table 2]

| Pr | Structure |
|---|---|
| 1 | 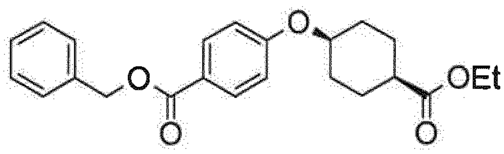 |
| 2 | 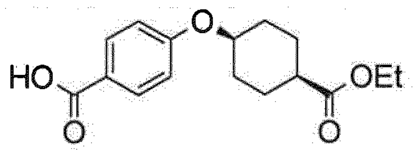 |
| 3 | 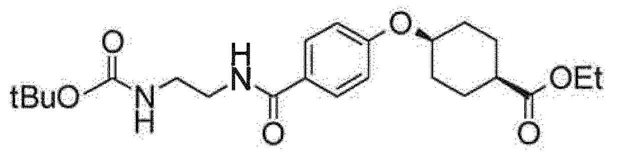 |
| 4/Cl | 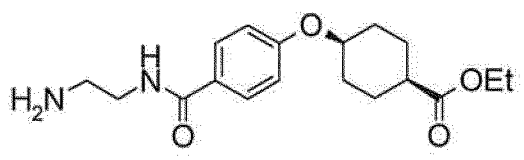 |
| 5 | 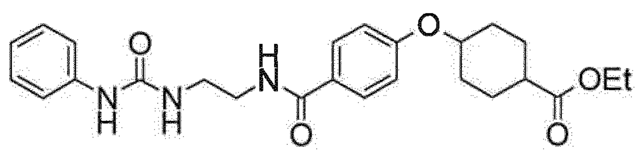 |
| 6 | 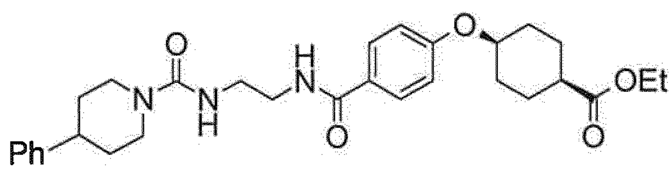 |
| 7 | 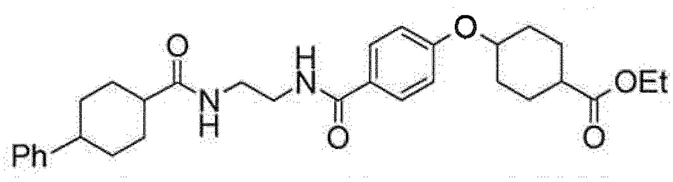 |
| 8 | 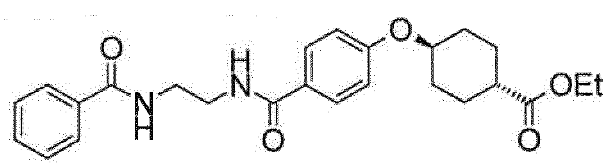 |

[Table 3]

| Pr | Structure |
|---|---|
| 9 | 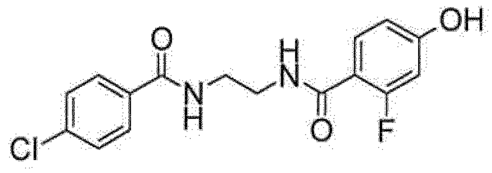 |
| 10 | 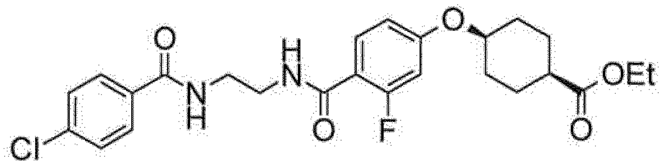 |
| 11 | 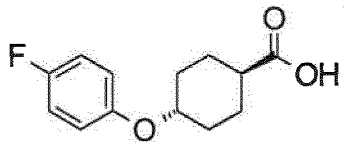 |
| 12 | 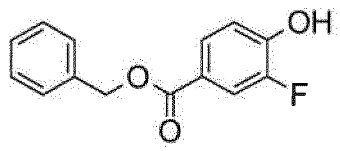 |
| 13 | 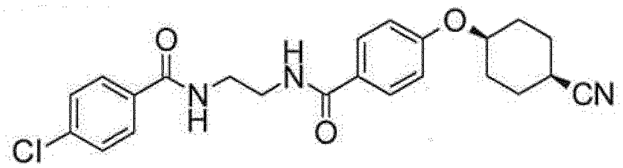 |
| 14 | 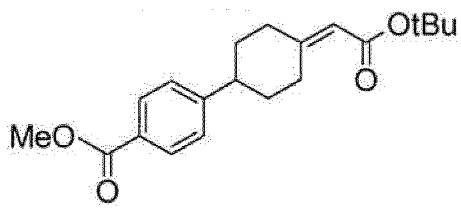 |
| 15 | 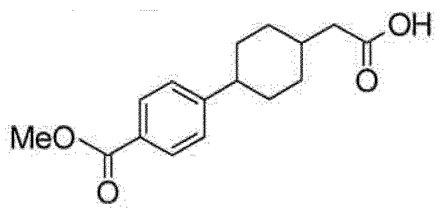 |

[Table 4]

| Pr | Structure |
|---|---|
| 16 | 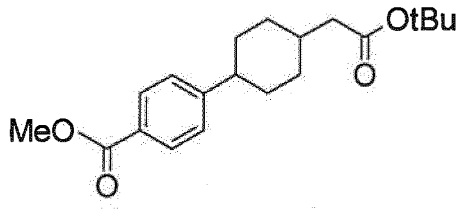 |
| 17 | 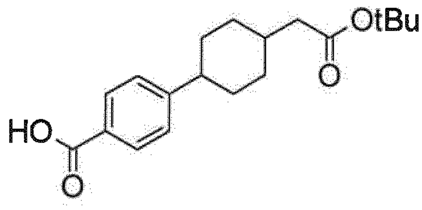 |
| 18 | 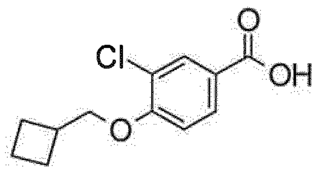 |
| 19 | 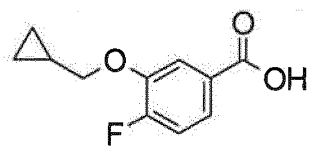 |
| 20 | 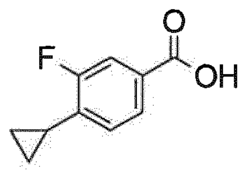 |
| 21 | 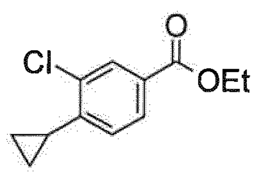 |
| 22 | 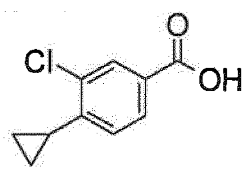 |
| 23 | 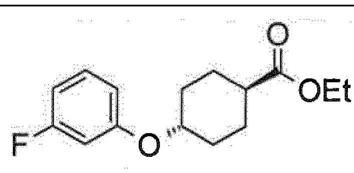 |

[Table 5]

| Pr | Structure |
|---|---|
| 24 | 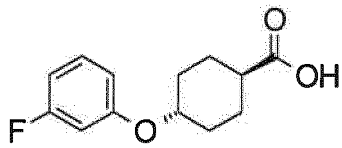 |
| 25 | 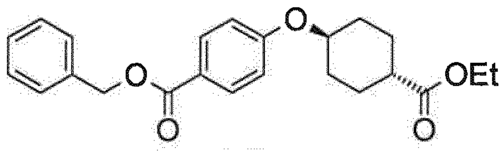 |
| 26 | 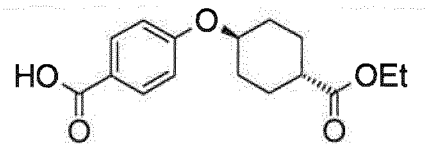 |
| 27 | 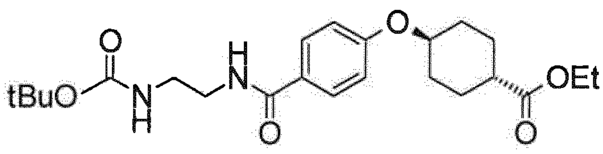 |
| 28/Cl | 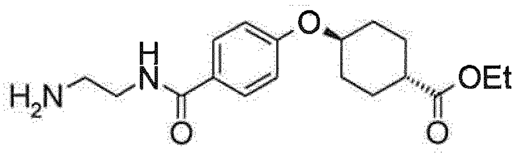 |
| 29 | 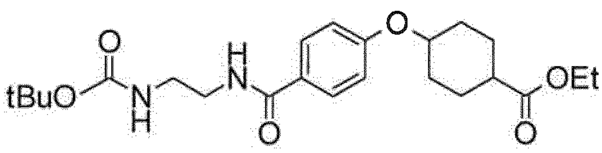 |
| 30/Cl | 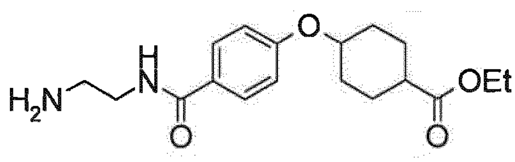 |
| 31 | 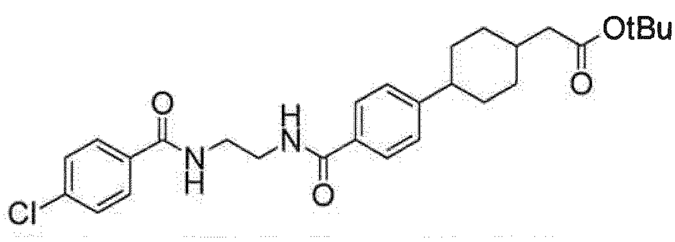 |

[Table 6]

| Pr | Structure |
|---|---|
| 32 | 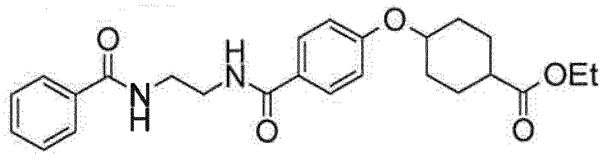 |
| 33 | 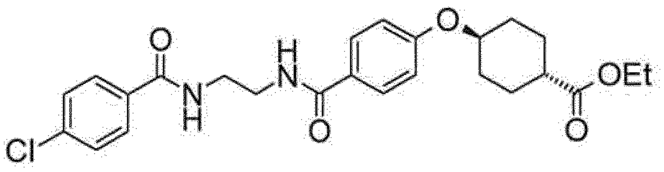 |
| 34 | 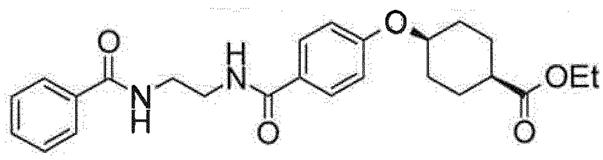 |
| 35 | 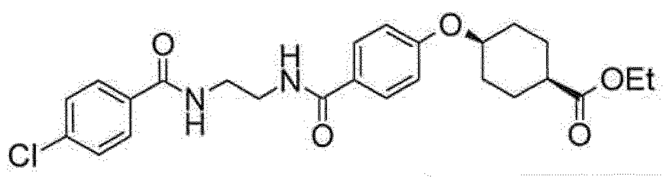 |
| 36 | 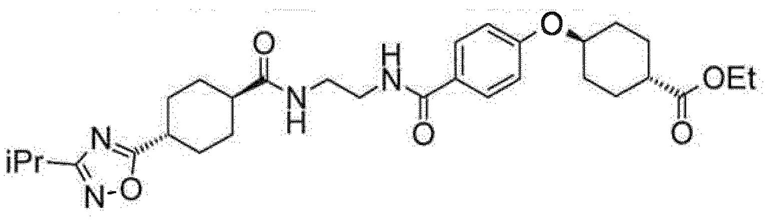 |
| 37 | 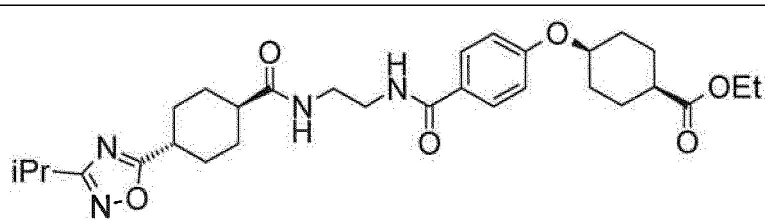 |
| 38 | 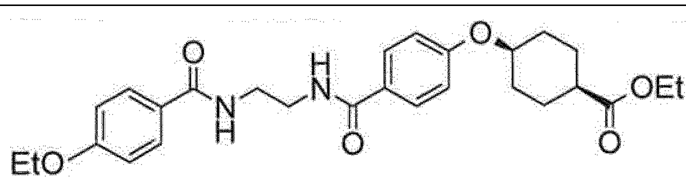 |

[Table 7]

| Pr | Structure |
|---|---|
| 39 | 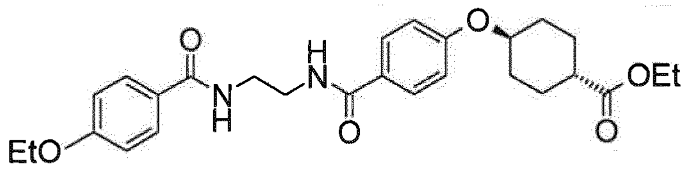 |
| 40 | 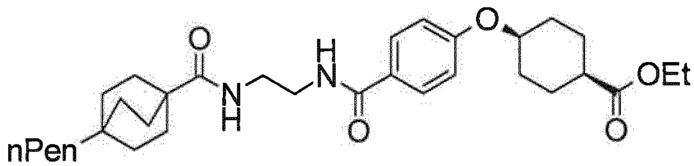 |
| 41 | 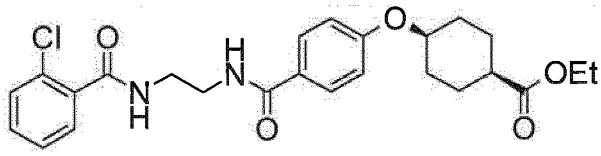 |
| 42 | 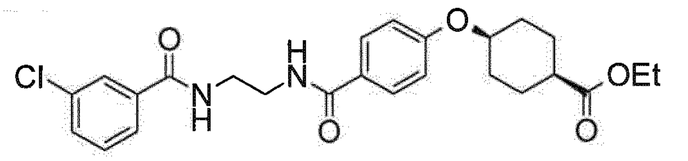 |
| 43 | 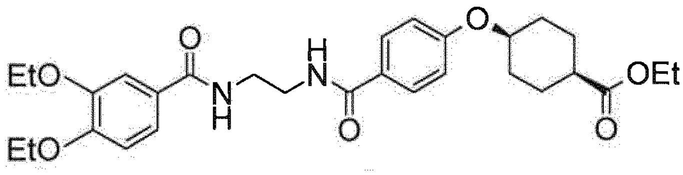 |
| 44 | 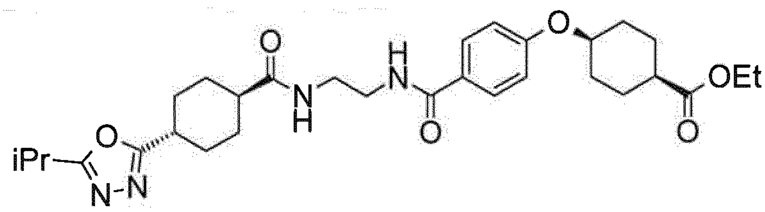 |
| 45 | 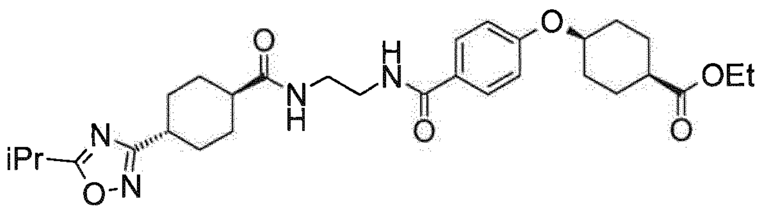 |

[Table 8]

| Pr | Structure |
|---|---|
| 46 | 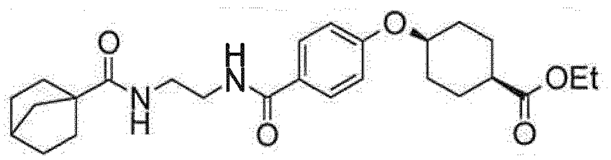 |

(continued)

| Pr | Structure |
|---|---|
| 47 | 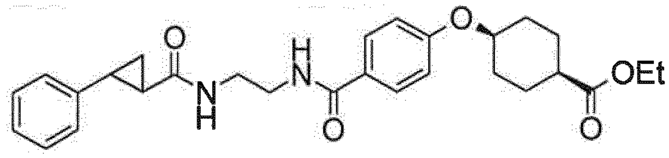 |
| 48 | 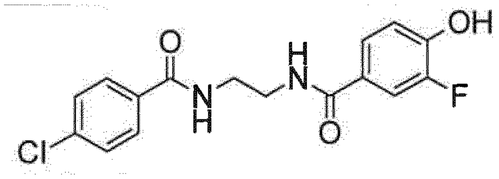 |
| 49 | 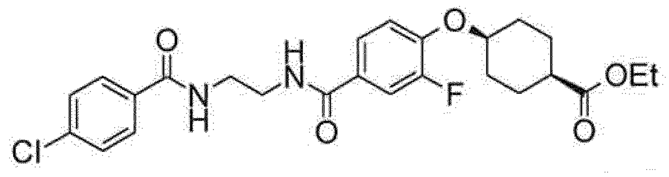 |
| 50 | 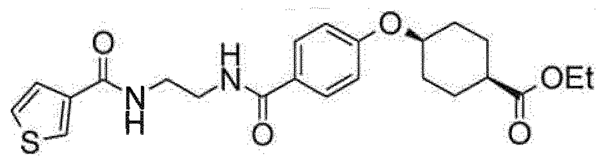 |
| 51 | 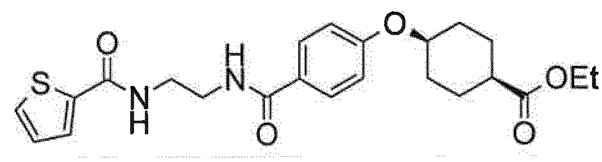 |
| at’ | 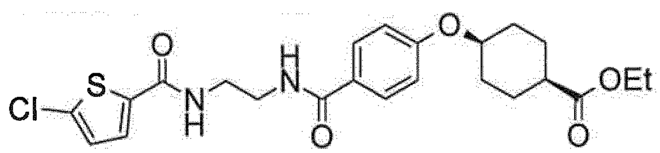 |
| 53 | 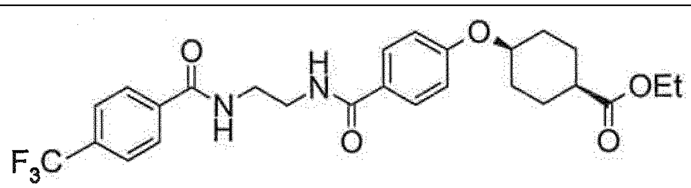 |

[Table 9]

| Pr | Structure |
|---|---|
| 54 | 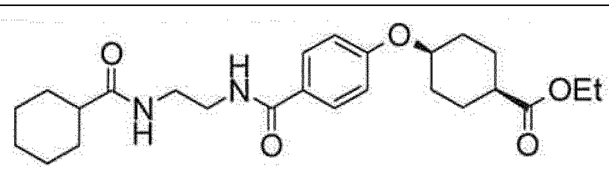 |

(continued)

| Pr | Structure |
|---|---|
| 55 | 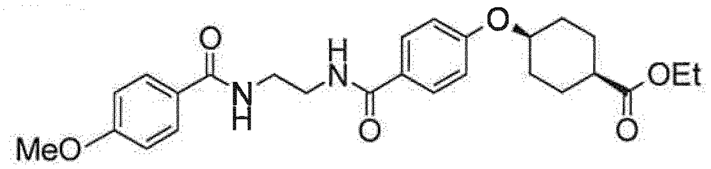 |
| 56 | 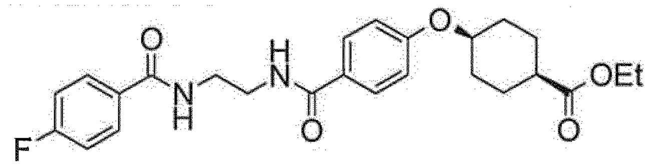 |
| 57 | 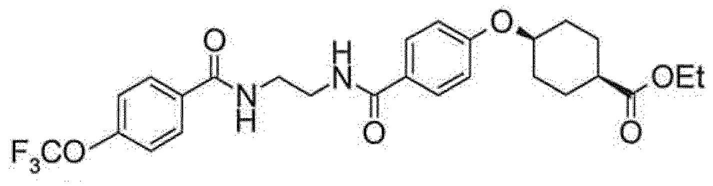 |
| 58 | 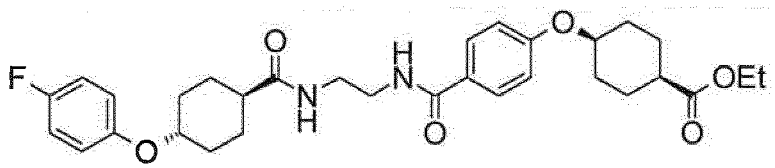 |
| 59 | 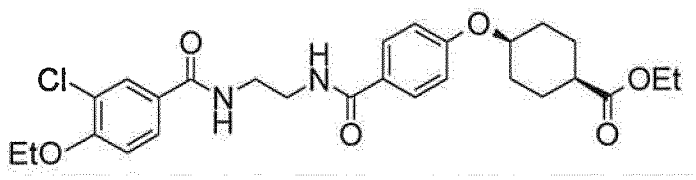 |
| 60 | 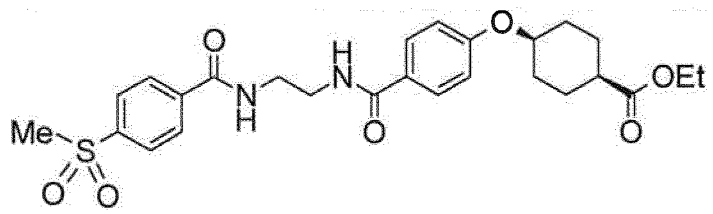 |

[Table 10]

| Pr | Structure |
|---|---|
| 61 | 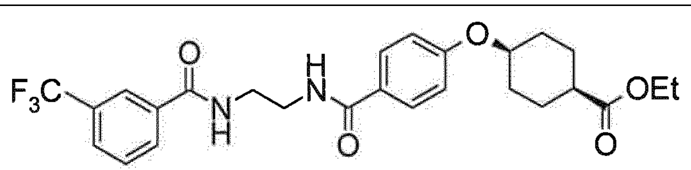 |
| 62 | 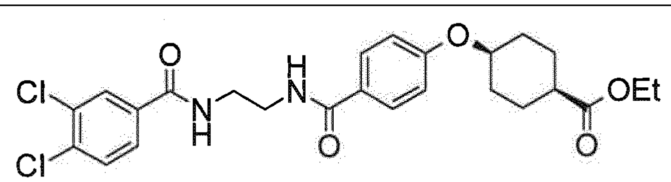 |

(continued)

| Pr | Structure |
|---|---|
| 63 | 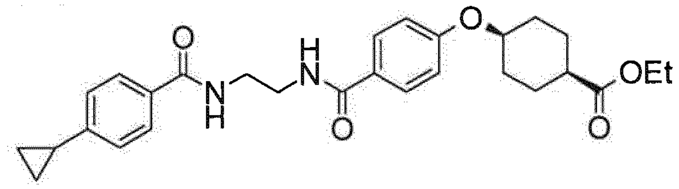 |
| 64 | 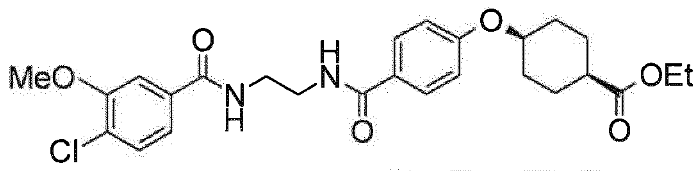 |
| 65 | 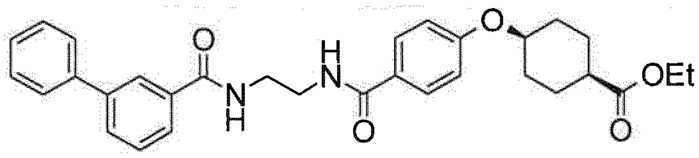 |
| 66 | 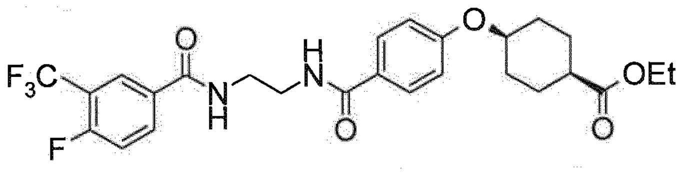 |
| 67 | 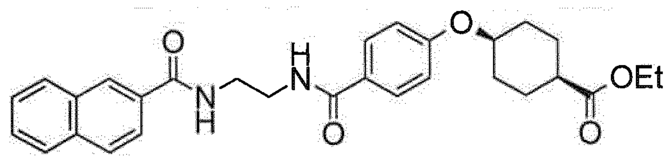 |
| 68 | 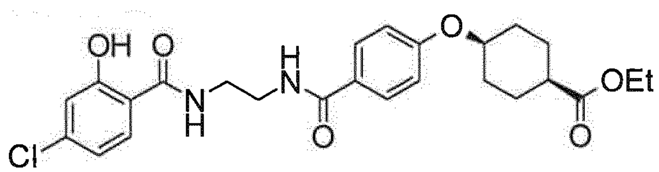 |

[Table 11]

| Pr | Structure |
|---|---|
| 69 | 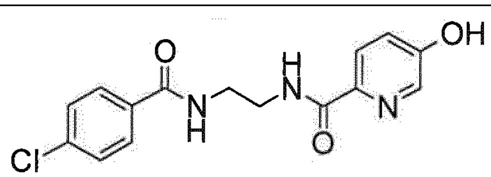 |
| 70 | 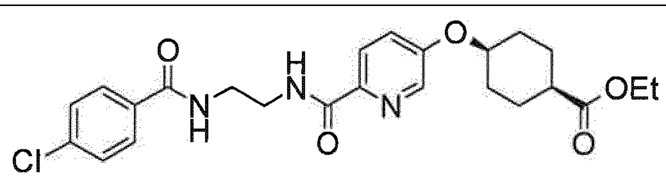 |

(continued)

| Pr | Structure |
|---|---|
| 71 | 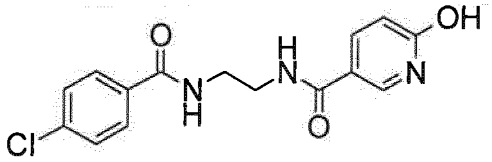 |
| 72 | 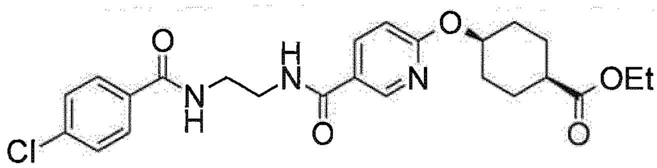 |
| 73 | 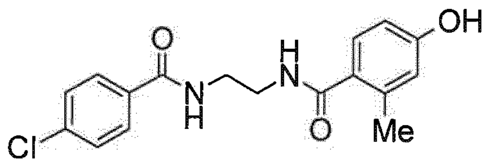 |
| 74 | 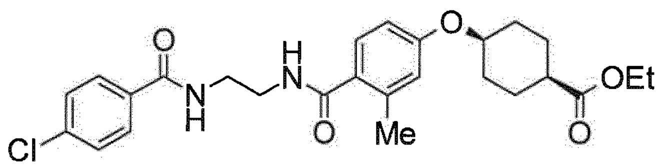 |
| 75 | 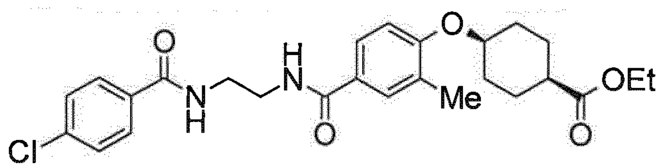 |
| 76 | 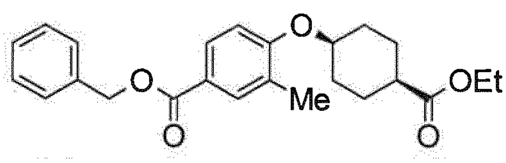 |

[Table 12]

| Pr | Structure |
|---|---|
| 77 | 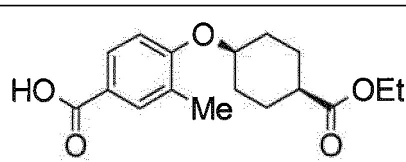 |
| 78 | 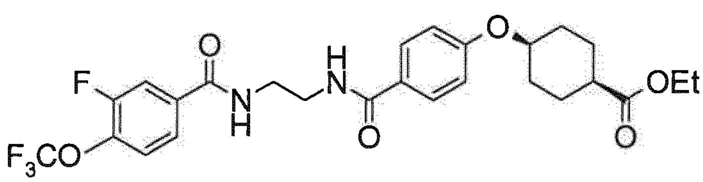 |

(continued)

| Pr | Structure |
|---|---|
| 79 | 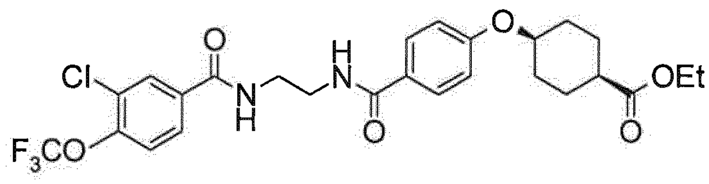 |
| 80 | 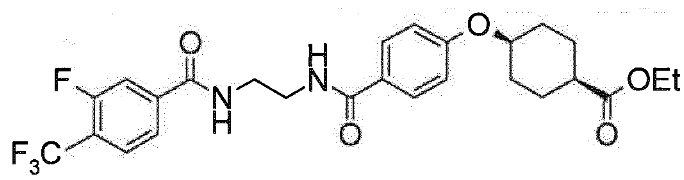 |
| 81 | 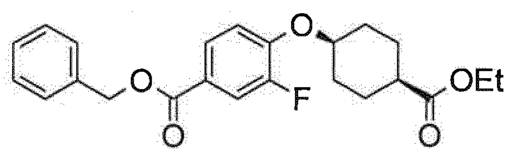 |
| 82 | 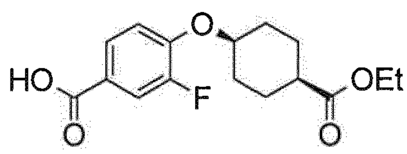 |
| 83 | 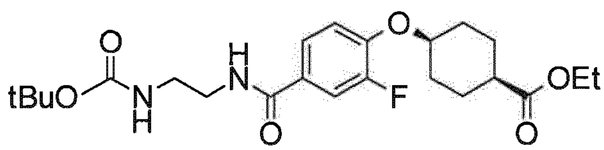 |
| 84/Cl | 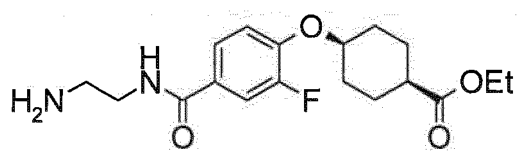 |

[Table 13]

| Pr | Structure |
|---|---|
| 85 | 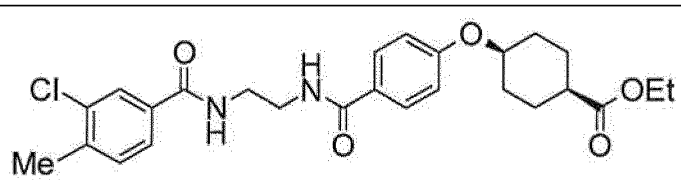 |
| 86 | 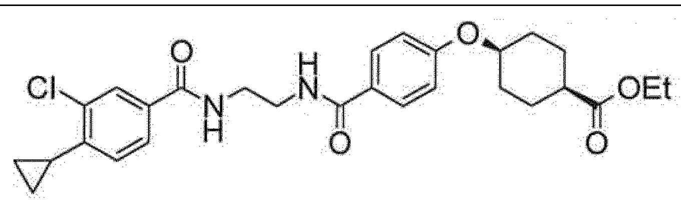 |

(continued)

| Pr | Structure |
|---|---|
| 87 | 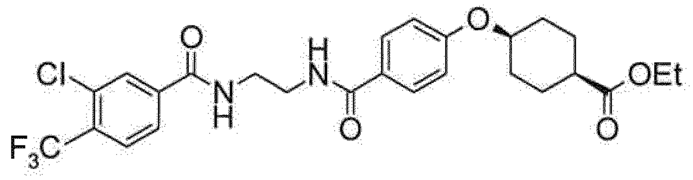 |
| 88 | 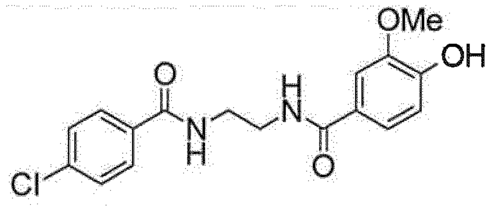 |
| 89 | 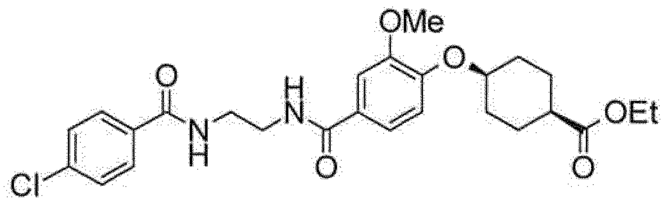 |
| 90 | 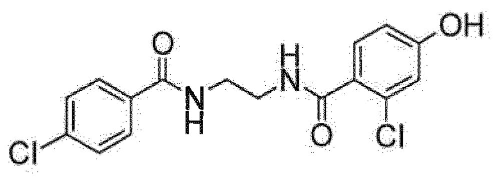 |
| 91 | 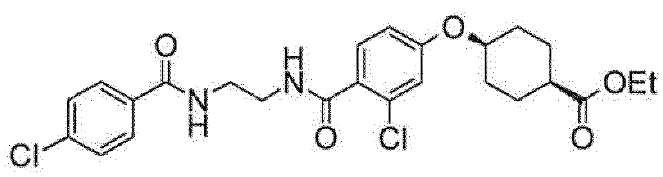 |

[Table 14]

| Pr | Structure |
|---|---|
| 92 | 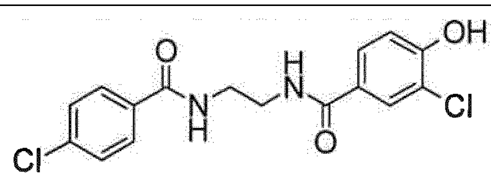 |
| 93 | 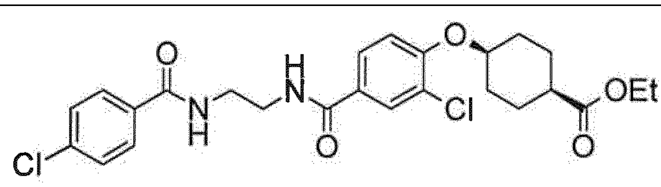 |

(continued)

| Pr | Structure |
|---|---|
| 94 | 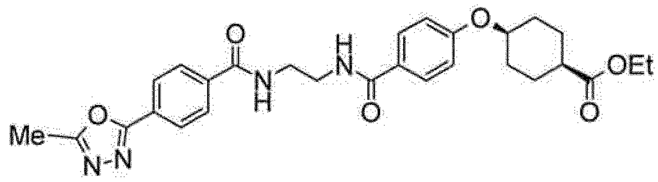 |
| 95 | 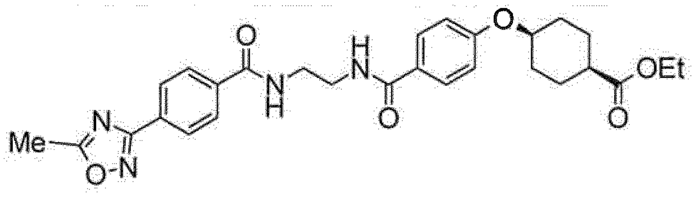 |
| 96 | 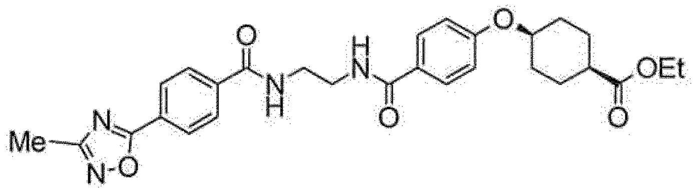 |
| 97 | 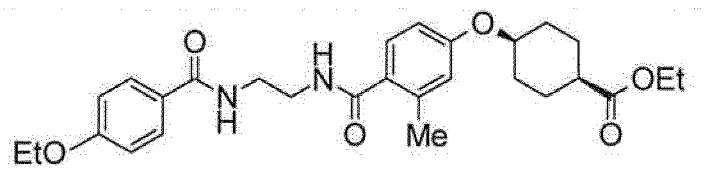 |

[Table 15]

| Pr | Structure |
|---|---|
| 98 | 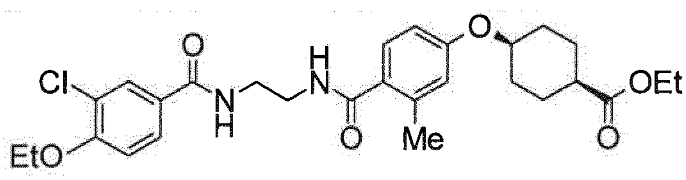 |
| 99 | 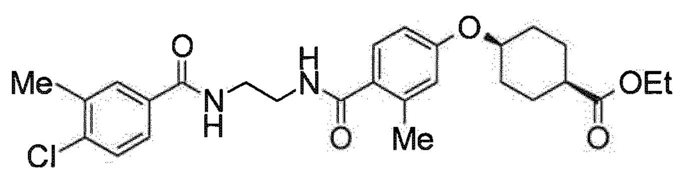 |
| 100 | 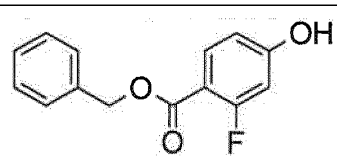 |
| 101 | 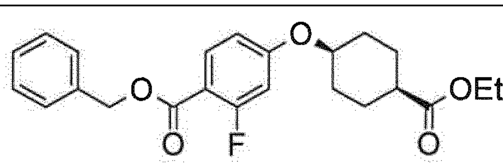 |

(continued)

| Pr | Structure |
|---|---|
| 102 | 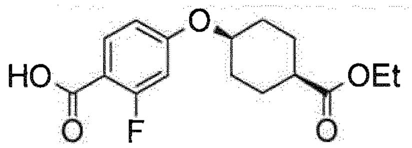 |
| 103 | 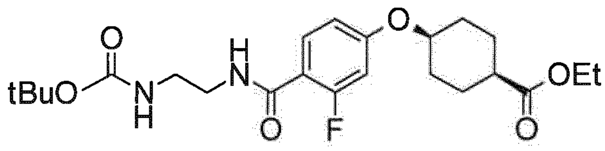 |
| 104/Cl | 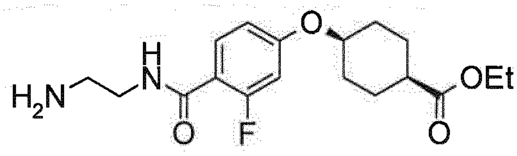 |
| 105 | 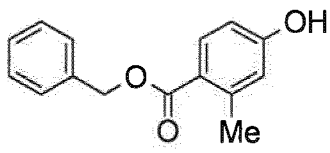 |
| 106 | 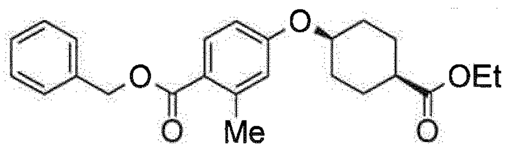 |

[Table 16]

| Pr | Structure |
|---|---|
| 107 | 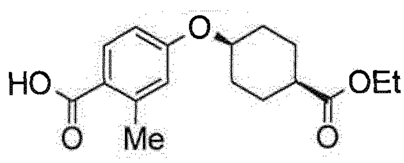 |
| 108 | 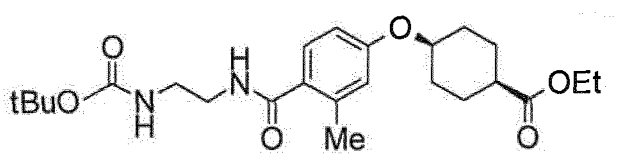 |
| 109/Cl | 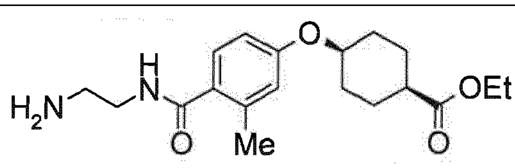 |
| 110 | 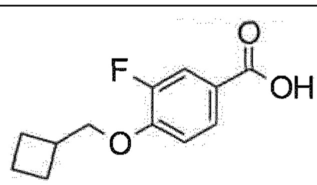 |

(continued)

| Pr | Structure |
|---|---|
| 111 | 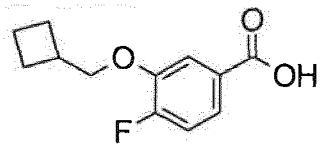 |
| 112 | 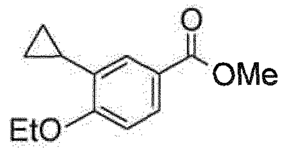 |
| 113 | 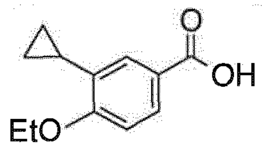 |
| 114 | 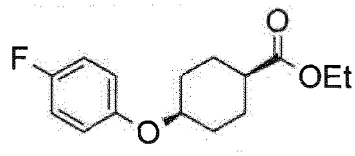 |
| 115 | 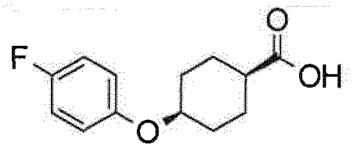 |

[Table 17]

| Pr | Structure |
|---|---|
| 116 | 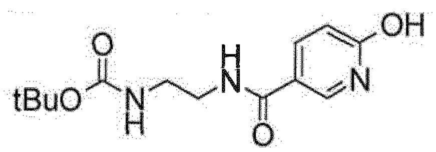 |
| 117 | 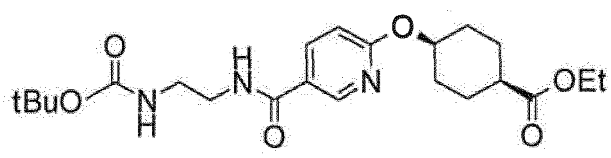 |
| 118 | 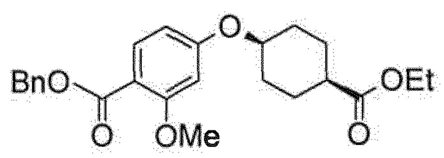 |
| 119 | 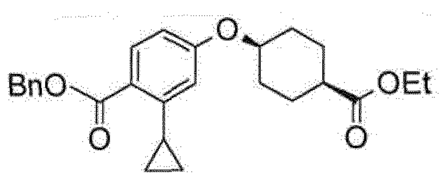 |

(continued)

| Pr | Structure |
|---|---|
| 120 | 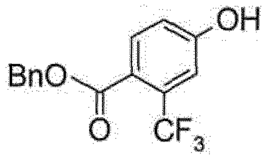 |
| 121/Cl | 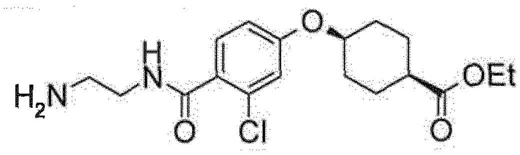 |
| 122 | 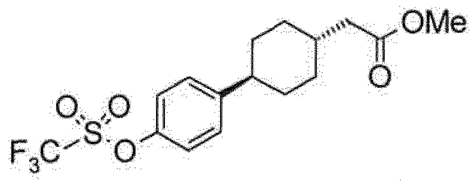 |
| 123 | 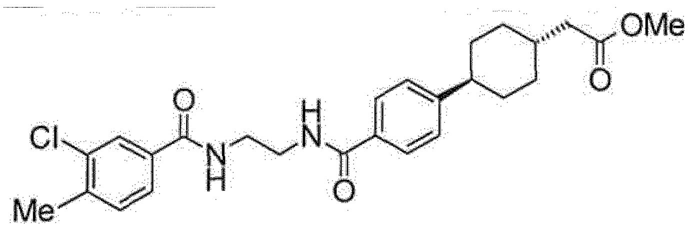 |

[Table 18]

| Pr | Structure |
|---|---|
| 124 | 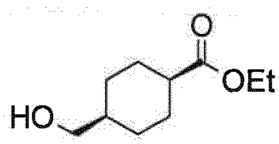 |
| 125 | 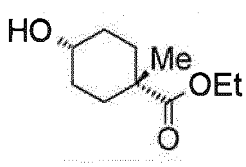 |
| 126 | 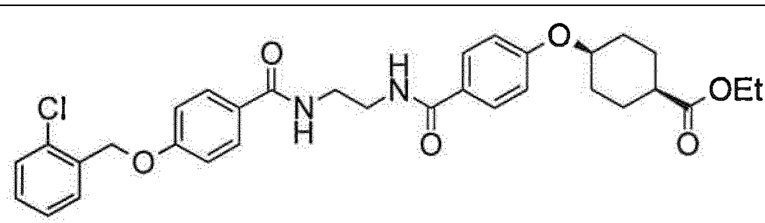 |

(continued)

| Pr | Structure |
|---|---|
| 127 | 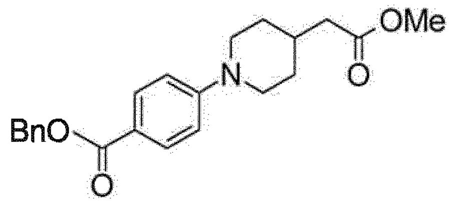 |
| 128 | 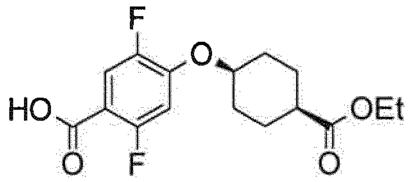 |
| 129 | 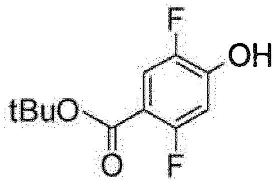 |
| 130/Cl | 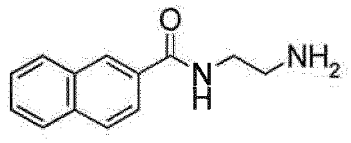 |
| 131/Cl | 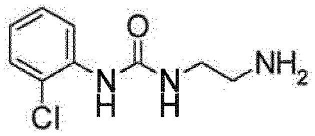 |

[Table 19]

| Pr | Structure |
|---|---|
| 132 | 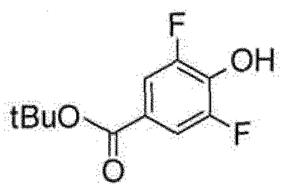 |
| 133 | 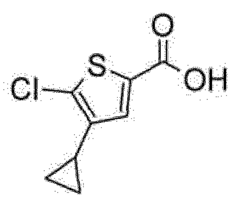 |
| 134 | 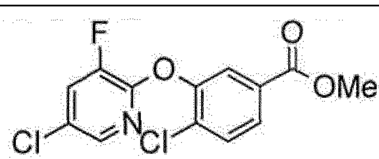 |

(continued)

| Pr | Structure |
|---|---|
| 135 | 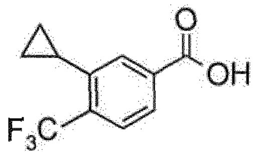 |
| 136 | 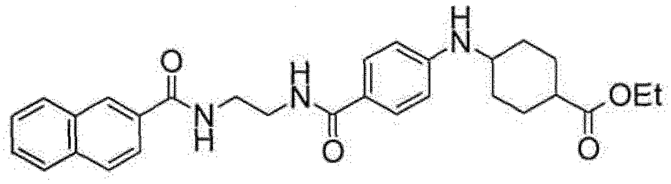 |
| 137 | 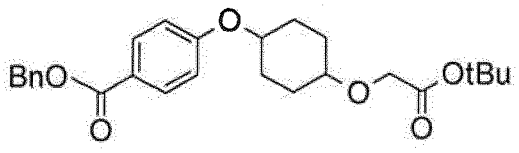 |
| 138 | 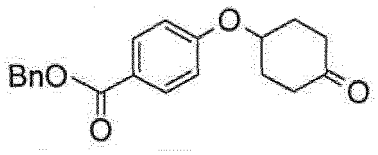 |
| 139 | 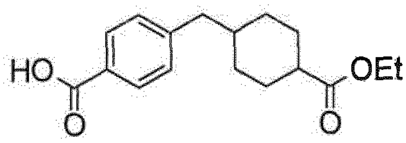 |

[Table 20]

| Pr | Structure |
|---|---|
| 140 | 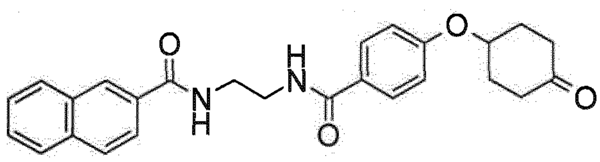 |
| 141/TF | 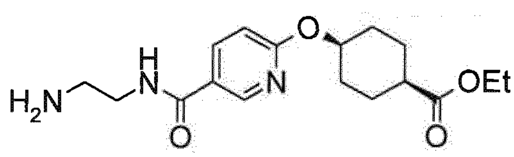 |
| 142 | 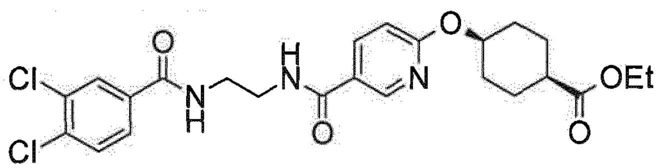 |

(continued)

| Pr | Structure |
|---|---|
| 143 | 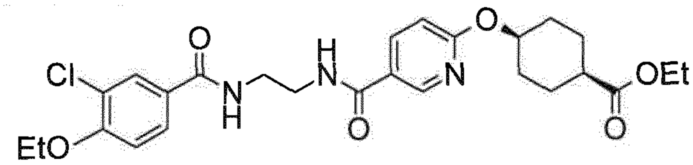 |
| 144 | 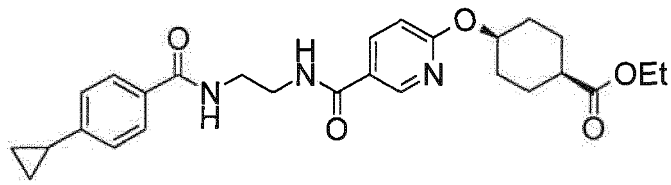 |
| 145 | 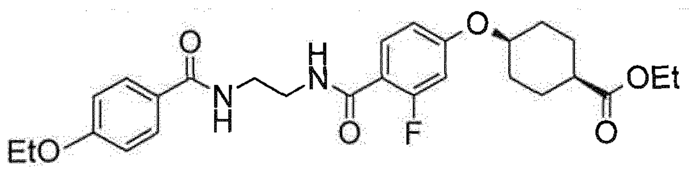 |
| 146 | 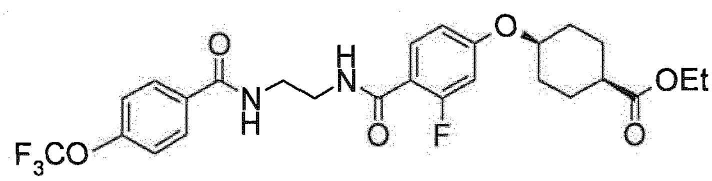 |
| 147 | 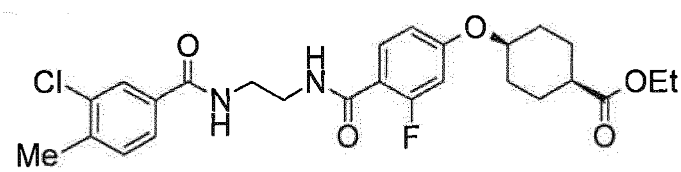 |

[Table 21]

| Pr | Structure |
|---|---|
| 148 | 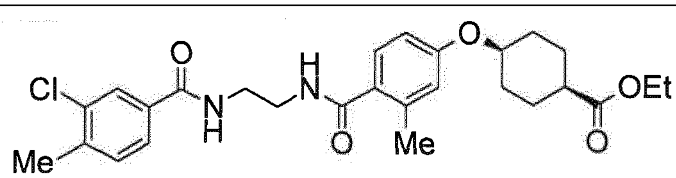 |
| 149 | 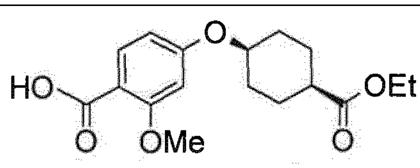 |
| 150 | 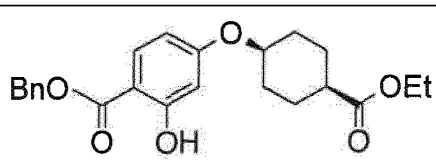 |

(continued)

| Pr | Structure |
|---|---|
| 151 | 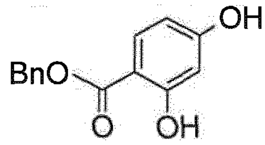 |
| 152 | 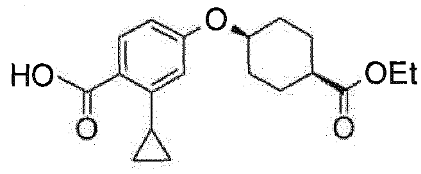 |
| 153 | 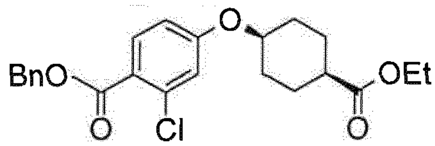 |
| 154 | 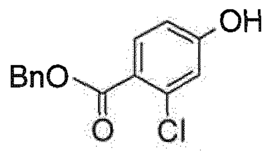 |
| 155 | 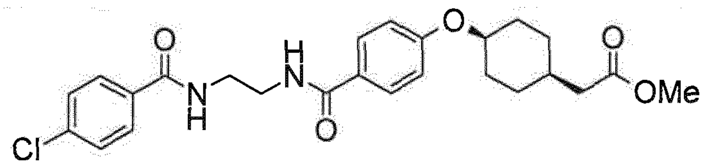 |
| 156 | 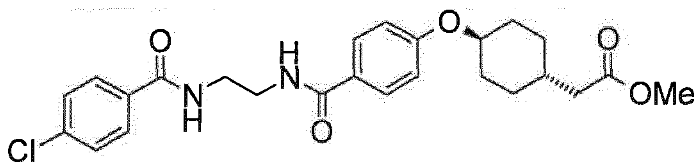 |

[Table 22]

| Pr | Structure |
|---|---|
| 157 | 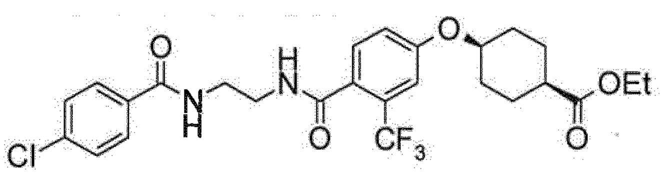 |
| 158 | 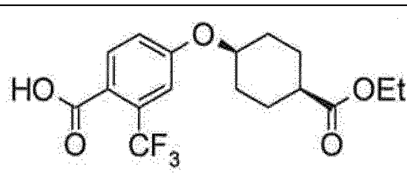 |

(continued)

| Pr | Structure |
|---|---|
| 159 | 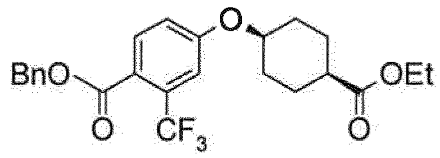 |
| 160 | 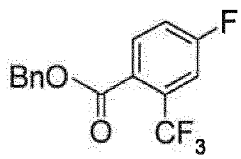 |
| 161 | 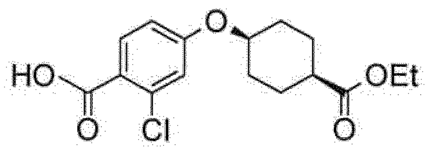 |
| 162 | 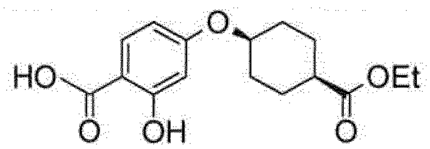 |
| 163 | 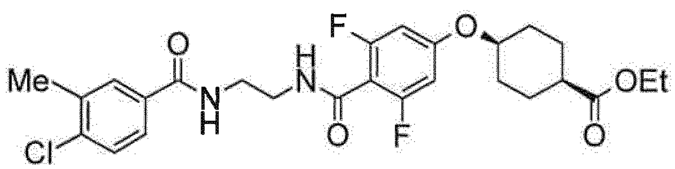 |
| 164/Cl | 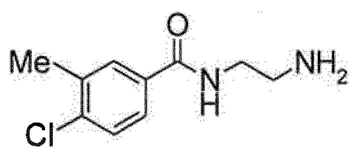 |
| 165 | 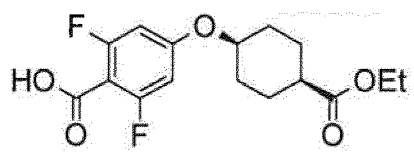 |

[Table 23]

| Pr | Stricture |
|---|---|
| 166 | 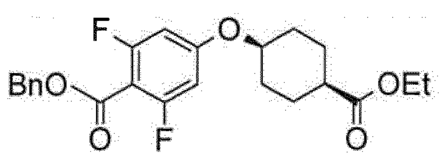 |
| 167 | 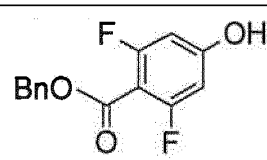 |

(continued)

| Pr | Stricture |
|---|---|
| 168 | 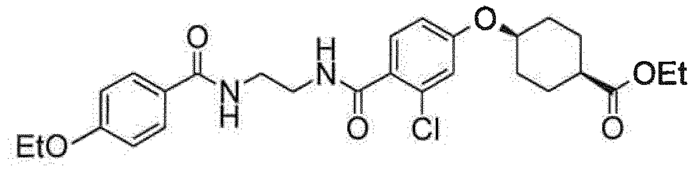 |
| 169 | 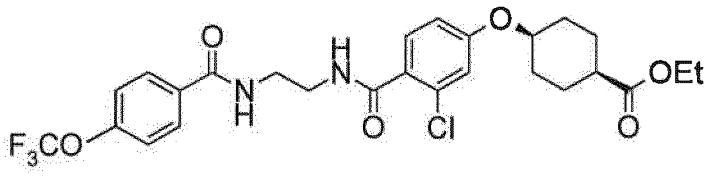 |
| 170 | 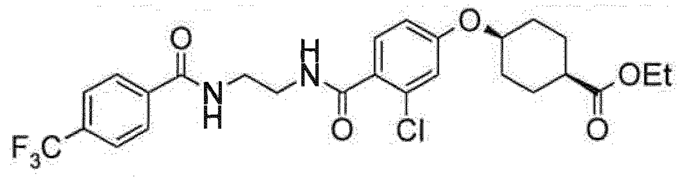 |
| 171 | 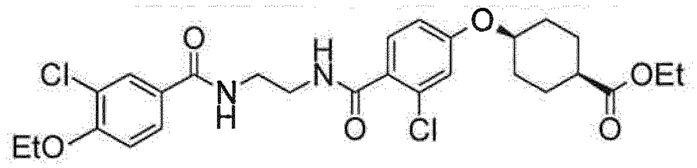 |
| 172 | 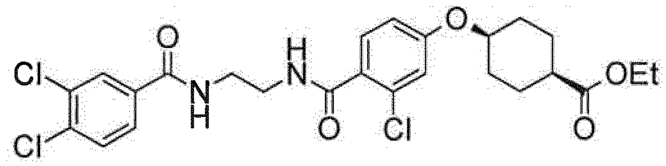 |
| 173 | 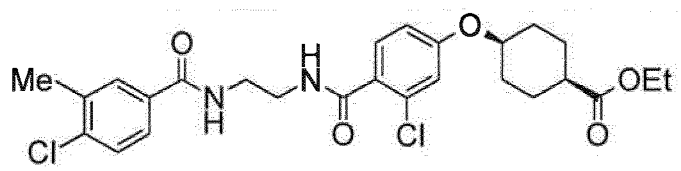 |

[Table 24]

| Pr | Structure |
|---|---|
| 174 | 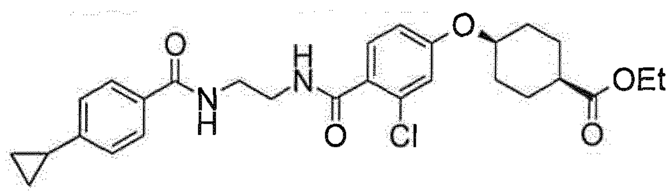 |
| 175 | 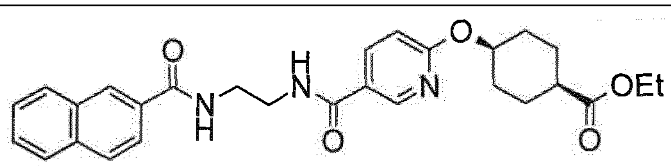 |

(continued)

| Pr | Structure |
|---|---|
| 176 | 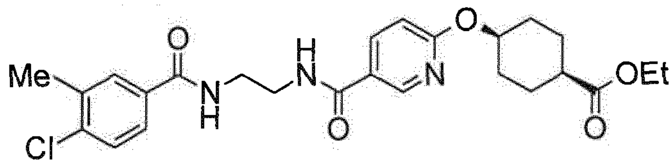 |
| 177 | 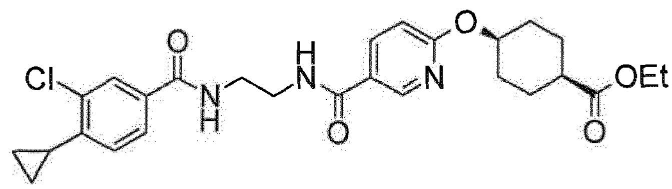 |
| 178/Cl | 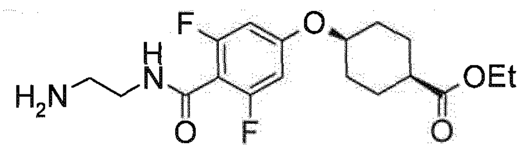 |
| 179 | 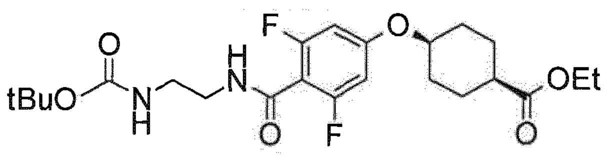 |
| 180/Cl | 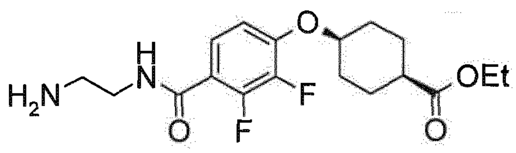 |
| 181 | 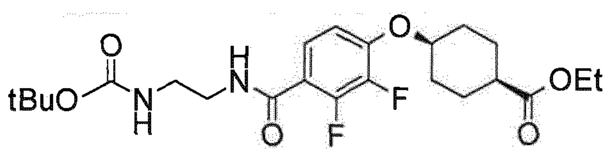 |

[Table 25]

| Pr | Structure |
|---|---|
| 182 | 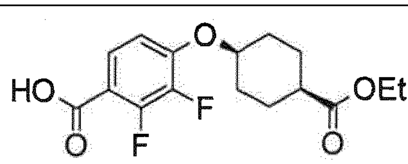 |
| 183 | 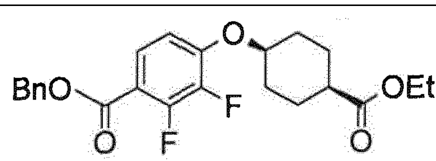 |
| 184 | 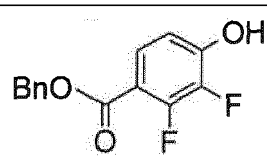 |

(continued)

| Pr | Structure |
|---|---|
| 185/Cl | 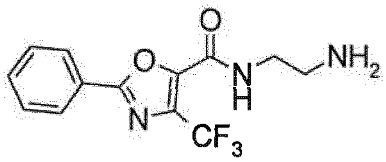 |
| 186/Cl | 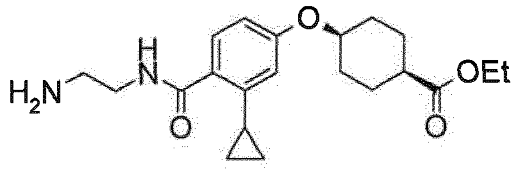 |
| 187/Cl | 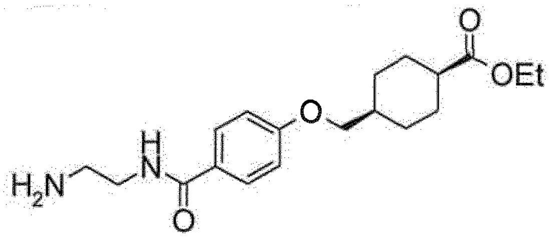 |
| 188 | 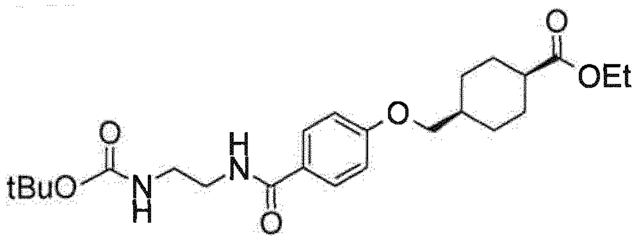 |

[Table 26]

| Pr | Structure |
|---|---|
| 189 | 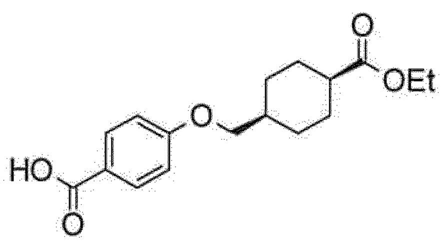 |
| 190 | 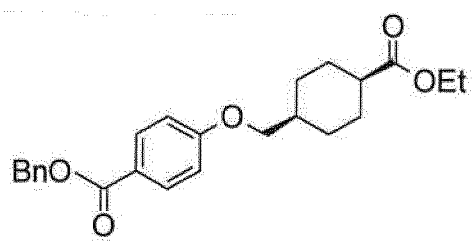 |

(continued)

| Pr | Structure |
|---|---|
| 191 | 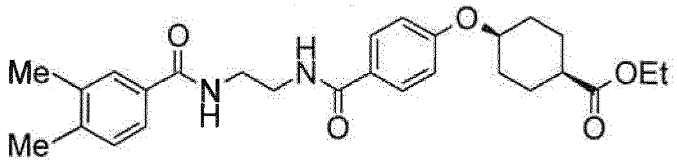 |
| 192 | 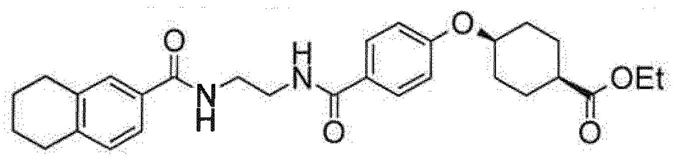 |
| 193 | 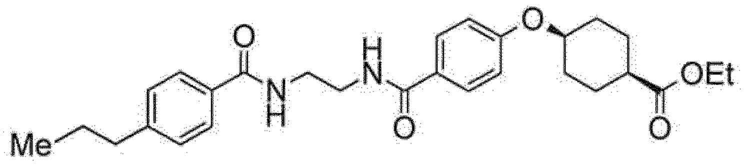 |
| 194 | 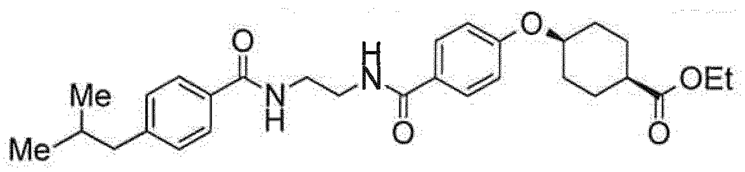 |
| 195 | 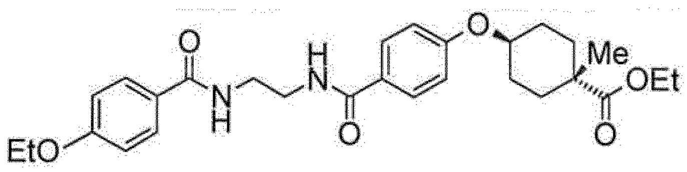 |

[Table 27]

| Pr | Structure |
|---|---|
| 196/Cl | 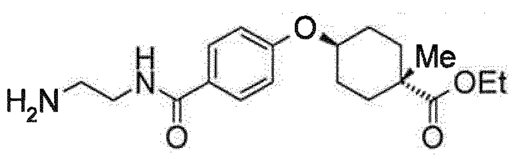 |
| 197 | 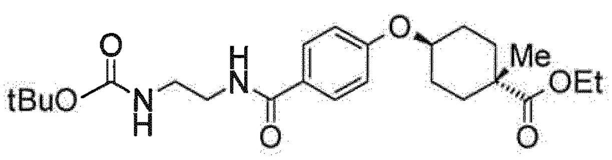 |
| 198 | 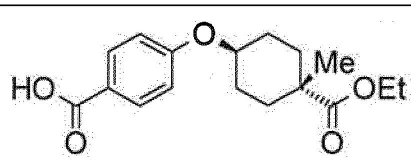 |

(continued)

| Pr | Structure |
|---|---|
| 199 | 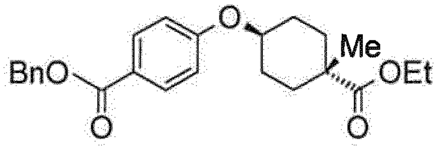 |
| 200 | 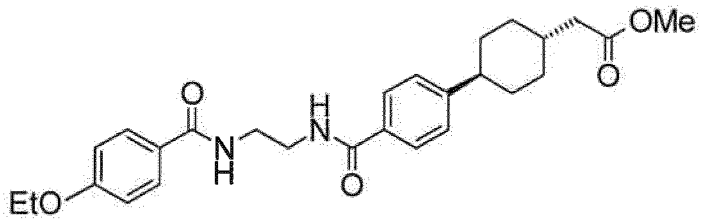 |
| 201/Cl | 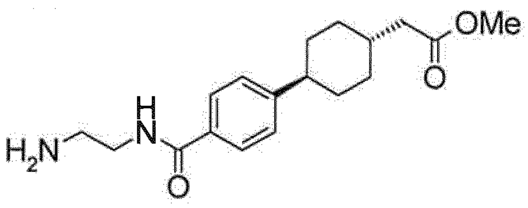 |
| 202 | 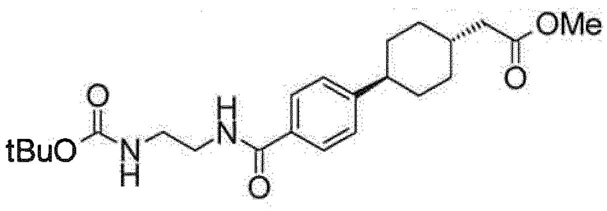 |

[Table 28]

| Pr | Structure |
|---|---|
| 203 | 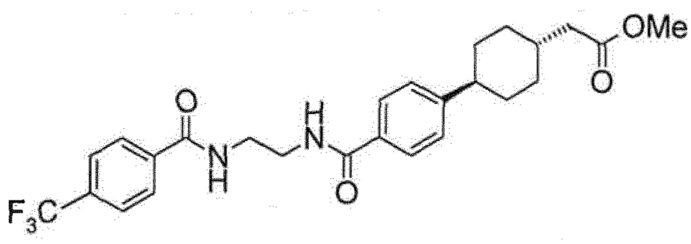 |
| 204 | 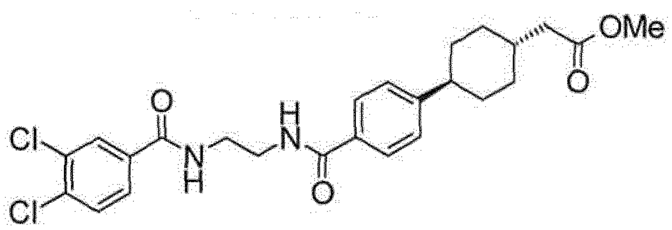 |

(continued)

| Pr | Structure |
|---|---|
| 205 | 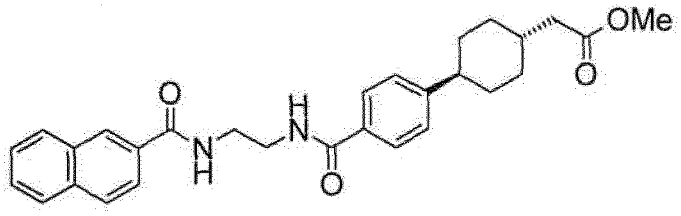 |
| 206 | 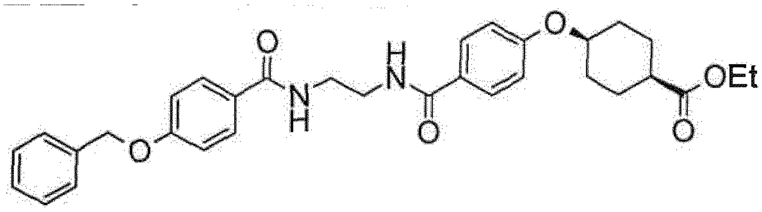 |
| 207 | 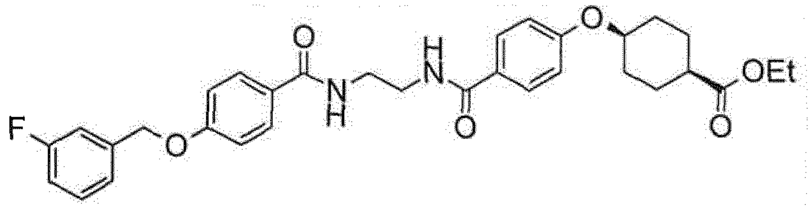 |
| 208 | 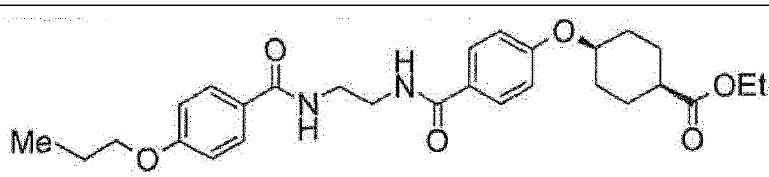 |

[Table 29]

| Pr | Structure |
|---|---|
| 209 | 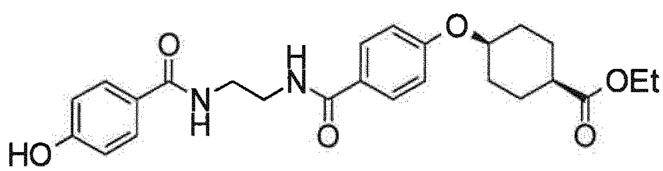 |
| 210 | 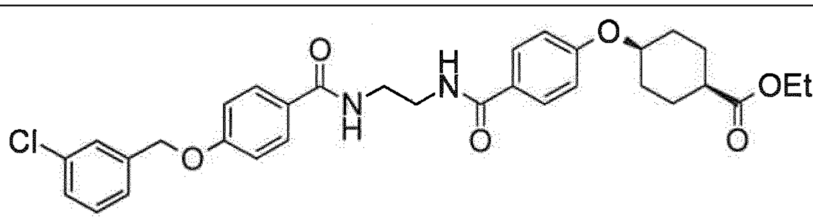 |

(continued)

| Pr | Structure |
|---|---|
| 211 | 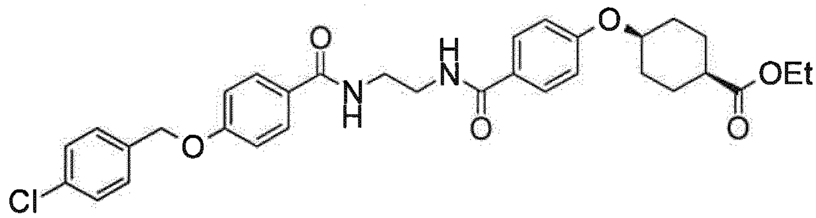 |
| 212* | 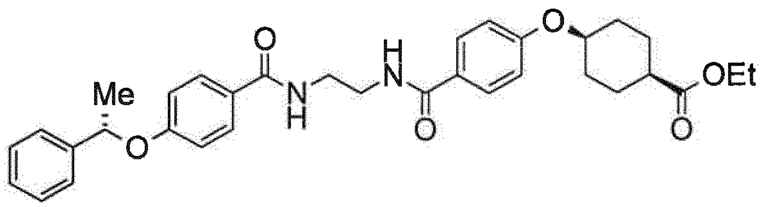 |
| 213* | 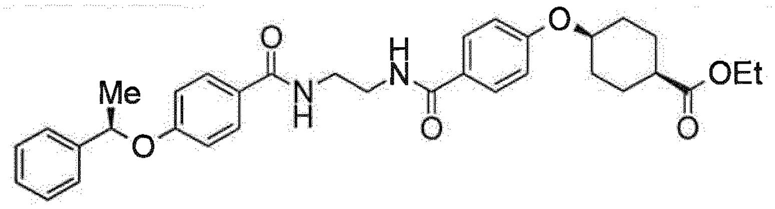 |
| 214 | 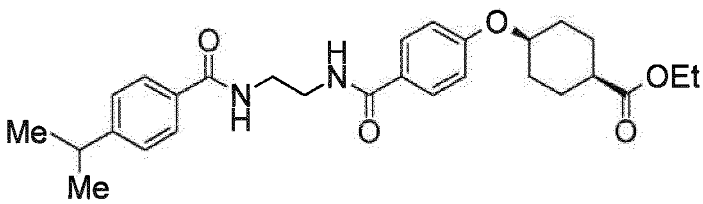 |

[Table 30]

| Pr | Structure |
|---|---|
| 215 | 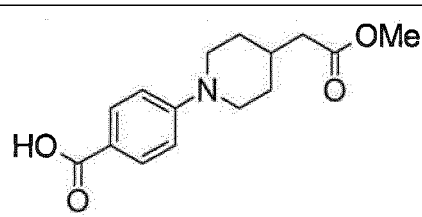 |
| 216/Cl | 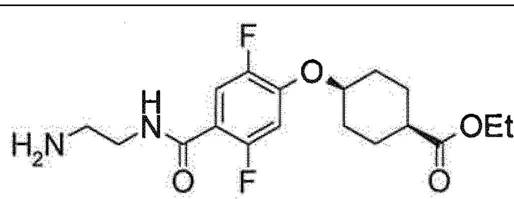 |

(continued)

| Pr | Structure |
|---|---|
| 217 | 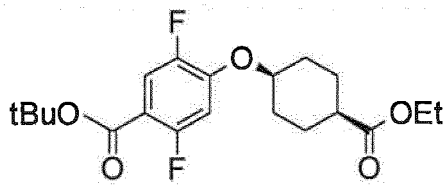 |
| 218 | 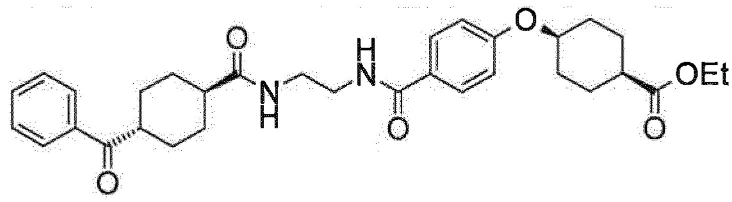 |
| 219/Cl | 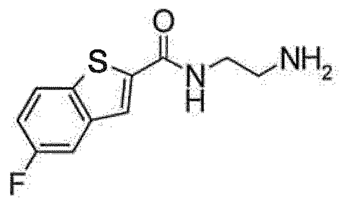 |
| 220 | 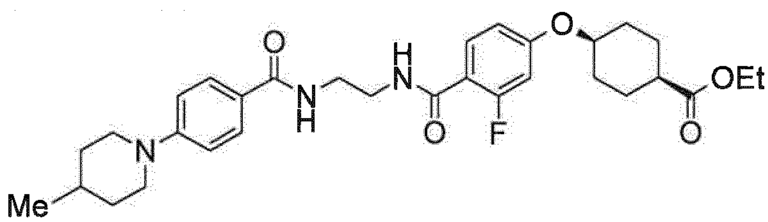 |

[Table 31]

| Pr | Structure |
|---|---|
| 221 | 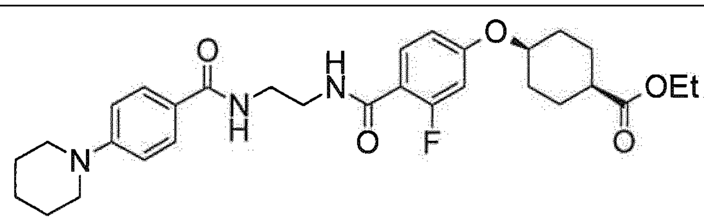 |
| 222 | 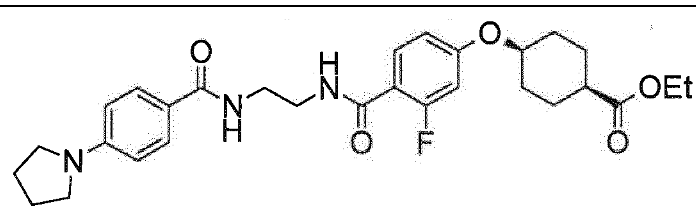 |
| 223 | 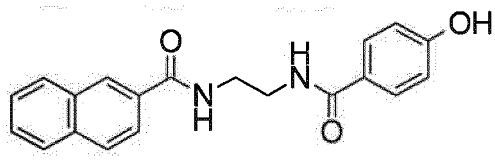 |

(continued)

| Pr | Structure |
|---|---|
| 224 | 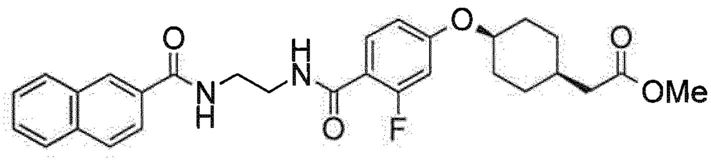 |
| 225/Cl | 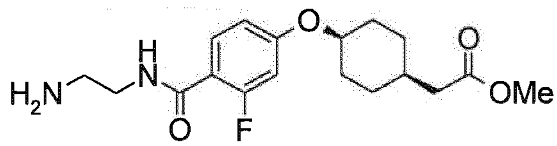 |
| 226 | 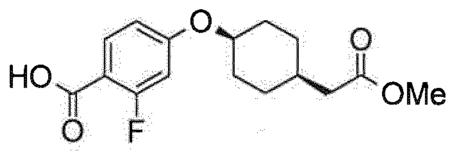 |
| 227 | 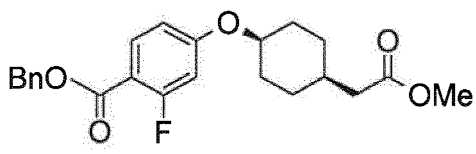 |
| 228/Cl | 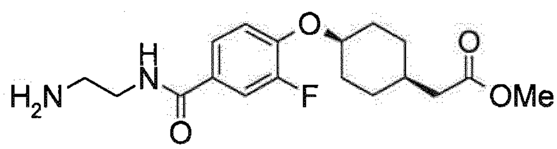 |

[Table 32]

| Pr | Structure |
|---|---|
| 229 | 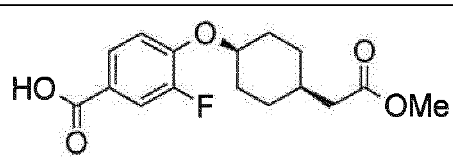 |
| 230 | 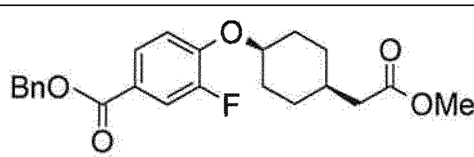 |
| 231/Cl | 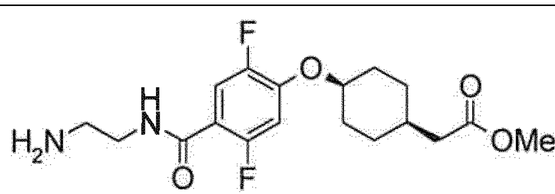 |

(continued)

| Pr | Structure |
|---|---|
| 232 | 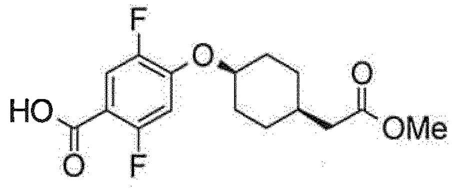 |
| 233 | 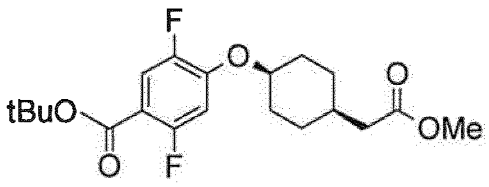 |
| 234 | 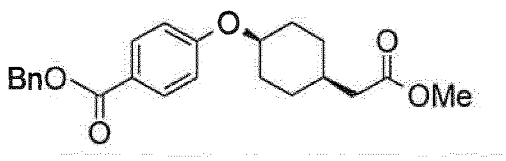 |
| 235 | 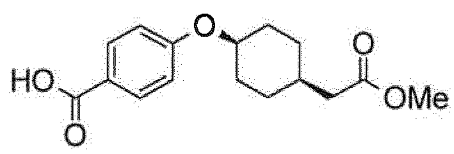 |
| 236/Cl | 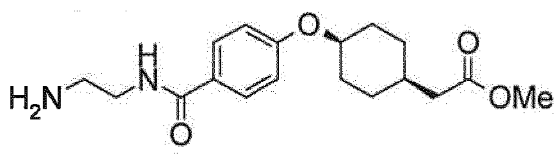 |

[Table 33]

| Pr | Structure |
|---|---|
| 237 | 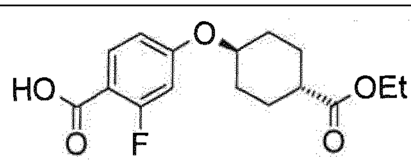 |
| 238 | 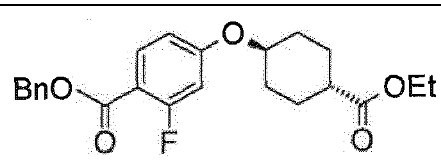 |
| 239 | 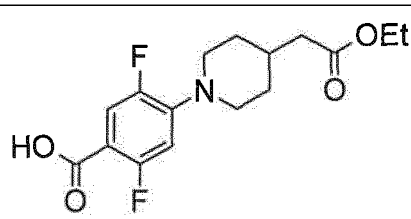 |

(continued)

| Pr | Structure |
|---|---|
| 240 | 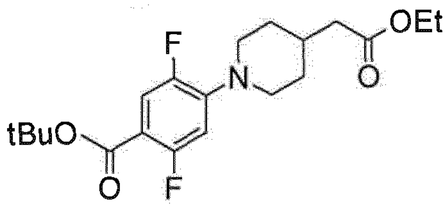 |
| 241 | 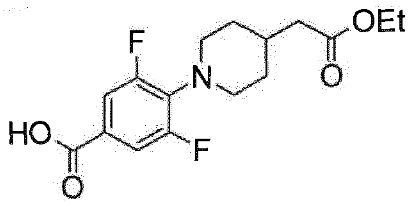 |
| 242 | 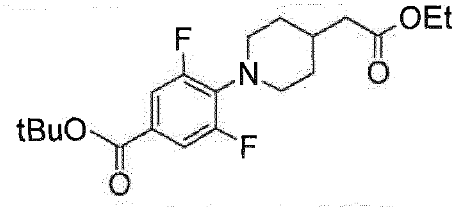 |
| 243 | 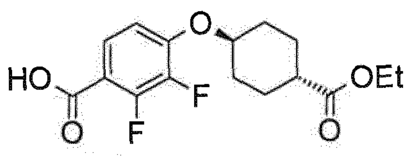 |
| 244 | 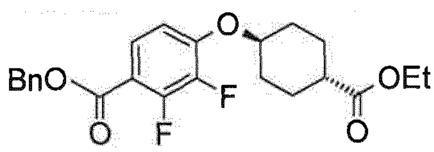 |

[Table 34]

| Pr | Structure |
|---|---|
| 245 | 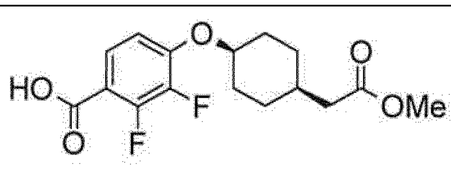 |
| 246 | 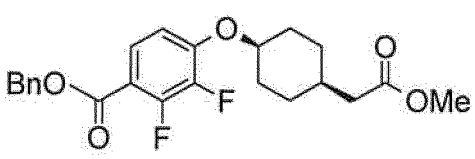 |
| 247/Cl | 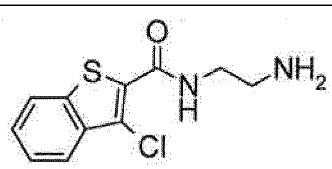 |

(continued)

| Pr | Structure |
|---|---|
| 248 | 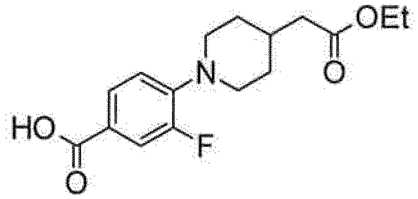 |
| 249 | 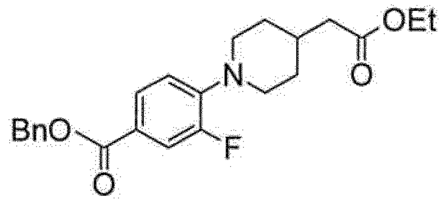 |
| 250/Cl | 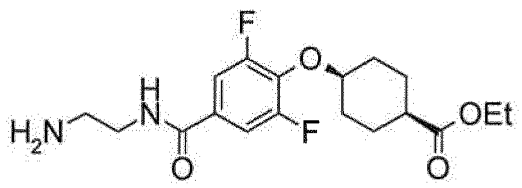 |
| 251 | 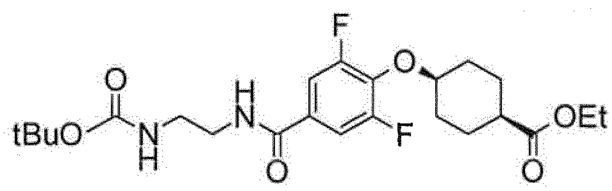 |

[Table 35]

| Pr | Structure |
|---|---|
| 252 | 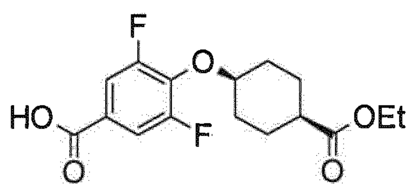 |
| 253 | 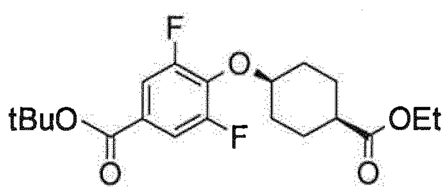 |
| 254 | 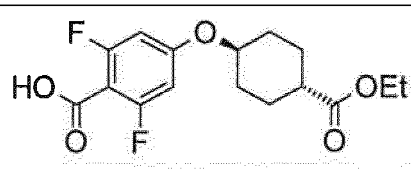 |

(continued)

| Pr | Structure |
|---|---|
| 255 | 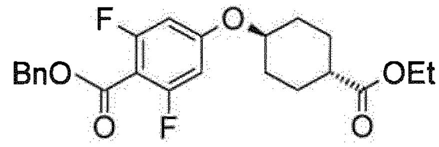 |
| 256 | 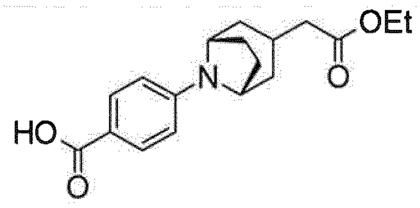 |
| 257 | 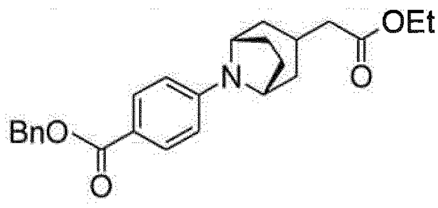 |
| 258 | 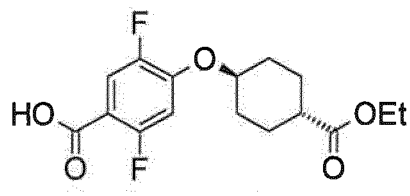 |

[Table 36]

| Pr | Structure |
|---|---|
| 259 | 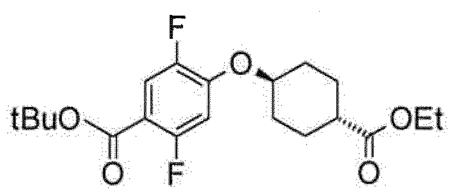 |
| 260 | 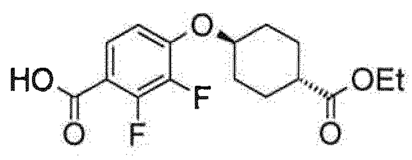 |
| 261 | 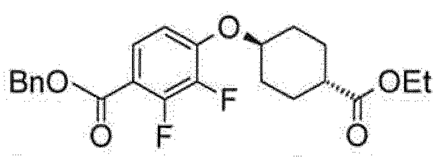 |
| 262/Cl | 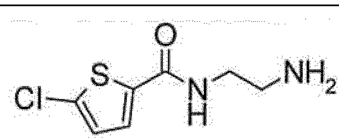 |

(continued)

| Pr | Structure |
|---|---|
| 263 | 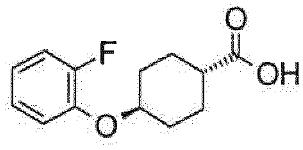 |
| 264 | 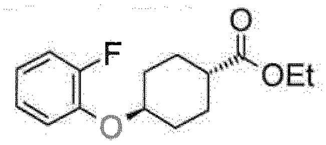 |
| 265 | 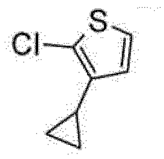 |
| 266 | 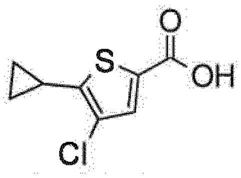 |
| 267 | 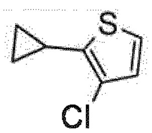 |

[Table 37]

| Pr | Structure |
|---|---|
| 268 | 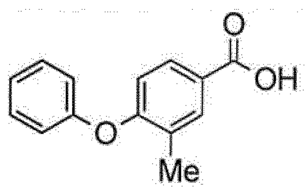 |
| 269 | 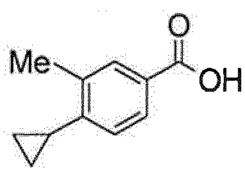 |
| 270 | 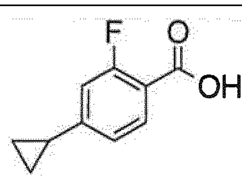 |

(continued)

| Pr | Structure |
|---|---|
| 271 | 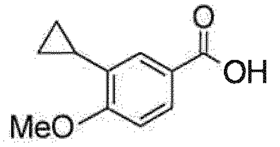 |
| 272 | 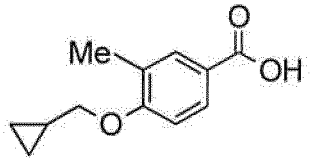 |
| 273 | 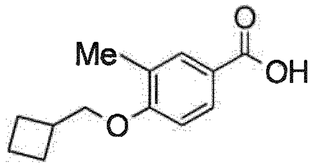 |
| 274 | 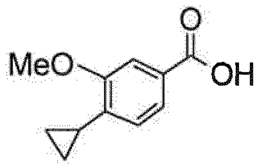 |
| 275 | 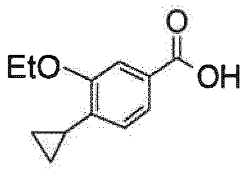 |

[Table 38]

| Pr | Structure |
|---|---|
| 276 | 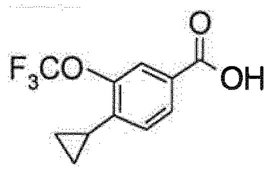 |
| 277 | 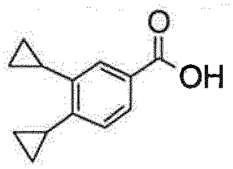 |
| 278 | 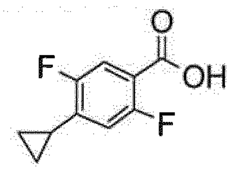 |

(continued)

| Pr | Structure |
|---|---|
| 279 | 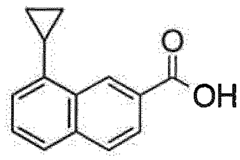 |
| 280 | 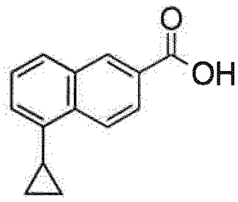 |
| 281 | 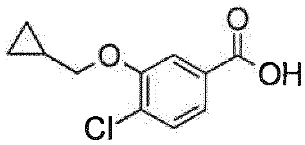 |
| 282 | 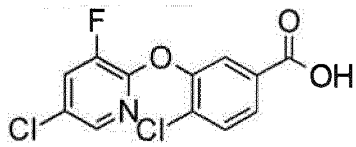 |
| 283 | 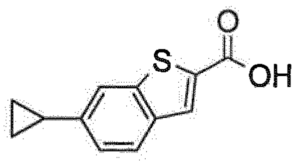 |

[Table 39]

| Pr | Structure |
|---|---|
| 284 | 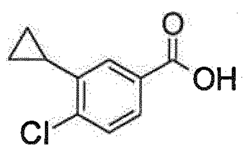 |
| 285 | 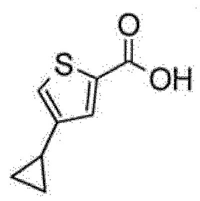 |
| 286 | 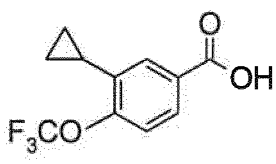 |

(continued)

| Pr | Structure |
|---|---|
| 287 | 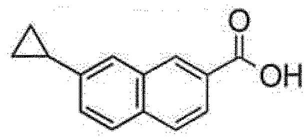 |
| 288* | 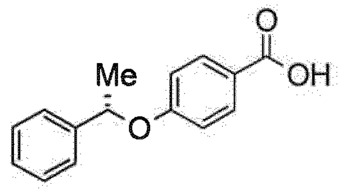 |
| 289* | 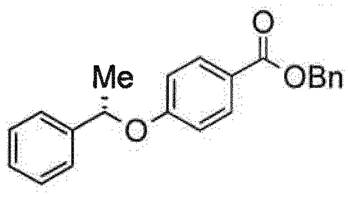 |
| 290* | 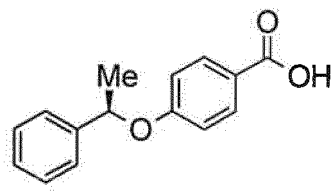 |
| 291* | 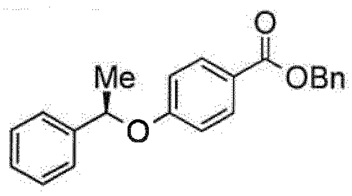 |

[Table 40]

| Pr | Structure |
|---|---|
| 292 | 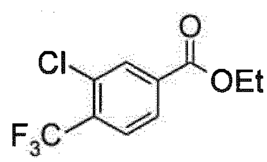 |
| 293 | 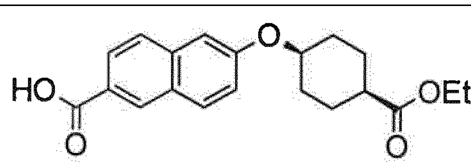 |
| 294 | 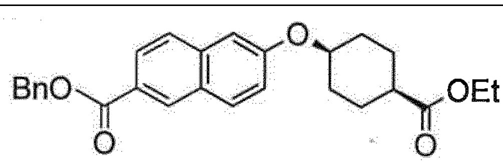 |

(continued)

| Pr | Structure |
|---|---|
| 295 | 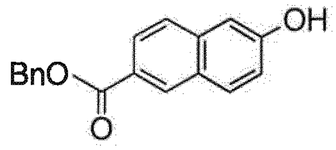 |
| 296/Cl | 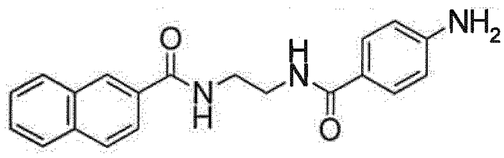 |
| 297 | 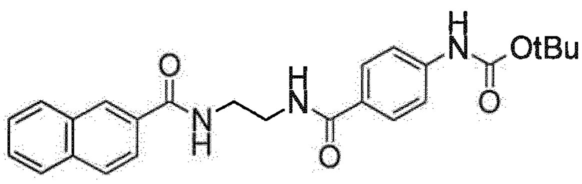 |
| 298 | 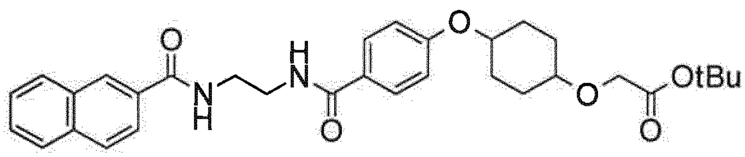 |
| 299 | 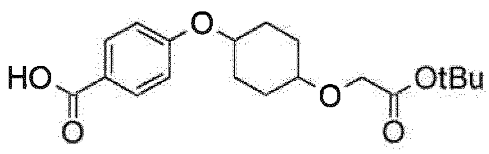 |
| 300 | 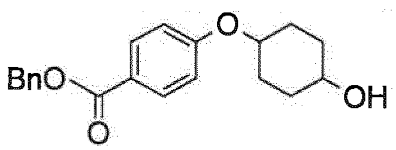 |

[Table 41]

| Pr | Structure |
|---|---|
| 301 | 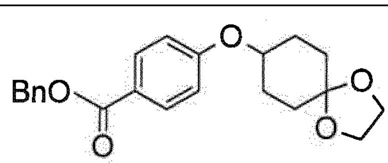 |
| 302 | 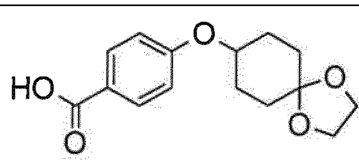 |
| 303 | 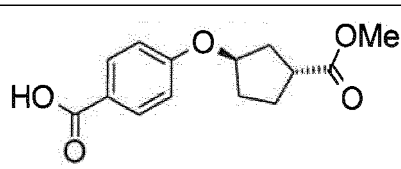 |

(continued)

| Pr | Structure |
|---|---|
| 304 | 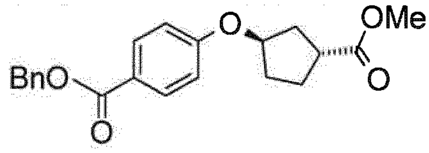 |
| 305 | 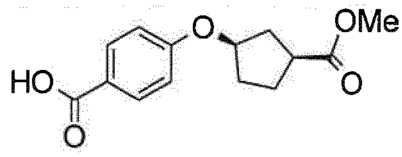 |
| 306 | 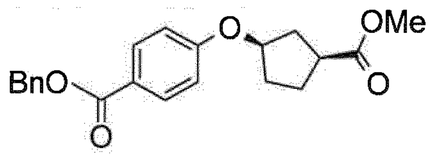 |
| 307 | 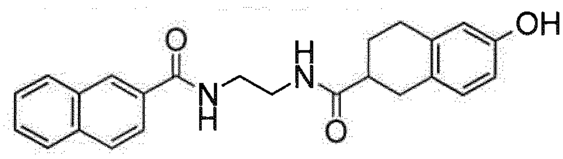 |
| 308 | 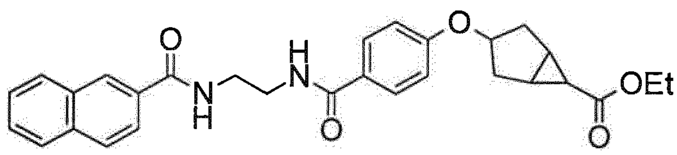 |
| 309 | 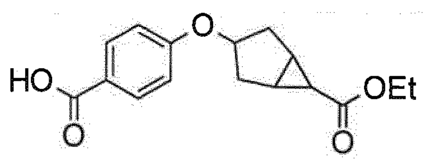 |

[Table 42]

| Pr | Structure |
|---|---|
| 310 | 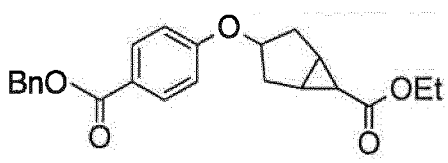 |
| 311 | 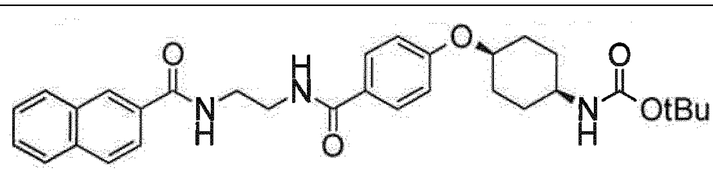 |
| 312 | 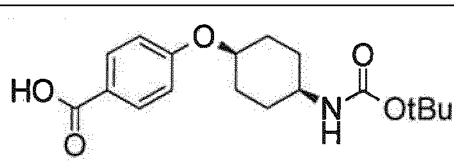 |

(continued)

| Pr | Structure |
|---|---|
| 313 | 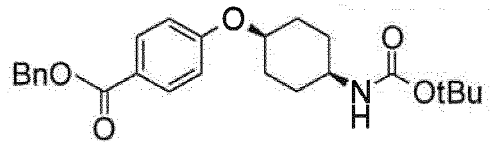 |
| 314 | 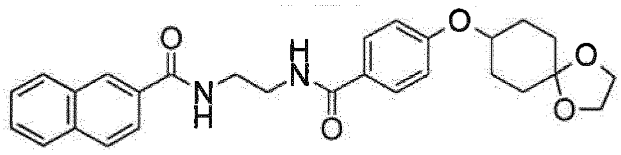 |

[Table 43]

| Ex | Structure |
|---|---|
| 1 | 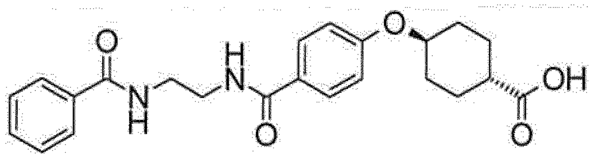 |
| 2 | 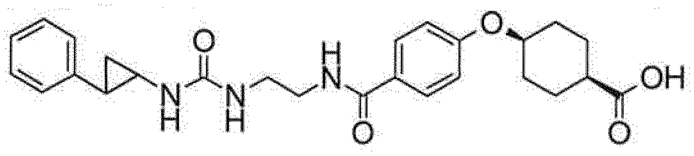 |
| 3 | 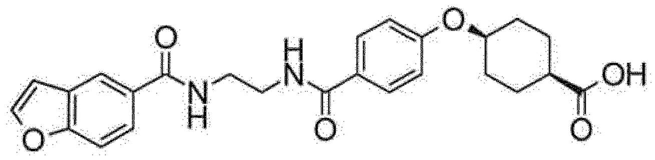 |
| 4 | 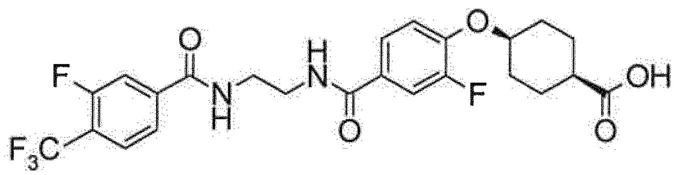 |
| 5 | 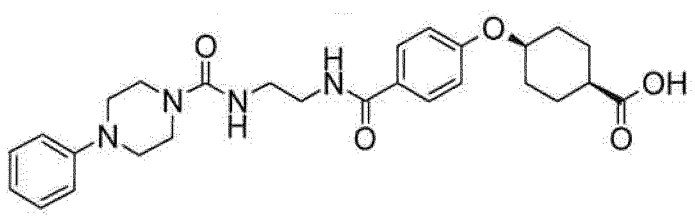 |
| 6 | 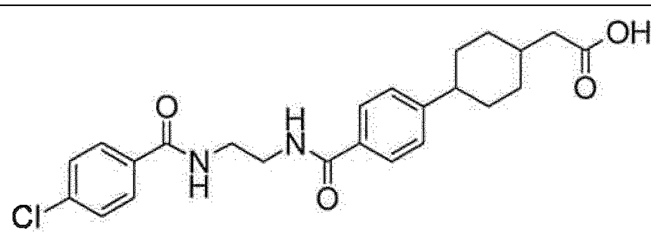 |

(continued)

| Ex | Structure |
|---|---|
| 7 | 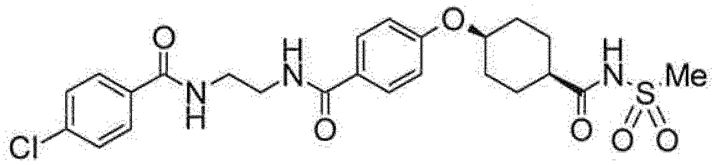 |

[Table 44]

| Ex | Structure |
|---|---|
| 8 | 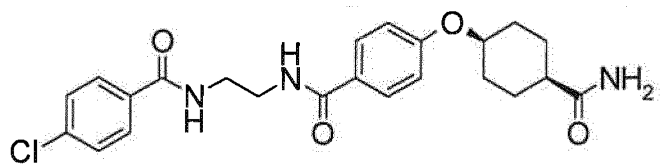 |
| 9 | 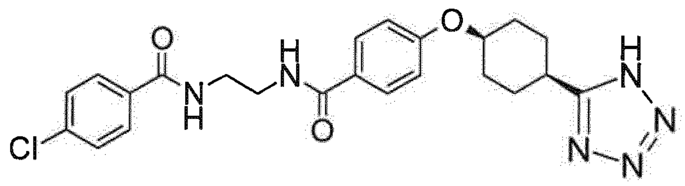 |
| 10 | 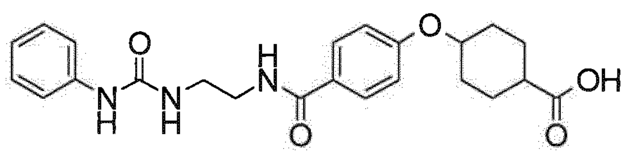 |
| 11 | 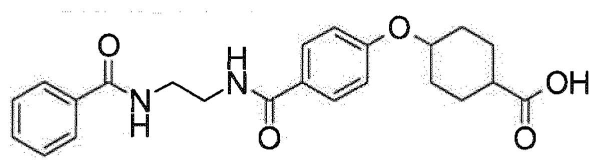 |
| 12 | 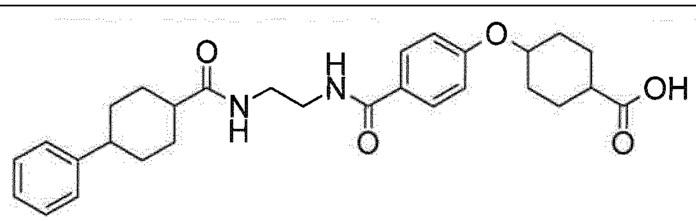 |
| 13 | 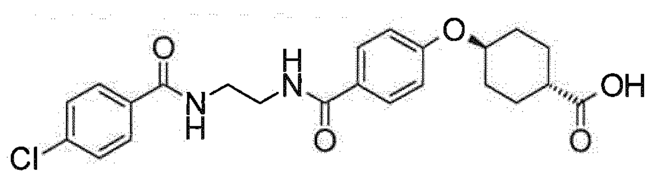 |
| 14 | 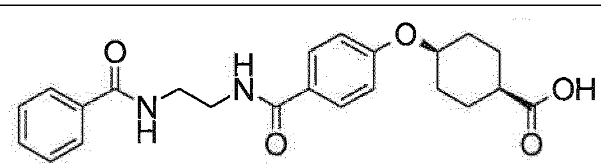 |

(continued)

| Ex | Structure |
|---|---|
| 15 | 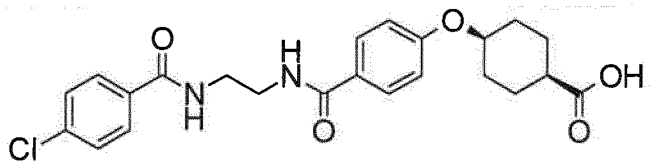 |

[Table 45]

| Ex | Structure |
|---|---|
| 16 | 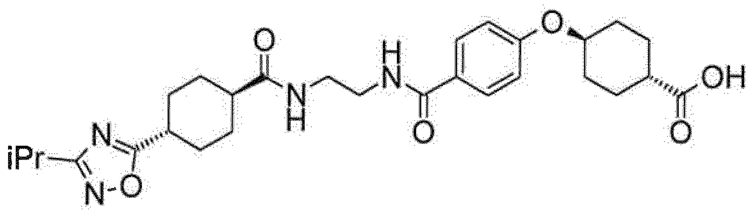 |
| 17 | 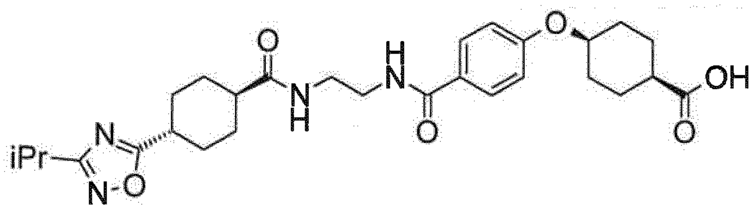 |
| 18 | 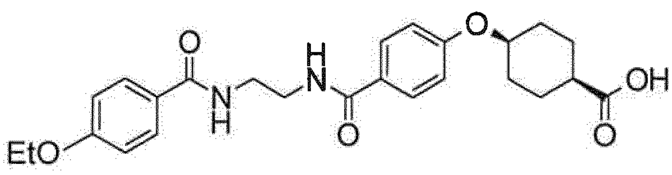 |
| 19 | 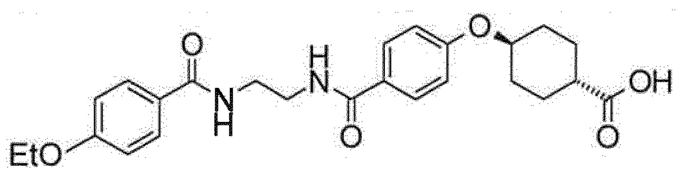 |
| 20 | 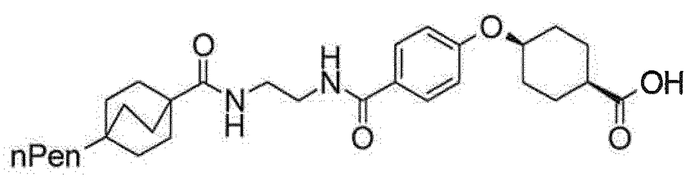 |
| 21 | 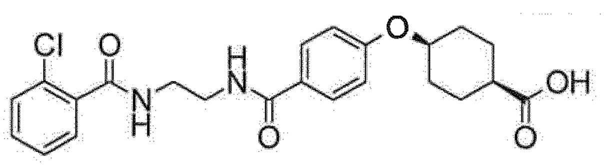 |

(continued)

| Ex | Structure |
|---|---|
| 22 | 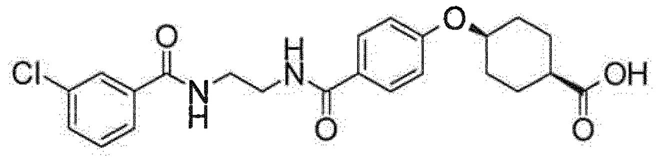 |

[Table 46]

| Ex | Structure |
|---|---|
| 23 | 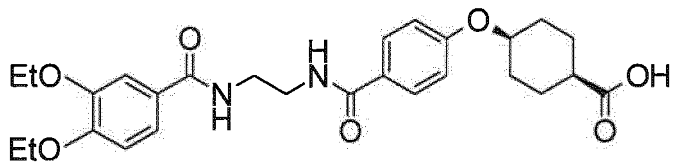 |
| 24 | 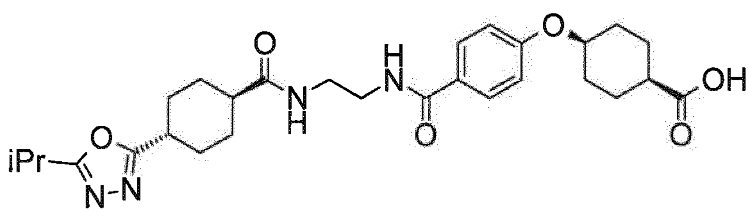 |
| 25 | 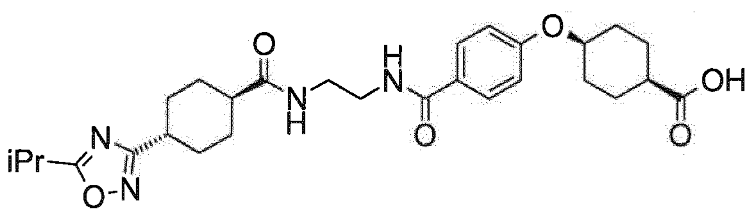 |
| 26 | 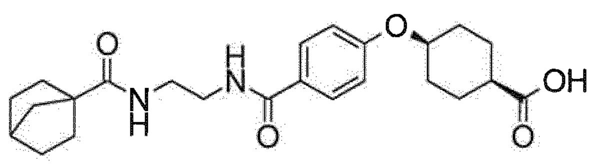 |
| 27 | 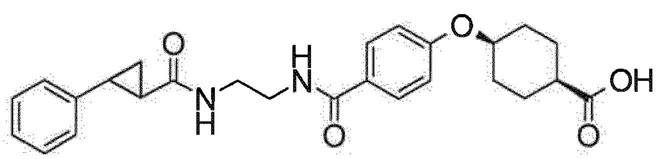 |
| 28 | 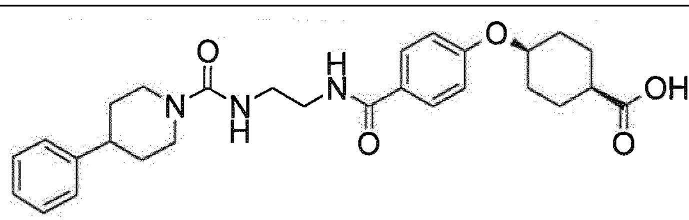 |

(continued)

| Ex | Structure |
|---|---|
| 29 | 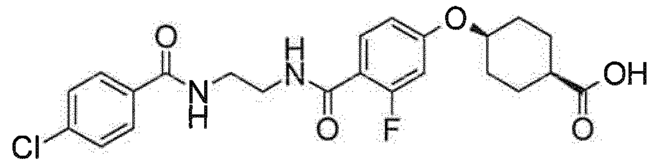 |

[Table 47]

| Ex | Structure |
|---|---|
| 30 | 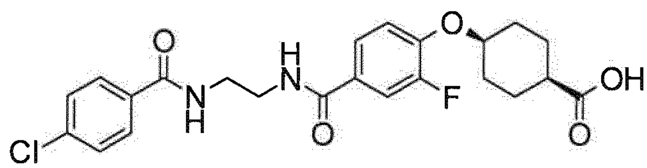 |
| 31 | 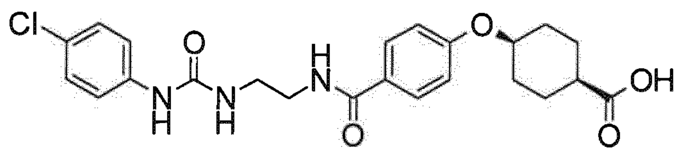 |
| 32 | 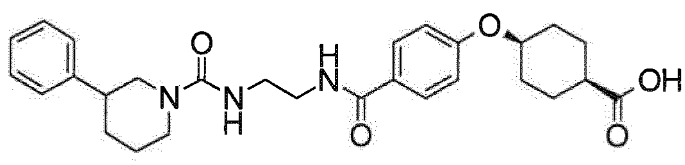 |
| 33 | 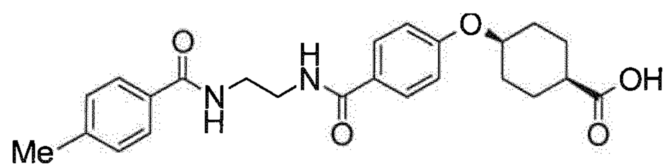 |
| 34 | 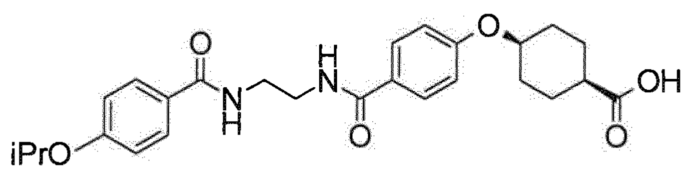 |
| 35 | 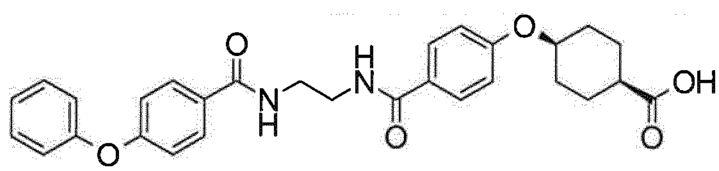 |

(continued)

| Ex | Structure |
|---|---|
| 36 | 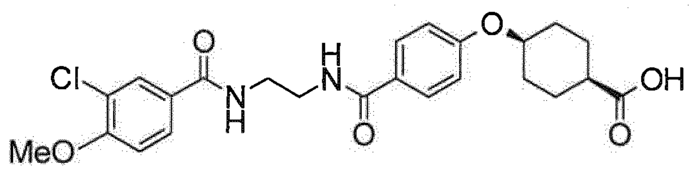 |
| 37 | 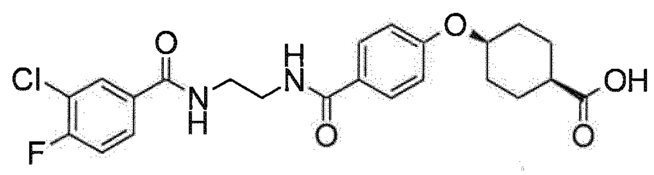 |

[Table 48]

| Ex | Structure |
|---|---|
| 38 | 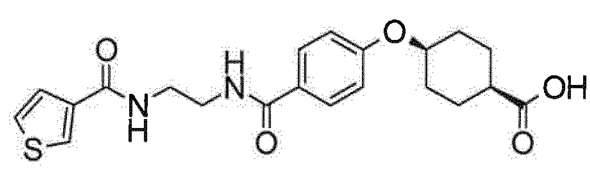 |
| 39 | 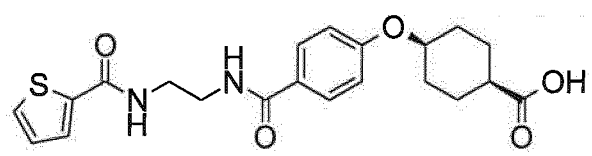 |
| 40 | 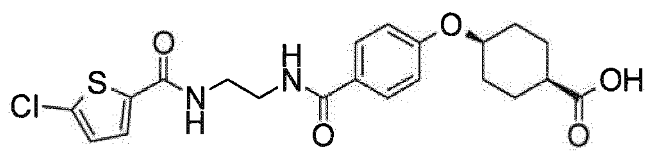 |
| 41 | 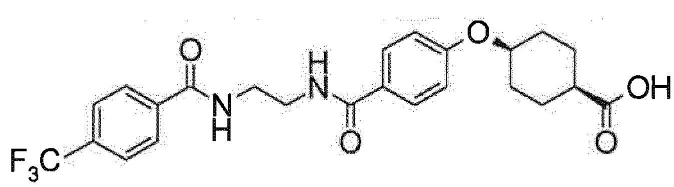 |
| 42 | 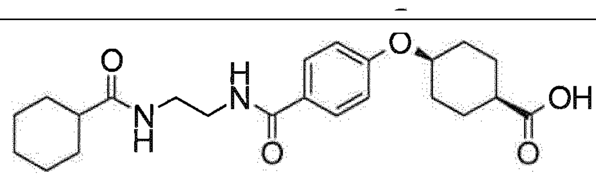 |

(continued)

| Ex | Structure |
|---|---|
| 43 | 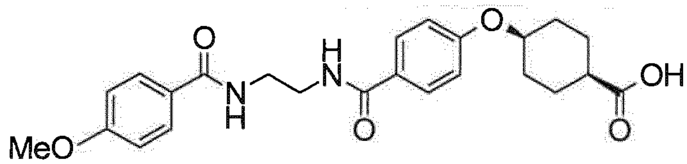 |
| 44 | 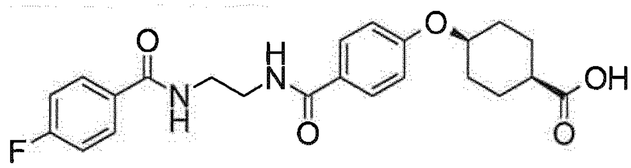 |
| 45 | 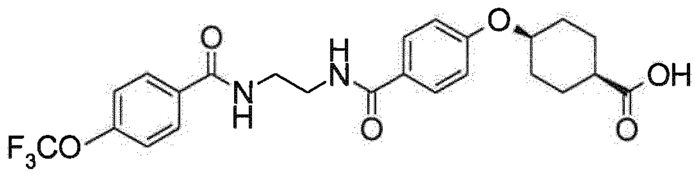 |

[Table 49]

| Ex | Structure |
|---|---|
| 46 | 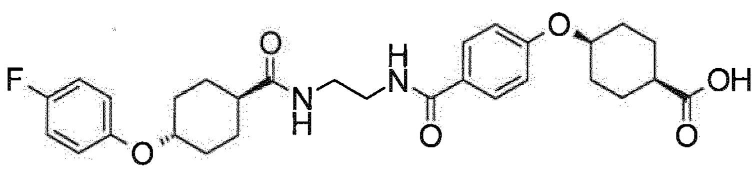 |
| 47 | 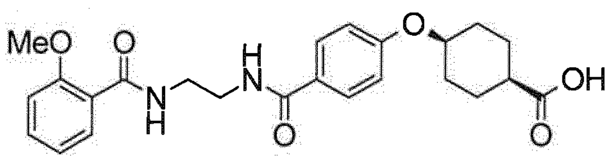 |
| 48 | 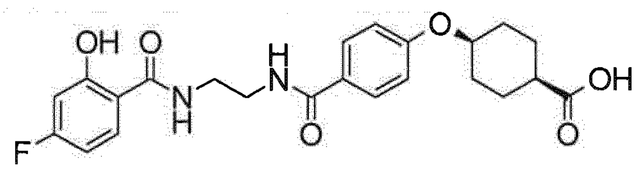 |
| 49 | 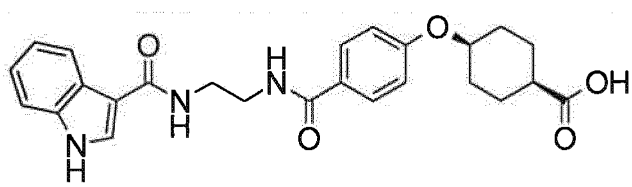 |

(continued)

| Ex | Structure |
|---|---|
| 50 | 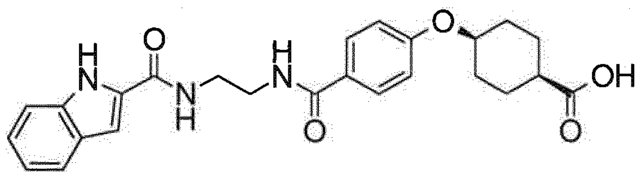 |
| 51 | 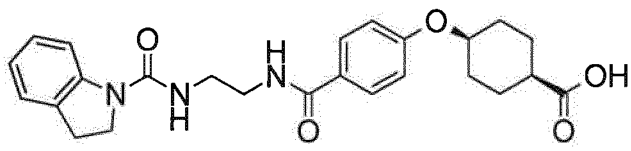 |
| 52 | 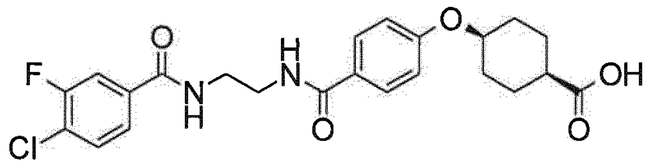 |
| 53 | 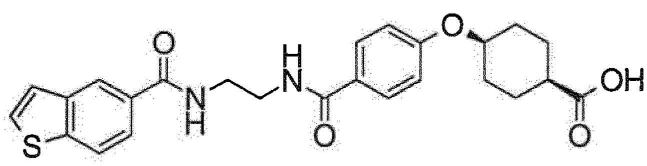 |

[Table 50]

| Ex | Structure |
|---|---|
| 54 | 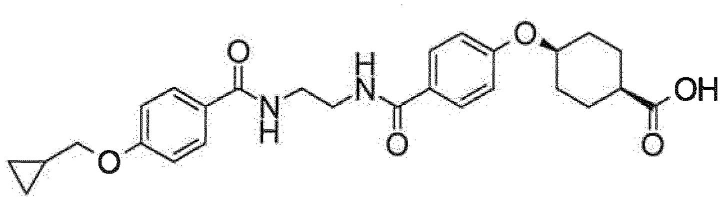 |
| 55 | 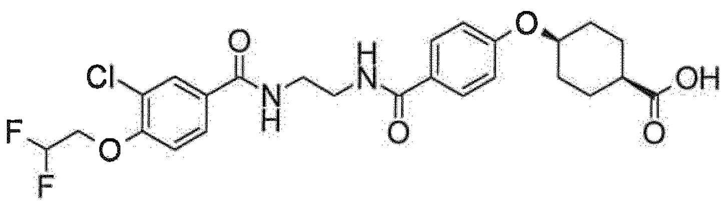 |
| 56 | 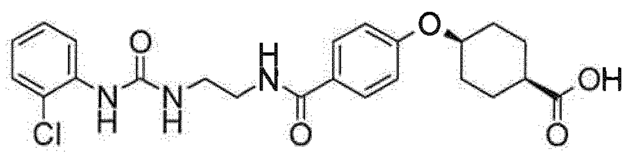 |

(continued)

| Ex | Structure |
|---|---|
| 57 | 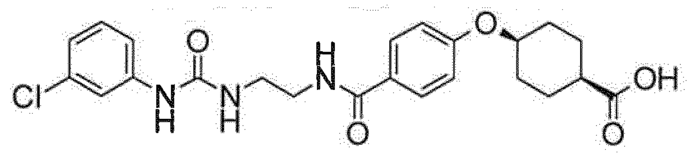 |
| 58 | 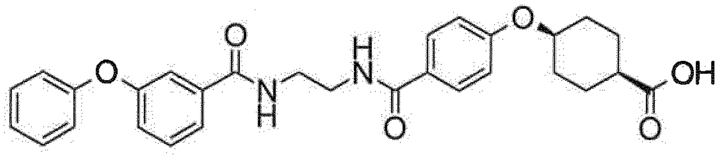 |
| 59 | 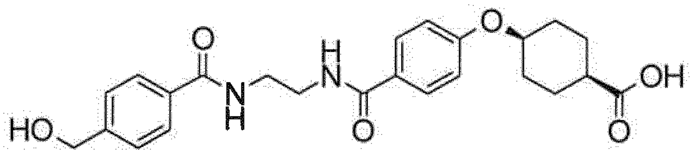 |
| 60 | 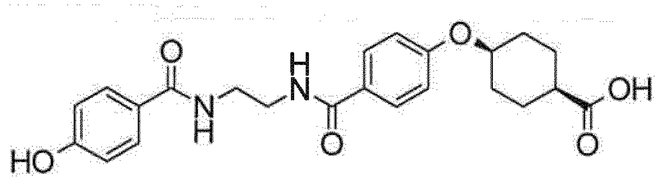 |
| 61 | 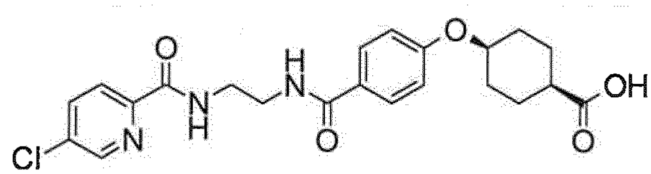 |

[Table 51]

| Ex | Structure |
|---|---|
| 62 | 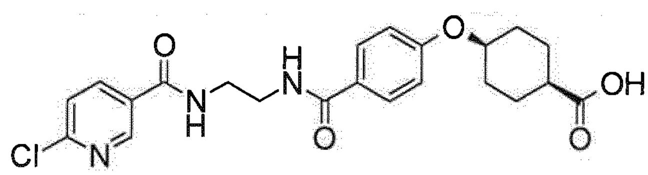 |
| 63 | 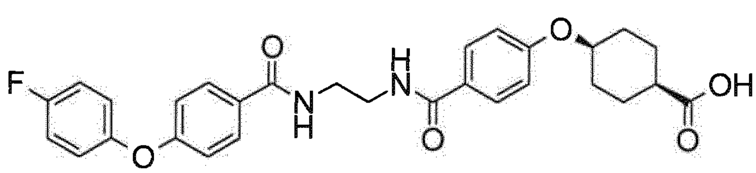 |

(continued)

| Ex | Structure |
|---|---|
| 64 | 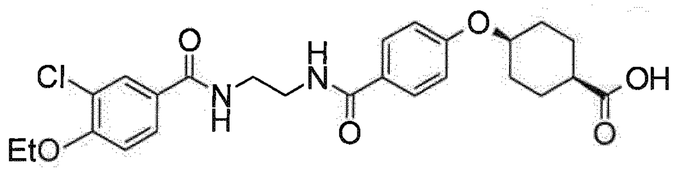 |
| 65 | 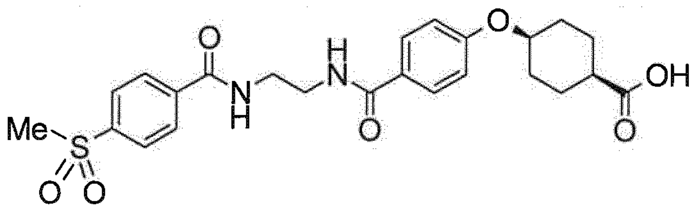 |
| 66 | 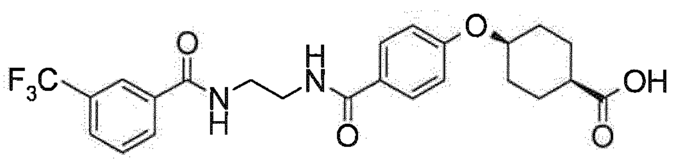 |
| 67 | 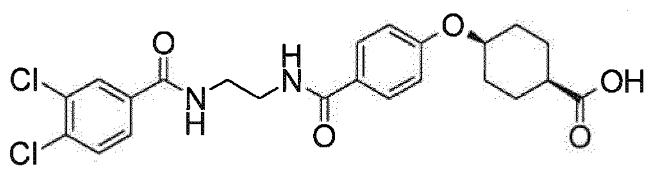 |
| 68 | 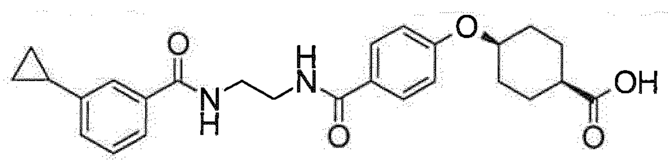 |
| 69 | 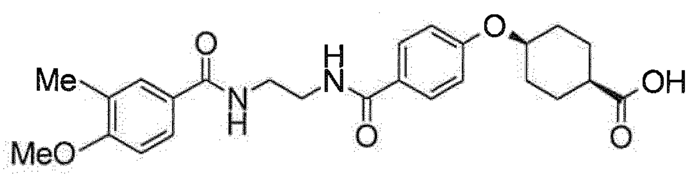 |

[Table 52]

| Ex | Structure |
|---|---|
| 70 | 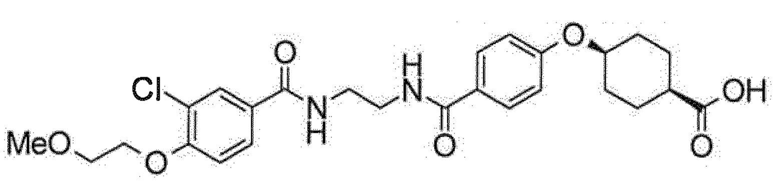 |

(continued)

| Ex | Structure |
|---|---|
| 71 | 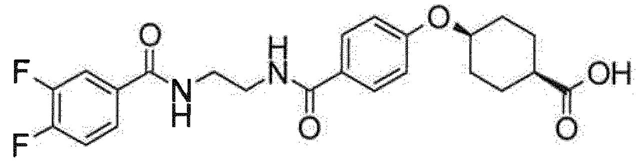 |
| 72 | 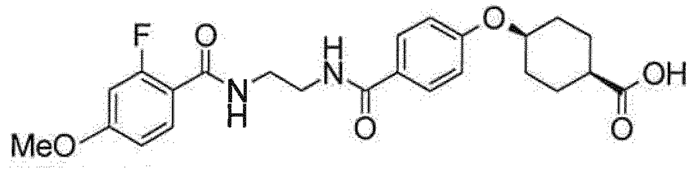 |
| 73 | 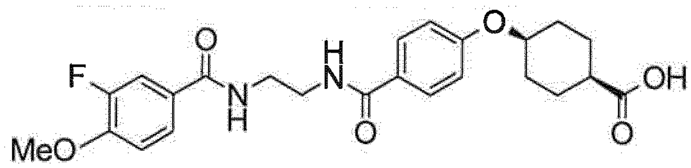 |
| 74 | 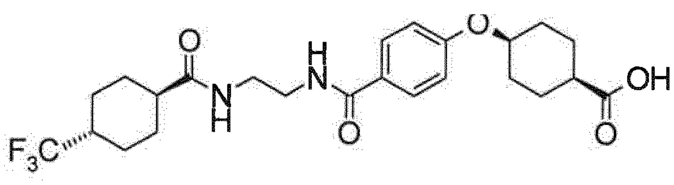 |
| 75 | 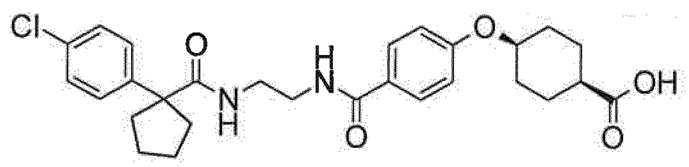 |
| 76 | 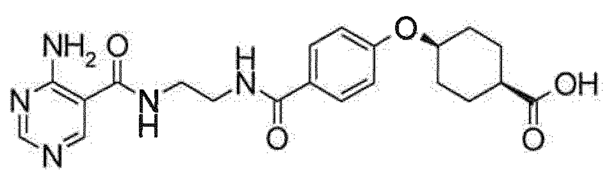 |

[Table 53]

| Ex | Structure |
|---|---|
| 77 | 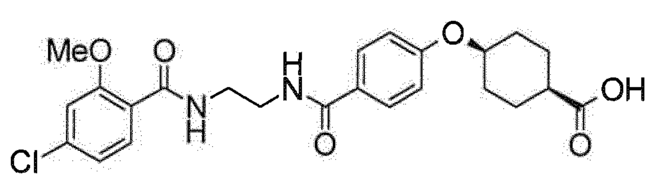 |

(continued)

| Ex | Structure |
|---|---|
| 78 | 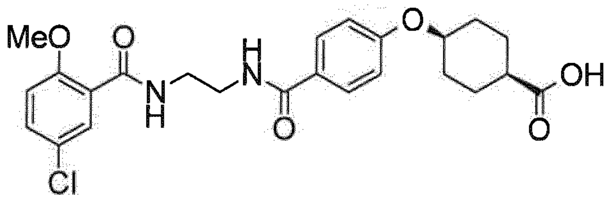 |
| 79 | 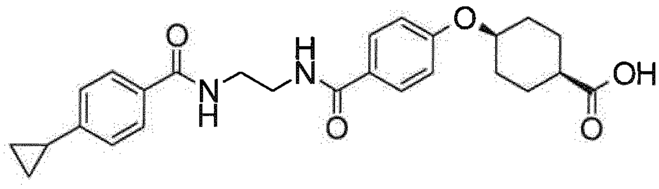 |
| 80 | 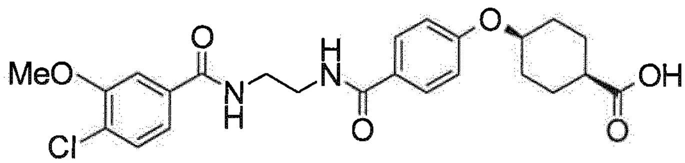 |
| 81 | 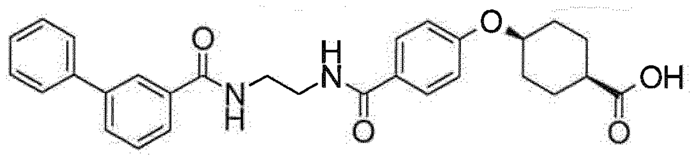 |
| 82 | 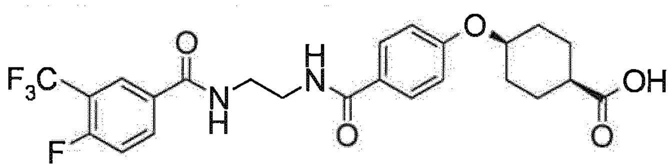 |
| 83 | 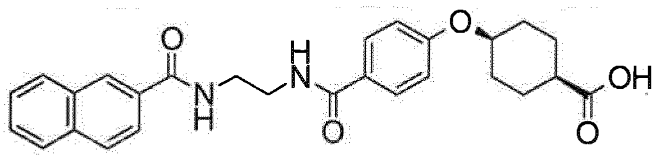 |
| 84 | 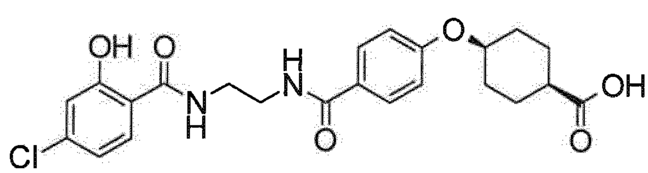 |

[Table 54]

| Ex | Structure |
|---|---|
| 85 | 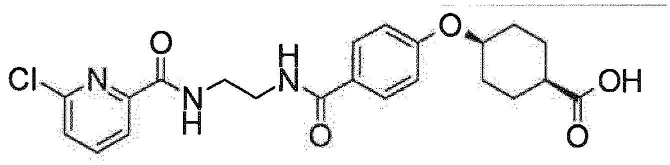 |
| 86 | 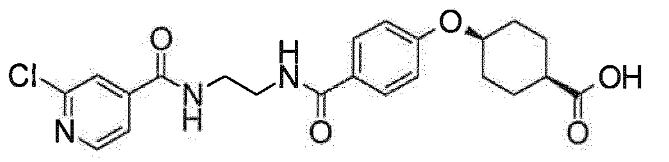 |
| 87 | 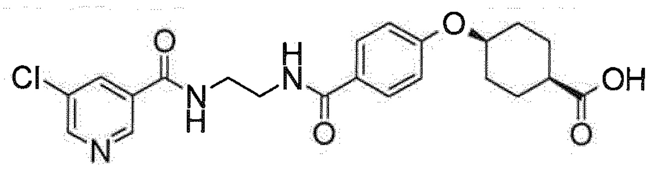 |
| 88 | 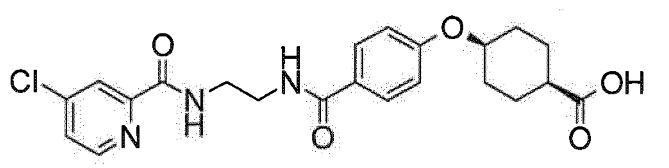 |
| 89 | 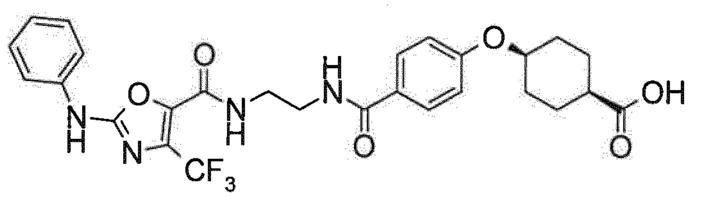 |
| 90 | 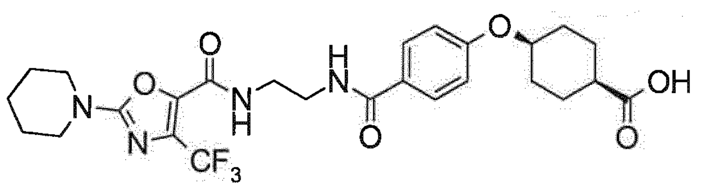 |
| 91 | 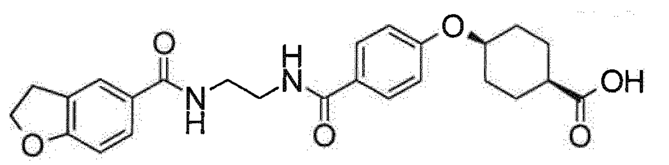 |
| 92 | 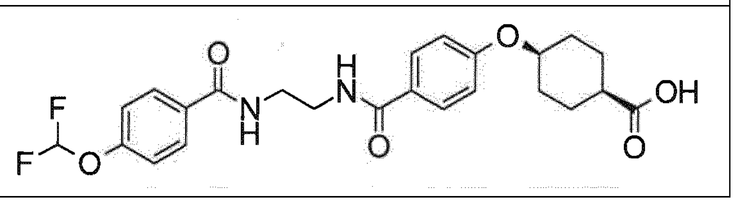 |

[Table 55]

| Ex | Structure |
|---|---|
| 93 | 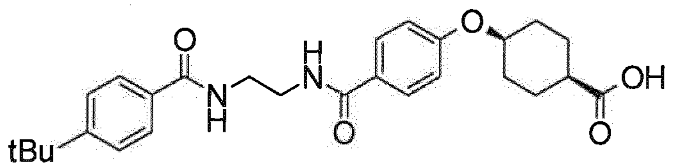 |
| 94 | 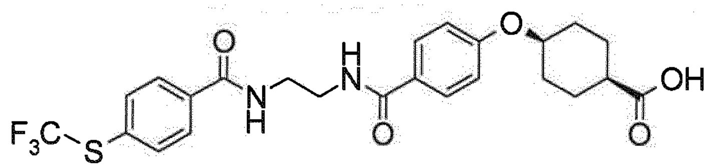 |
| 95 | 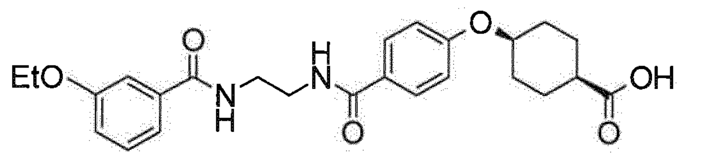 |
| 96 | 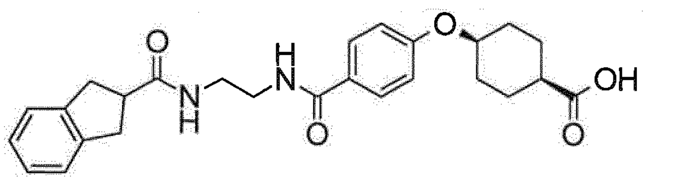 |
| 97 | 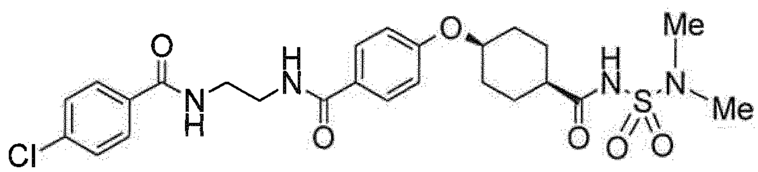 |
| 98 | 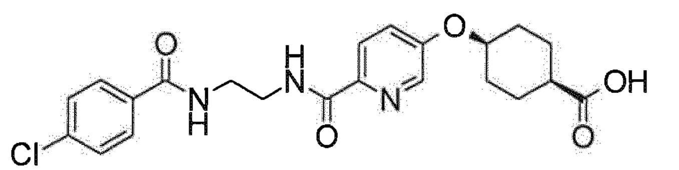 |
| 99 | 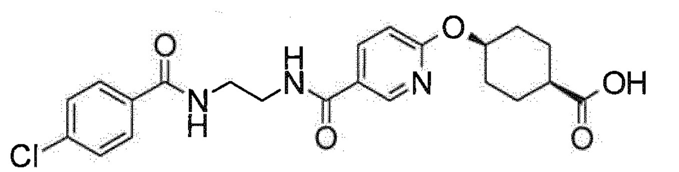 |
| 100 | 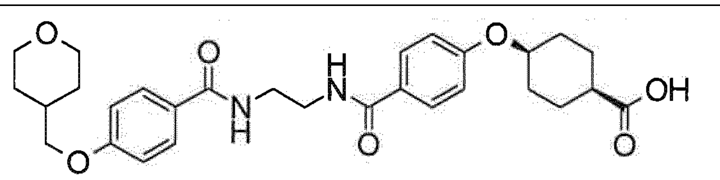 |

[Table 56]

| Ex | Structure |
|---|---|
| 101 | 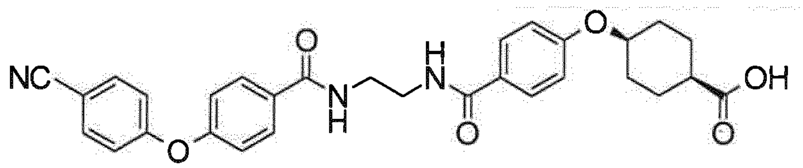 |
| 102 | 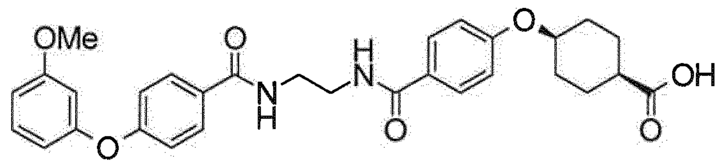 |
| 103 | 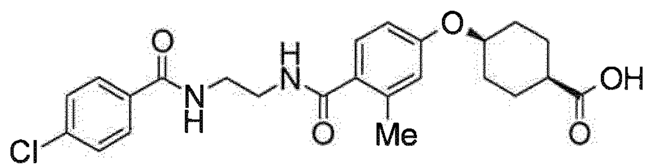 |
| 104 | 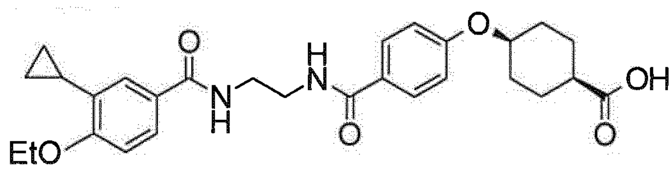 |
| 105 | 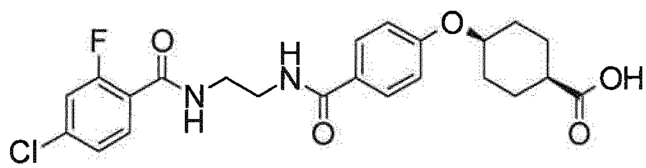 |
| 106 | 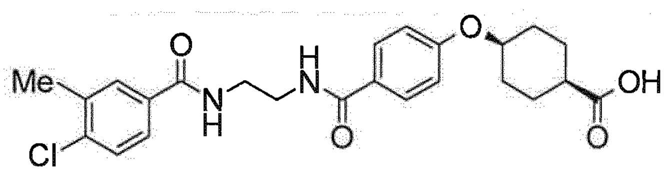 |
| 107 | 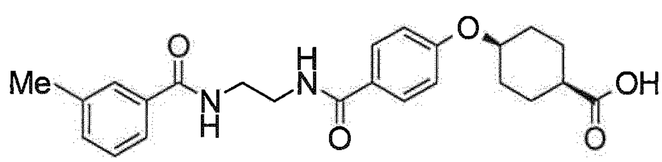 |
| 108 | 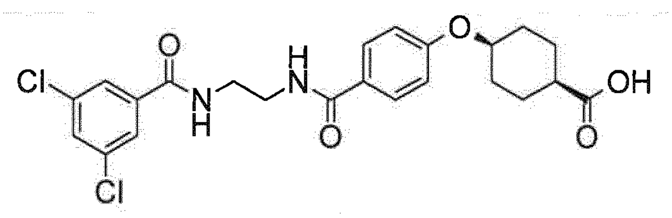 |

[Table 57]

| Ex | Structure |
|---|---|
| 109 | 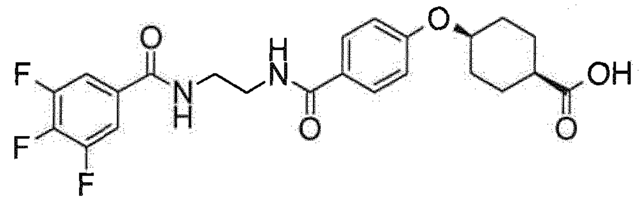 |
| 110 | 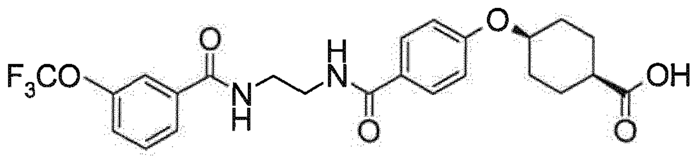 |
| 111 | 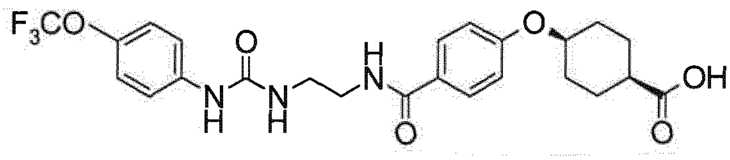 |
| 112 | 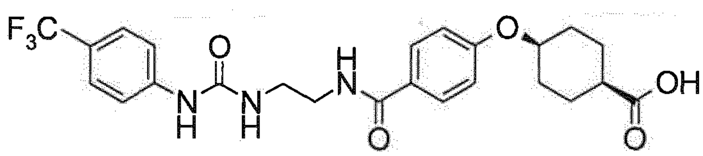 |
| 113 | 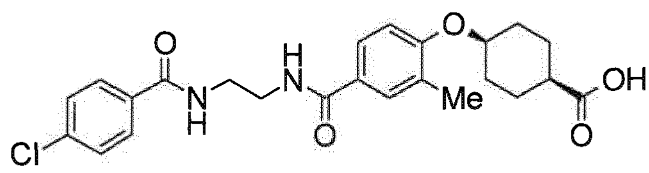 |
| 114 | 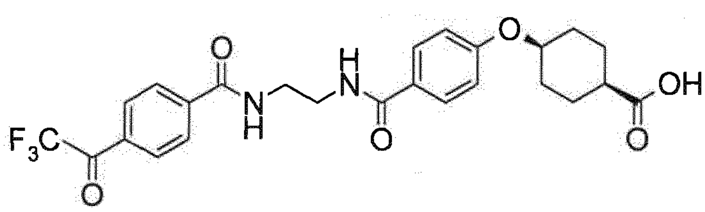 |
| 115 | 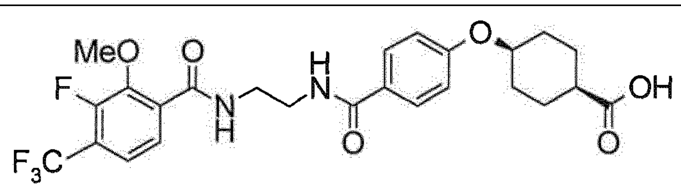 |
| 116 | 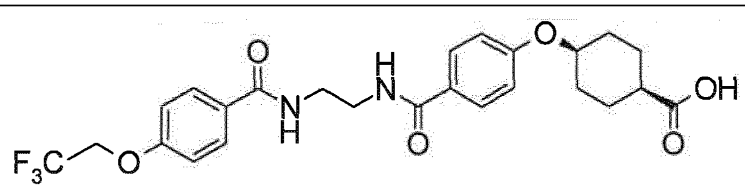 |

[Table 58]

| Ex | Structure |
|---|---|
| 117 | 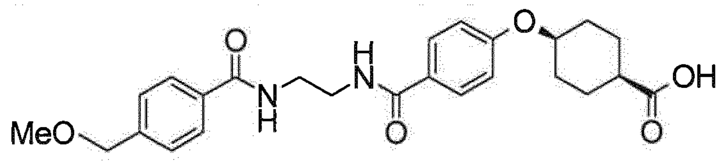 |
| 118 | 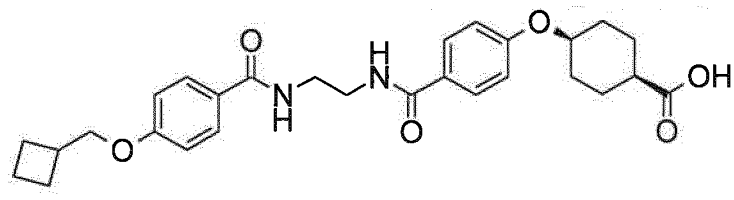 |
| 119 | 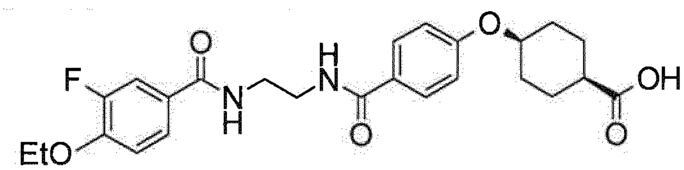 |
| 120 | 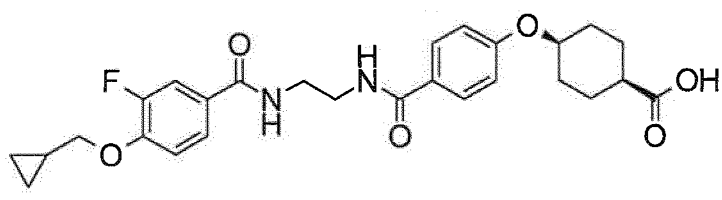 |
| 121 | 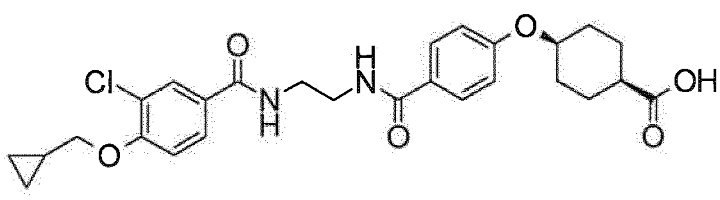 |
| 122 | 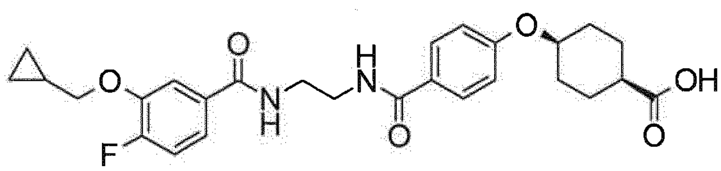 |
| 123 | 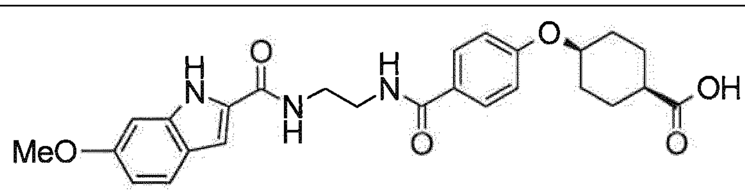 |

[Table 59]

| Ex | Structure |
|---|---|
| 124 | 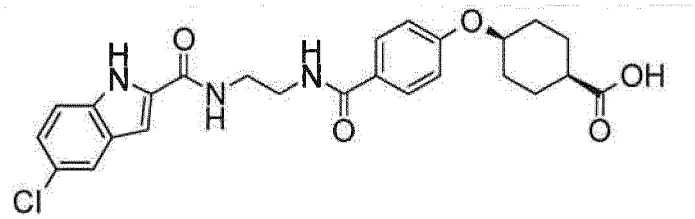 |
| 125 | 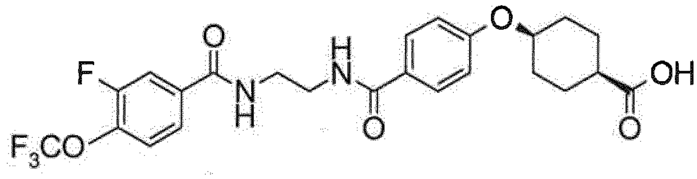 |
| 126 | 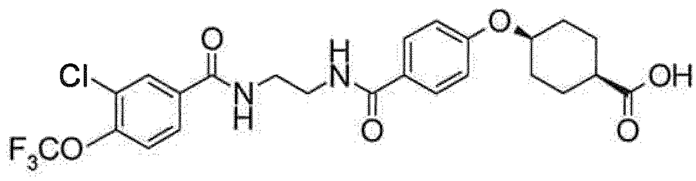 |
| 127 | 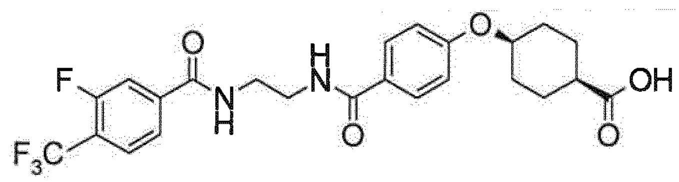 |
| 128 | 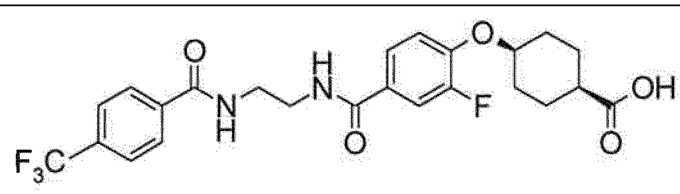 |
| 129 | 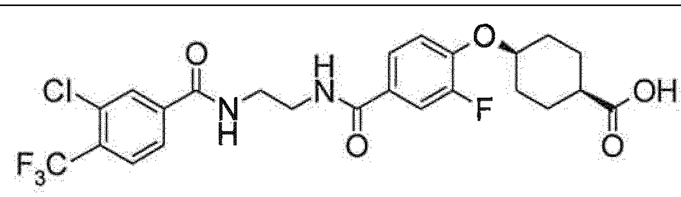 |
| 130 | 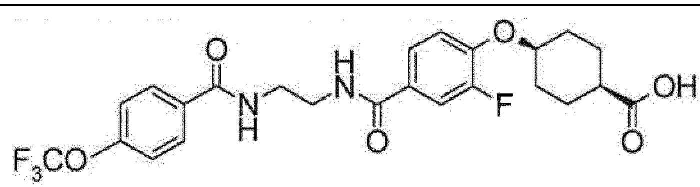 |

[Table 60]

| Ex | Structure |
|---|---|
| 131 | 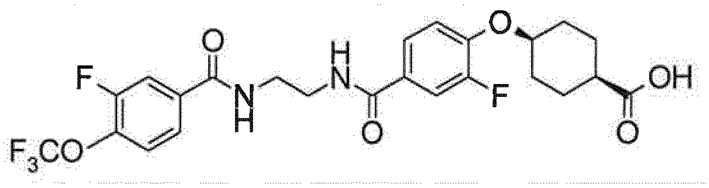 |
| 132 | 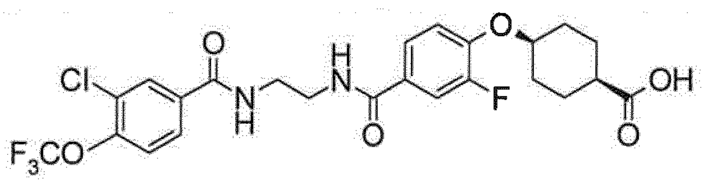 |
| 133 | 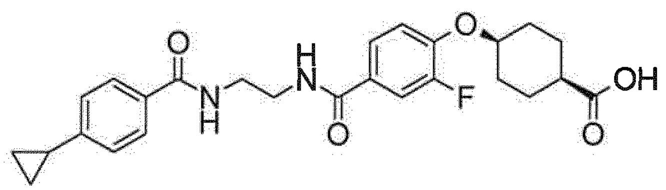 |
| 134 | 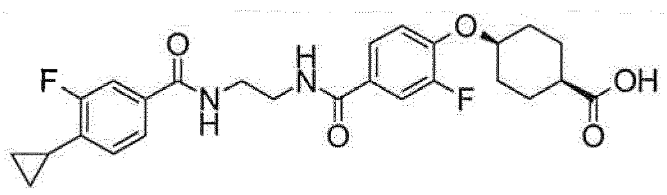 |
| 135 | 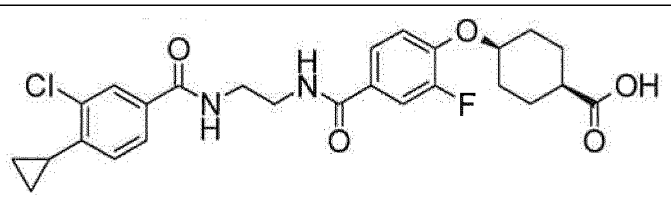 |
| 136 | 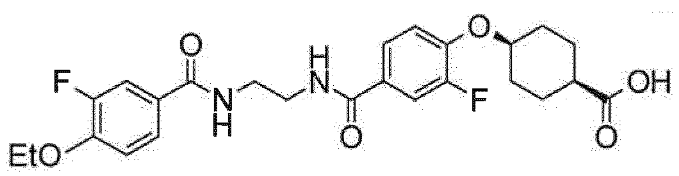 |
| 137 | 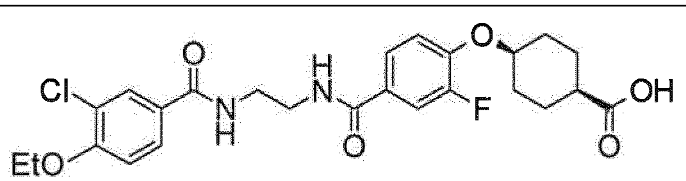 |

[Table 61]

| Ex | Structure |
|---|---|
| 138 | 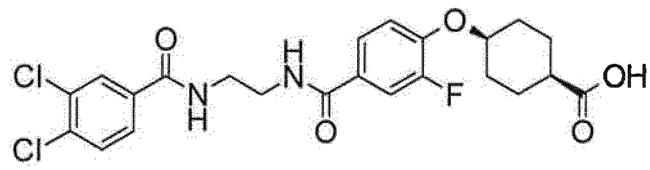 |
| 139 | 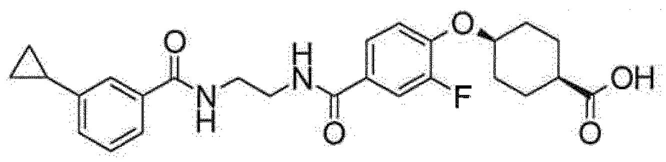 |
| 140 | 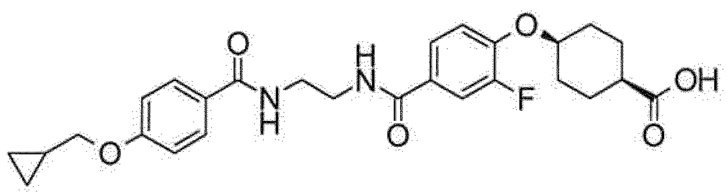 |
| 141 | 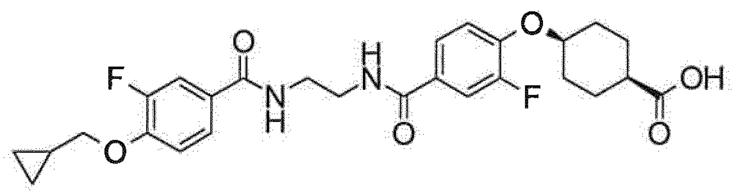 |
| 142 | 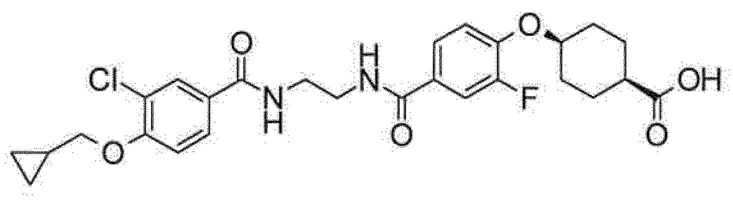 |
| 143 | 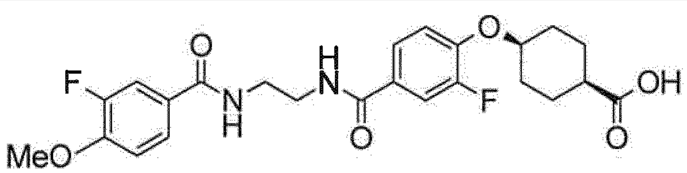 |
| 144 | 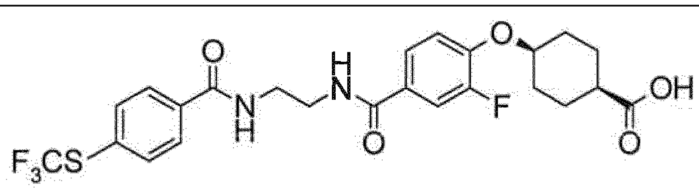 |

[Table 62]

| Ex | Structure |
|---|---|
| 145 | 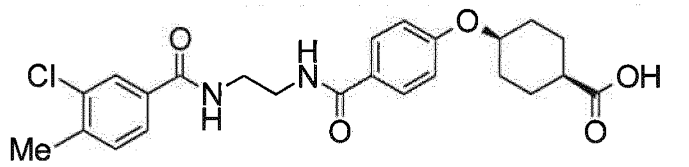 |
| 146 | 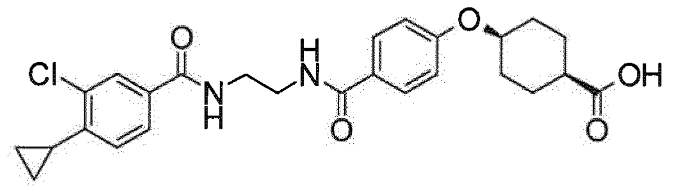 |
| 147 | 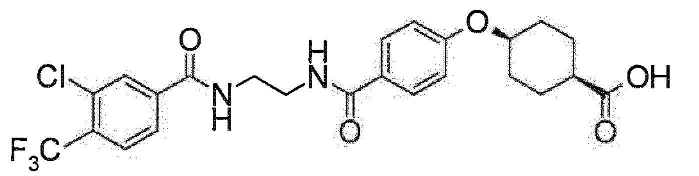 |
| 148 | 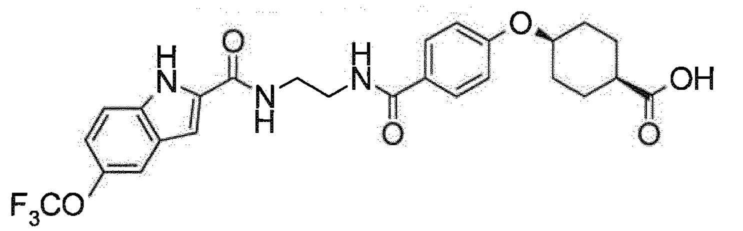 |
| 149 | 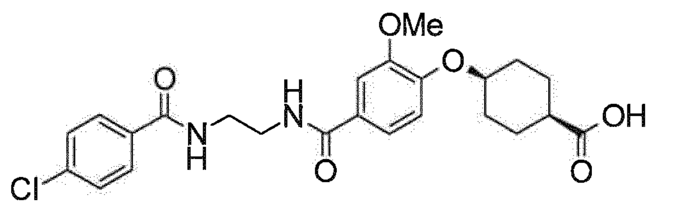 |
| 150 | 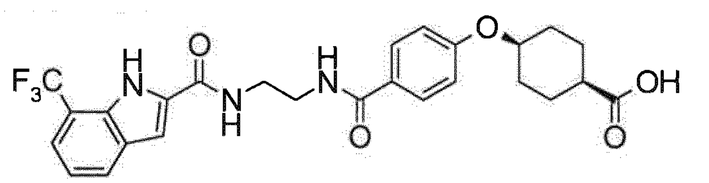 |
| 151 | 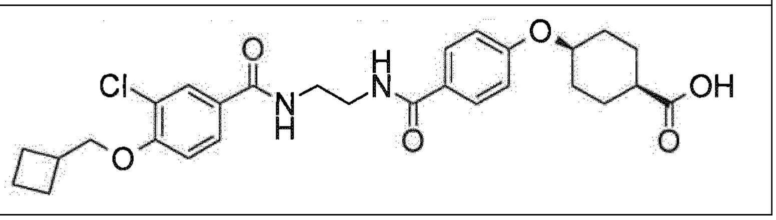 |

[Table 63]

| Ex | Structure |
|---|---|
| 152 | 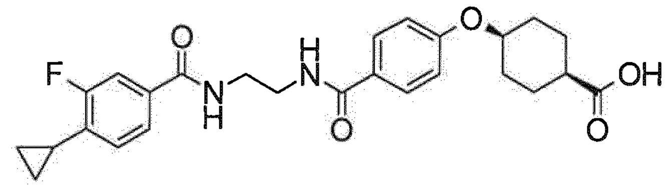 |
| 153 | 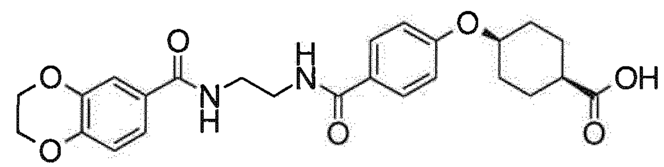 |
| 154 | 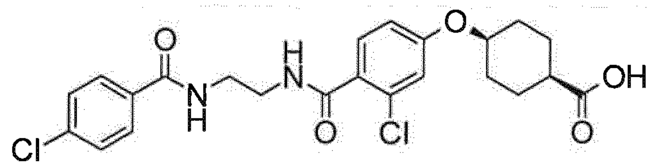 |
| 155 | 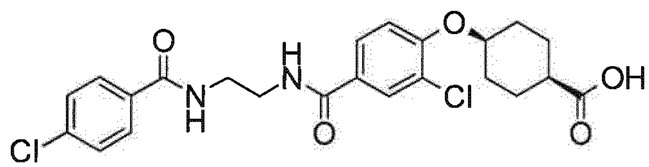 |
| 156 | 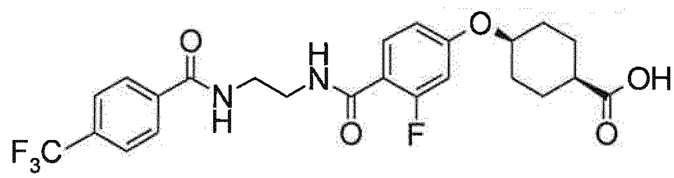 |
| 157 | 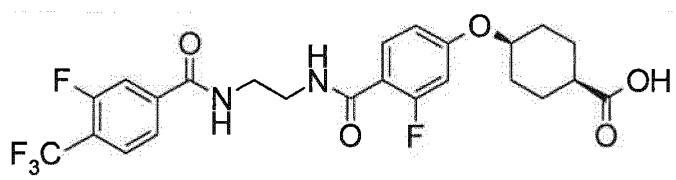 |
| 158 | 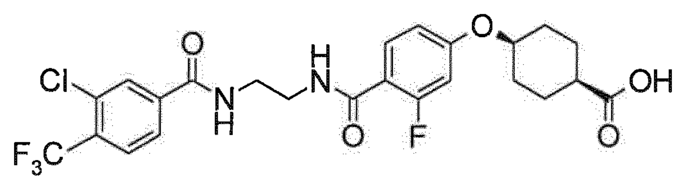 |

[Table 64]

| Ex | Structure |
|---|---|
| 159 | 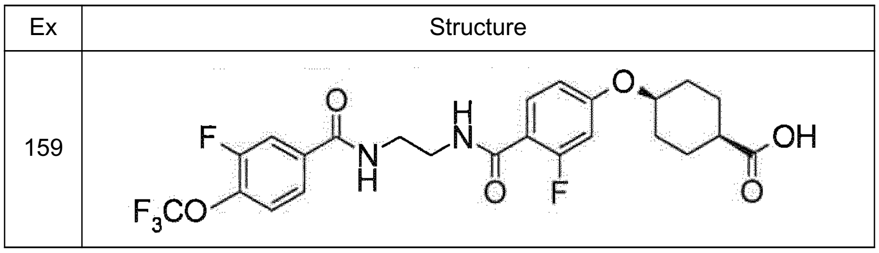 |

(continued)

| Ex | Structure |
|---|---|
| 160 | 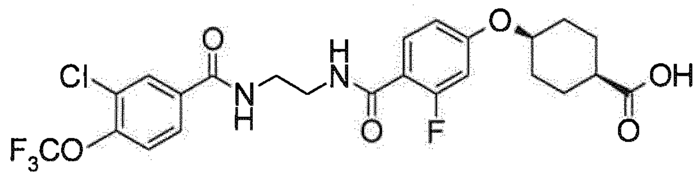 |
| 161 | 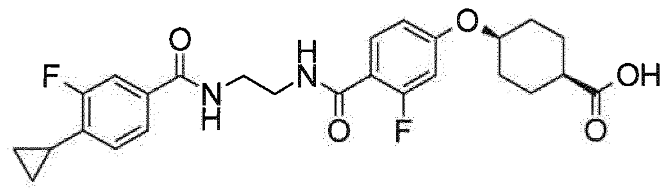 |
| 162 | 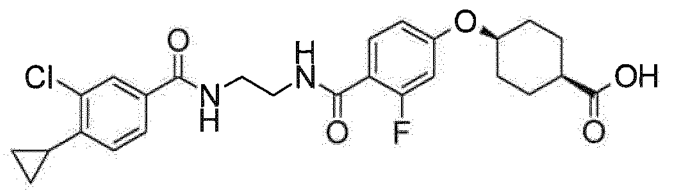 |
| 163 | 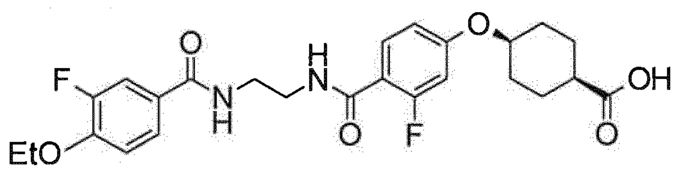 |
| 164 | 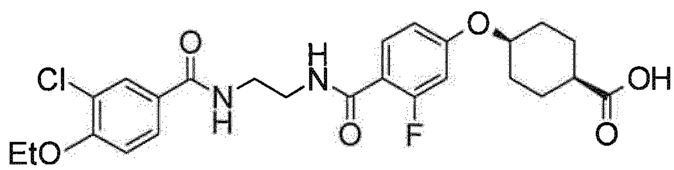 |
| 165 | 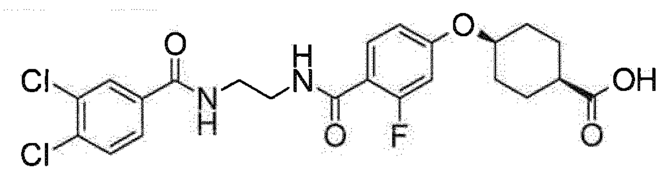 |

[Table 65]

| Ex | Structure |
|---|---|
| 166 | 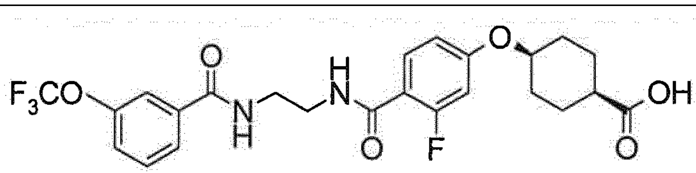 |
| 167 | 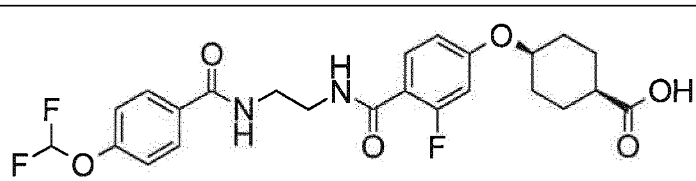 |

(continued)

| Ex | Structure |
|---|---|
| 168 | 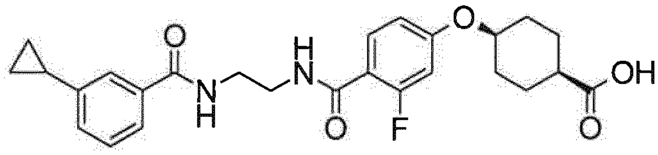 |
| 169 | 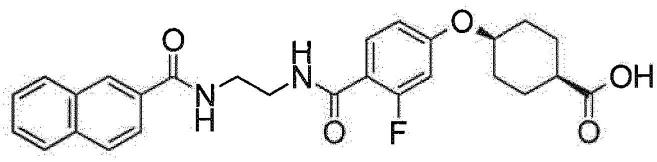 |
| 170 | 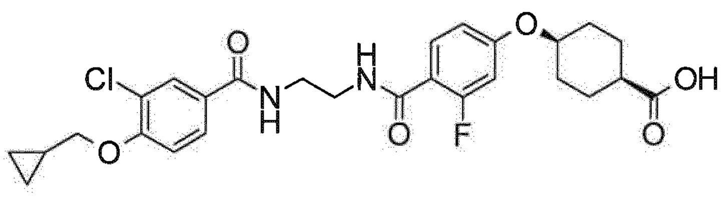 |
| 171 | 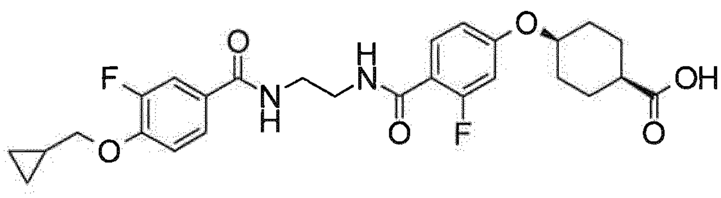 |
| 172 | 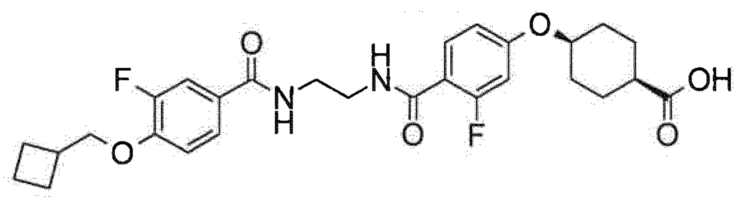 |

[Table 66]

| Ex | Structure |
|---|---|
| 173 | 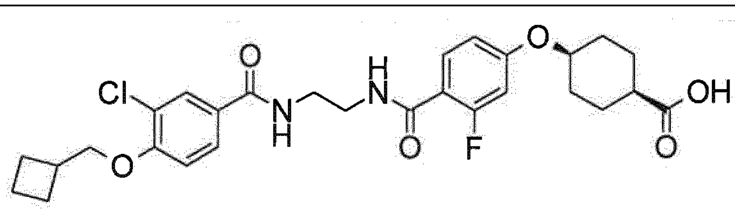 |
| 174 | 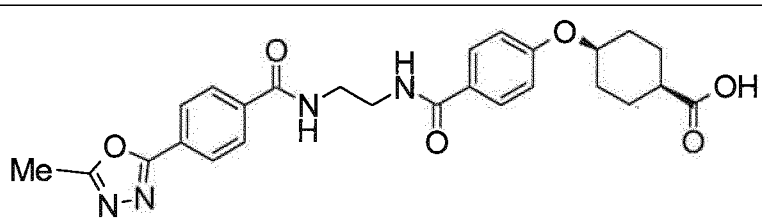 |

(continued)

| Ex | Structure |
|---|---|
| 175 | 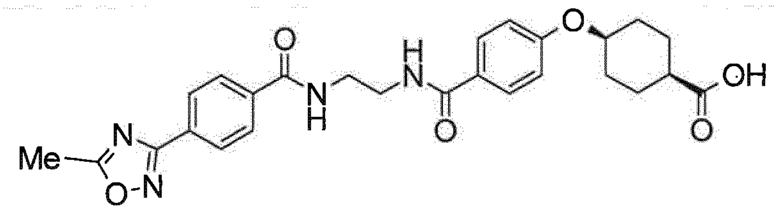 |
| 176 | 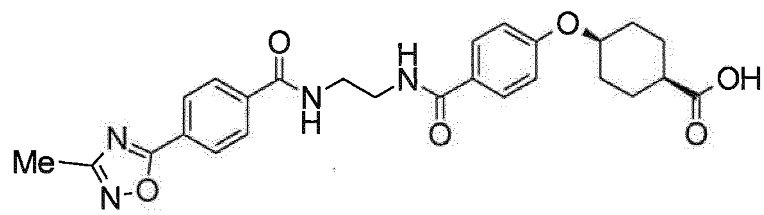 |
| 177 | 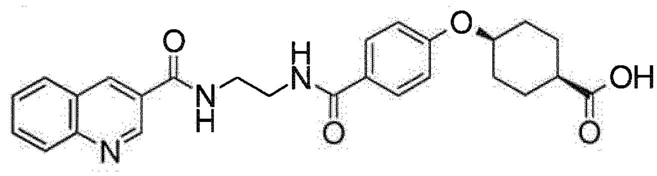 |
| 178 | 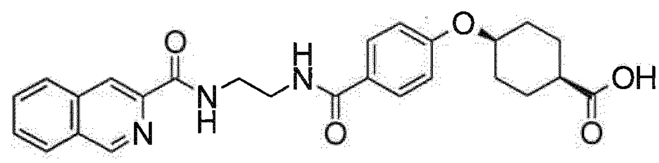 |
| 179 | 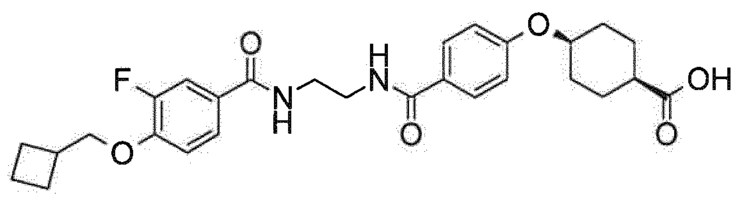 |

[Table 67]

| Ex | Structure |
|---|---|
| 180 | 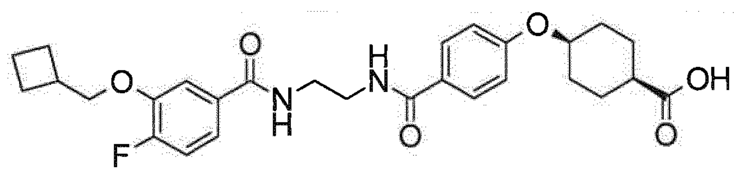 |
| 181 | 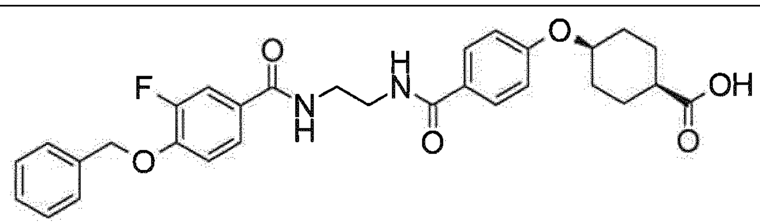 |

(continued)

| Ex | Structure |
|---|---|
| 182 | 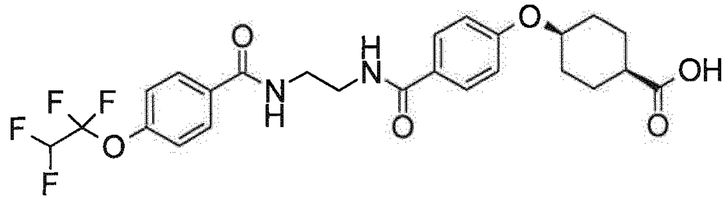 |
| 183 | 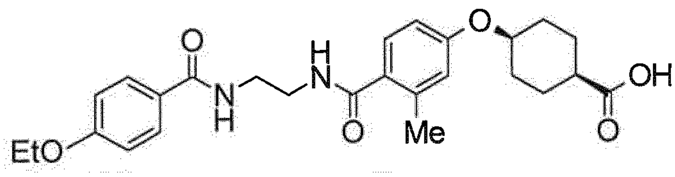 |
| 184 | 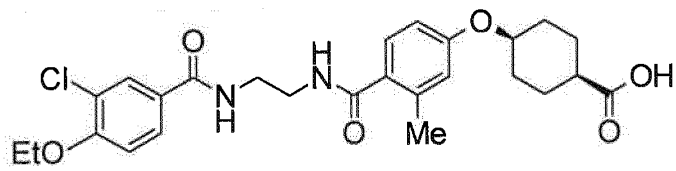 |
| 185 | 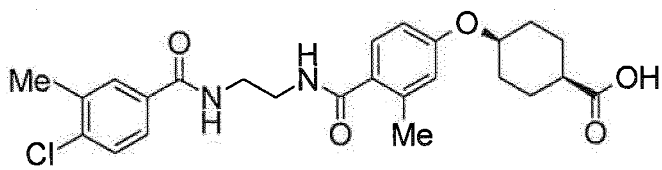 |
| 186 | 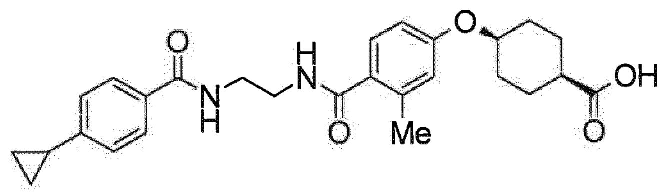 |

[Table 68]

| Ex | Structure |
|---|---|
| 187 | 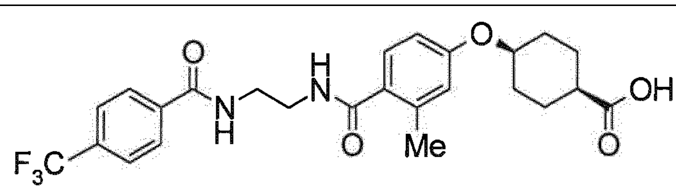 |
| 188 | 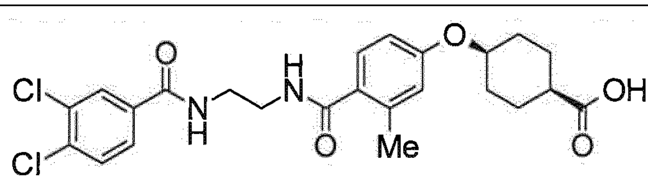 |

(continued)

| Ex | Structure |
|---|---|
| 189 | 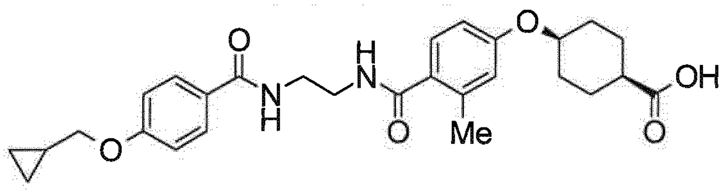 |
| 190 | 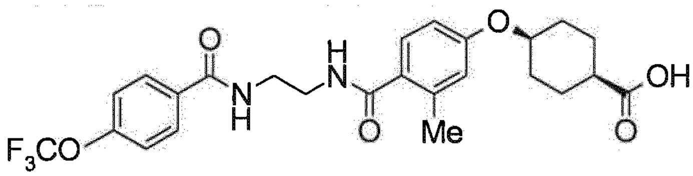 |
| 191 | 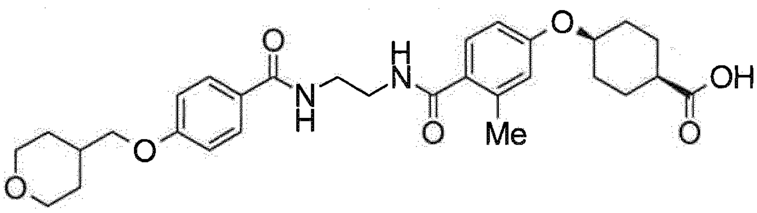 |
| 192 | 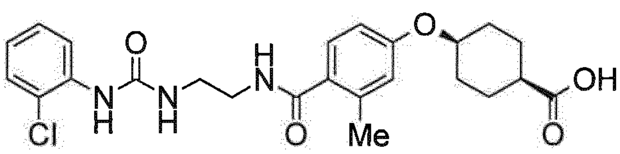 |
| 193 | 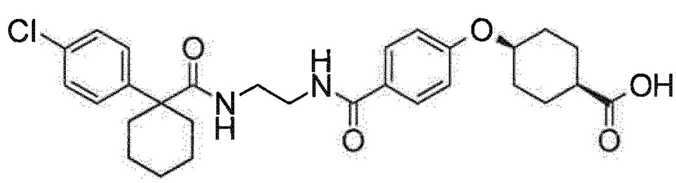 |

[Table 69]

| Ex | Structure |
|---|---|
| 194 | 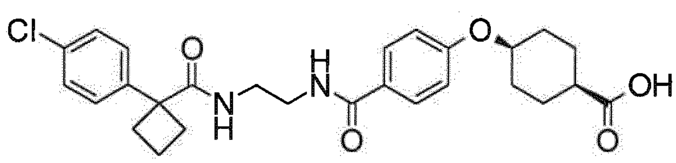 |
| 195 | 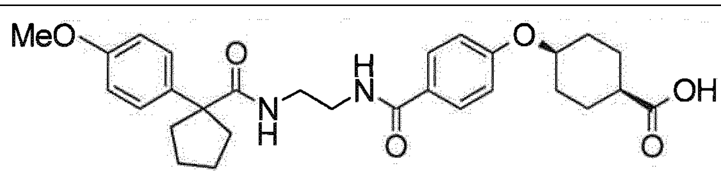 |

(continued)

| Ex | Structure |
|---|---|
| 196 | 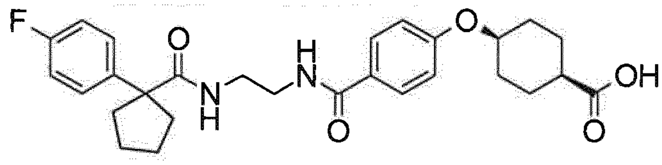 |
| 197 | 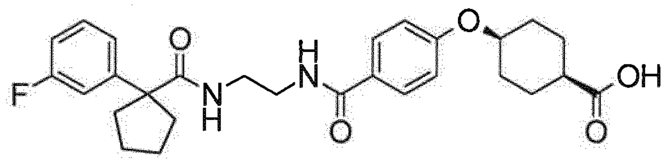 |
| 198 | 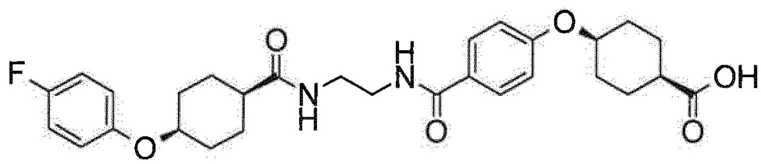 |
| 199 | 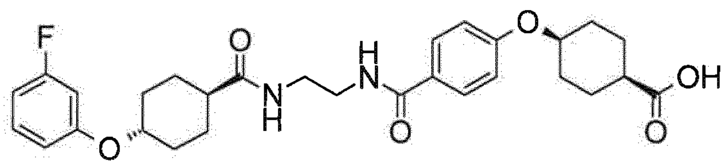 |
| 200 | 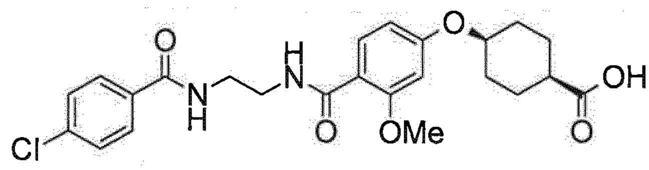 |
| 201 | 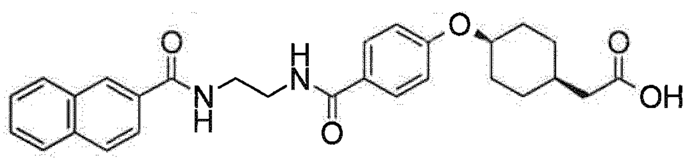 |

[Table 70]

| Ex | Structure |
|---|---|
| 202 | 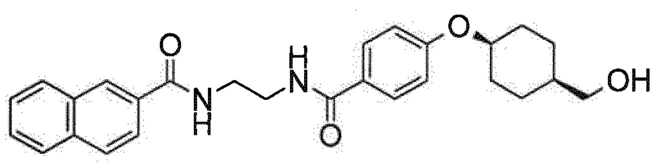 |
| 203 | 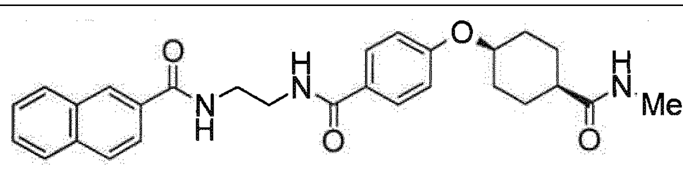 |

(continued)

| Ex | Structure |
|---|---|
| 204/Cl | 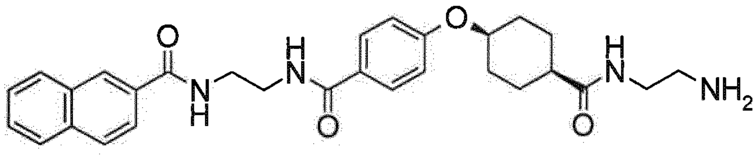 |
| 205 | 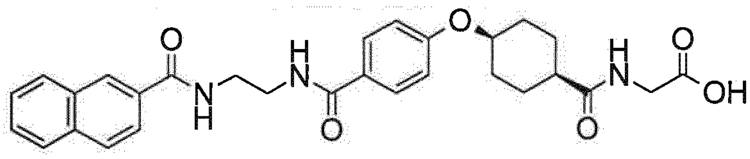 |
| 206 | 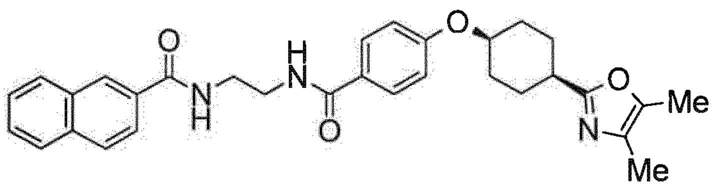 |
| 207 | 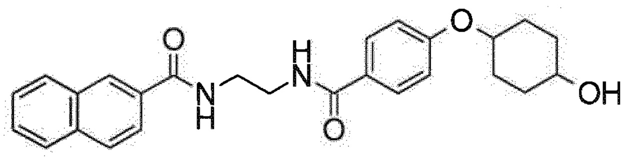 |
| 208 | 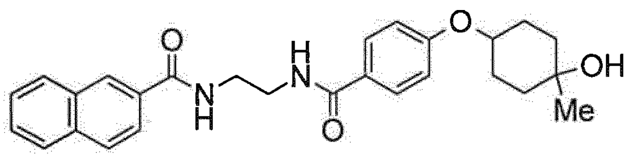 |
| 209/Cl | 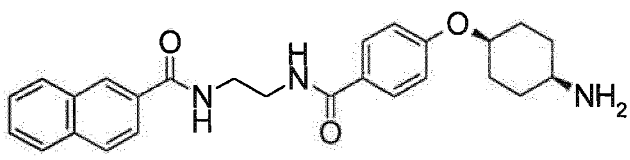 |

[Table 71]

| Ex | Structure |
|---|---|
| 210 | 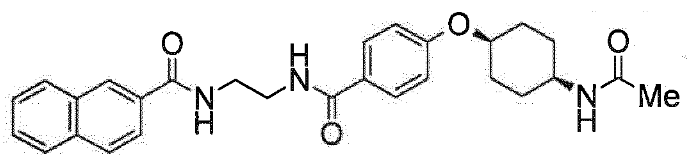 |
| 211 | 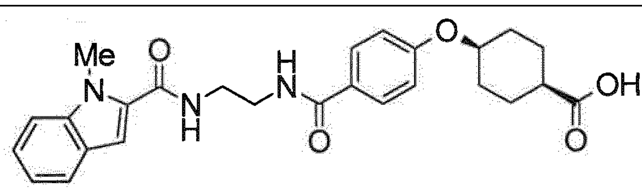 |

(continued)

| Ex | Structure |
|---|---|
| 212 | 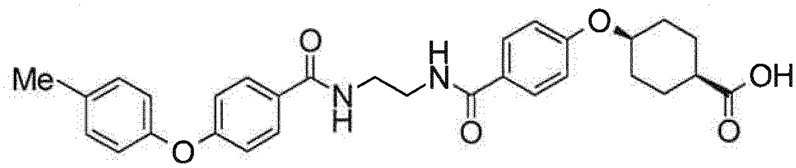 |
| 213 | 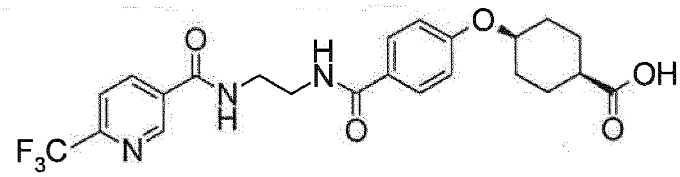 |
| 214 | 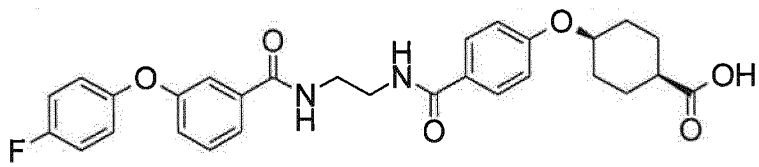 |
| 215 | 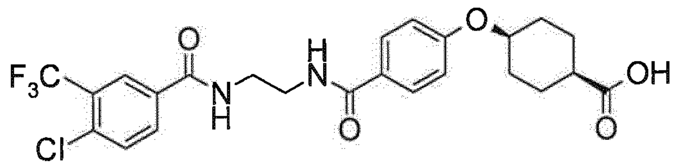 |
| 216 | 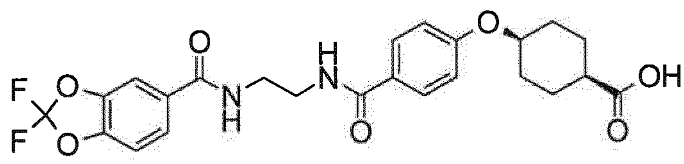 |
| 217 | 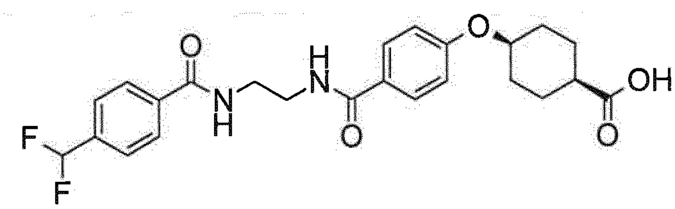 |

[Table 72]

| Ex | Structure |
|---|---|
| 218 | 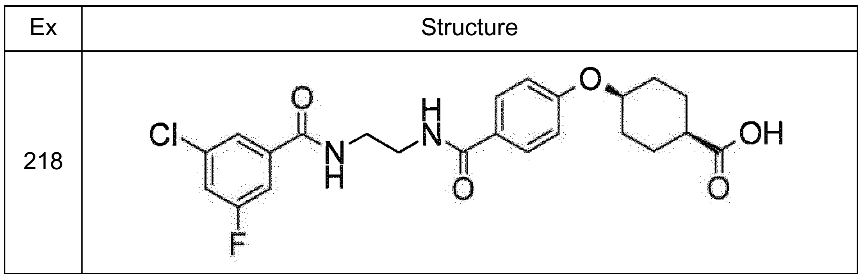 |

(continued)

| Ex | Structure |
|---|---|
| 219 | 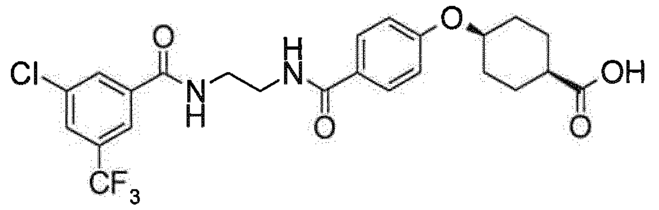 |
| 220 | 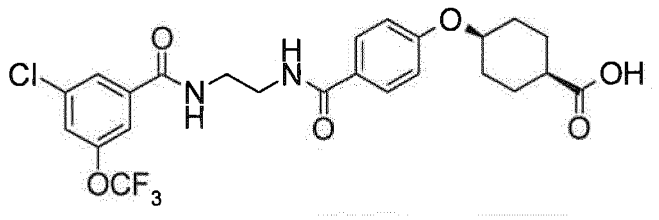 |
| 221 | 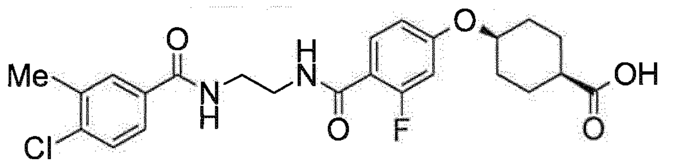 |
| 222 | 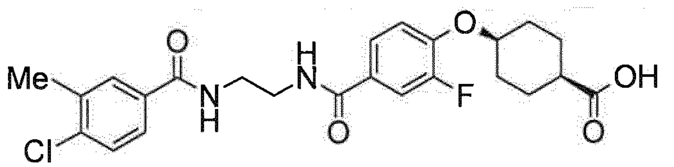 |
| 223 | 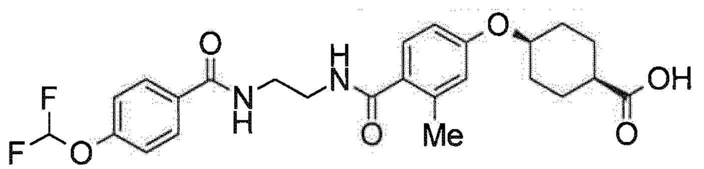 |
| 224 | 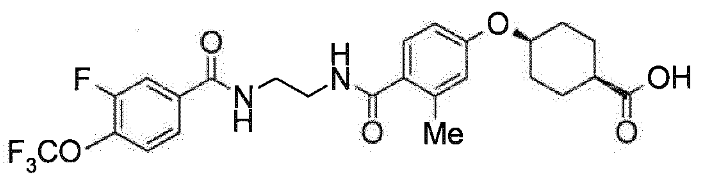 |

[Table 73]

| Ex | Structure |
|---|---|
| 225 | 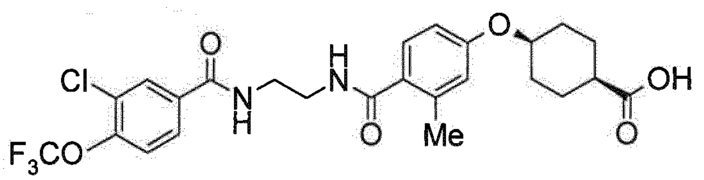 |

(continued)

| Ex | Structure |
|---|---|
| 226 | 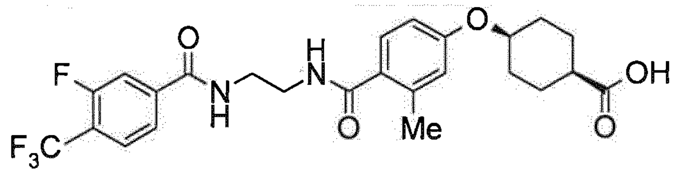 |
| 227 | 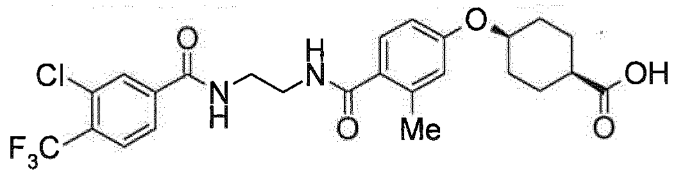 |
| 228 | 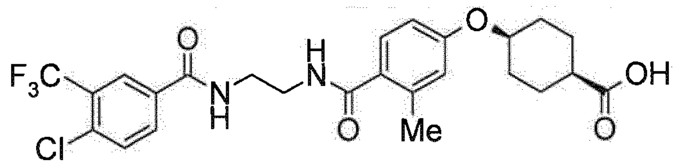 |
| 229 | 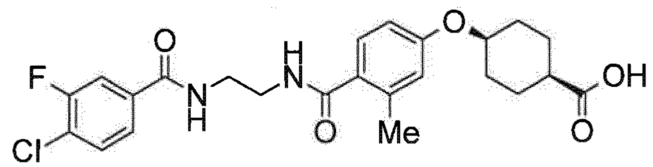 |
| 230 | 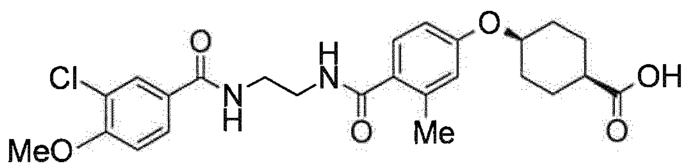 |
| 231 | 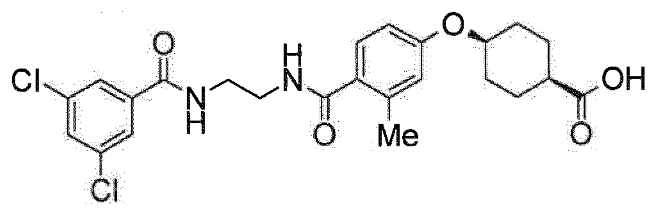 |
| 232 | 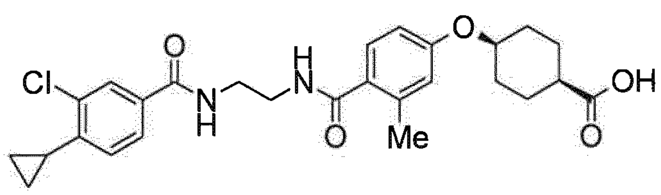 |

[Table 74]

| Ex | Structure |
|---|---|
| 233 | 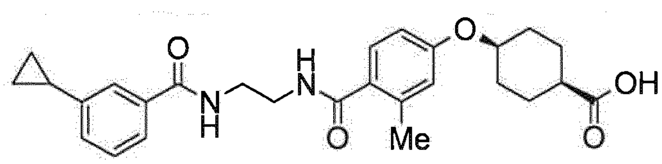 |

(continued)

| Ex | Structure |
|---|---|
| 234 | 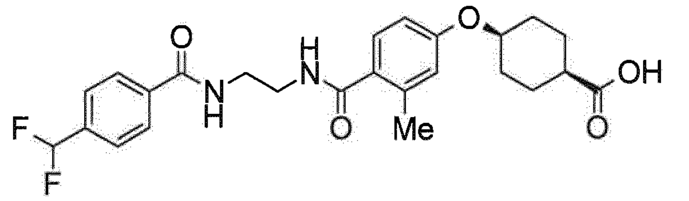 |
| 235 | 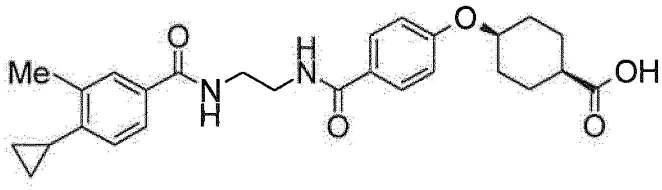 |
| 236 | 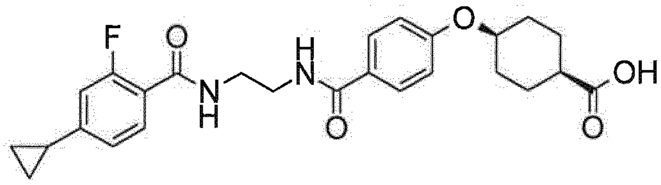 |
| 237 | 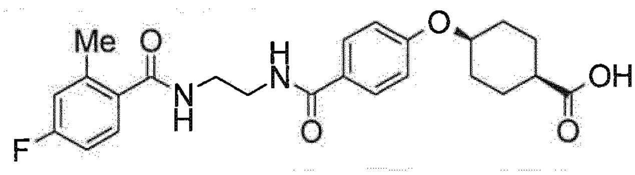 |
| 238 | 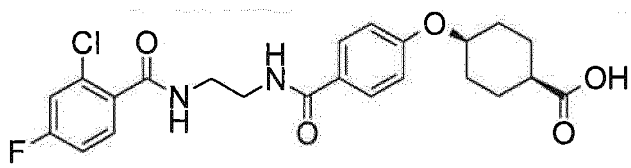 |
| 239 | 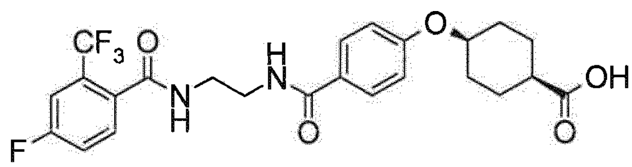 |
| 240 | 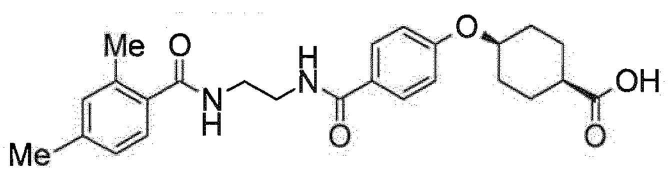 |

[Table 75]

| Ex | Structure |
|---|---|
| 241 | 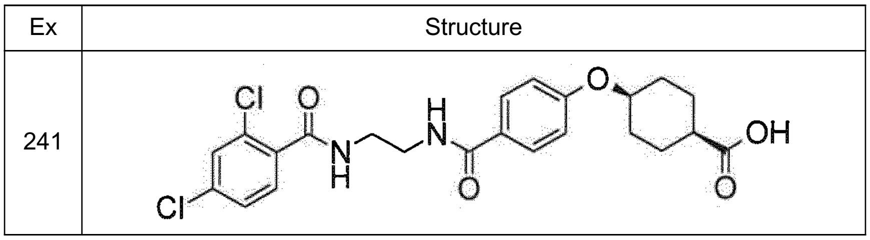 |

(continued)

| Ex | Structure |
|---|---|
| 242 | 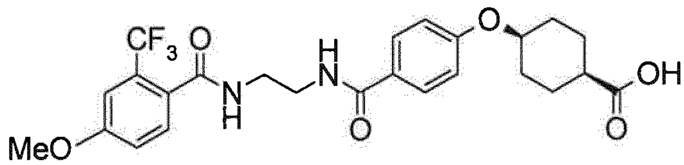 |
| 243 | 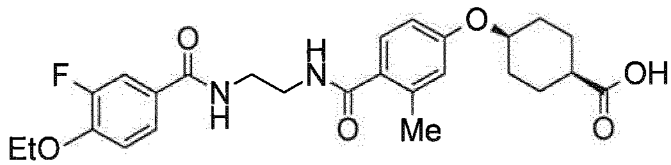 |
| 244 | 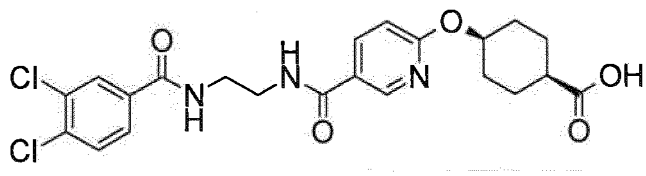 |
| 245 | 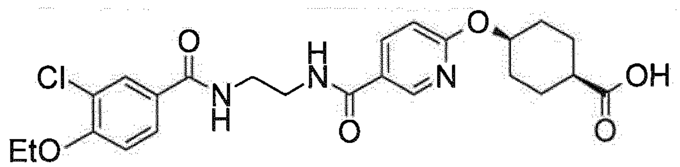 |
| 246 | 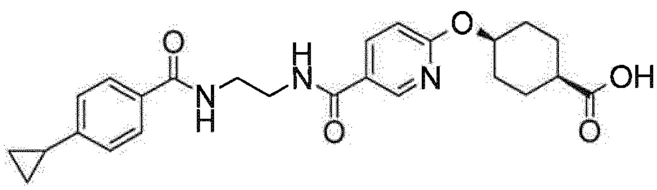 |
| 247 | 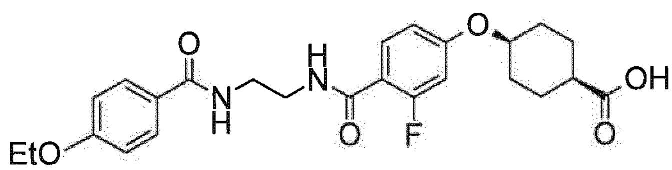 |
| 248 | 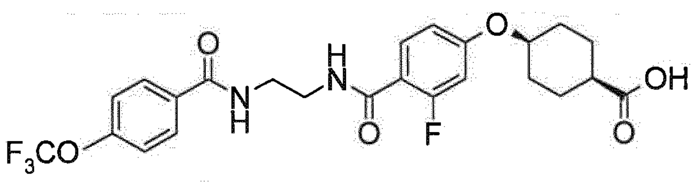 |

[Table 76]

| Ex | Structure |
|---|---|
| 249 | 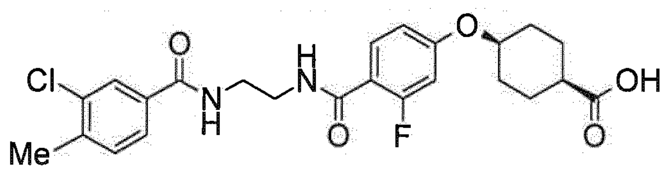 |

(continued)

| Ex | Structure |
|---|---|
| 250 | 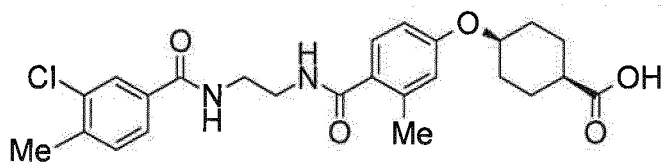 |
| 251 | 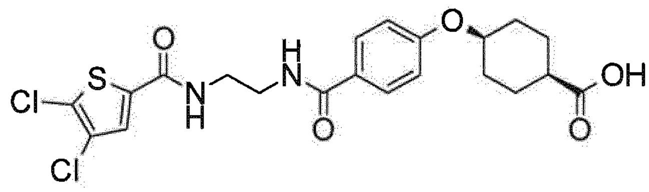 |
| 252 | 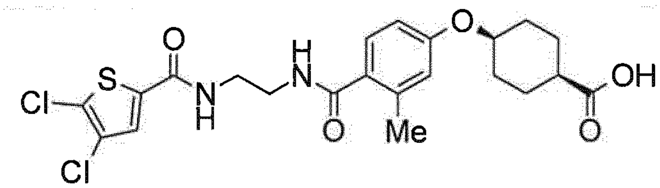 |
| 253 | 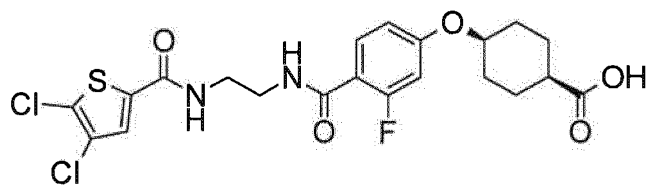 |
| 254 | 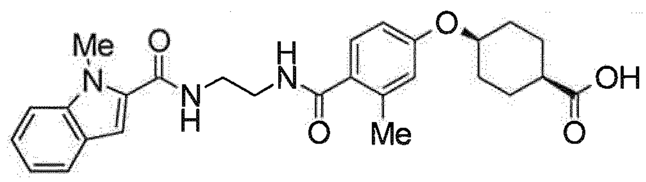 |
| 255 | 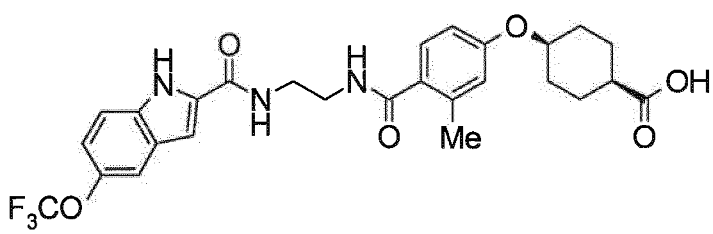 |

[Table 77]

| Ex | Structure |
|---|---|
| 256 | 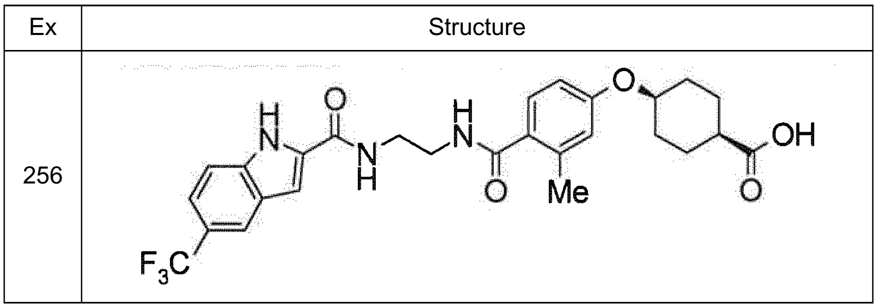 |

(continued)

| Ex | Structure |
|---|---|
| 257 | 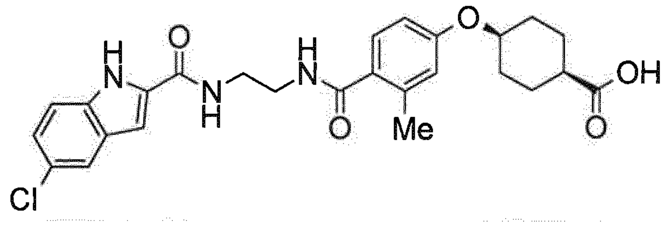 |
| 258 | 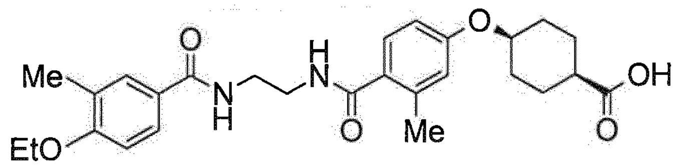 |
| 259 | 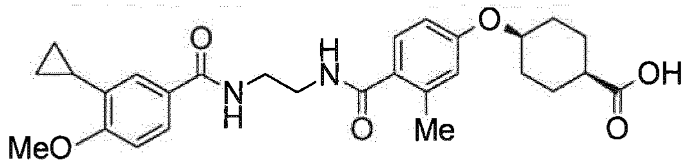 |
| 260 | 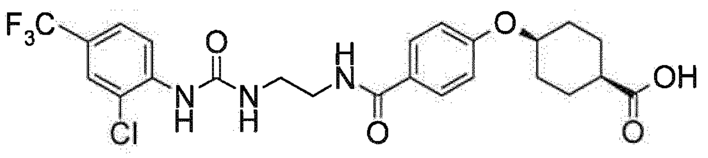 |
| 261 | 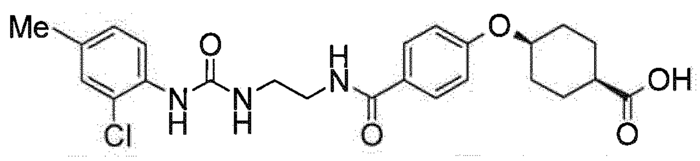 |
| 262 | 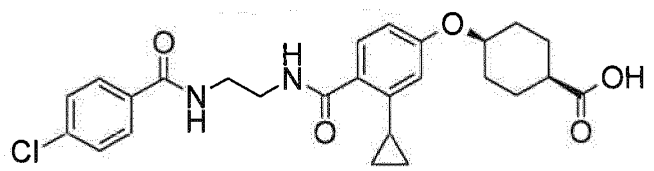 |
| 263 | 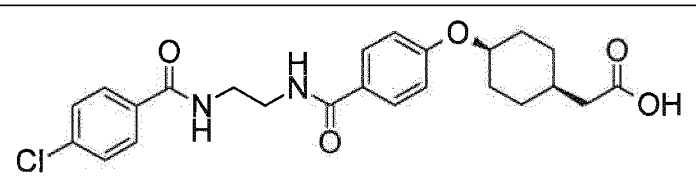 |

[Table 78]

| Ex | Structure |
|---|---|
| 264 | 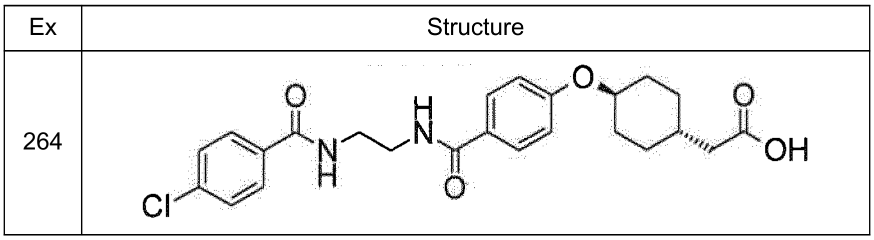 |

(continued)

| Ex | Structure |
|---|---|
| 265 | 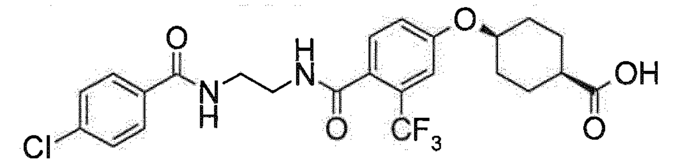 |
| 266 | 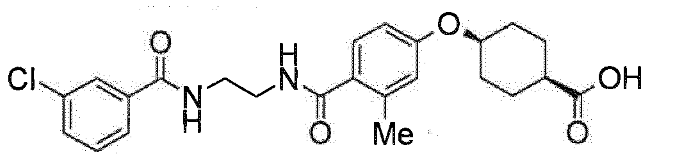 |
| 267 | 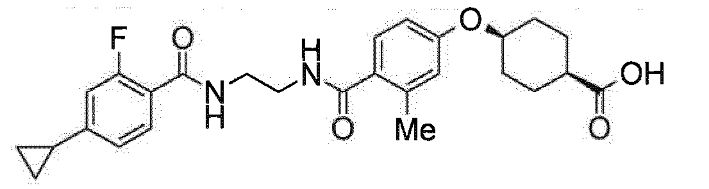 |
| 268 | 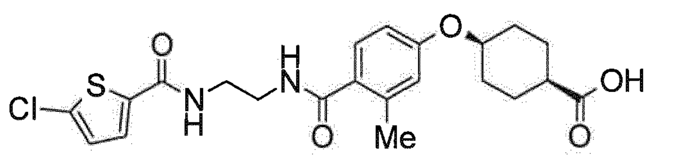 |
| 269 | 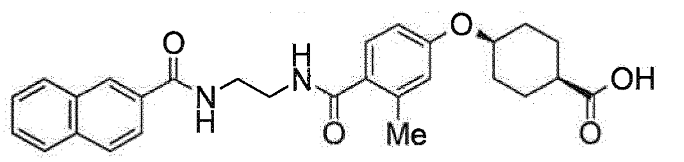 |
| 270 | 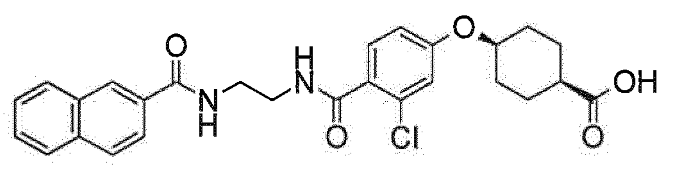 |
| 271 | 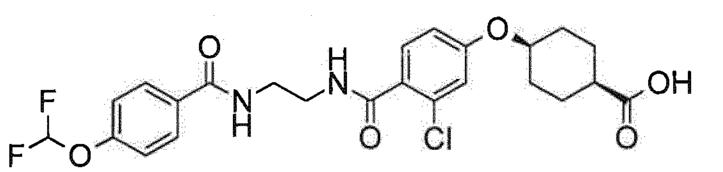 |

[Table 79]

| Ex | Structure |
|---|---|
| 272 | 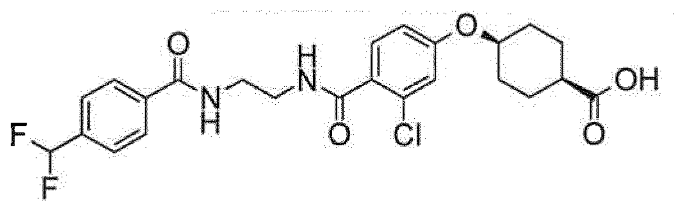 |

(continued)

| Ex | Structure |
|---|---|
| 273 | 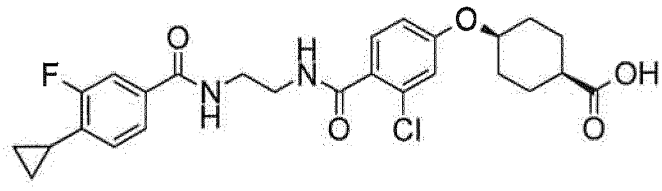 |
| 274 | 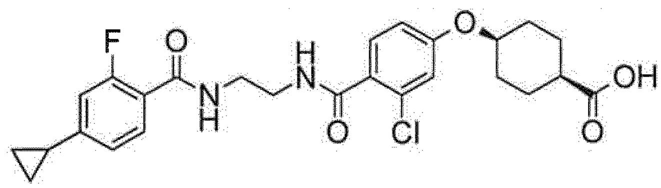 |
| 275 | 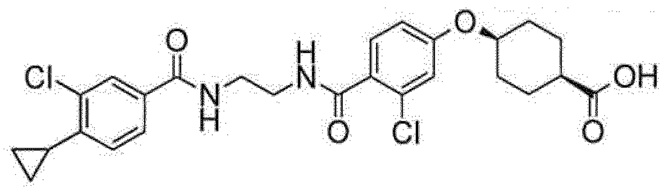 |
| 276 | 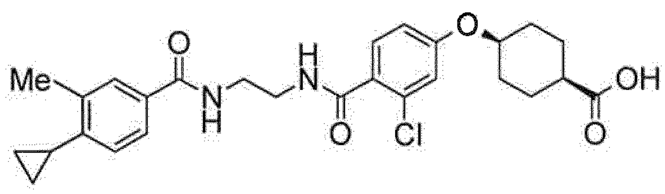 |
| 277 | 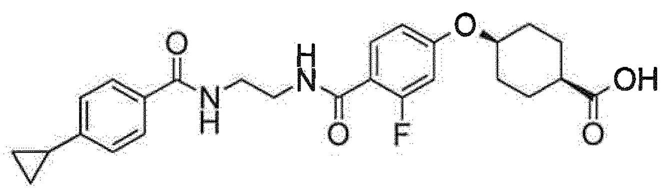 |
| 278 | 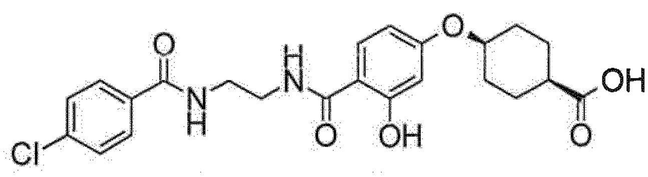 |

[Table 80]

| Ex | Structure |
|---|---|
| 279 | 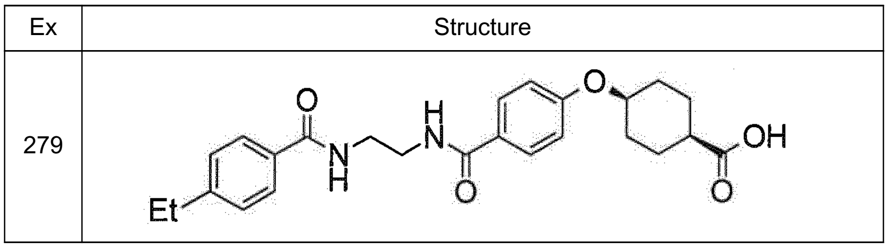 |

(continued)

| Ex | Structure |
|---|---|
| 280 | 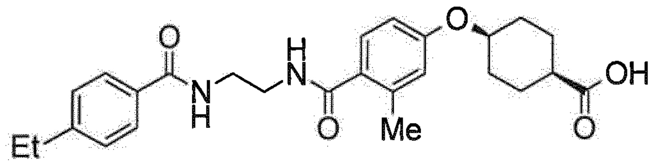 |
| 281 | 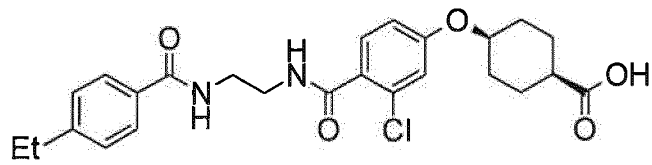 |
| 282 | 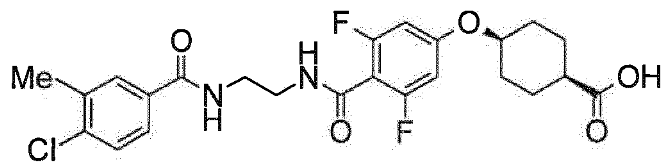 |
| 283 | 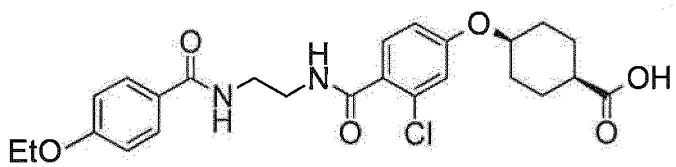 |
| 284 | 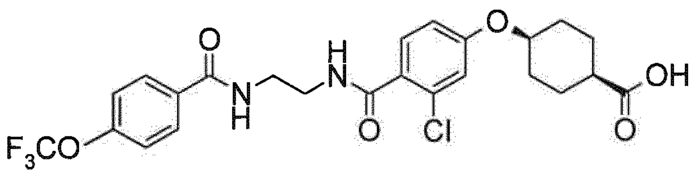 |
| 285 | 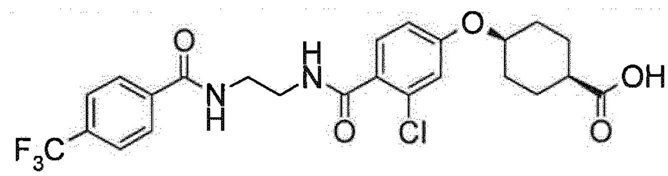 |
| 286 | 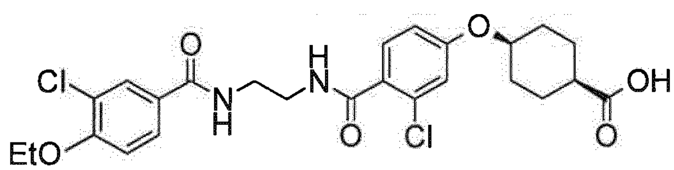 |

[Table 81]

| Ex | Structure |
|---|---|
| 287 | 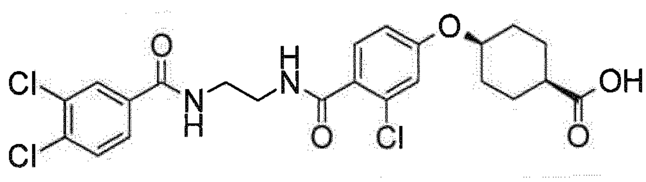 |

(continued)

| Ex | Structure |
|---|---|
| 288 | 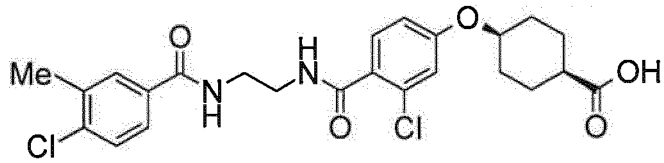 |
| 289 | 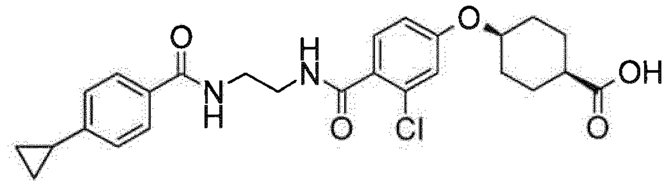 |
| 290 | 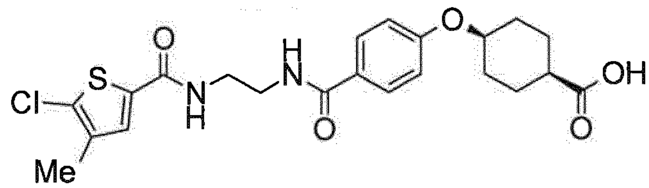 |
| 291 | 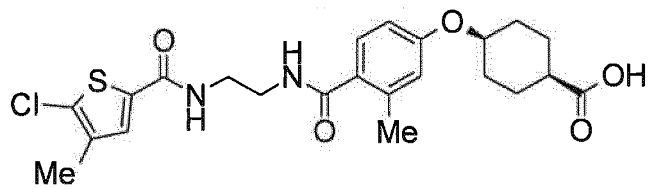 |
| 292 | 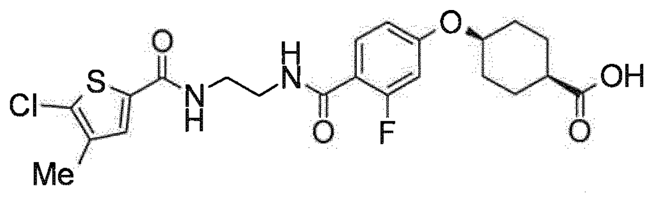 |
| 293 | 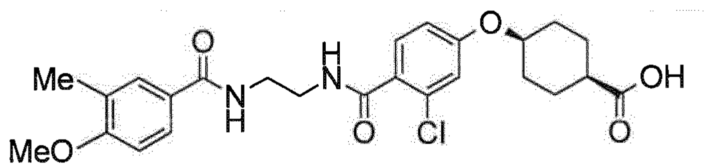 |
| 294 | 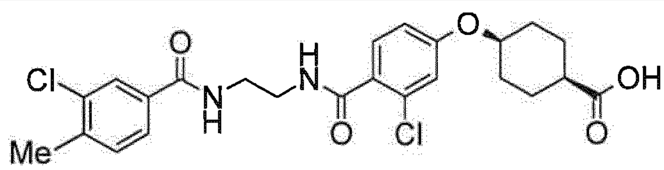 |

[Table 82]

| Ex | Structure |
|---|---|
| 295 | 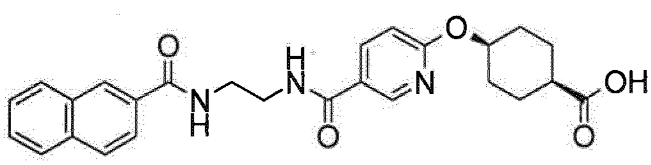 |

(continued)

| Ex | Structure |
|---|---|
| 296 | 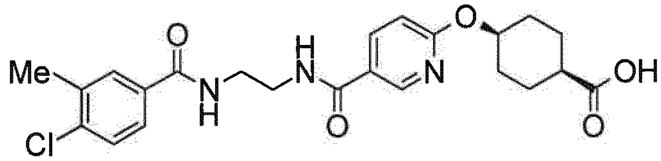 |
| 297 | 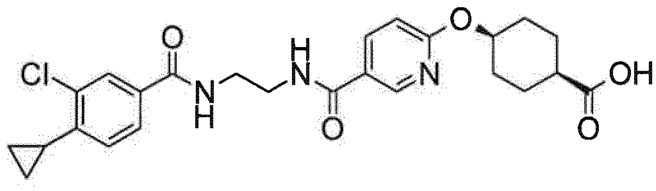 |
| 298 | 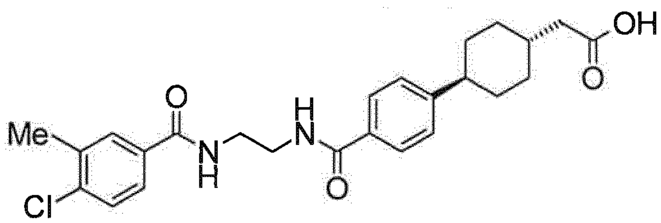 |
| 299 | 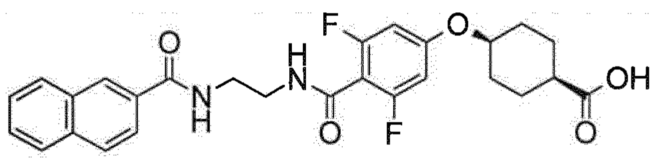 |
| 300 | 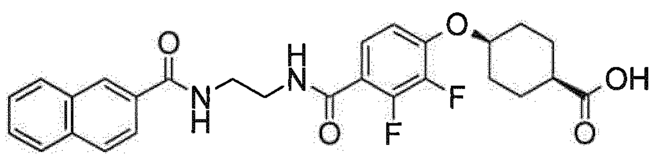 |
| 301 | 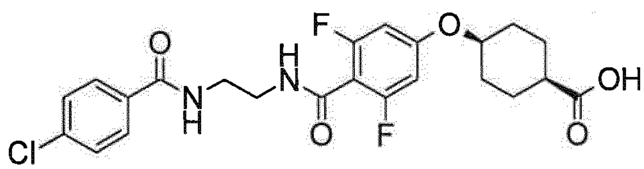 |
| 302 | 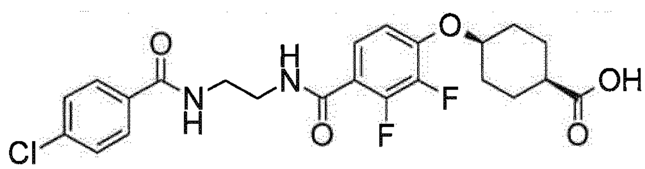 |

[Table 83]

| Ex | Structure |
|---|---|
| 303 | 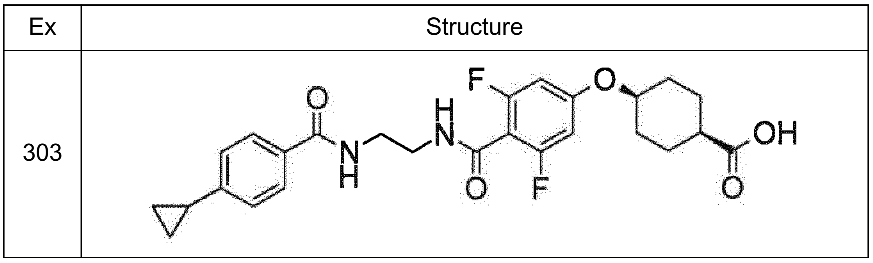 |

(continued)

| Ex | Structure |
|---|---|
| 304 | 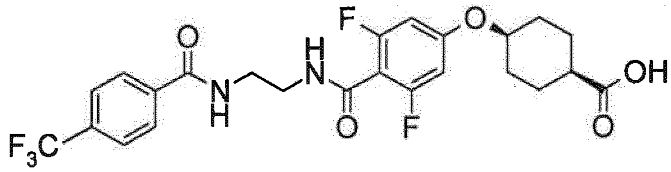 |
| 305 | 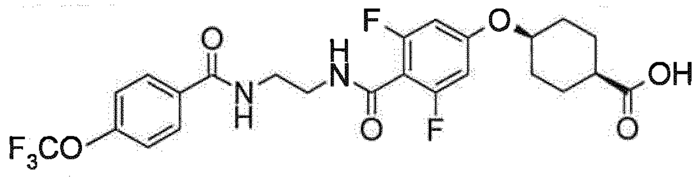 |
| 306 | 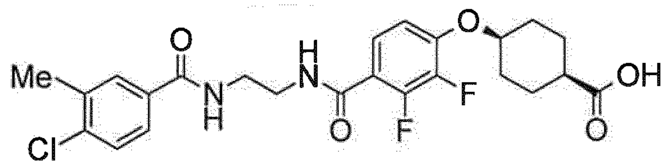 |
| 307 | 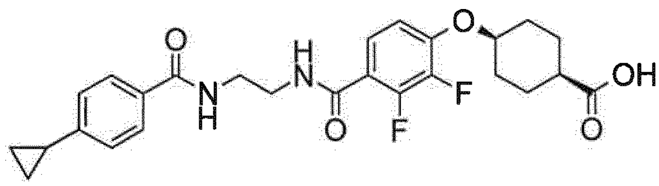 |
| 308 | 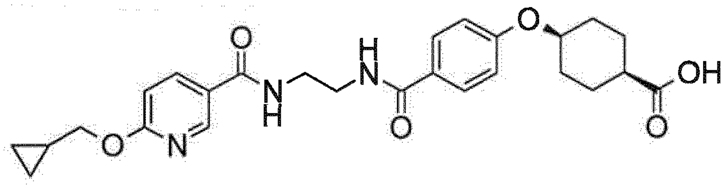 |
| 309 | 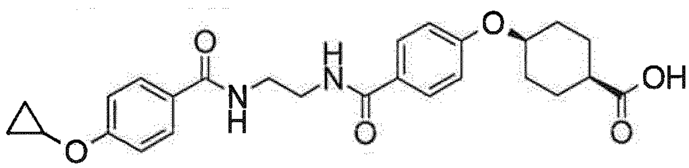 |

[Table 84]

| Ex | Structure |
|---|---|
| 310 | 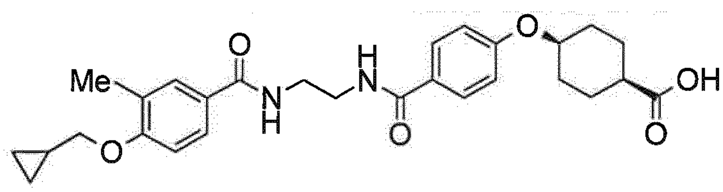 |

(continued)

| Ex | Structure |
|---|---|
| 311 | 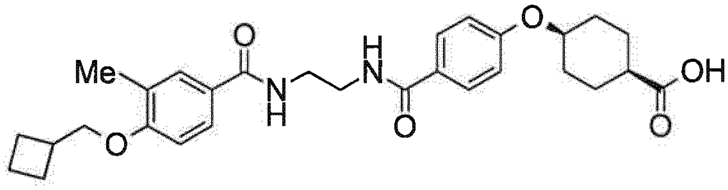 |
| 312 | 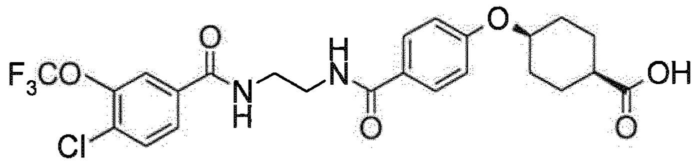 |
| 313 | 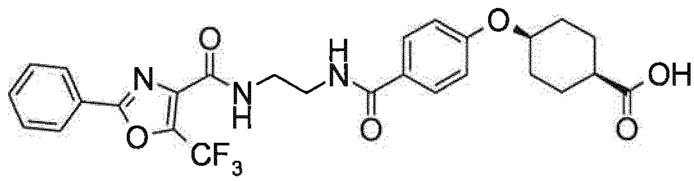 |
| 314 | 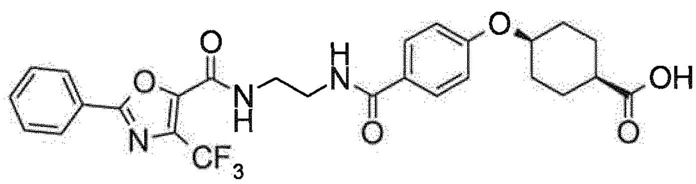 |
| 315 | 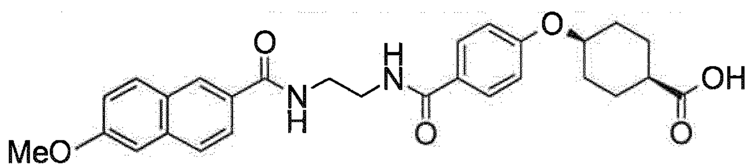 |
| 316 | 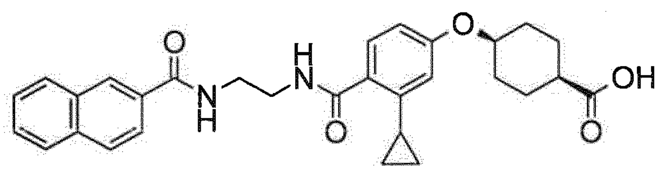 |
| 317* | 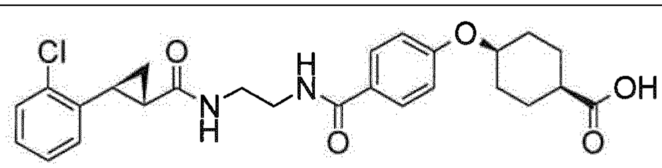 |

[Table 85]

| Ex | Structure |
|---|---|
| 318* | 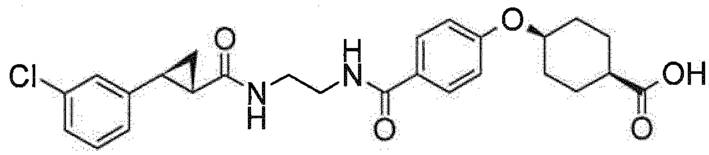 |

(continued)

| Ex | Structure |
|---|---|
| 319* | 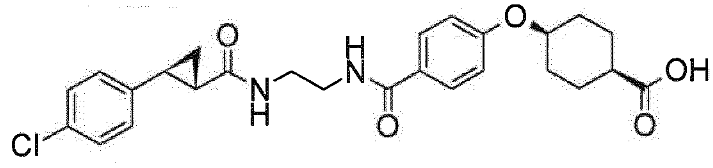 |
| 320* | 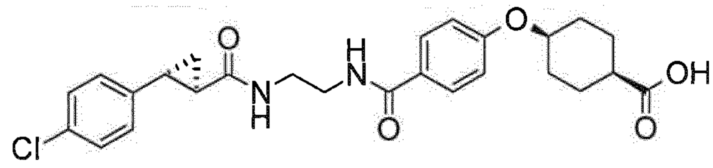 |
| 321 | 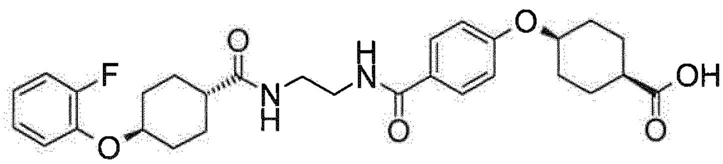 |
| 322 | 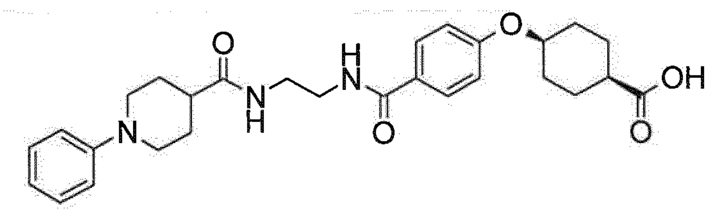 |
| 323 | 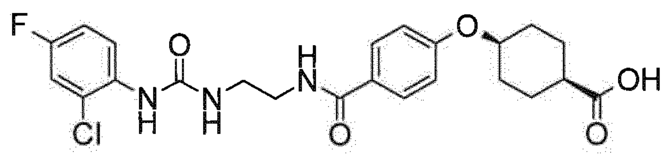 |
| 324 | 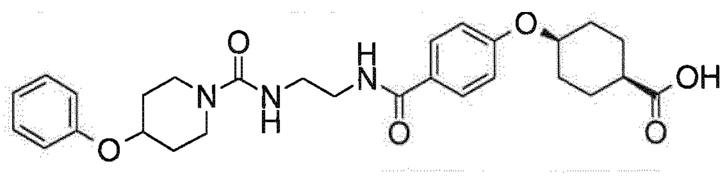 |
| 325 | 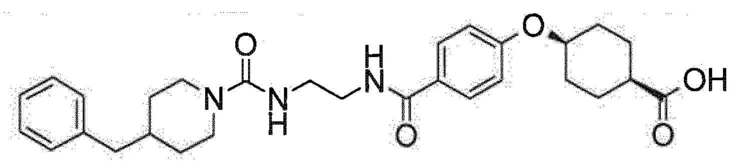 |

[Table 86]

| Ex | Structure |
|---|---|
| 326 | 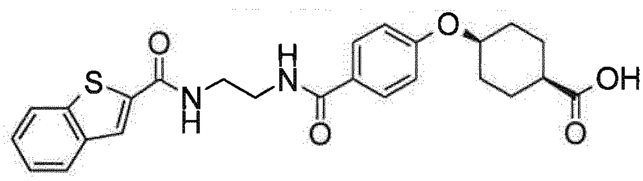 |

(continued)

| Ex | Structure |
|---|---|
| 327 | 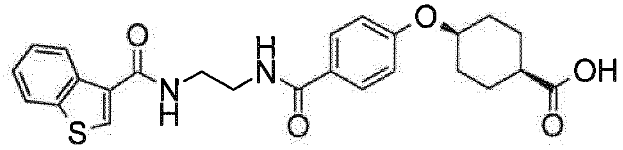 |
| 328 | 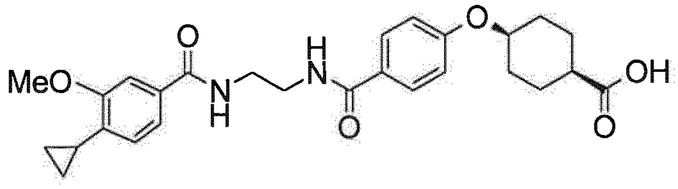 |
| 329 | 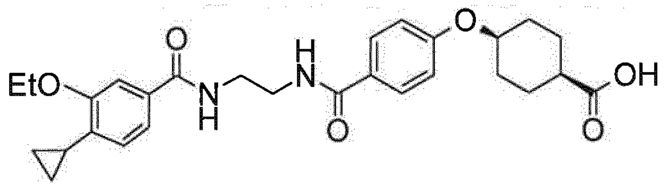 |
| 330 | 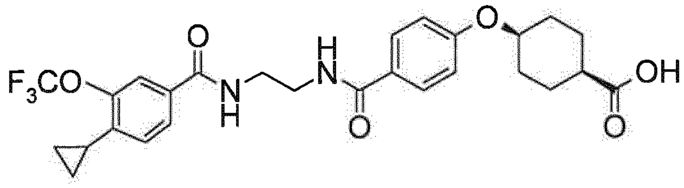 |
| 331 | 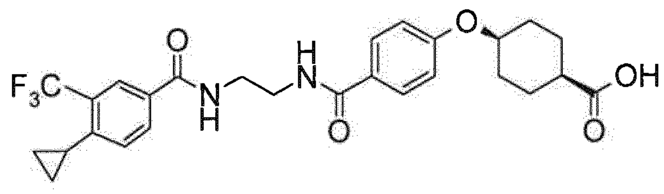 |
| 332 | 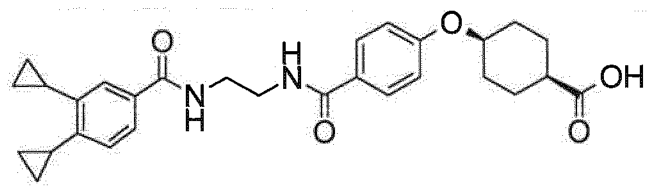 |

[Table 87]

| Ex | Structure |
|---|---|
| 333 | 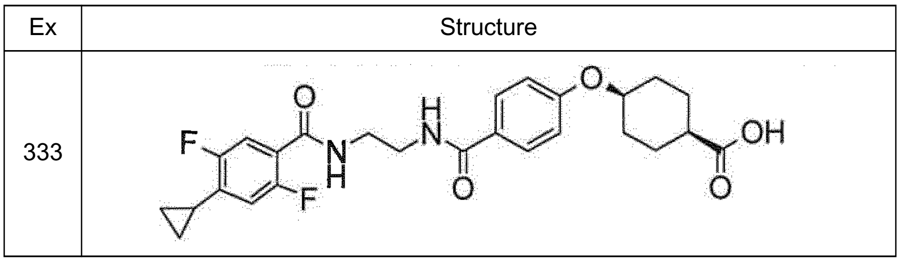 |

(continued)

| Ex | Structure |
|---|---|
| 334 | 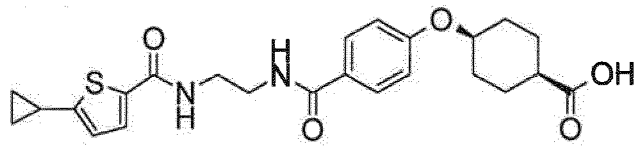 |
| 335 | 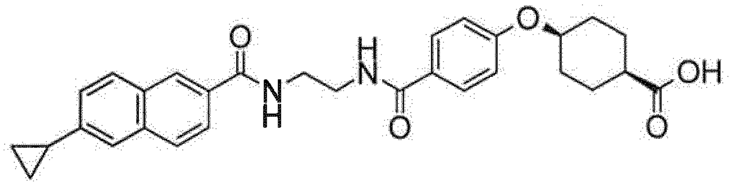 |
| 336 | 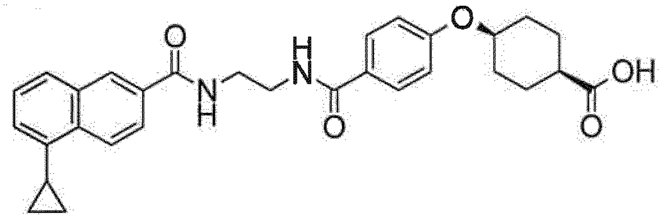 |
| 337 | 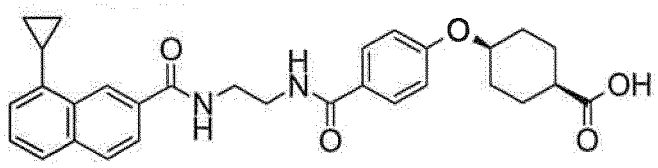 |
| 338 | 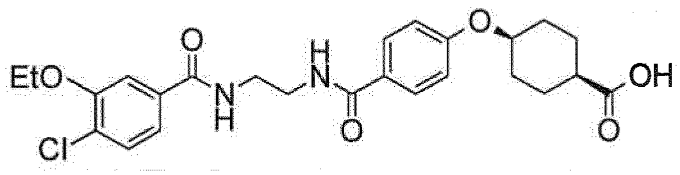 |
| 339 | 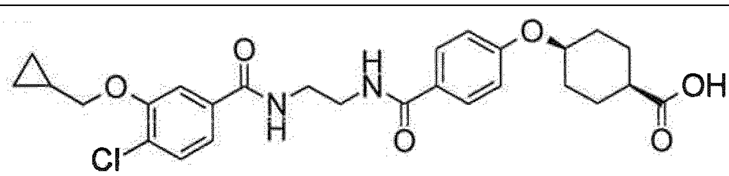 |

[Table 88]

| Ex | Structure |
|---|---|
| 340 | 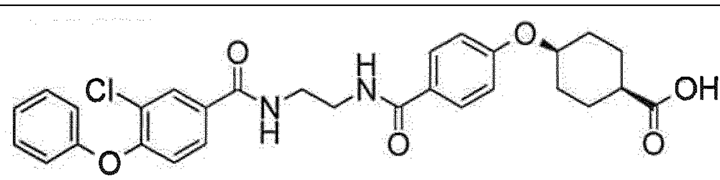 |
| 341 | 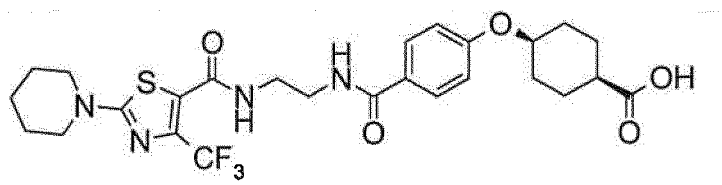 |

(continued)

| Ex | Structure |
|---|---|
| 342 | 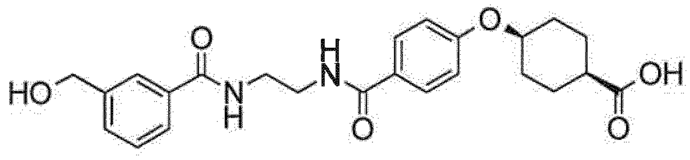 |
| 343 | 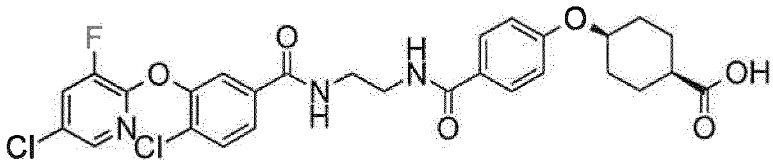 |
| 344 | 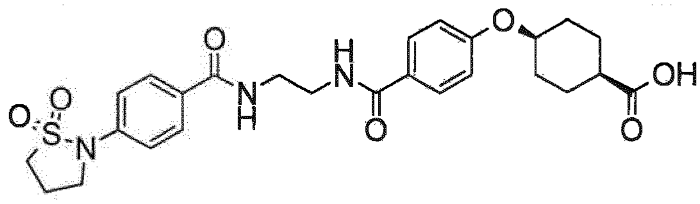 |
| 345 | 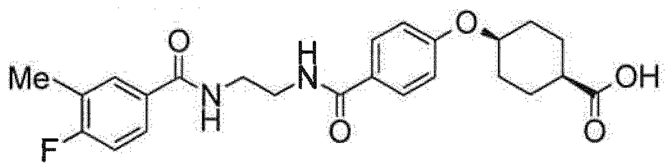 |
| 346 | 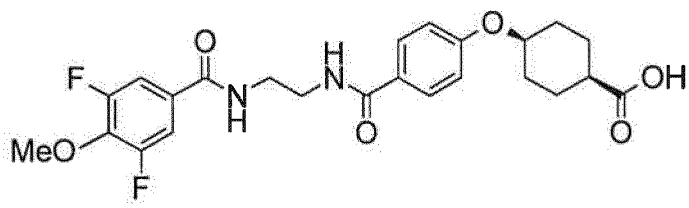 |

[Table 89]

| Ex | Structure |
|---|---|
| 347 | 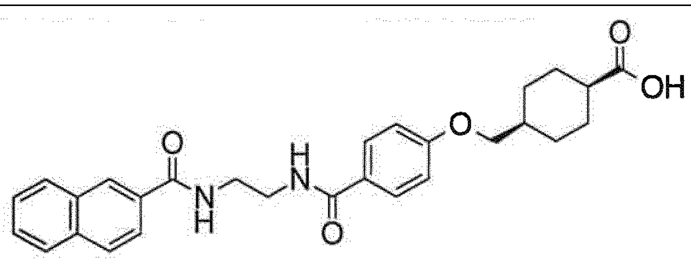 |
| 348 | 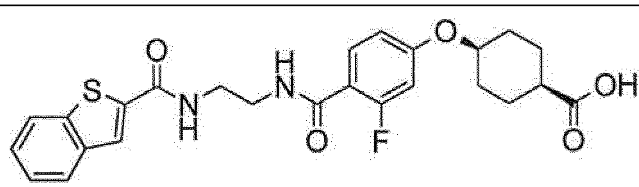 |

(continued)

| Ex | Structure |
|---|---|
| 349 | 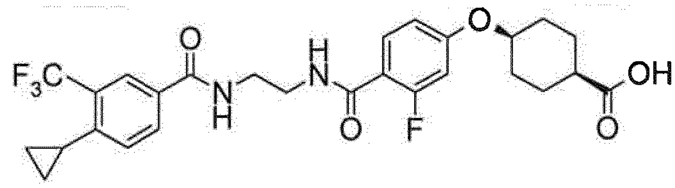 |
| 350 | 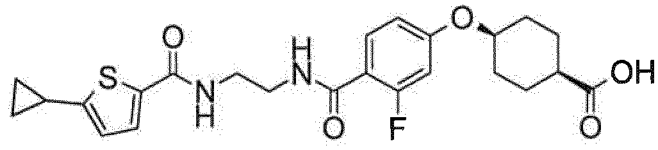 |
| 351 | 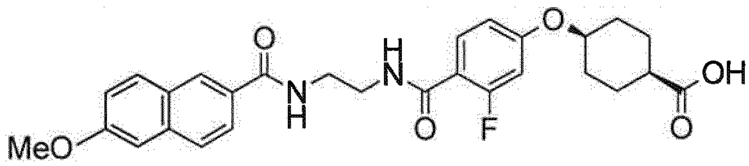 |
| 352 | 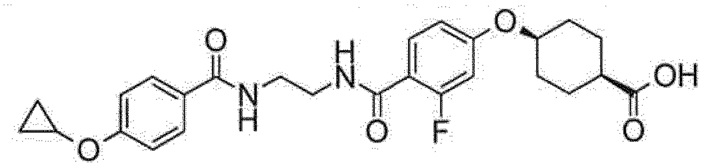 |
| 353 | 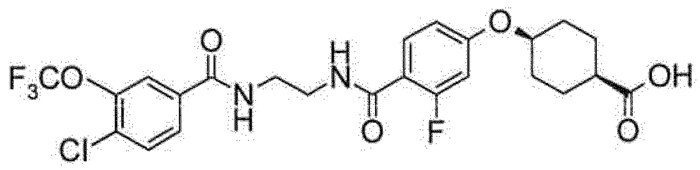 |

[Table 90]

| Ex | Structure |
|---|---|
| 354 | 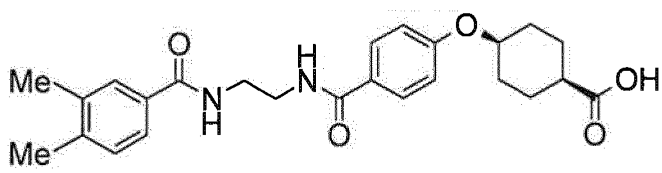 |
| 355 | 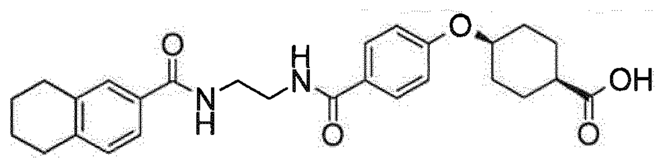 |
| 356 | 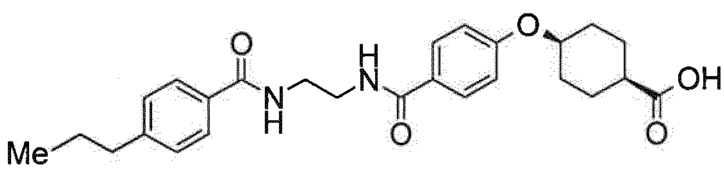 |

(continued)

| Ex | Structure |
|---|---|
| 357 | 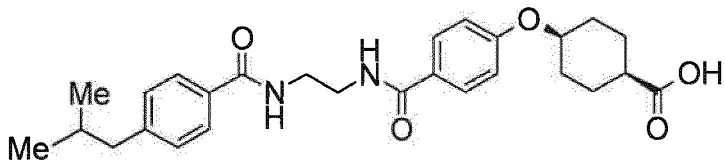 |
| 358 | 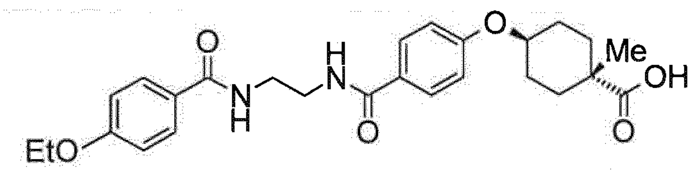 |
| 359 | 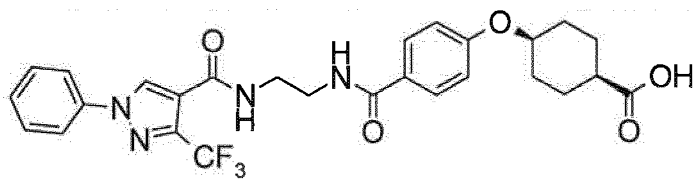 |
| 360 | 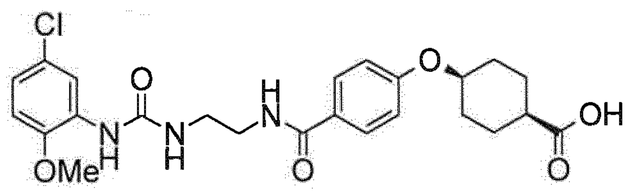 |
| 361 | 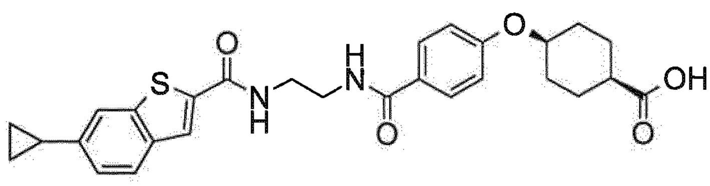 |

[Table 91]

| Ex | Structure |
|---|---|
| 362 | 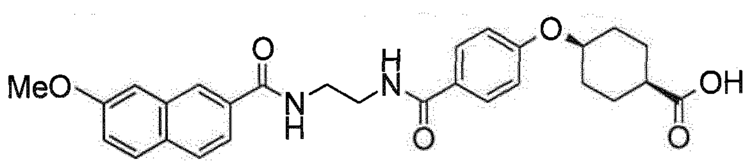 |
| 363 | 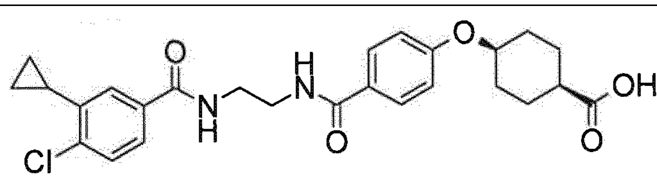 |

(continued)

| Ex | Structure |
|---|---|
| 364 | 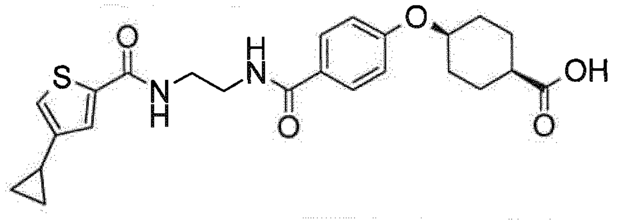 |
| 365 | 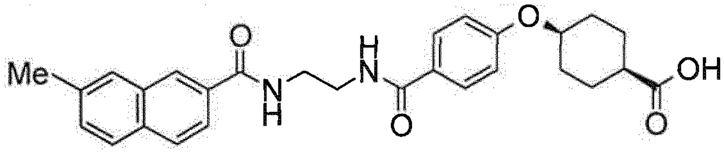 |
| 366 | 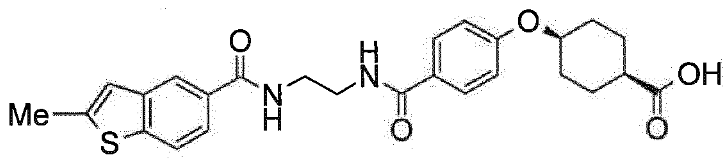 |
| 367 | 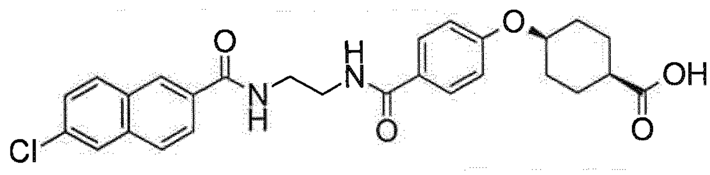 |
| 368 | 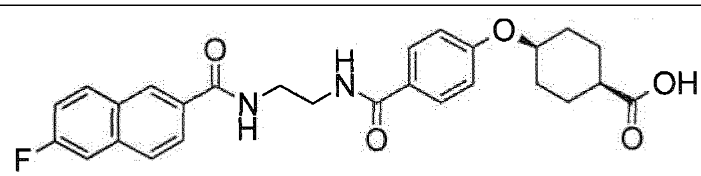 |
| 369 | 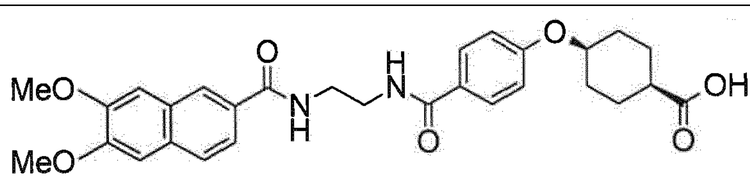 |

[Table 92]

| Ex | Structure |
|---|---|
| 370 | 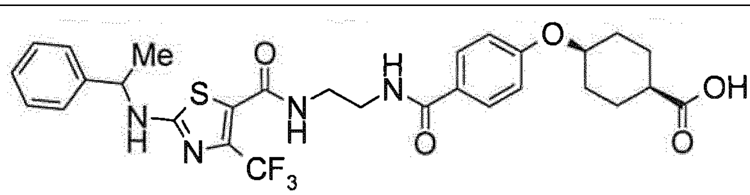 |
| 371 | 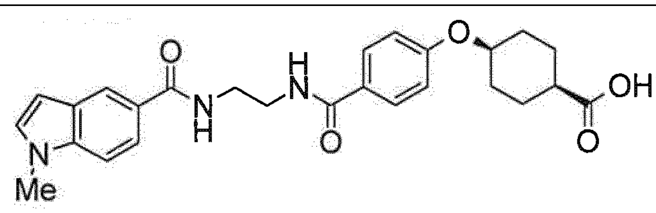 |

(continued)

| Ex | Structure |
|---|---|
| 372 | 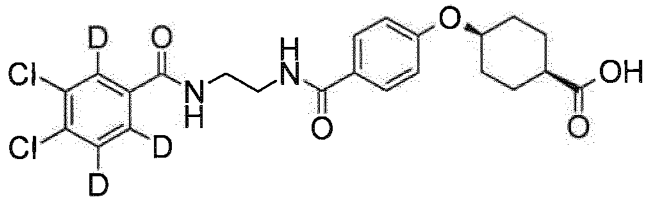 |
| 373 | 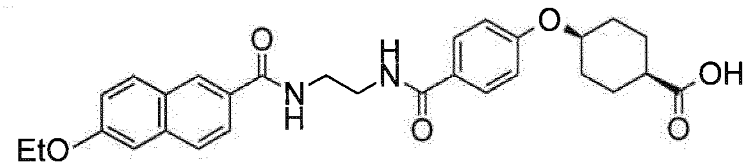 |
| 374 | 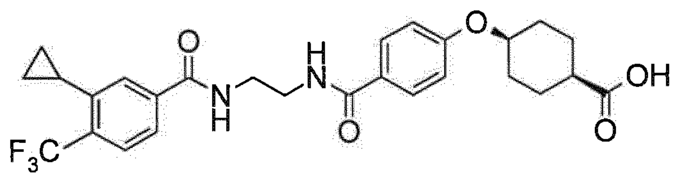 |
| 375 | 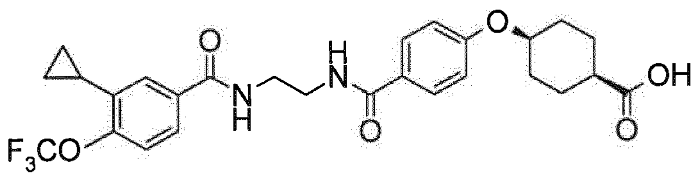 |
| 376 | 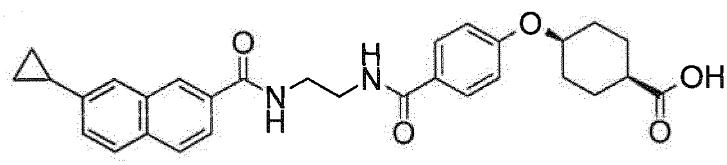 |

[Table 93]

| Ex | Structure |
|---|---|
| 377 | 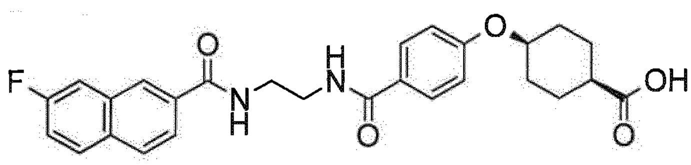 |
| 378 | 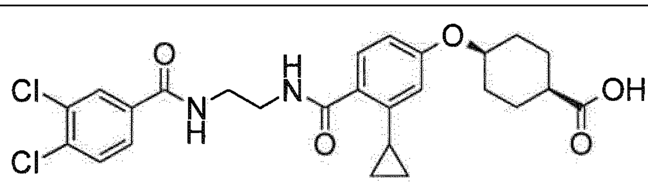 |
| 379 | 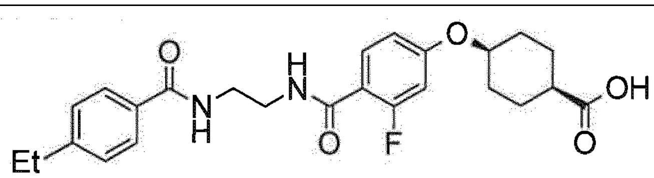 |

(continued)

| Ex | Structure |
|---|---|
| 380 | 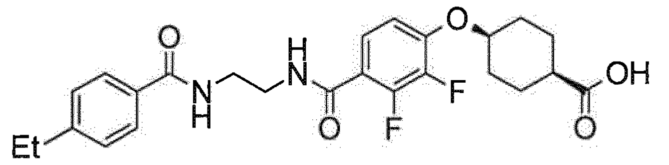 |
| 381 | 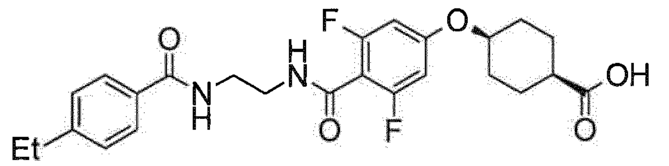 |
| 382 | 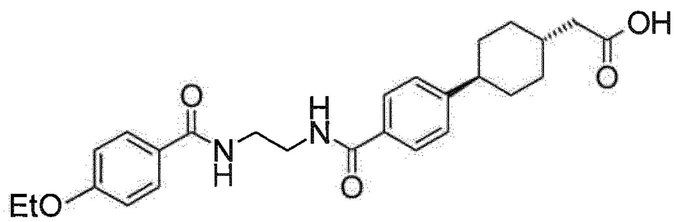 |
| 383 | 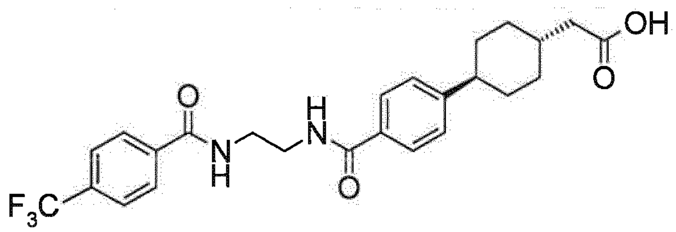 |

[Table 94]

| Ex | Structure |
|---|---|
| 384 | 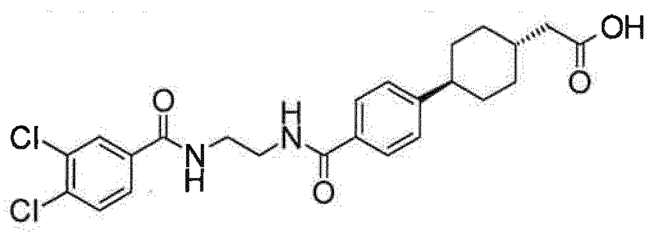 |
| 385 | 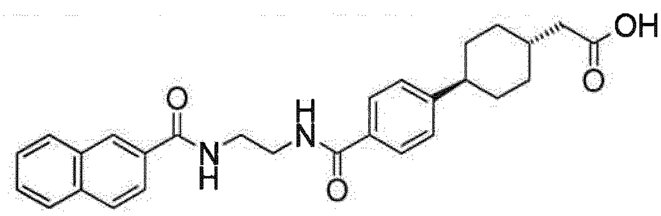 |

(continued)

| Ex | Structure |
|---|---|
| 386 | 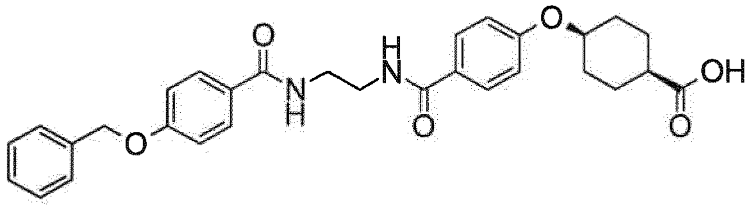 |
| 387 | 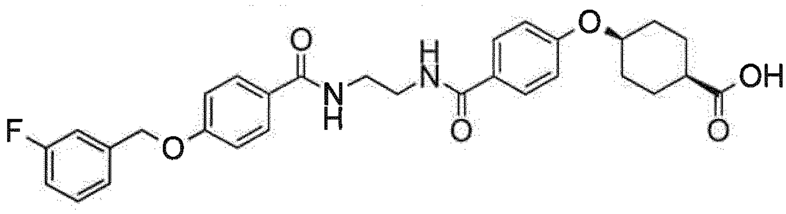 |
| 388 | 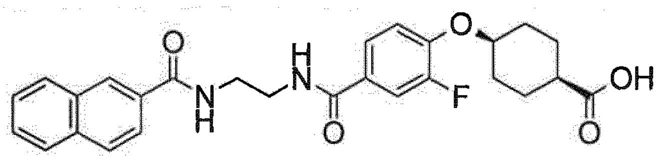 |
| 389 | 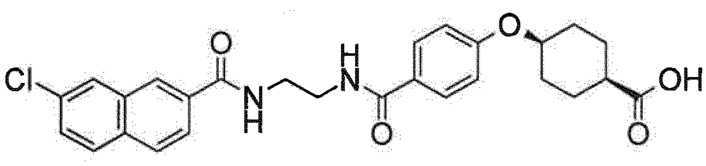 |

[Table 95]

| Ex | Structure |
|---|---|
| 390 | 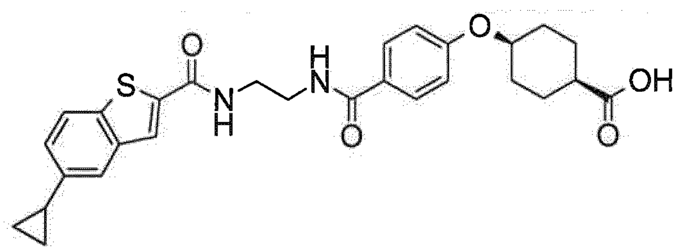 |
| 391 | 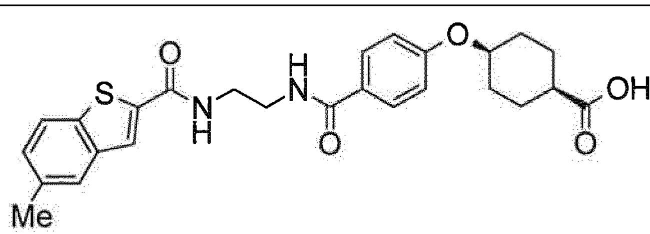 |
| 392 | 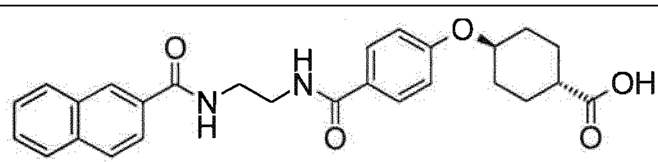 |

(continued)

| Ex | Structure |
|---|---|
| 393 | 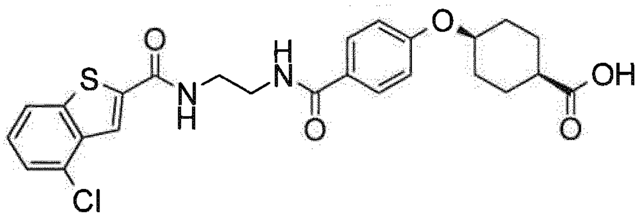 |
| 394 | 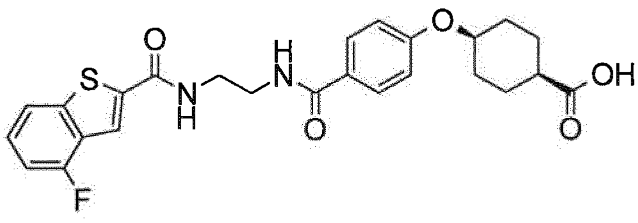 |
| 395 | 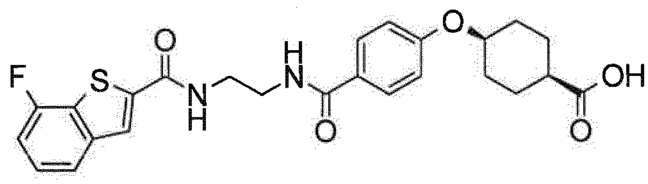 |
| 396 | 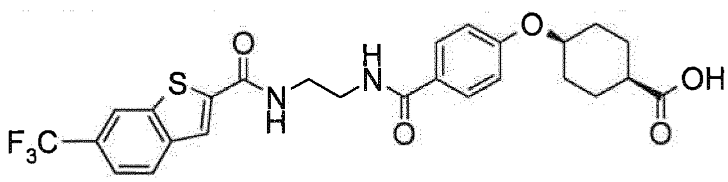 |

[Table 96]

| Ex | Structure |
|---|---|
| 397 | 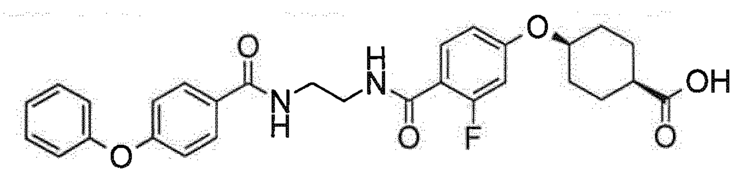 |
| 398 | 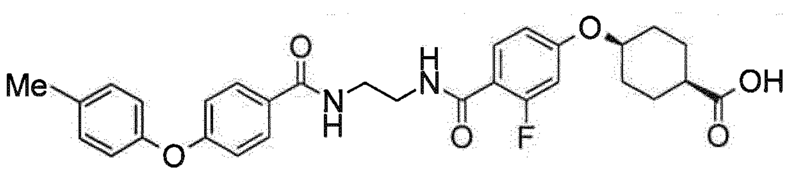 |
| 399 | 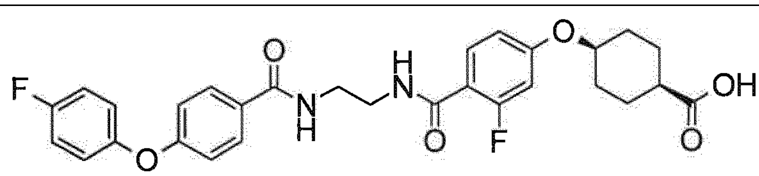 |

(continued)

| Ex | Structure |
|---|---|
| 400 | 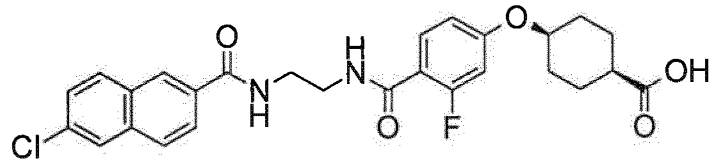 |
| 401 | 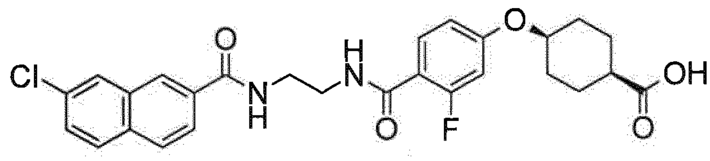 |
| 402 | 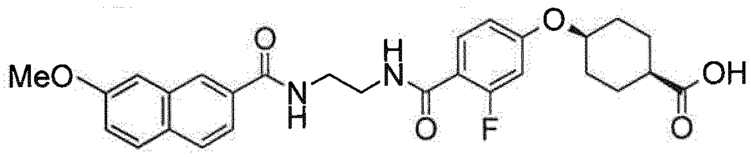 |
| 403 | 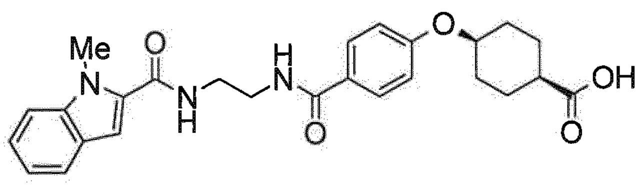 |
| 404 | 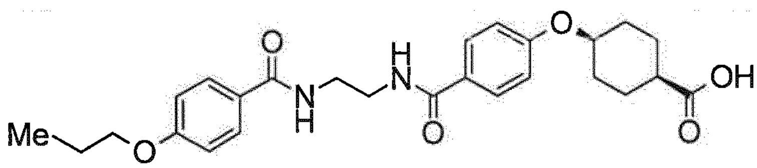 |

[Table 97]

| Ex | Structure |
|---|---|
| 405 | 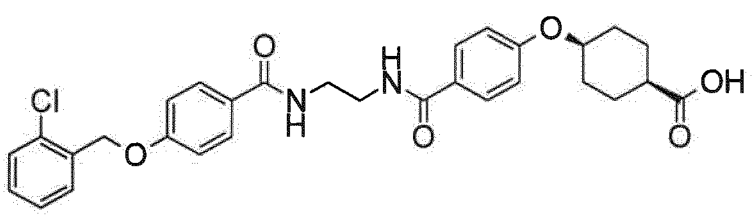 |
| 406 | 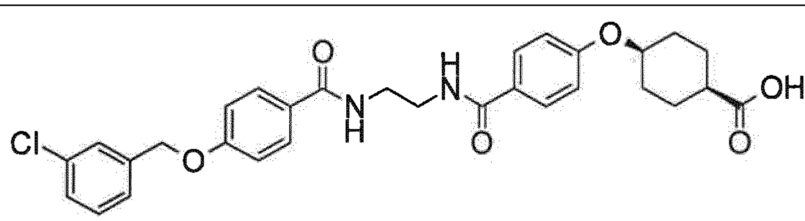 |

(continued)

| Ex | Structure |
|---|---|
| 407 | 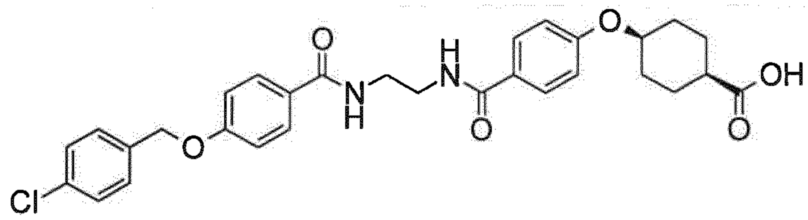 |
| 408* | 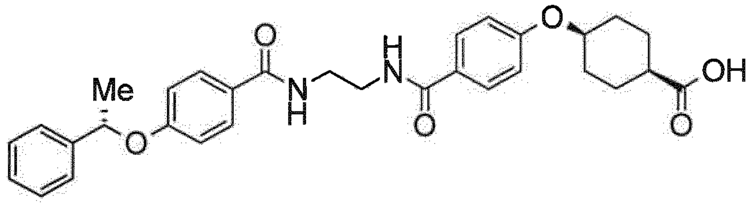 |
| 409* | 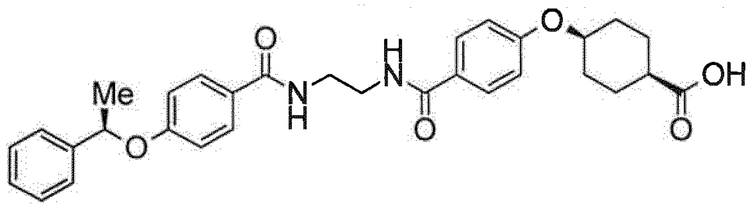 |
| 410 | 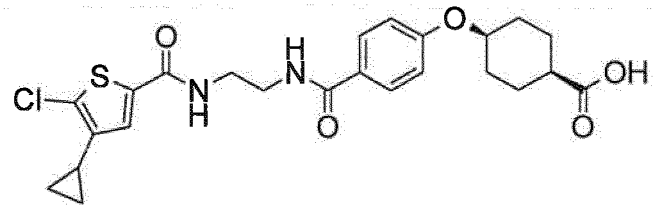 |

[Table 98]

| Ex | Structure |
|---|---|
| 411 | 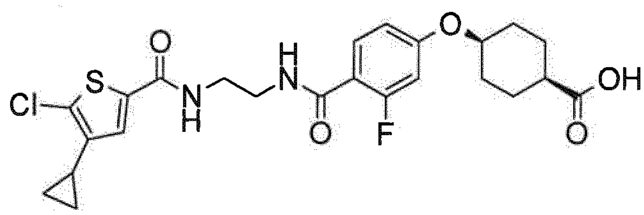 |
| 412 | 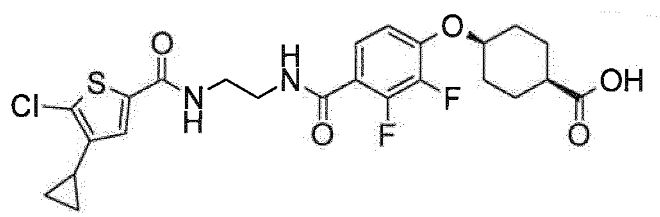 |

(continued)

| Ex | Structure |
|---|---|
| 413 | 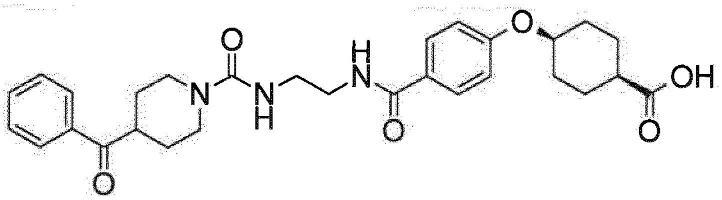 |
| 414 | 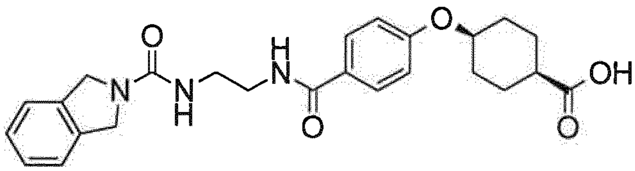 |
| 415 | 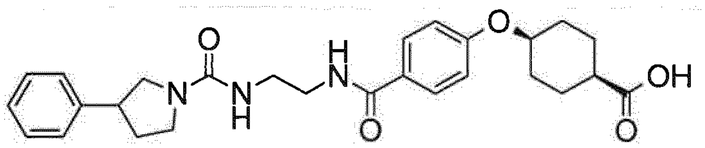 |
| 416 | 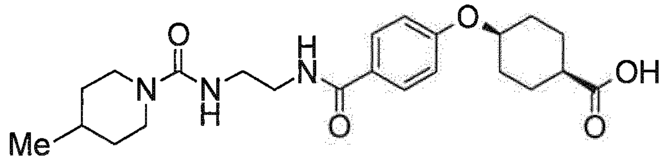 |
| 417* | 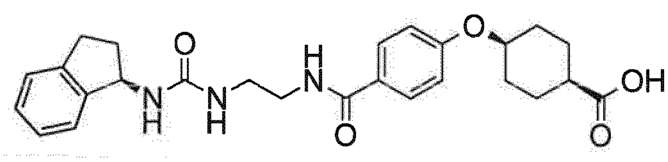 |
| 418 | 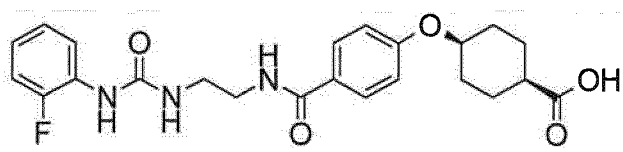 |

[Table 99]

| Ex | Structure |
|---|---|
| 419 | 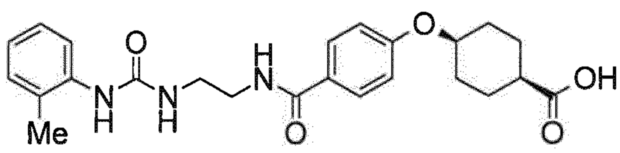 |
| 420 | 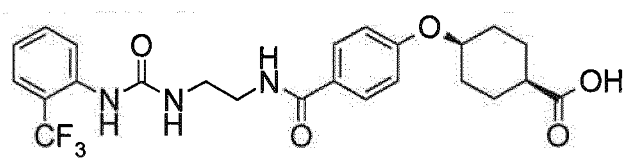 |

(continued)

| Ex | Structure |
|---|---|
| 421 | 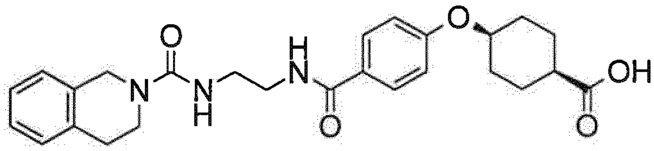 |
| 422 | 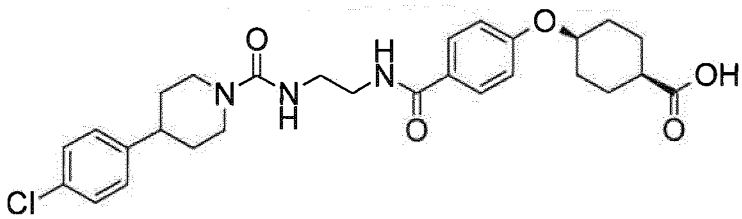 |
| 423 | 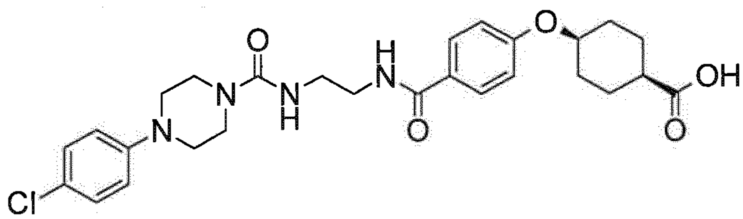 |
| 424 | 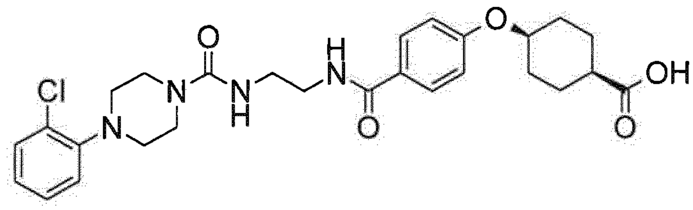 |
| 425 | 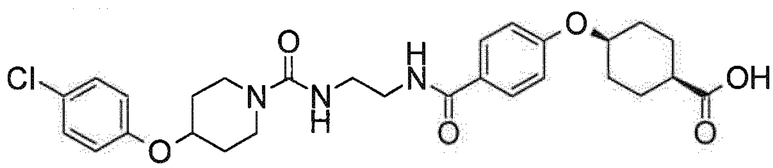 |

[Table 100]

| Ex | Structure |
|---|---|
| 426 | 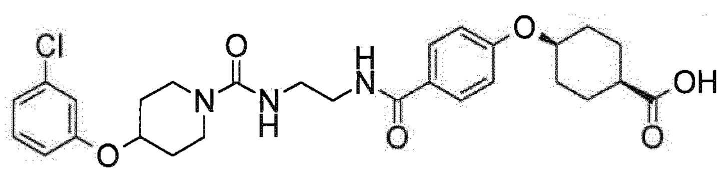 |
| 427 | 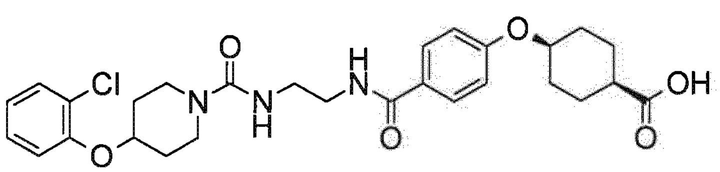 |

(continued)

| Ex | Structure |
|---|---|
| 428 | 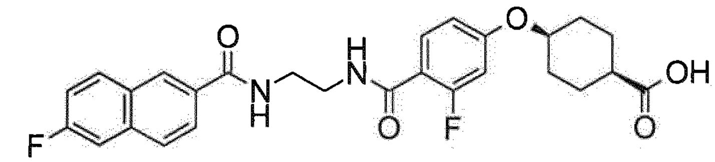 |
| 429 | 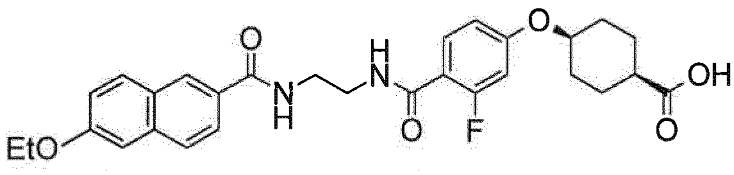 |
| 430 | 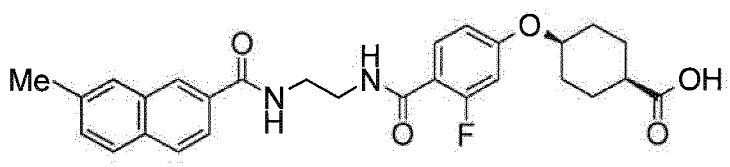 |
| 431 | 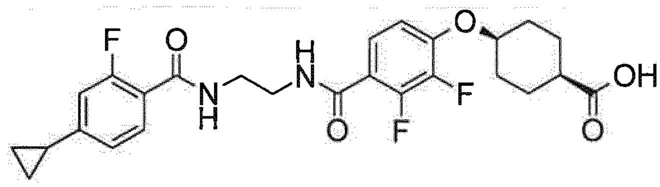 |
| 432 | 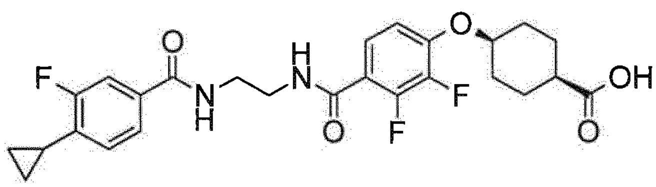 |
| 433 | 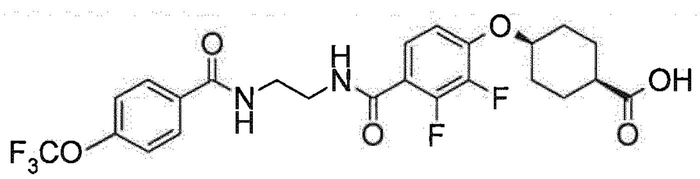 |

[Table 101]

| Ex | Structure |
|---|---|
| 434 | 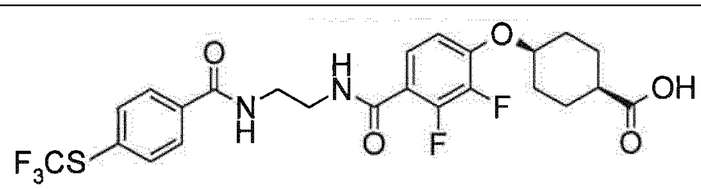 |
| 435 | 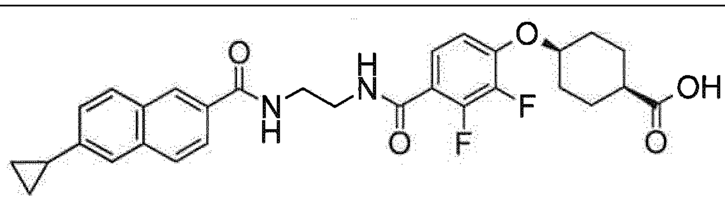 |

(continued)

| Ex | Structure |
|---|---|
| 436 | 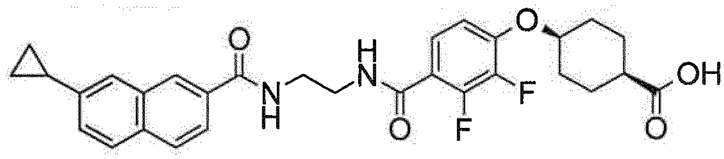 |
| 437 | 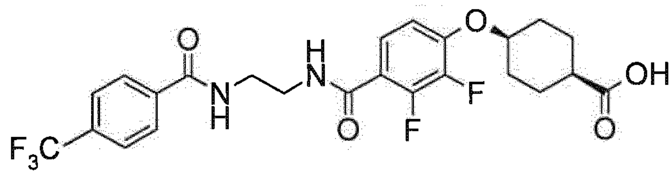 |
| 438 | 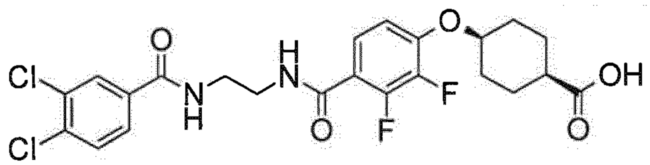 |
| 439 | 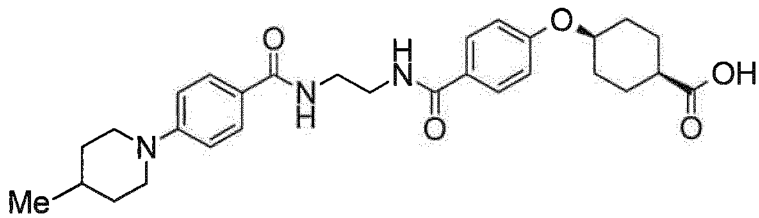 |
| 440 | 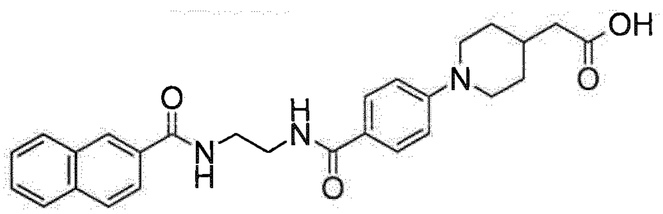 |

[Table 102]

| Ex | Structure |
|---|---|
| 441 | 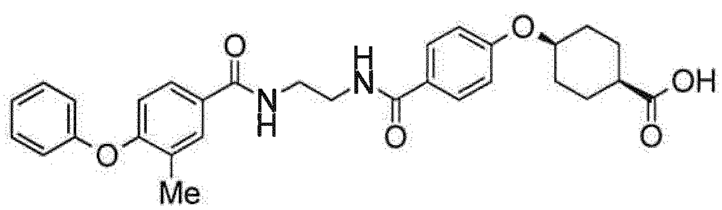 |
| 442 | 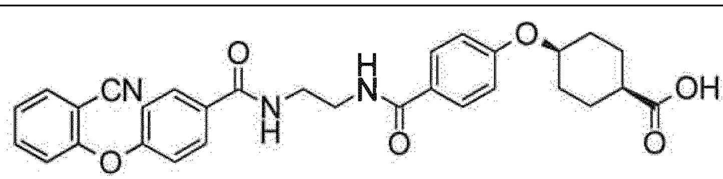 |

(continued)

| Ex | Structure |
|---|---|
| 443 | 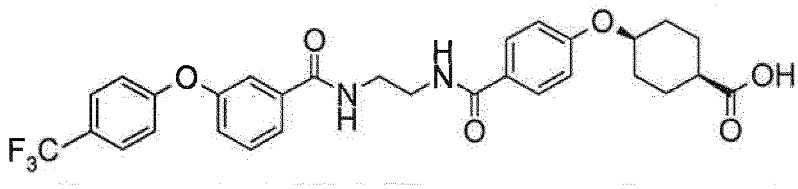 |
| 444 | 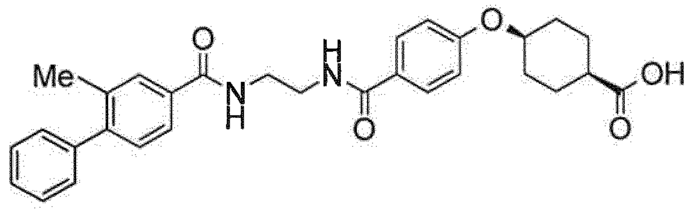 |
| 445 | 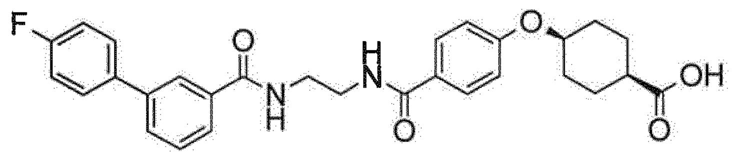 |
| 446 | 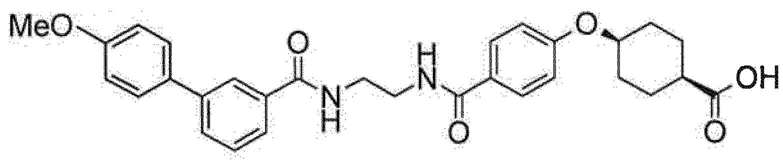 |
| 447 | 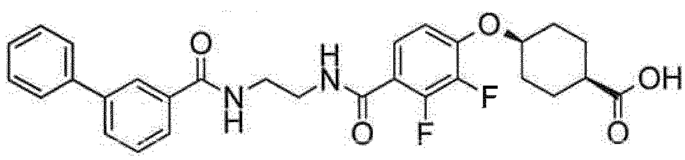 |

[Table 103]

| Ex | Structure |
|---|---|
| 448 | 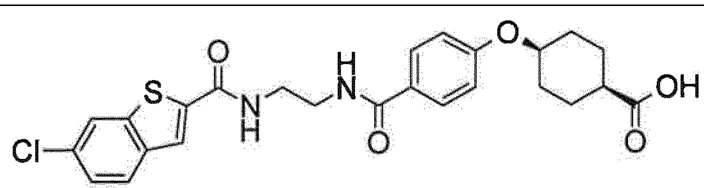 |
| 449 | 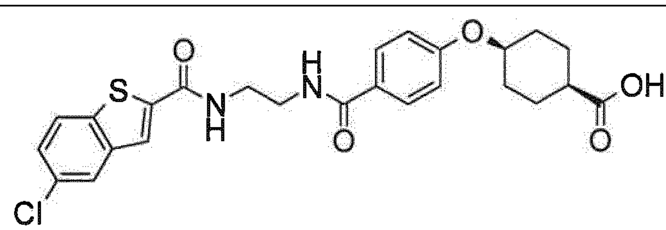 |

(continued)

| Ex | Structure |
|---|---|
| 450 | 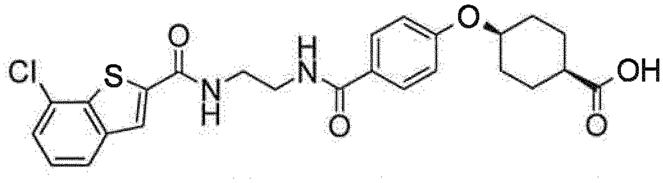 |
| 451 | 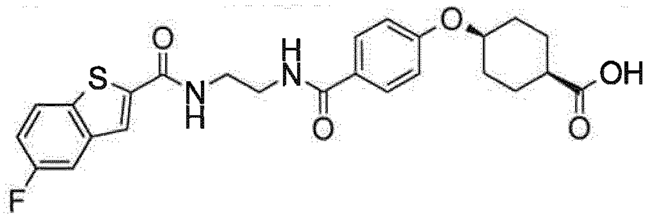 |
| 452 | 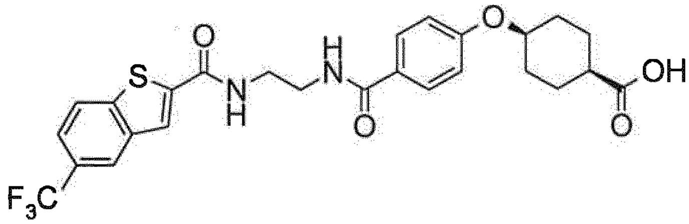 |
| 453 | 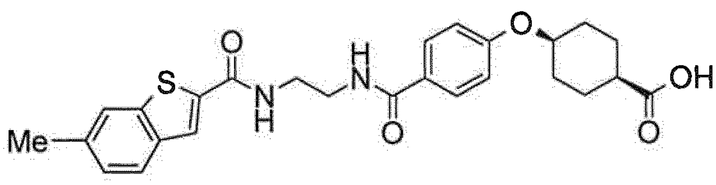 |

[Table 104]

| Ex | Structure |
|---|---|
| 454 | 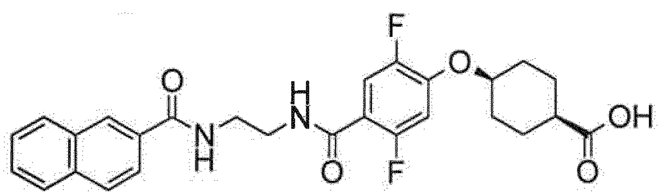 |
| 455 | 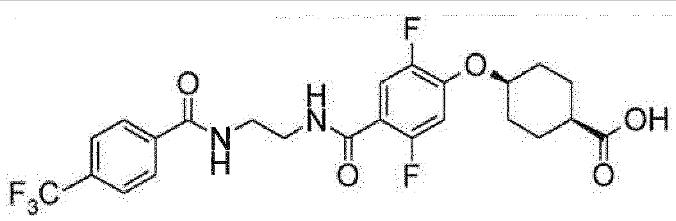 |
| 456 | 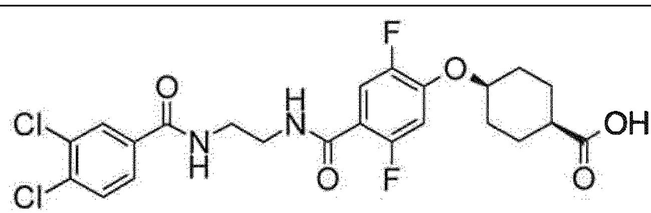 |

(continued)

| Ex | Structure |
|---|---|
| 457 | 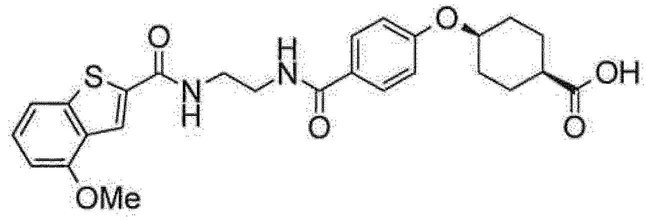 |
| 458 | 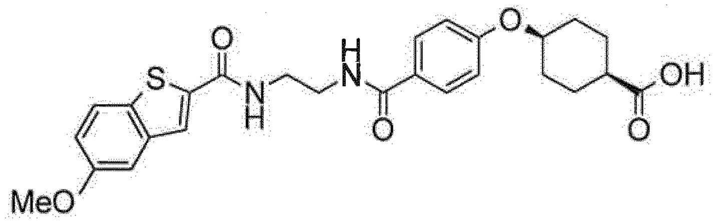 |
| 459 | 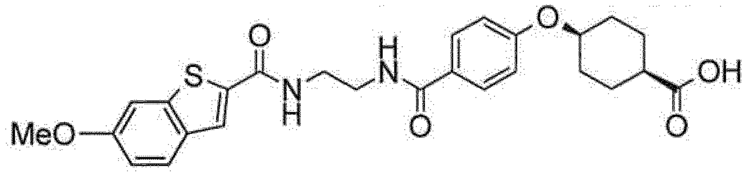 |

[Table 105]

| Ex | Structure |
|---|---|
| 460 | 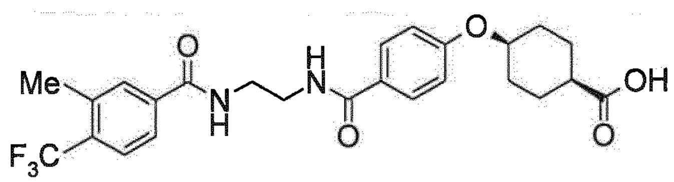 |
| 461 | 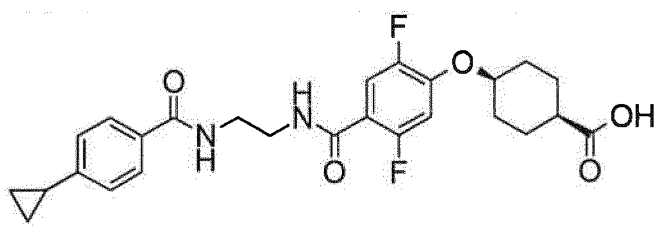 |
| 462 | 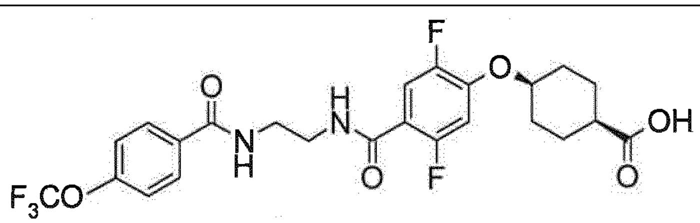 |
| 463 | 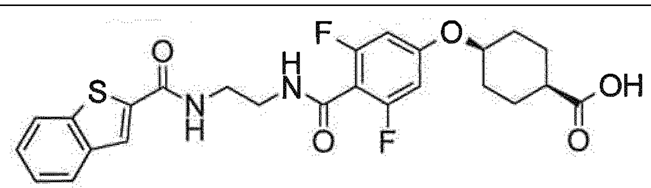 |

(continued)

| Ex | Structure |
|---|---|
| 464 | 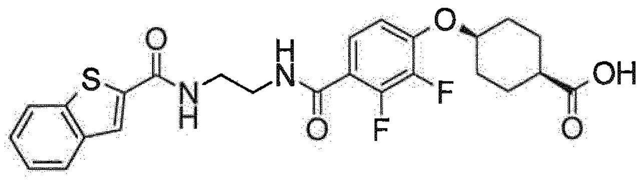 |
| 465 | 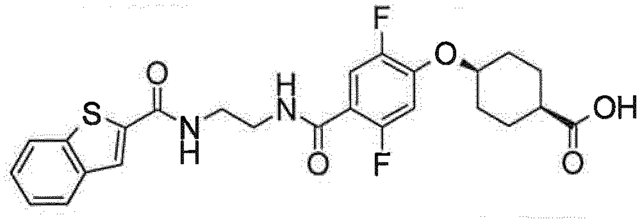 |
| 466 | 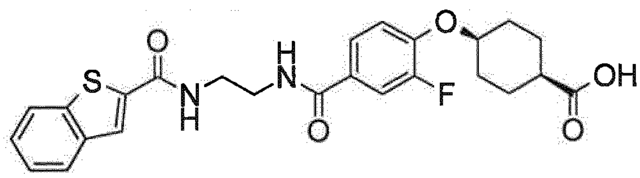 |

[Table 106]

| Ex | Structure |
|---|---|
| 467 | 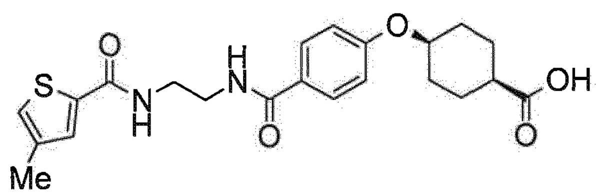 |
| 468 | 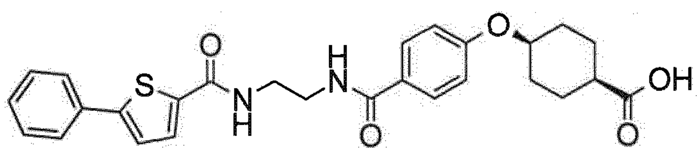 |
| 469 | 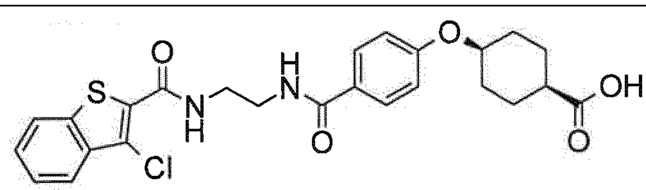 |
| 470 | 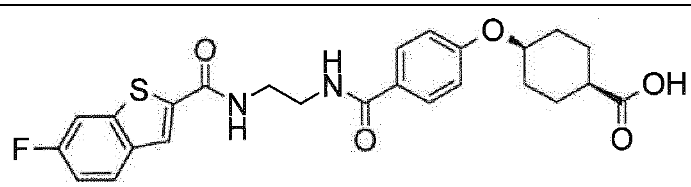 |

(continued)

| Ex | Structure |
|---|---|
| 471 | 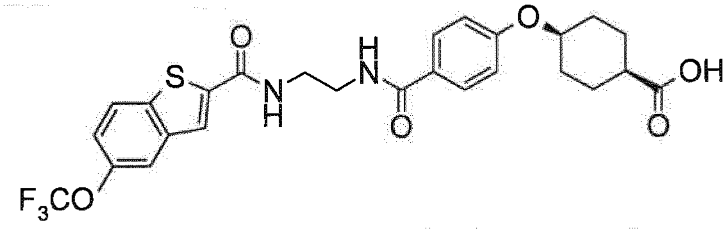 |
| 472 | 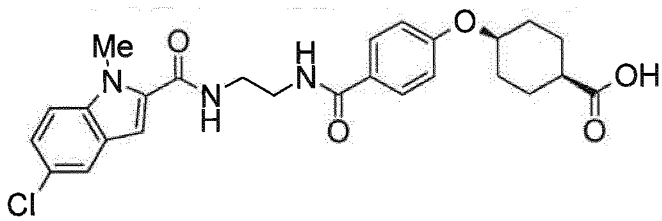 |
| 473 | 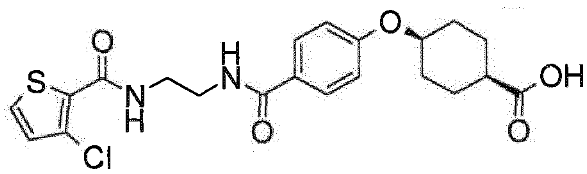 |

[Table 107]

| Ex | Structure |
|---|---|
| 474 | 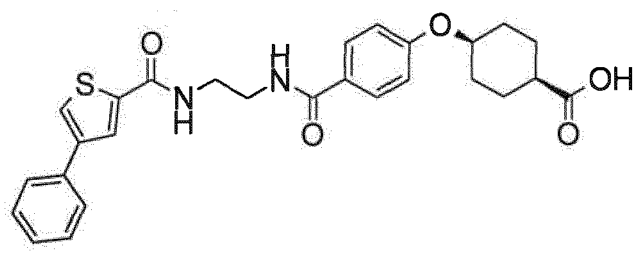 |
| 475 | 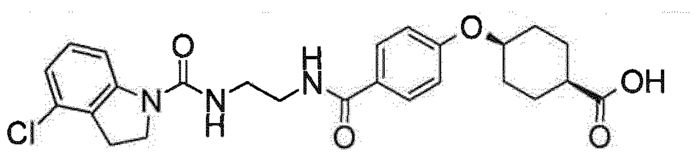 |
| 476 | 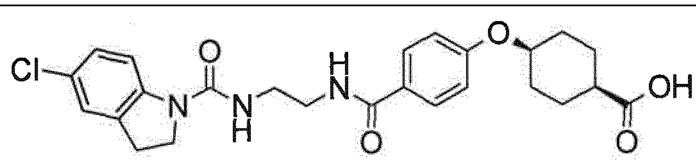 |
| 477 | 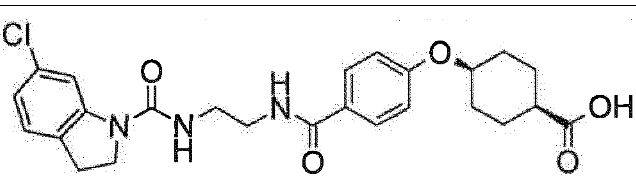 |

(continued)

| Ex | Structure |
|---|---|
| 478 | 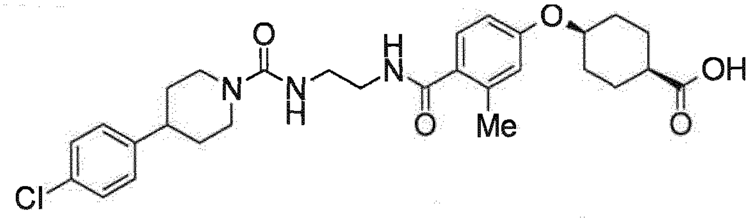 |
| 479 | 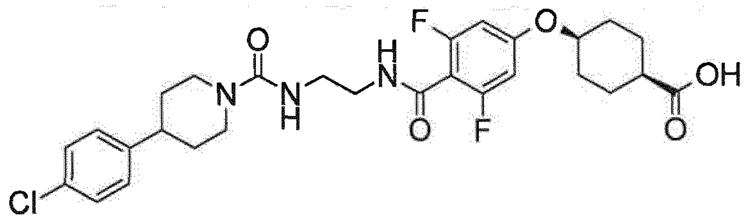 |

[Table 108]

| Ex | Structure |
|---|---|
| 480 | 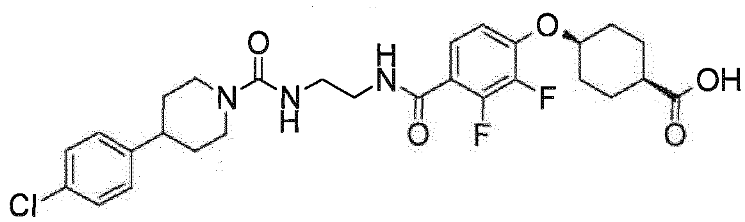 |
| 481 | 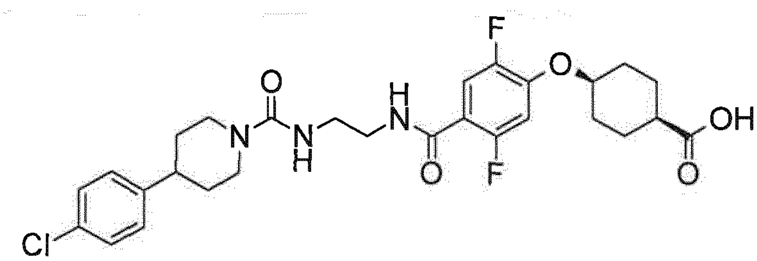 |
| 482 | 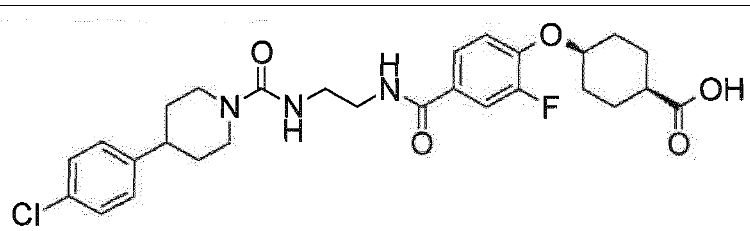 |
| 483 | 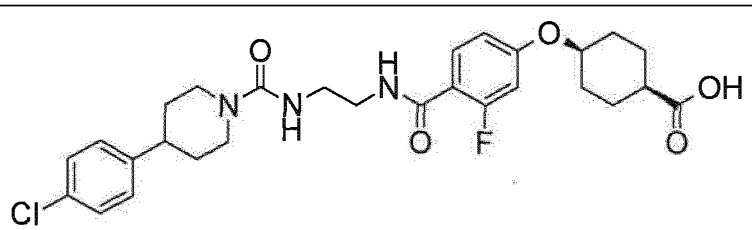 |

(continued)

| Ex | Structure |
|---|---|
| 484 | 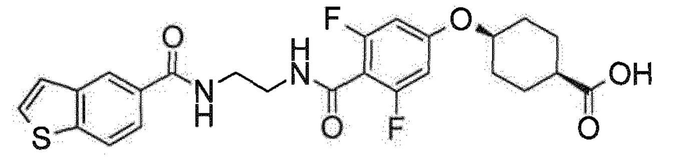 |
| 485 | 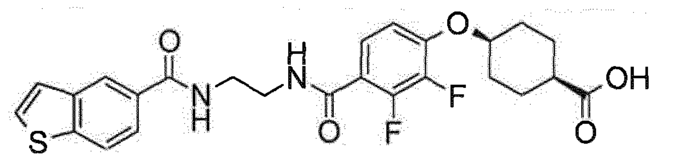 |
| 486 | 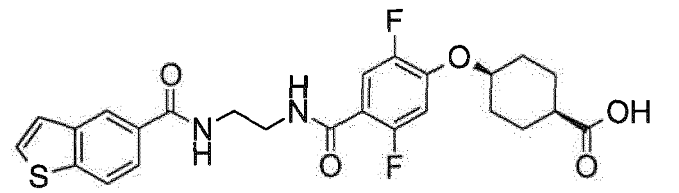 |

[Table 109]

| Ex | Structure |
|---|---|
| 487 | 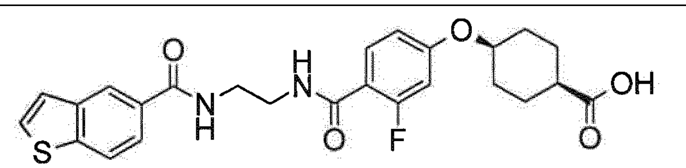 |
| 488 | 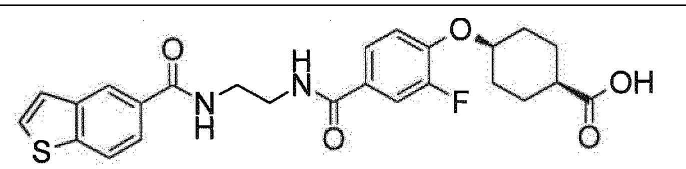 |
| 489 | 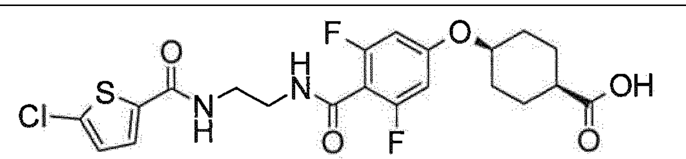 |
| 490 | 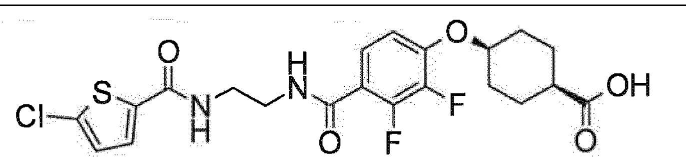 |
| 491 | 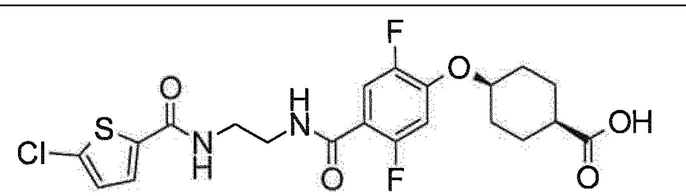 |

(continued)

| Ex | Structure |
|---|---|
| 492 | 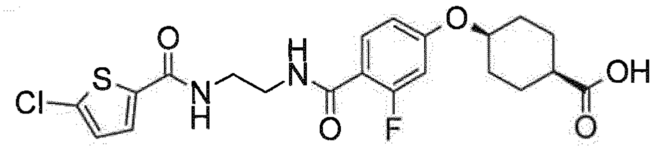 |
| 493 | 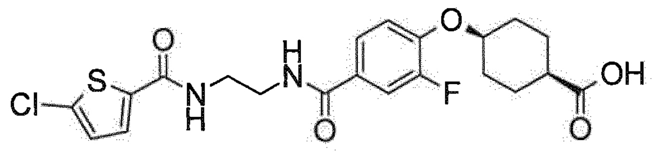 |
| 494 | 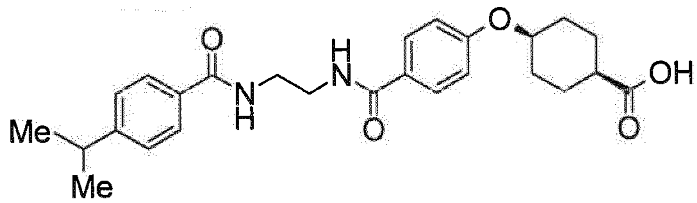 |

[Table 110]

| Ex | Structure |
|---|---|
| 495 | 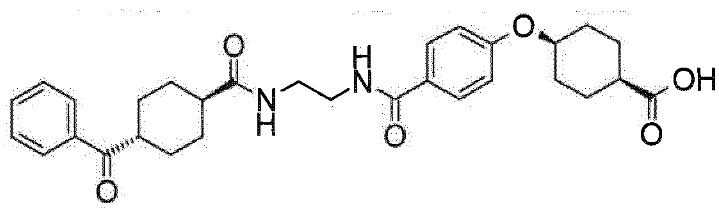 |
| 496 | 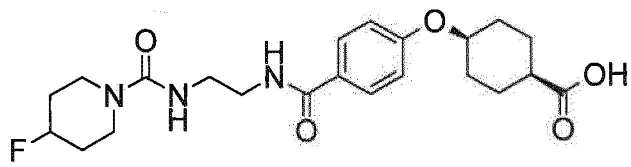 |
| 497 | 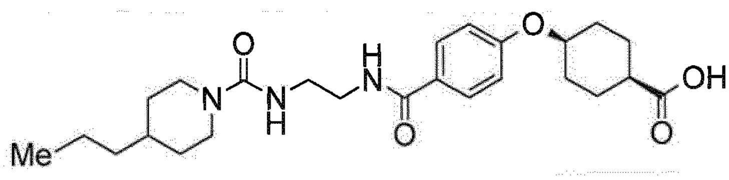 |
| 498* | 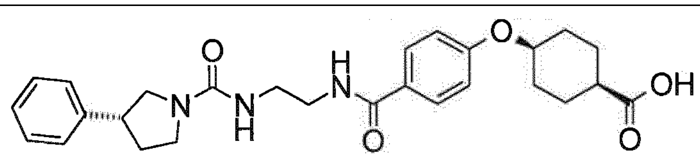 |
| 499 | 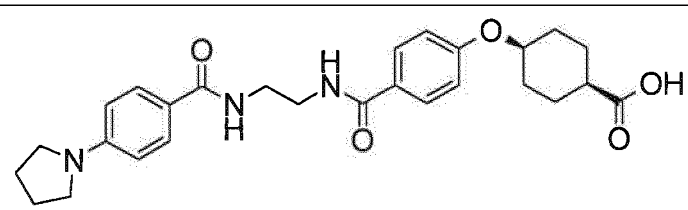 |

(continued)

| Ex | Structure |
|---|---|
| 500 | 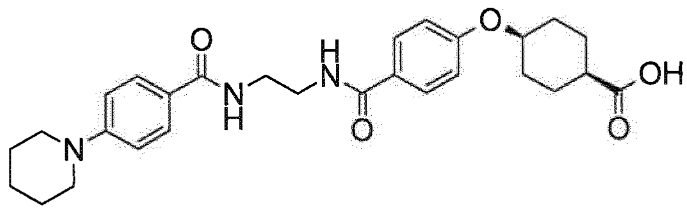 |
| 501 | 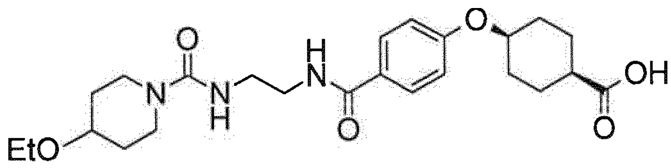 |

[Table 111]

| Ex | Structure |
|---|---|
| 502 | 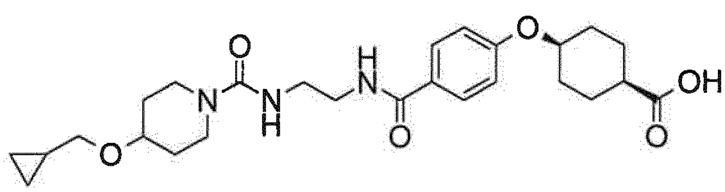 |
| 503 | 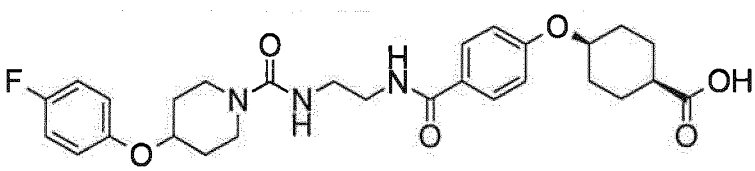 |
| 504 | 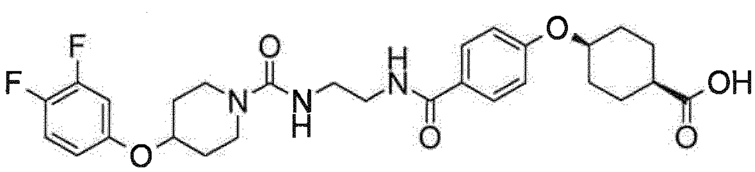 |
| 505 | 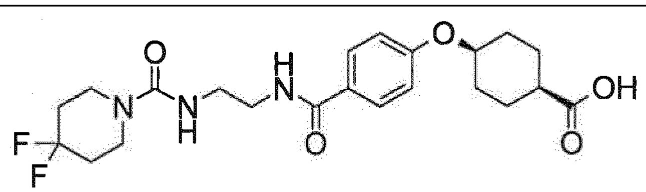 |
| 506 | 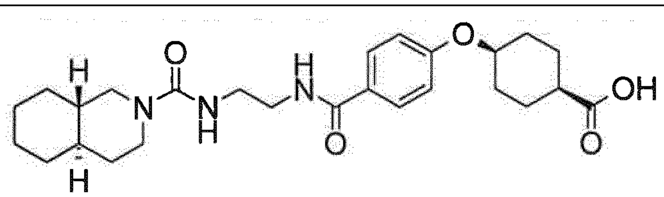 |

(continued)

| Ex | Structure |
|---|---|
| 507 | 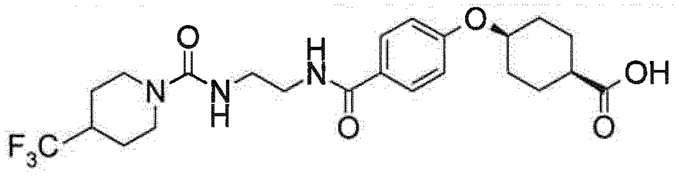 |

[Table 112]

| Ex | Structure |
|---|---|
| 508 | 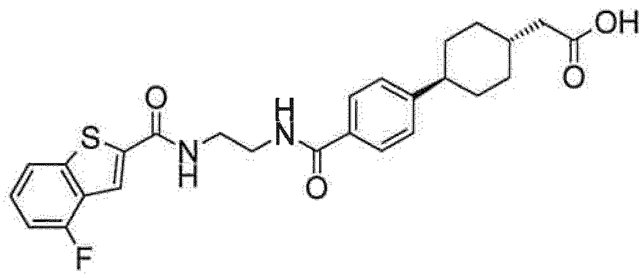 |
| 509 | 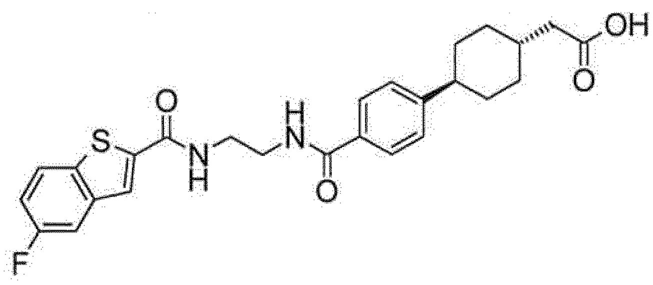 |
| 510 | 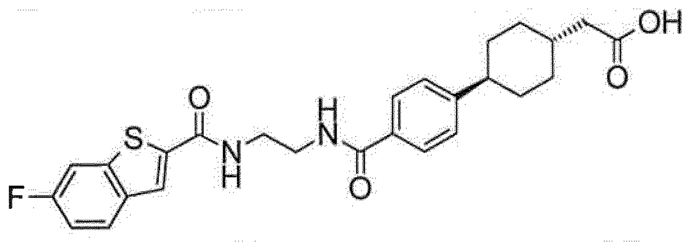 |
| 511 | 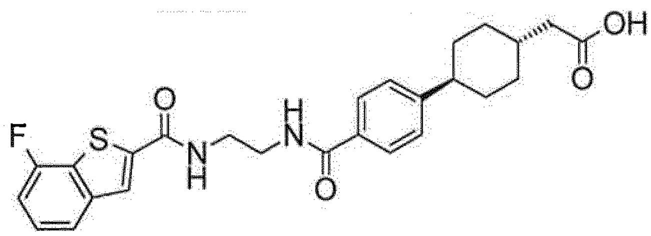 |
| 512 | 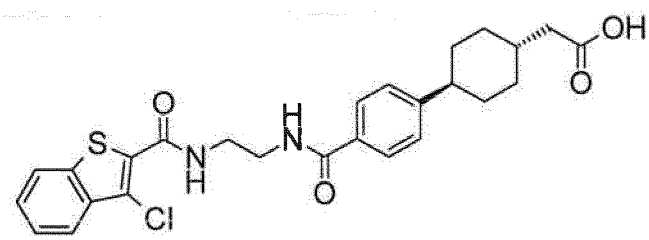 |

[Table 113]

| Ex | Structure |
|---|---|
| 513 | 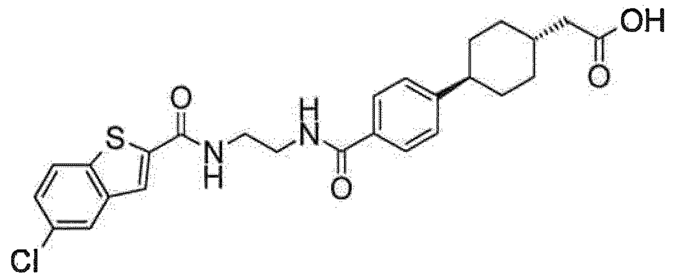 |
| 514 | 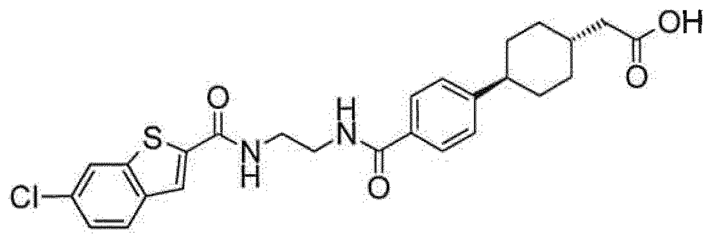 |
| 515 | 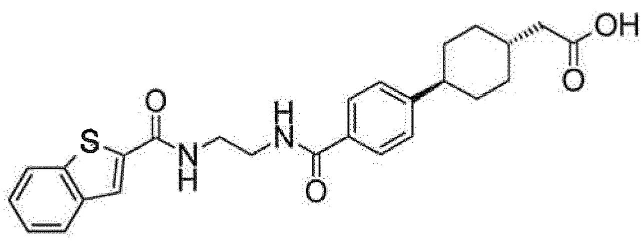 |
| 516 | 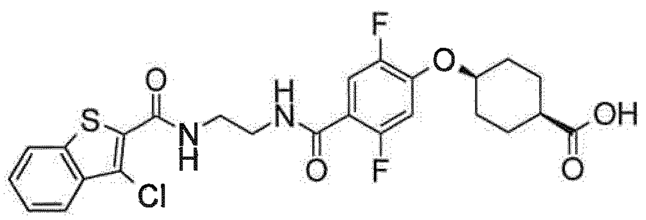 |
| 517 | 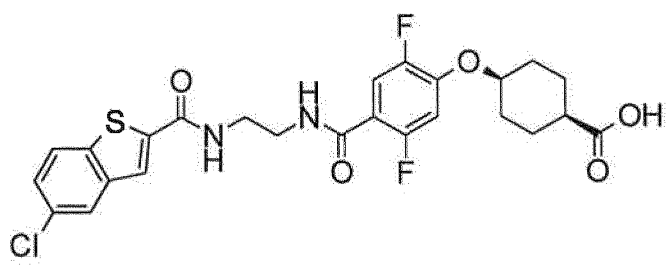 |

[Table 114]

| Ex | Structure |
|---|---|
| 518 | 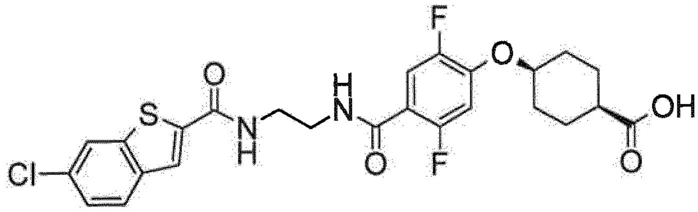 |
| 519 | 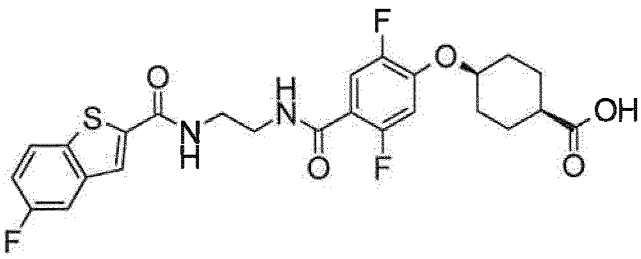 |
| 520 | 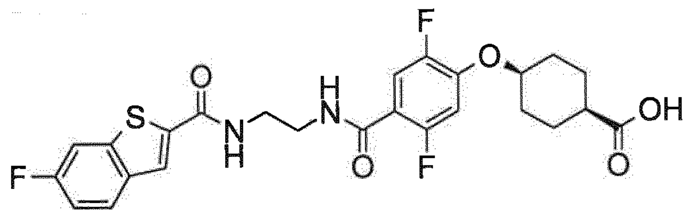 |
| 521 | 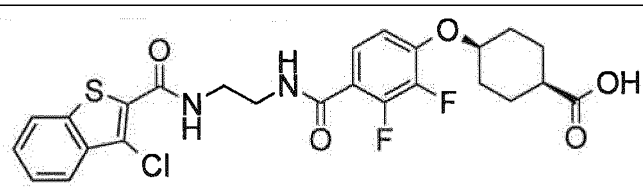 |
| 522 | 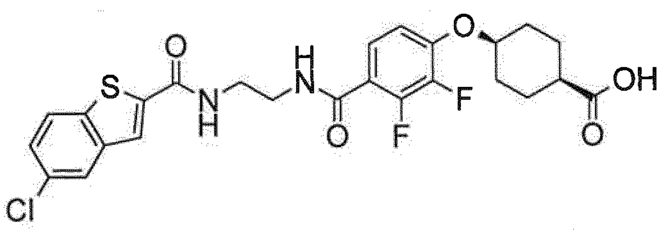 |
| 523 | 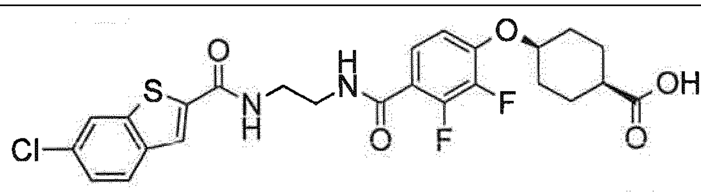 |

[Table 115]

| Ex | Structure |
|---|---|
| 524 | 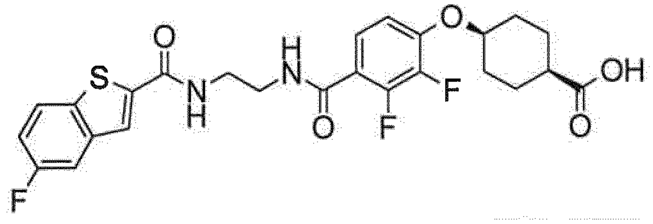 |
| 525 | 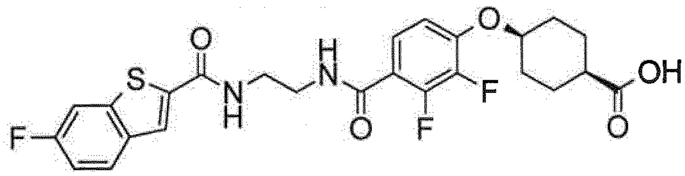 |
| 526 | 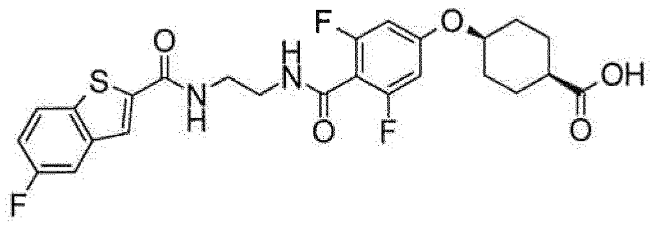 |
| 527 | 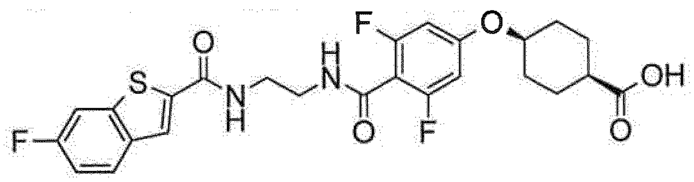 |
| 528 | 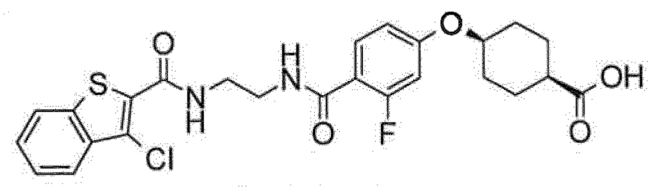 |
| 529 | 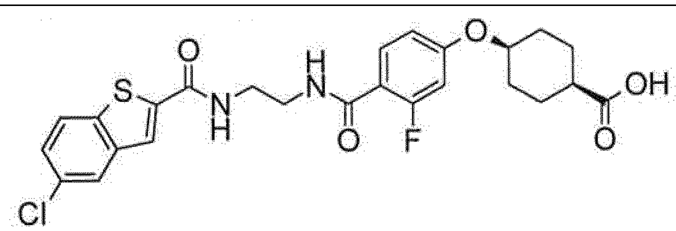 |

[Table 116]

| Ex | Structure |
|---|---|
| 530 | 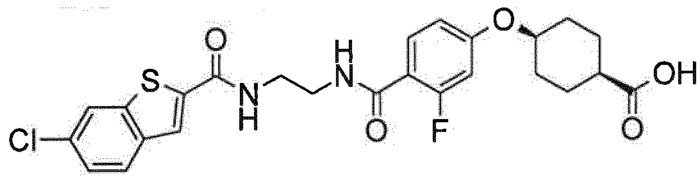 |

(continued)

| Ex | Structure |
|---|---|
| 531 | 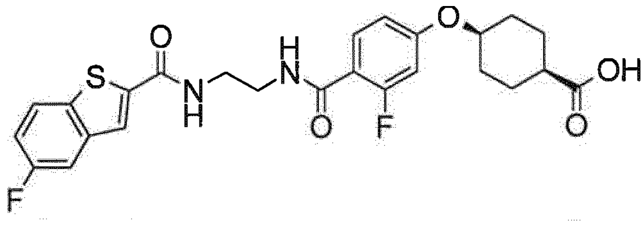 |
| 532 | 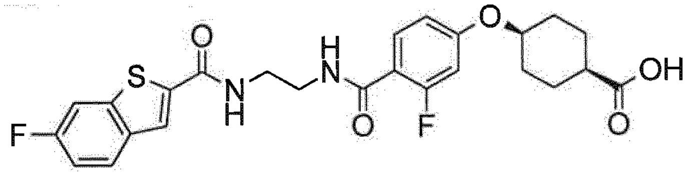 |
| 533 | 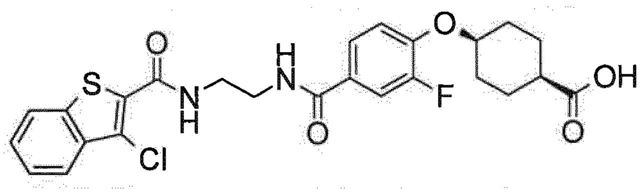 |
| 534 | 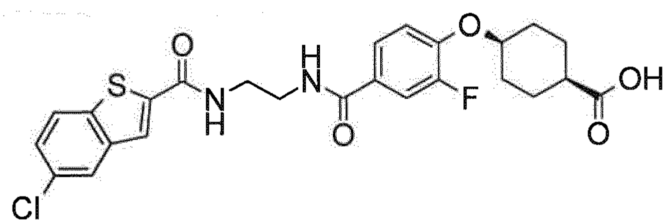 |
| 535 | 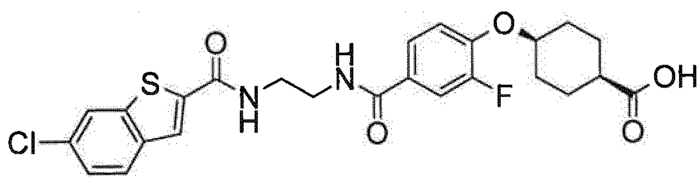 |
| 536 | 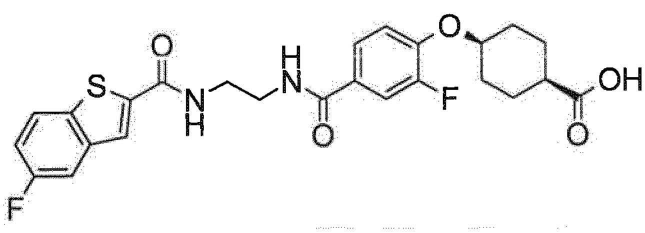 |

[Table 117]

| Ex | Structure |
|---|---|
| 537 | 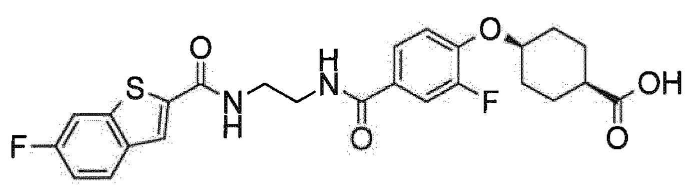 |

(continued)

| Ex | Structure |
|---|---|
| 538 | 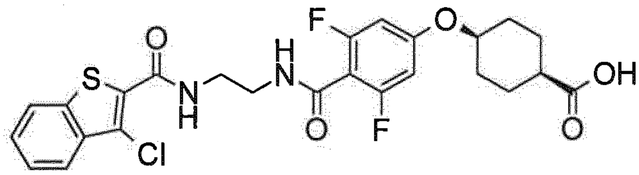 |
| 539 | 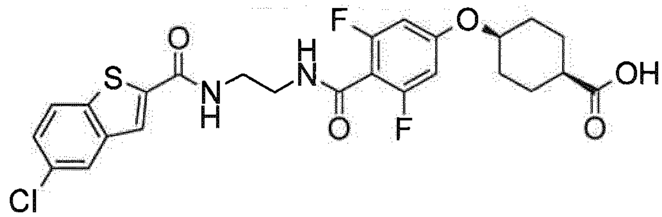 |
| 540 | 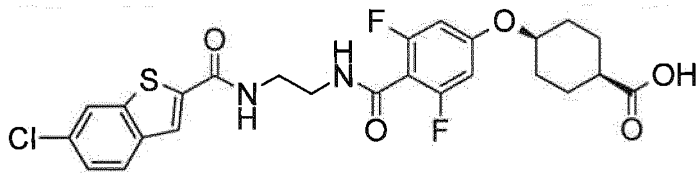 |
| 541 | 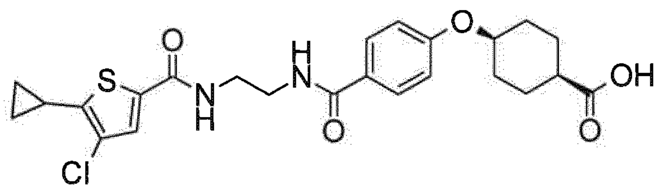 |
| 542 | 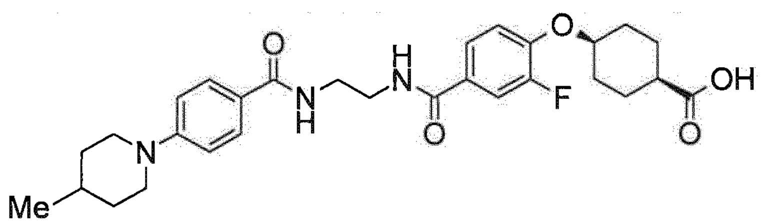 |
| 543 | 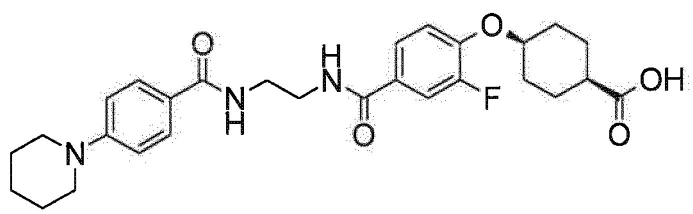 |

[Table 118]

| Ex | Structure |
|---|---|
| 544 | 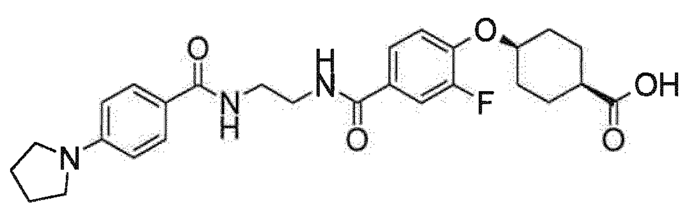 |

(continued)

| Ex | Structure |
|---|---|
| 545 | 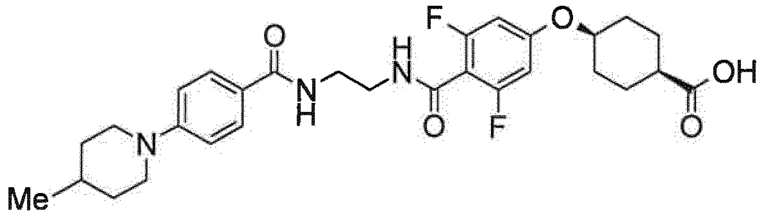 |
| 546 | 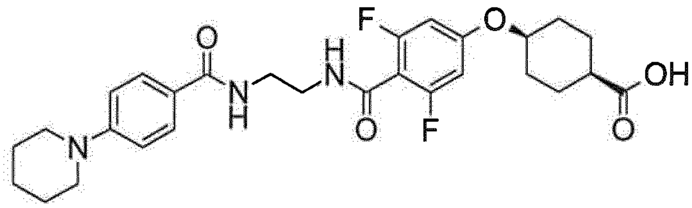 |
| 547 | 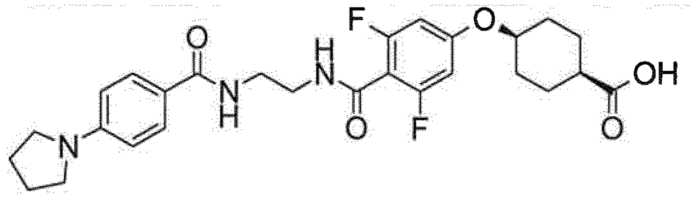 |
| 548 | 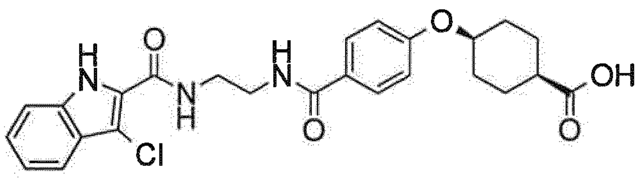 |
| 549 | 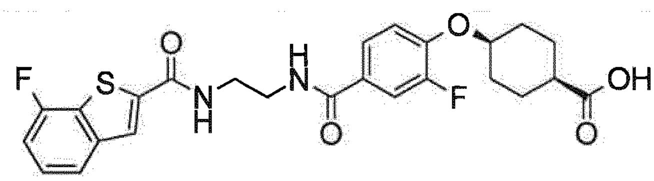 |

[Table 119]

| Ex | Structure |
|---|---|
| 550 | 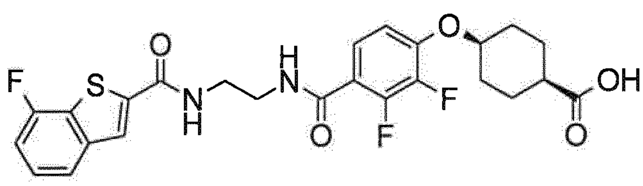 |
| 551 | 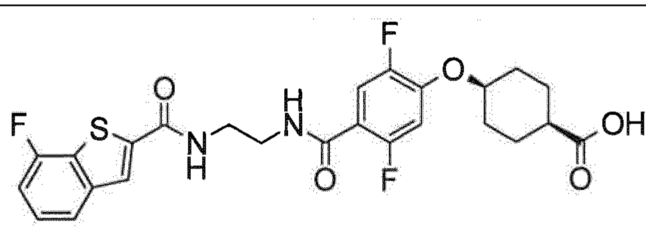 |

(continued)

| Ex | Structure |
|---|---|
| 552 | 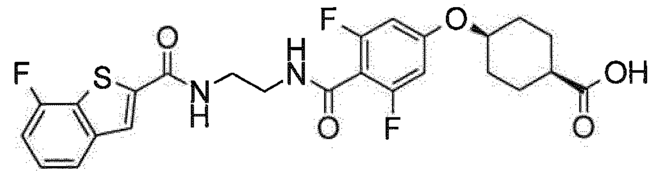 |
| 553 | 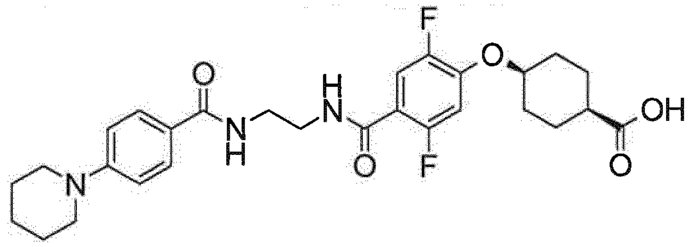 |
| 554 | 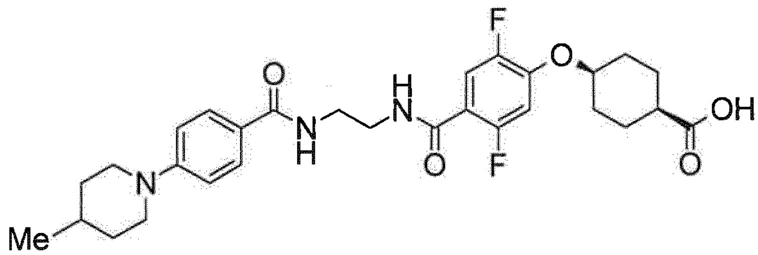 |
| 555 | 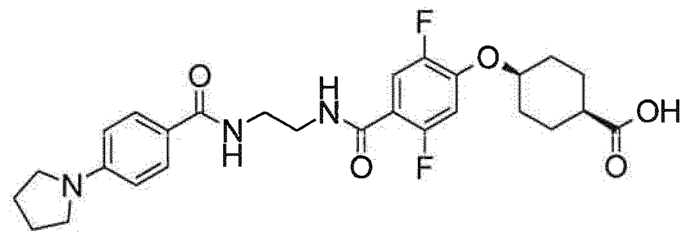 |

[Table 120]

| Ex | Structure |
|---|---|
| 556 | 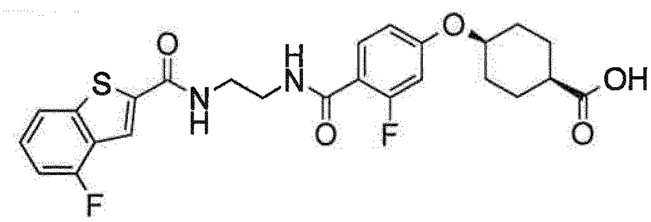 |
| 557 | 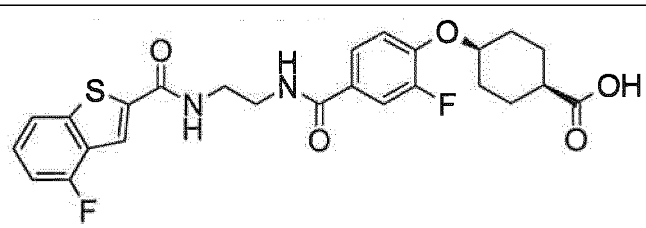 |

(continued)

| Ex | Structure |
|---|---|
| 558 | 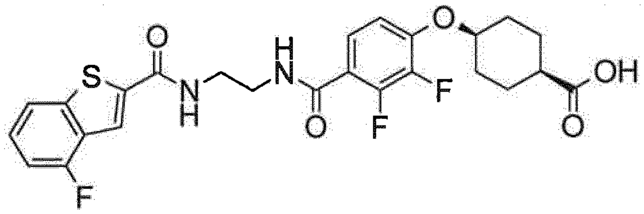 |
| 559 | 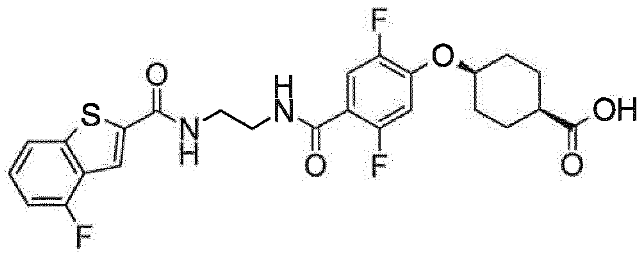 |
| 560 | 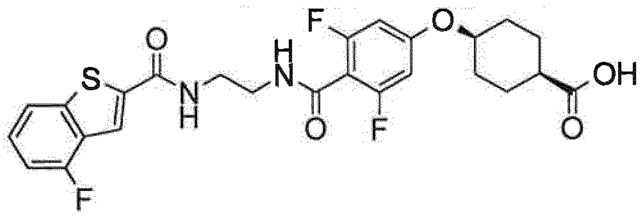 |
| 561 | 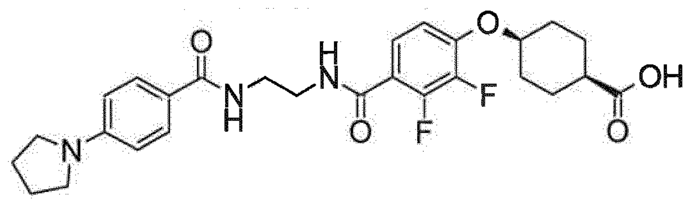 |

[Table 121]

| Ex | Structure |
|---|---|
| 562 | 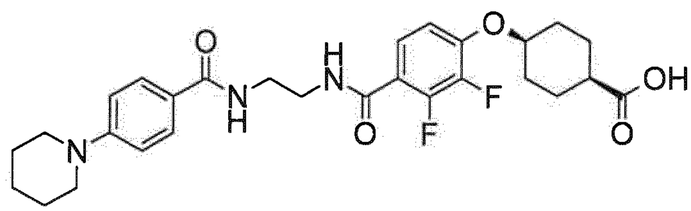 |
| 563 | 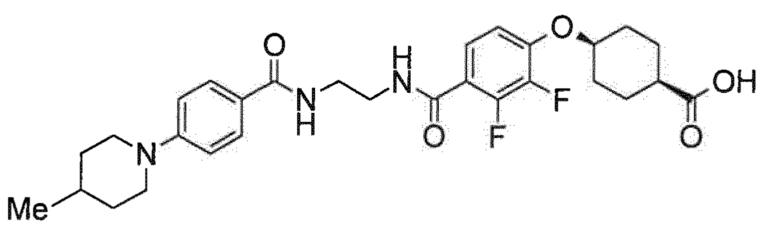 |

(continued)

| Ex | Structure |
|---|---|
| 564 | 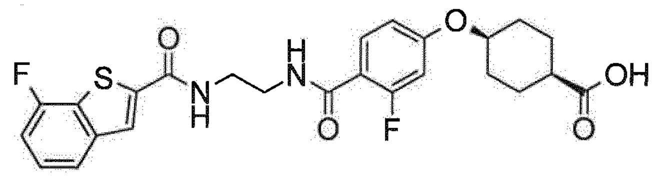 |
| 565 | 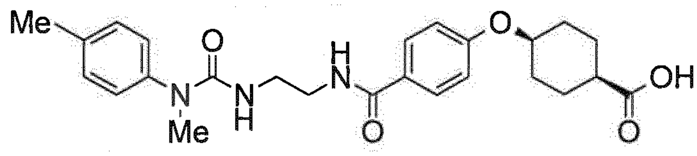 |
| 566 | 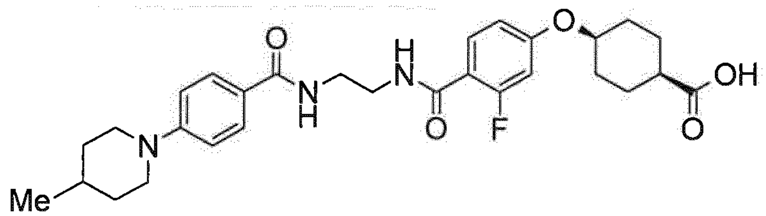 |
| 567 | 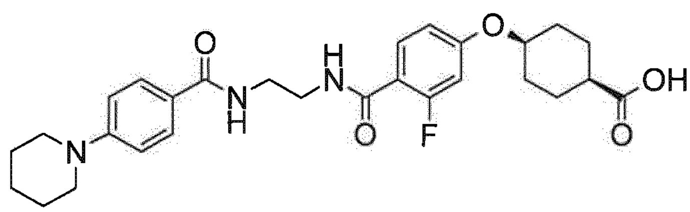 |
| 568 | 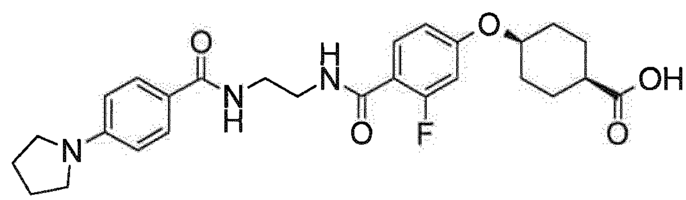 |

[Table 122]

| Ex | Structure |
|---|---|
| 569 | 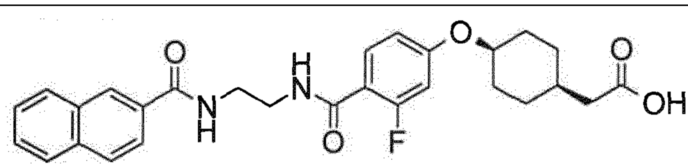 |
| 570 | 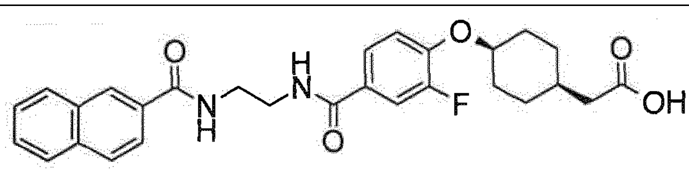 |

(continued)

| Ex | Structure |
|---|---|
| 571 | 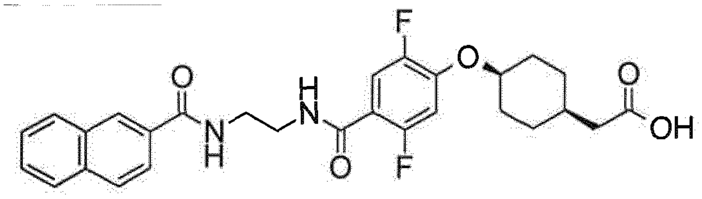 |
| 572 | 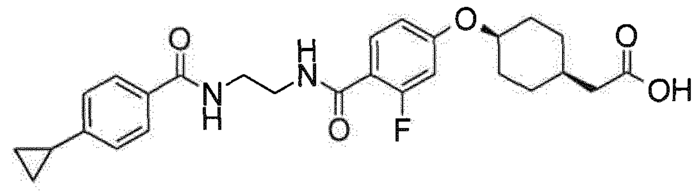 |
| 573 | 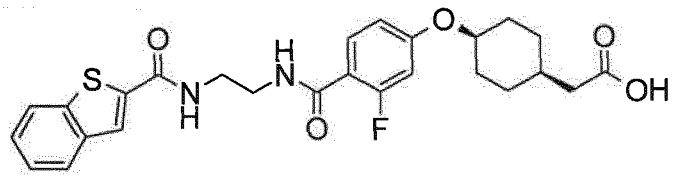 |
| 574 | 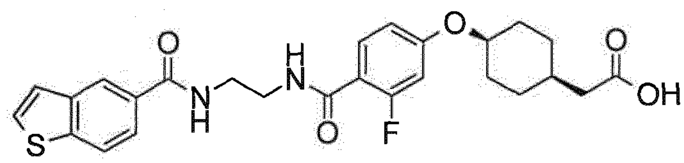 |
| 575 | 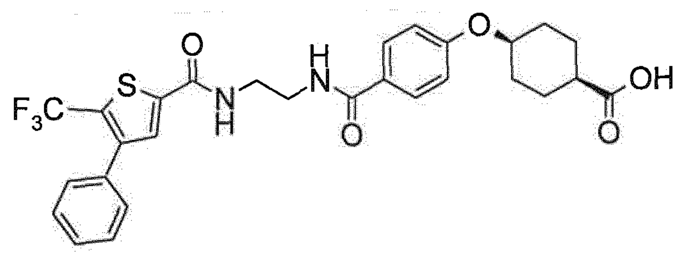 |

[Table 123]

| Ex | Structure |
|---|---|
| 576 | 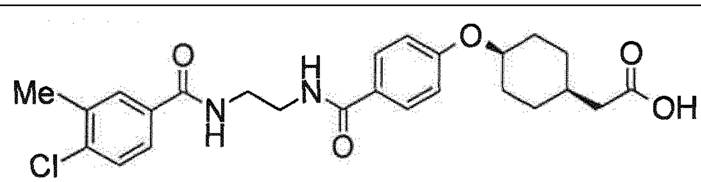 |
| 577 | 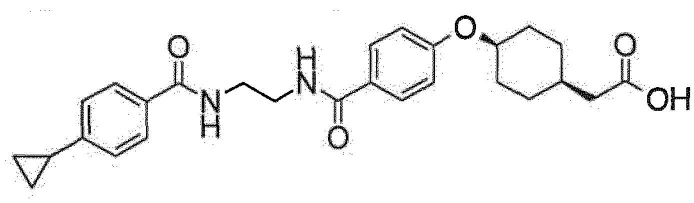 |

(continued)

| Ex | Structure |
|---|---|
| 578 | 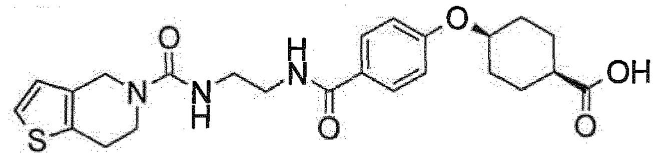 |
| 579 | 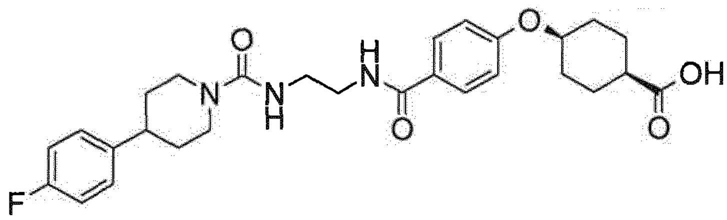 |
| 580 | 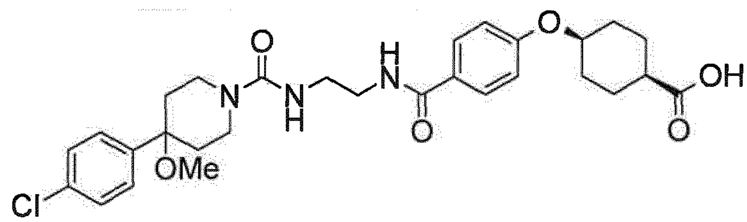 |
| 581 | 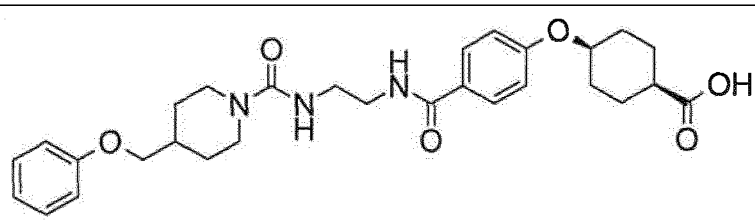 |
| 582 | 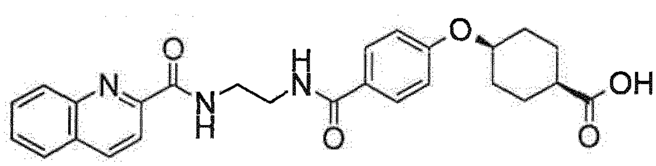 |

[Table 124]

| Ex | Structure |
|---|---|
| 583 | 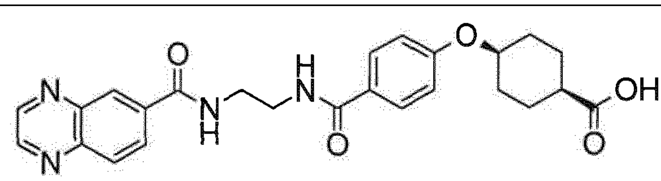 |
| 584 | 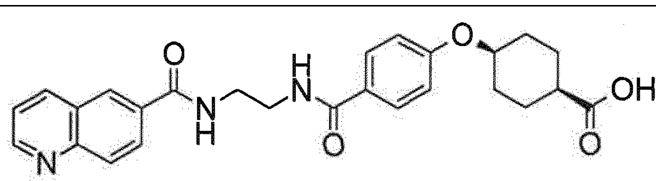 |

(continued)

| Ex | Structure |
|---|---|
| 585 | 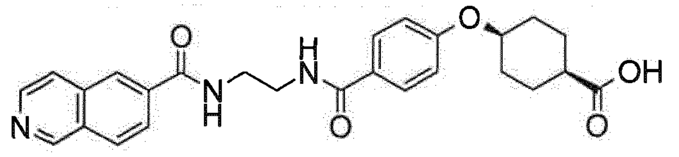 |
| 586 | 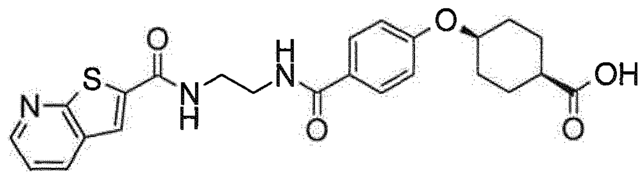 |
| 587 | 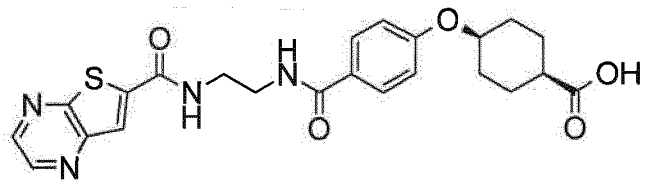 |
| 588 | 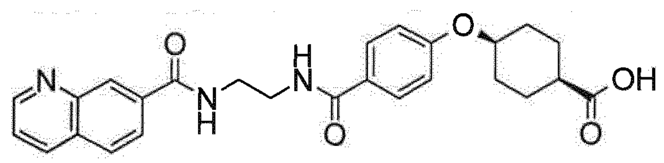 |
| 589 | 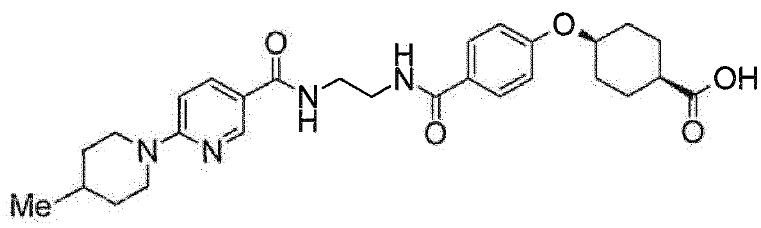 |

[Table 125]

| Ex | Structure |
|---|---|
| 590 | 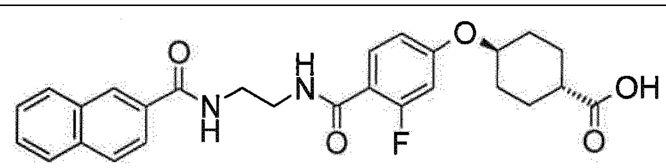 |
| 591 | 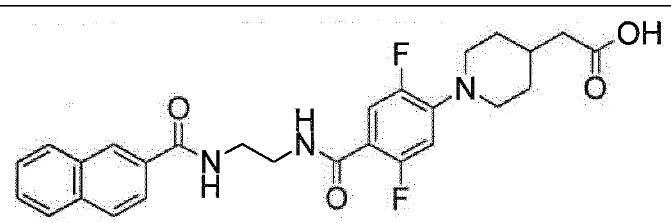 |

(continued)

| Ex | Structure |
|---|---|
| 592 | 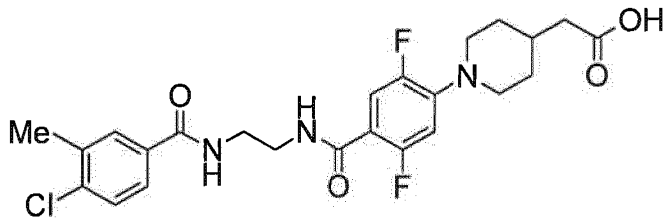 |
| 593 | 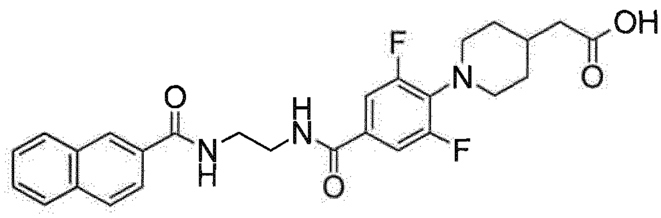 |
| 594 | 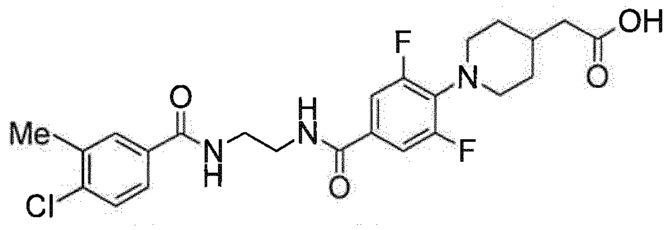 |
| 595 | 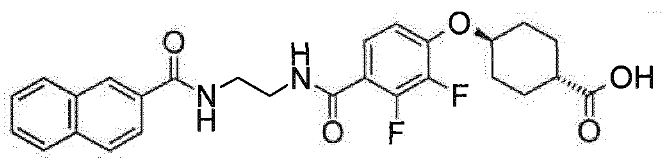 |
| 596 | 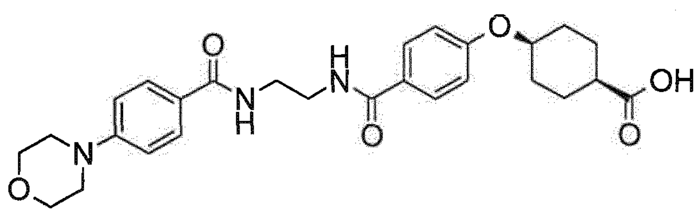 |

[Table 126]

| Ex | Structure |
|---|---|
| 597 | 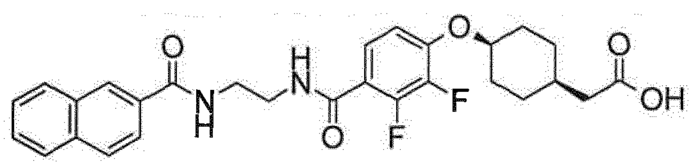 |
| 598 | 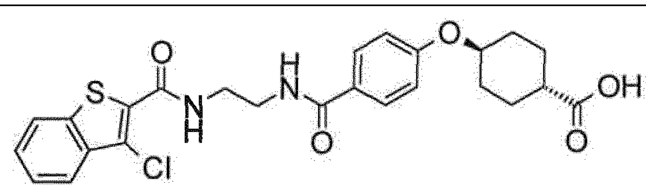 |

(continued)

| Ex | Structure |
|---|---|
| 599 | 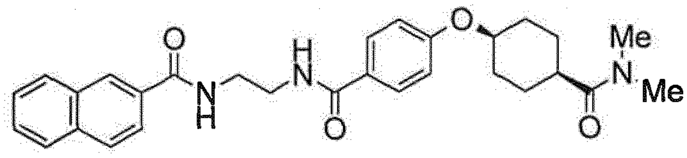 |
| 600 | 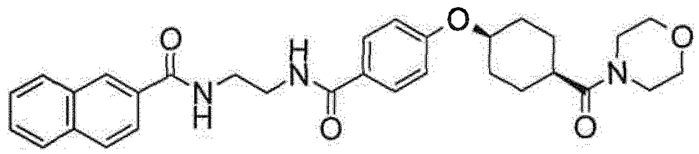 |
| 601 | 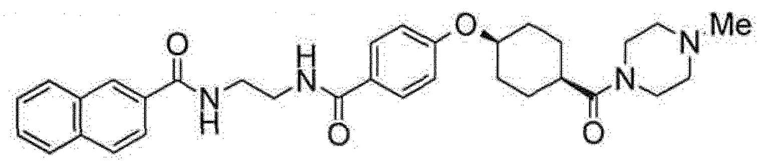 |
| 602 | 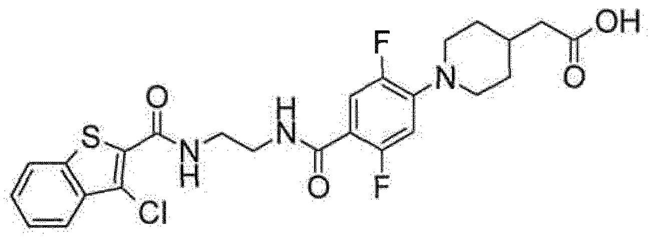 |
| 603 | 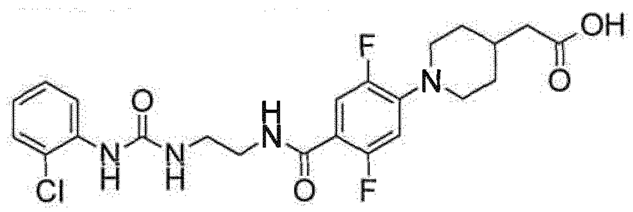 |

[Table 127]

| Ex | Structure |
|---|---|
| 604 | 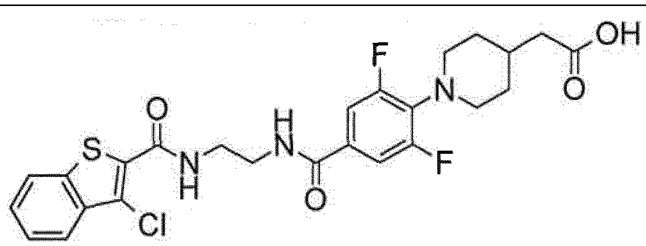 |
| 605 | 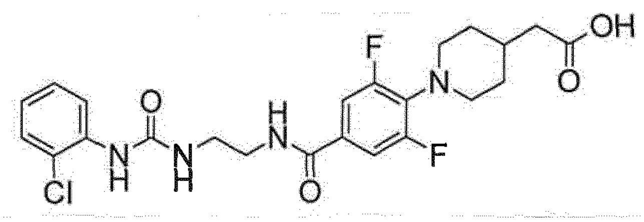 |

(continued)

| Ex | Structure |
|---|---|
| 606 | 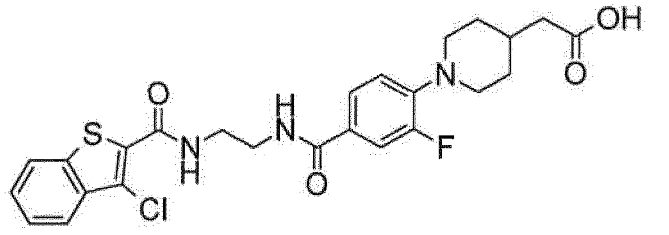 |
| 607 | 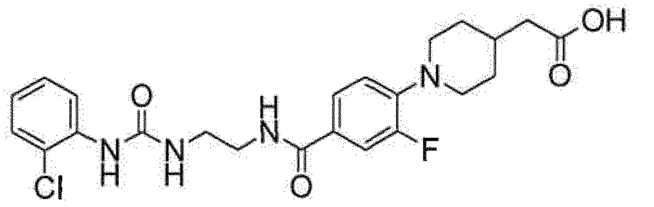 |
| 608 | 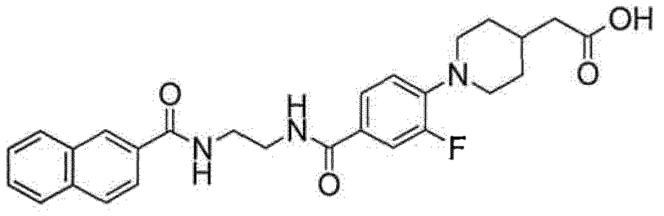 |
| 609 | 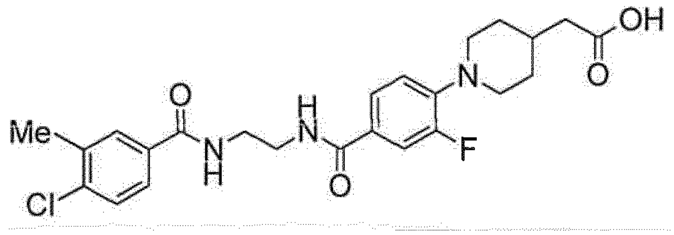 |

[Table 128]

| Ex | Structure |
|---|---|
| 610 | 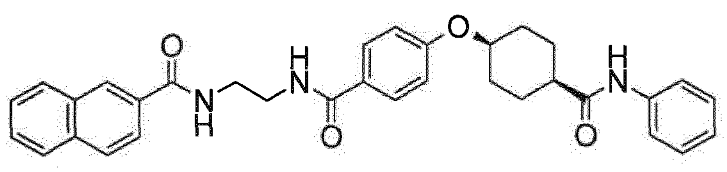 |
| 611 | 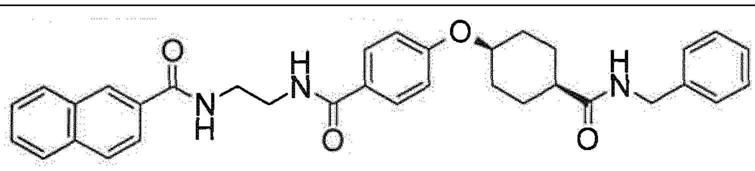 |
| 612 | 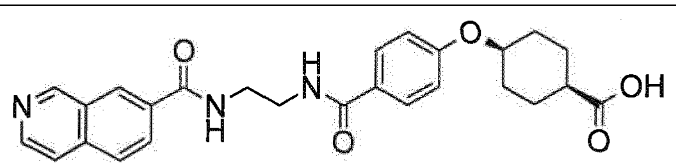 |

(continued)

| Ex | Structure |
|---|---|
| 613 | 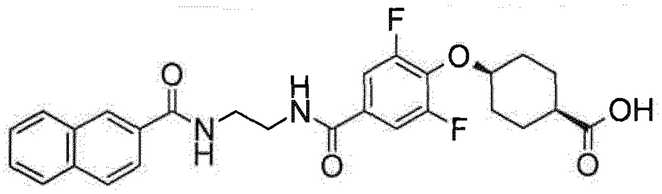 |
| 614 | 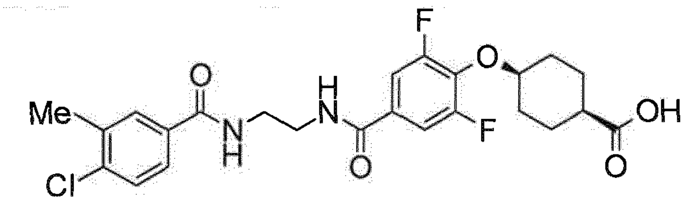 |
| 615 | 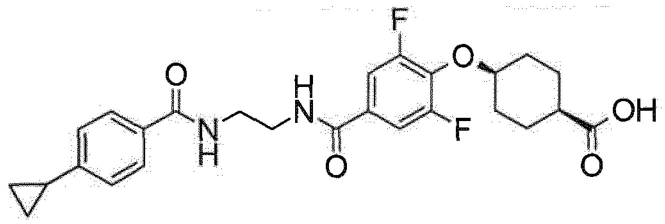 |
| 616 | 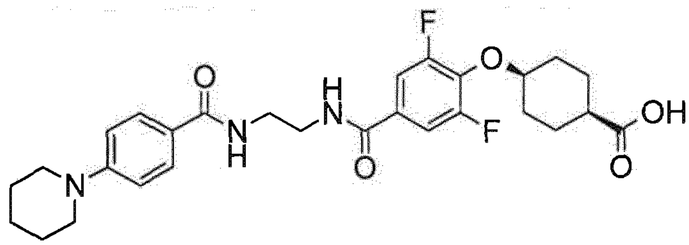 |

[Table 129]

| Ex | Structure |
|---|---|
| 617 | 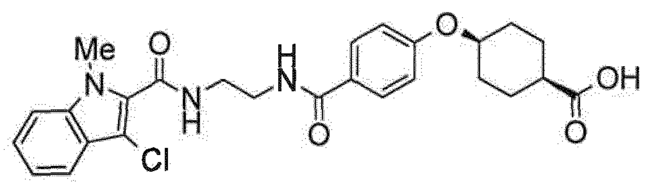 |
| 618 | 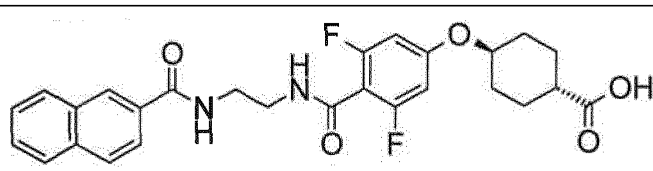 |
| 619 | 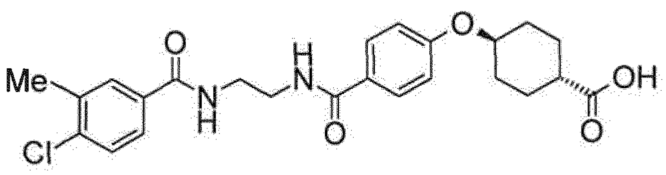 |

(continued)

| Ex | Structure |
|---|---|
| 620 | 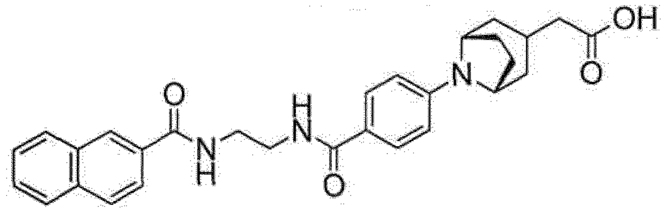 |
| 621 | 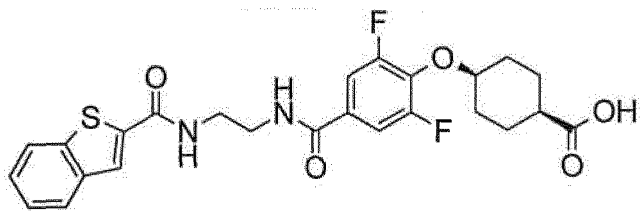 |
| 622 | 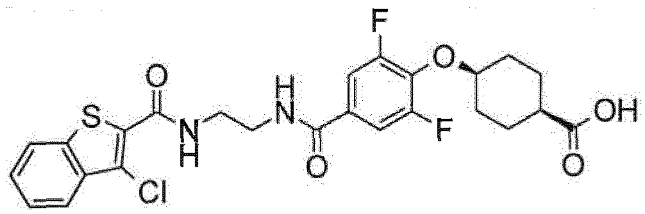 |

[Table 130]

| Ex | Structure |
|---|---|
| 623 | 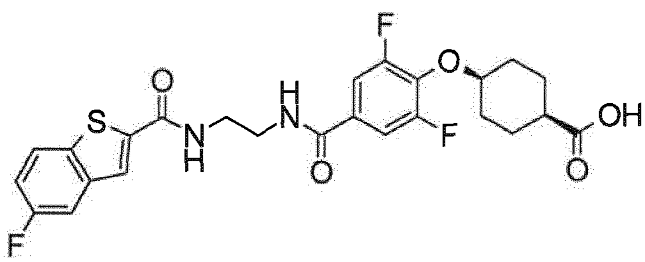 |
| 624 | 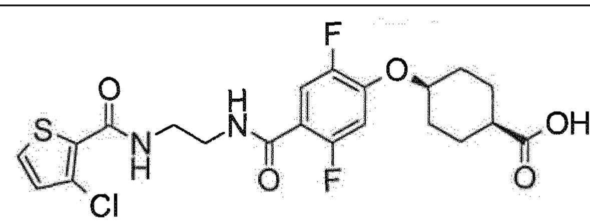 |
| 625 | 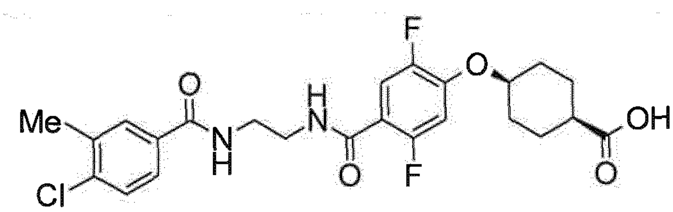 |

(continued)

| Ex | Structure |
|---|---|
| 626 | 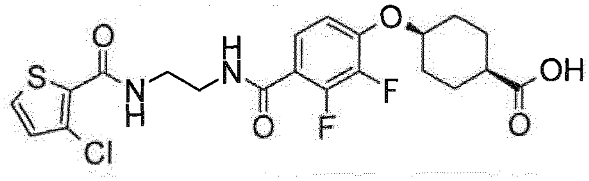 |
| 627 | 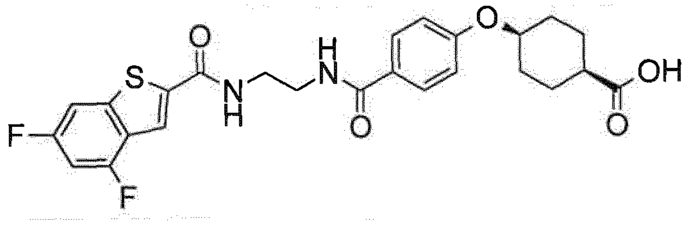 |
| 628 | 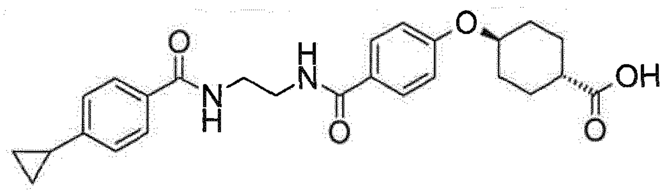 |

[Table 131]

| Ex | Structure |
|---|---|
| 629 | 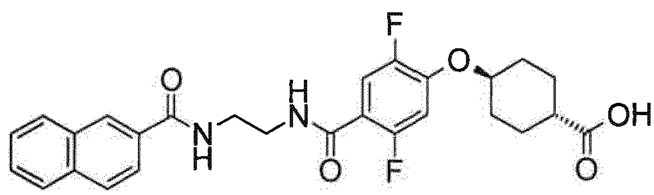 |
| 630 | 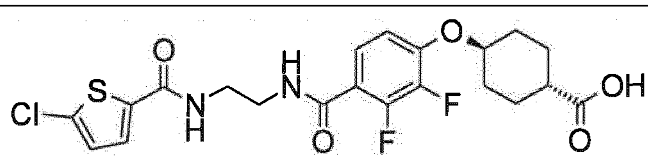 |
| 631 | 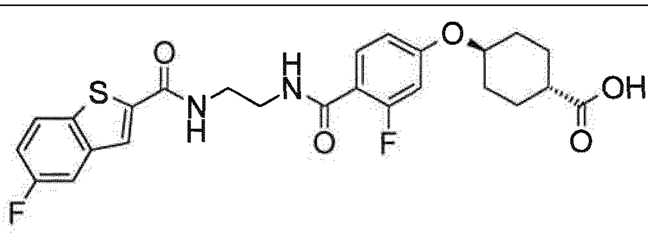 |
| 632* | 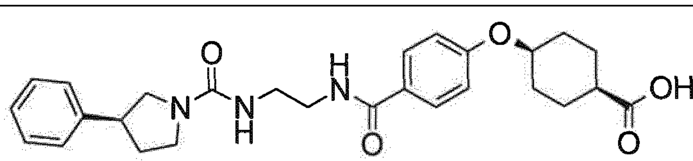 |

(continued)

| Ex | Structure |
|---|---|
| 633 | 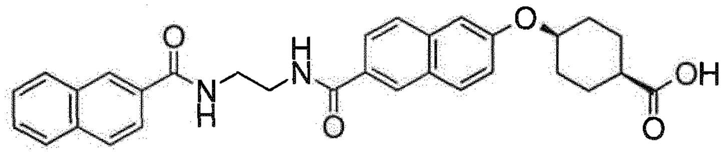 |
| 634 | 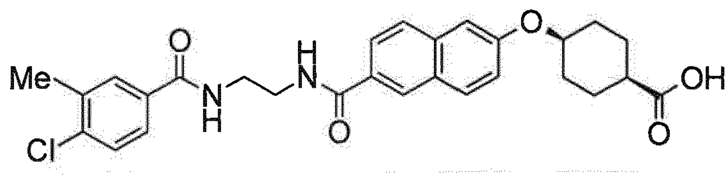 |
| 635 | 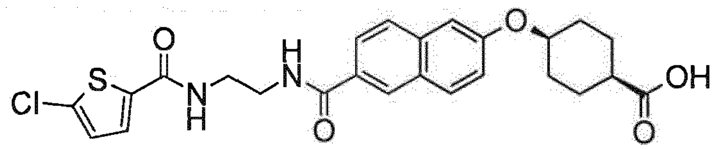 |
| 636 | 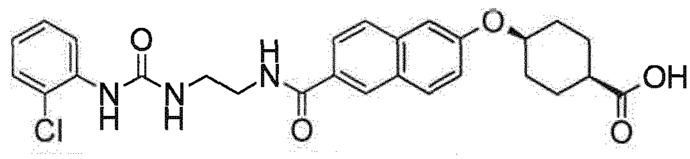 |

[Table 132]

| Ex | Structure |
|---|---|
| 637 | 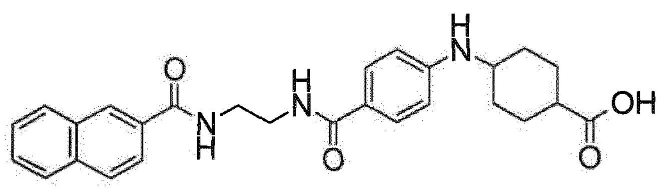 |
| 638 | 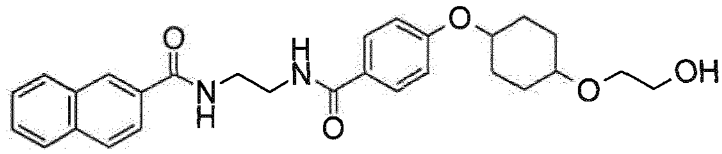 |
| 639 | 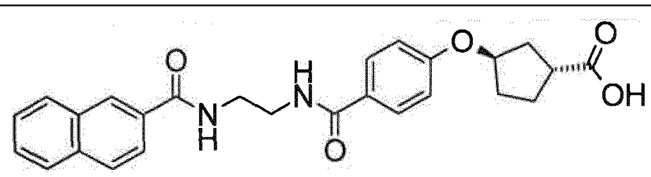 |
| 640 | 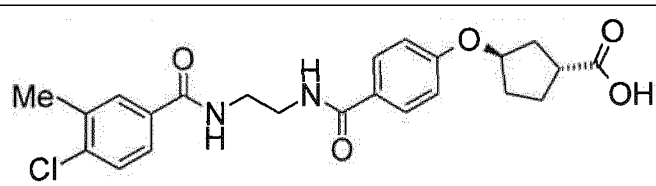 |

(continued)

| Ex | Structure |
|---|---|
| 641 | 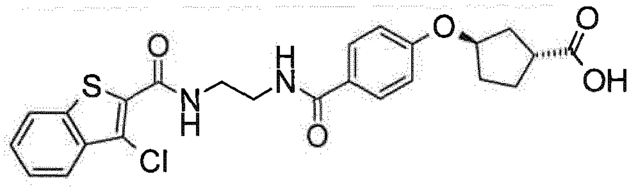 |
| 642 | 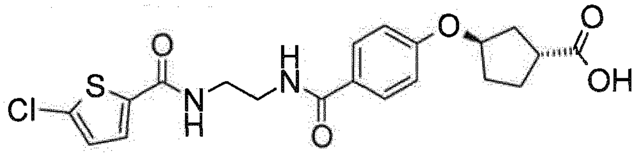 |
| 643 | 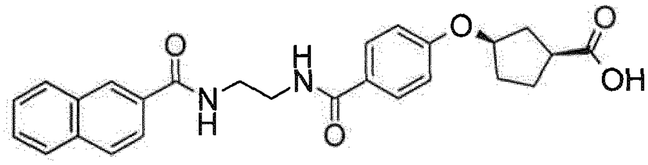 |
| 644 | 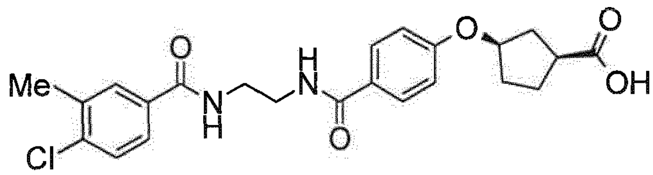 |

[Table 133]

| Ex | Structure |
|---|---|
| 645 | 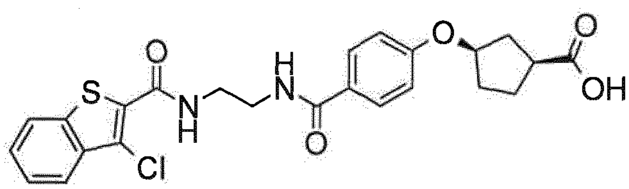 |
| 646 | 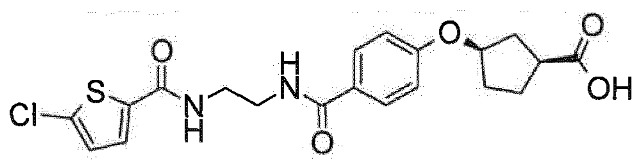 |
| 647 | 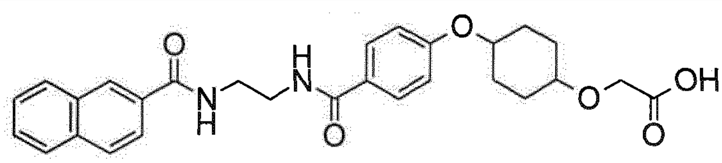 |
| 648 | 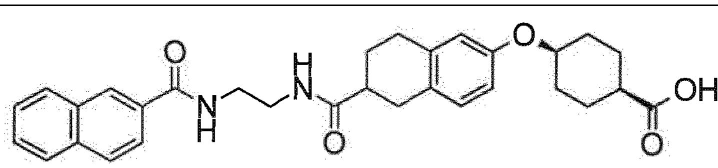 |

(continued)

| Ex | Structure |
|---|---|
| 649 | 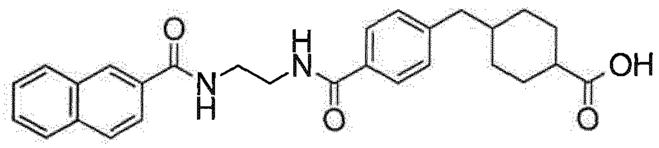 |
| 650 | 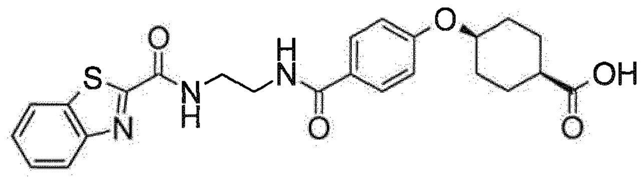 |
| 651 | 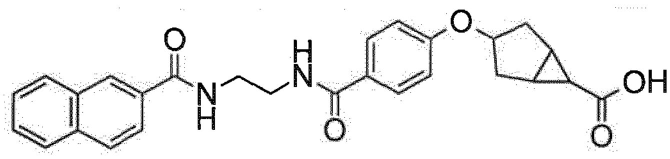 |
| 652 | 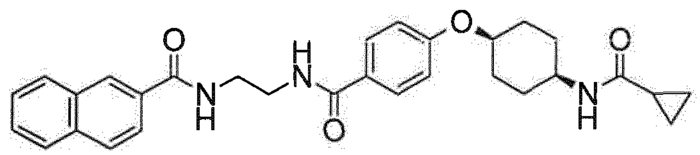 |

[Table 134]

| Pr | PSy | Data |
|---|---|---|
| 1 | 1 | NMR2 : 1.26(3H,t),1.60-1.81(4H,m),1.89-2.06(4H,m),2.36-2.4 5(1H,m),4.15(2H,q),4.54-4.60(1H,m), 5.34(2H,s),6.91(2H,d),7. 30-7.46(5H,m),8.01(2H,d). |
| 2 | 2 | NMR2 : 1.27(3H,t),1.64-1.83(4H,m),1.91-2.08(4H,m),2.38-2.4 6(1H,m),4.15(2H,q),4.57-4.63(1H,m), 6.94(2H,d), 8.04(2H,d). |
| 3 | 3 | ESP : 435. |
| 4 | 4 | ESP : 335. |
| 5 | 5 | ESP : 454. |
| 6 | 6 | ESP : 522. |
| 7 | 7 | ESP : 521. |
| 8 | 8 | ESP : 439. |
| 9 | 9 | ESN : 335. |
| 10 | 10 | ESP : 491. |
| 11 | 11 | EI : 238 |
| 12 | 12 | ESN : 245. |
| 13 | 13 | ESN : 424. |
| 14 | 14 | NMR2 : 1.49(9H,s),1.60-1.70(2H,m),1.96-2.10(3H,m),2.28-2. 42(2H,m),2.84(1H,tt),3.90(3H,s), 3.91-3.99(1H,m),5.62(1H,s), 7.27(2H,d),7.96(2H,d). |
| 15 | 15 | NMR2 : 1.13-1.27(1.4H,m),1.42-1.60(1.4H,m),1.66-2.00(5.9 H,m),2.29-2.38(1.7H,m),2.48-2.59 (1.3H,m),2.62-2.72(0.3H, m),3.89-3.91(3H,m),7.27(1.4H,d),7.30(0.6H,d),7.94-7.99(2H, m). |

(continued)

| Pr | PSy | Data |
|---|---|---|
| 16 | 16 | NMR2 : 1.09-1.22(1.4H,m),1.48-1.57(1.4H,m),1.61-1.94(5.9 H,m),2.18(1.4H,d),2.24-2.36(0.9H,m), 2.52(0.7H,tt),2.60-2.70 (0.3H,m),3.89-3.91(3H,m),7.27(1.4H,d),7.30(0.6H,d),7.94-7.98 (2H,m). |
| 17 | 17 | NMR1 : 1.05-1.19(1.4H,m);1.41-1.54(1.4H,m),1.55-1.84(5.9 H,m),2.09-2.18(1.7H,m),2.32-2.36 (0.6H,m),2.51-2.56(1H,m), 7.34(1.4H,d),7.39(0.6H,d),7.83-7.87(2H,m). |
| 18 | 18 | ESN : 239. |
| 19 | 19 | ESN : 209. |
| 20 | 20 | ESN : 179. |
| 21 | 21 | NMR2 : 0.73-0.78(2H,m),1.07-1.13(2H,m),1.39(3H,t),2.24-2.3 2(1H,m),4.36(2H,q),6.93(1H,d),7.82 (1H,dd),8.02(1H,d). |
| 22 | 22 | NMR1 : 0.77-0.82(2H,m),1.06-1.12(2H,m),2.19-2.27(1H,m),7. 12(1H,d),7.77(1H,dd),7.88(1H,d). |
| 23 | 23 | FP : 267. |
| 24 | 24 | FN : 237. |

[Table 135]

| Pr | PSy | Data |
|---|---|---|
| 25 | 1 | NMR2 : 1.26(3H,t),1.45-1.67(4H,m),2.05-2.23(4H,m),2.31-2.4 0(1H,m),4.14(2H,q),4.25-4.34(1H,m), 5.33(2H,s),6.89(2H,d),7. 30-7.46(5H,m),8.01(2H,d). |
| 26 | 2 | NMR2 : 1.27(3H,t),1.47-1.69(4H,m),2.06-2.24(4H,m),2.32-2.4 1(1H,m),4.15(2H,q),4.28-4.37(1H,m), 6.92(2H,d), 8.04(2H,d). |
| 27 | 3 | NMR2 : 1.27(3H,t),1.43(9H,s),1.44-1.68(4H,m),2.04-2.22(4H, m),2.30-2.40(1H,m),3.34-3.44(2H,m), 3,50-3.57(2H,m),4.14(2 H,q),4.23-4.32(1H,m),4.92-5.01(1H,m),6.89(2H,d),6.96-7.04(1 H,m),7.76 (2H,d). |
| 28 | 4 | NMR1 : 1.18(3H,t),1.35-1.62(4H,m),1.90-2.11(4H,m),2.31-2.4 1(1H,m),2.93-3.01(2H,m),3,44-3.52 (2H,m),4.07(2H,q),4.38-4. 47(1H,m),7.02(2H,d),7.84(2H,d),7.93(2H,bs). |
| 29 | 3 | NMR2 : 1.27(3H,t),1.43(9H,s),1.44-1.80(4H,m),1.90-2.22(4H, m),2.31-2.45(1H,m),3.36-3.44(2H,m), 3.51-3.57(2H,m),4.11-4. 18(2H,m),4.28(0.5H,tt),4.53-4.57(0.5H,m),4.93-5.00(1H,m),6. 87-6.93(2H, m),6.97-7.04(1H,m),7.75(2H,d). |
| 30 | 4 | NMR1 : 1.18(3H,t),1.42(1H,dq),1.55(1H,dq),1.64-1.85(4H,m), 1.91-1.98(1H,m),2.03-2.11(1H,m), 2.36(0.5H,tt),2.47-2.51(0.5 H,m),2.97(2H,t),3.49(2H,q),4.03-4.10(2H,m),4.43(0.5H,tt),4.6 2-4.67 (0.5H,m),6.99-7.04(2H,m),7.82-7.86(2H,m),7.93(2H,bs), 8.52-8.57(1H,m). |
| 31 | 9 | ESP : 499. |
| 32 | 8 | ESP : 439. |
| 33 | 8 | ESP : 473. |
| 34 | 8 | ESP : 439. |
| 35 | 8 | ESN : 471. |
| 36 | 7 | ESP : 555. |
| 37 | 7 | ESP : 555. |
| 38 | 8 | ESP : 483. |
| 39 | 8 | ESP : 483. |
| 40 | 7 | ESP : 541. |
| 41 | 8 | ESP : 473. |

(continued)

| Pr | PSy | Data |
|---|---|---|
| 42 | 8 | ESP : 473. |
| 43 | 7 | ESP : 527. |
| 44 | 7 | ESP : 555. |
| 45 | 7 | ESP : 555. |
| 46 | 7 | ESP : 457. |
| 47 | 7 | ESP : 479. |
| 48 | 9 | ESN : 335. |

[Table 136]

| Pr | PSy | Data |
|---|---|---|
| 49 | 10 | ESP : 491. |
| 50 | 7 | ESP : 445. |
| 51 | 7 | ESP : 445. |
| 52 | 7 | ESP : 479. |
| 53 | 7 | ESP : 507. |
| 54 | 8 | ESP : 445. |
| 55 | 8 | ESP : 469. |
| 56 | 8 | ESP : 457. |
| 57 | 8 | ESP : 523. |
| 58 | 7 | ESP : 555. |
| 59 | 7 | ESP : 517. |
| 60 | 7 | ESP : 517. |
| 61 | 7 | ESP : 507. |
| 62 | 7 | ESP : 507. |
| 63 | 7 | ESP : 479. |
| 64 | 7 | ESP : 503. |
| 65 | 7 | ESP : 515. |
| 66 | 8 | ESP : 525. |
| 67 | 8 | ESP : 489. |
| 68 | 7 | ESP : 489. |
| 69 | 9 | ESP : 320. |
| 70 | 10 | ESP : 474. |
| 71 | 9 | ESP : 320. |
| 72 | 10 | ESP : 474. |
| 73 | 9 | ESP : 333. |
| 74 | 10 | ESP : 487. |
| 75 | 9 | ESP : 487. |

(continued)

| Pr | PSy | Data |
|---|---|---|
| 76 | 1 | ESP : 397. |
| 77 | 2 | ESP : 307. |
| 78 | 7 | ESP : 541. |
| 79 | 7 | ESP : 557. |
| 80 | 8 | ESP : 525. |
| 81 | 1 | ESP : 401. |
| 82 | 2 | ESN : 309. |
| 83 | 3 | ESP : 453. |
| 84 | 4 | ESP : 353. |

[Table 137]

| Pr | PSy | Data |
|---|---|---|
| 85 | 7 | ESP : 487. |
| 86 | 7 | ESP : 513. |
| 87 | 7 | ESP : 541. |
| 88 | 9 | ESP : 349. |
| 89 | 10 | ESP : 503. |
| 90 | 9 | ESN : 351. |
| 91 | 10 | ESN : 505. |
| 92 | 9 | ESN : 351. |
| 93 | 10 | ESN : 505. |
| 94 | 7 | ESP : 521. |
| 95 | 8 | ESP : 521. |
| 96 | 8 | ESP : 521. |
| 97 | 8 | ESP : 497. |
| 98 | 7 | ESP : 531. |
| 99 | 7 | ESP : 501. |
| 100 | 12 | ESN : 245. |
| 101 | 1 | ESP : 401. |
| 102 | 2 | ESN : 309. |
| 103 | 3 | ESP : 453. |
| 104 | 4 | ESP : 353. |
| 105 | 12 | ESP : 243. |
| 106 | 1 | ESP : 397. |
| 107 | 2 | ESP : 307. |
| 108 | 3 | ESP : 449. |

(continued)

| Pr | PSy | Data |
|---|---|---|
| 109 | 4 | NMR1 : 1.18(3H,t),1.60-1.89(8H,m),2.35(3H,s),2.89-3.01(2 H,m),3,44-3.51(2H,m),4.08(2H,q), 4.56-4.65(1H,m),6.77-6.8 ‘ 5(2H,m),7.42(1H,d),7.94(2H,bs),8.22-8.32(1H,m). ESP : 349. |
| 110 | 18 | ESN : 223. |
| 111 | 19 | ESN : 223. |
| 112 | 21 | FP : 221. |
| 113 | 22 | FP : 207. |
| 114 | 23 | FP : 267. |
| 115 | 24 | FP : 237. |
| 116 | 116 | ESN : 280. |
| 117 | 117 | NNM1:1.17(3H,t),1.36(9H,s),1.66-1.87(8H,m),2.44-2.48(1H, m),3.08(2H,q),3.26(2H,q),4.04(2H,q), 5.19-5.24(1H,m),6.86(1 H,d),6.92(1H,t),8.08(1H,dd),8.44(1H,t),8.60(1H,d). |

[Table 138]

| Pr | PSy | Data |
|---|---|---|
| 118 | 118 | ESN:411. |
| 119 | 119 | ESP:423. |
| 120 | 120 | ESN : 295. |
| 121 | 121 | ESP:369. |
| 122 | 122 | ESN:379. |
| 123 | 123 | ESP:471. |
| 124 | 124 | NMR2:1.23-1.36(2H,m),1.26(3H,t),1.45-1.67(6H,m),1.97-2. 06(2H,m),2.53-2.59(1H,m),3.50(2H,d), 4.14(2H,q). |
| 125 | 125 | NMR2:1.15(3H,s),1.15-1.24(2H,m),1.26(3H,t),1.30-1.39(2H, m),1.58(1H,s),1.83-1.90(2H,m), 2.18-2.26(2H,m),3.55-3.64(1 H,m),4.15(2H,q). |
| 126 | 126 | ESP:579. |
| 127 | 127 | ESP:368. |
| 128 | 128 | ESN:327. |
| 129 | 129 | ESN:229. |
| 130 | 130 | NMR1:3.01-3.06(2H,m),3.55-3.62(2H,m),7.57-7.65(2H,m), 7.97-8.04(4H,m),8.08(2H,s),8.55(1H,s), 8.91-9.56(1H,m). |
| 131 | 131 | NMR1: 2.82-2.96(2H,m),3.27-3.42(2H,m),6.93-6.99(1H,m), 7.22-7.27(1H,m),7.37-7.43(1H,m), 7.59-7.67(1H,m),8.08-8.28 (4H,m). |
| 132 | 132 | ESN : 229. |
| 133 | 133 | NMR1:0.72-0.78(2H,m),0.94-1.02(2H,m),1.86-1.96(1H,m), 7.25(1H,s),12.9-13.7(1H,br). |
| 134 | 134 | ESP:315. |
| 135 | 135 | ESN:229. |
| 136 | 136 | ESP:487. |
| 137 | 137 | NMR1:1.99-2.08(2H,m),2.10-2.20(2H,m),2.31-2.47(4H,m), 4.90-4.97(1H,m),5.32(2H,s),7.15(2H,d), 7.32-7.48(5H,m),7.96 (2H,d). |

(continued)

| Pr | PSy | Data |
|---|---|---|
| 138 | 138 | NMR1:1.20-2.06(8H,m),1.42(9H,s),3.38-3.51(0.7,m),3.99(1. 3H,d),4.15(0.7H,d),4.45-4.66(1H,m), 4.79-4.92(0.3H,m),5.31 (2H,s),7.03-7.11(2H,m),7.32-7.48(5H,m),7.90-7.95(2H,m). |
| 139 | 139 | ESN:289. |
| 140 | 140 | ESP:431. |
| 141 | 4 | ESP:336. |
| 142 | 7 | ESP:508. |
| 143 | 7 | ESP:518. |
| 144 | 7 | ESN:480. |
| 145 | 8 | ESP:501. |

[Table 139]

| Pr | PSy | Data |
|---|---|---|
| 146 | 8 | ESP:541. |
| 147 | 7 | ESP:505. |
| 148 | 7 | ESP:501. |
| 149 | 2 | ESP:323. |
| 150 | 1 | ESN:397. |
| 151 | 12 | NMR1:5.34(2H,s),6.34(1H,d),6.38(1H,dd),7.32-7.50(5H,m), 7.67(1H,d),10.38-10.62(1H,br),10.69 (1H,s). |
| 152 | 2 | ESN:391. |
| 153 | 1 | NMR2:1.26(3H,t),1.61-1.82(4H,m),1.86-2.04(4H,m),2.35-2. 45(1H,m),4.15(2H,q),4.50-4.56(1H,m), 5.34(2H,s),6.78-6.82 (m,1H),6.97(1H,d),7.31-7.47(5H,m),7.89(1H,d). |
| 154 | 12 | ESP:262, 264. |
| 155 | 10 | ESN:471. |
| 156 | 10 | ESN:471. |
| 157 | 9 | NMR1:1.18(3H,t),1.62-1.88(8H,m),2.42-2.55(1H,m),3.33-3. 48(4H,m),4.06(2H,q),4.67-4.78(1H,m), 7.21-7.35(2H,m),7.45-7.61(3H,m),7.85(2H,d),8.43-8.68(2H,m) |
| 158 | 2 | ESP:361. |
| 159 | 1 | ESN:449. |
| 160 | 12 | NMR2:5.37(2H,s),7.2-7.55(7H,m),7.82-7.96(1H,m) |
| 161 | 2 | ESN:325, 327. |
| 162 | 2 | ESN:307. |
| 163 | 9 | ESP:523. |
| 164 | 121 | NMR1:2.37(3H,s),2.90-3.03(2H,m),3.47-3.59(2H,m),7.52(1 H,d),7.77(1H,dd),7.95(1H,d),8.16(2H,s), 8.83-8.89(1H,s). |
| 165 | 2 | FP:329. |
| 166 | 1 | FP:419. |
| 167 | 12 | ESP:265. |

(continued)

| Pr | PSy | Data |
|---|---|---|
| 168 | 8 | ESP:517. |
| 169 | 8 | ESP:557. |
| 170 | 7 | ESN:539. |
| 171 | 7 | ESP:551. |
| 172 | 7 | ESP:541. |
| 173 | 7 | ESP:521. |
| 174 | 7 | ESP:513. |
| 175 | 8 | ESP:490. |
| 176 | 7 | ESN:488. |
| 177 | 7 | ESP:514. |

[Table 140]

| Pr | PSy | Data |
|---|---|---|
| 178 | 4 | ESP:371. |
| 179 | 3 | ESP:471. |
| 180 | 4 | ESP:371. |
| 181 | 3 | ESP:471. |
| 182 | 2 | ESP:329. |
| 183 | 1 | FP:419. |
| 184 | 12 | EI:264. |
| 185 | 121 | NMR1:2.97-3.01(2H,m),3.53-3.62(2H,m),7.60-7.71(3H,m), 7.96(2H,s),8.21-8.26(2H,s),9.23-9.29 (1H,m). |
| 186 | 121 | ESP:375. |
| 187 | 4 | ESP:349. |
| 188 | 3 | ESP:449. |
| 189 | 2 | NMR2:1.27(3H,t),1.38-1.49(2H,m),1.58-1.67(2H,m),1.70-1. 79(2H,m),1.90-2.00(1H,m),2.01-2.10 (2H,m),2.56-2.63(1H, m),3.87(2H,d),4.15(2H,m),6.92(2H,d),8.04(2H,m). |
| 190 | 1 | ESP:397. |
| 191 | 7 | ESP:467. |
| 192 | 7 | ESP:493. |
| 193 | 7 | ESP:481. |
| 194 | 7 | ESP:495. |
| 195 | 8 | ESP:497. |
| 196 | 4 | ESP:349. |
| 197 | 3 | NMR2:1.23(3H,s),1.28(3H,t),1.44(9H,s),1.62-1.80(4H,m),1. 83-1.99(4H,m),3.38-3.44(2H,m), 3.52-3.58(2H,m),4.17(2H,q), 4.46-4.52(1H,m),4.95-5.02(1H,m),6.91(2H,d),7.01(1H,s),7.77 (2H,d). |
| 198 | 2 | ESP:307. |

(continued)

| Pr | PSy | Data |
|---|---|---|
| 199 | 1 | NMR2:1.23(3H,s),1.27(3H,t),1.66(2H,tt),1.76(2H,tt),1.84-1.9 9(4H,m),4.17(2H,q),4.48-4.53(1H,m), 5.34(2H,s),6.91(2H,d), 7.32-7.47(5H,m),8.02(2H,d). |
| 200 | 8 | ESN:465. |
| 201 | 4 | ESP:319. |
| 202 | 123 | NMR2:1.17(2H,q),1.43(9H,s),1.52(2H,q),1.83-1.95(5H,m),2. 27(2H,d),2.52(1H,tt),3.37-3.44(2H,m), 3.53-3.59(2H,m),3.69 (3H,s),4.97(1H,s),7.05(1H,s),7.26(2H,d),7.74(2H,d). |
| 203 | 8 | ESN:489. |
| 204 | 8 | ESN:489. |
| 205 | 8 | ESN:471. |
| 206 | 7 | ESP:545. |

[Table 141]

| Pr | PSy | Data |
|---|---|---|
| 207 | 7 | ESP:563. |
| 208 | 7 | ESP:497. |
| 209 | 7 | ESN:453. |
| 210 | 126 | ESP:579. |
| 211 | 126 | ESP:579. |
| 212 | 7 | ESP:559. |
| 213 | 7 | ESP:559. |
| 214 | 7 | ESP:481. |
| 215 | 2 | ESP:278. |
| 216 | 121 | ESP:371. |
| 217 | 1 | ESP:385. |
| 218 | 7 | ESP:549. |
| 219 | 121 | NMR1:2.95-3.07(2H,m),3.48-3.60(2H,m),7.33-7.41(1H,m), 7,82(1H,dd),7.92-8.06(3H,br),8.08(1H, dd),8.13(1H,s),9.06-9. 14(1H,m). |
| 220 | 7 | ESP:554. |
| 221 | 7 | ESP:540. |
| 222 | 7 | ESP:526. |
| 223 | 9 | ESN:333 |
| 224 | 8 | ESP:507. |
| 225 | 121 | ESP:353. |
| 226 | 2 | ESN:309. |
| 227 | 1 | NMR2:1.38-1.49(2H,m),1.55-1.69(4H,m),1.86-2.07(3H,m), 2.28(2H,d),3.68(3H,s),4.55-4.61(1H,m), 5.36(2H,s),6.60-6.72 (2H,m),7.31-7.42(3H,m),7.43-7.47(2H,m),7.91(1H,t). |
| 228 | 121 | ESP:353. |
| 229 | 2 | ESN:309. |

(continued)

| Pr | PSy | Data |
|---|---|---|
| 230 | 1 | NMR2:1.43-1.69(6H,m),1.85-1.98(1H,m),1.98-2.08(2H,m), 2.28(2H,d),3.67(3H,s),4.62-4.67(1H,m), 5.33(2H,s),6.97(1H, t),7.32-7.45(5H,m),7.74-7.83(2H,m). |
| 231 | 121 | ESP:371. |
| 232 | 128 | NMR1:1.27-1.39(2H,m),1.45-1.71(4H,m),1.78-1.94(3H,m), 2.25(2H,d),3.59(3H,s),4.72-4.82(1H,m), 7.17-7.29(1H,m),7.5 5-7.67(1H,m). ESN:327. |
| 233 | 1 | NMR2:1.41-1.72(15H,m),1.83-1.98(1H,m),1.98-2.08(2H,m), 2.28(2H,d),3.68(3H,s),4.55-4.62(1H,m), 6.63-6.71(1H,m),7.5 4-7.63(1H,m). |

[Table 142]

| Pr | PSy | Data |
|---|---|---|
| 234 | 1 | NMR1:1.24-1.97(9H,m),2.25(2H,d),3.58(3H,s),4.65-4.73(1 H,m),5.31(2H,s),7.05(2H,d),7.32-7.47 (5H,m),7.92(2H,d). |
| 235 | 2 | ESN:291. |
| 236 | 121 | ESP:335. |
| 237 | 2 | NMR1:1.18(3H,t),1.36-1.61(4H,m),1.90-1.97(2H,m),2.03-2. 09(2H,m),2.35(1H,tt),4.06(2H,q),4.46 (1H,tt),6.85(1H,dd),6.9 2(1H,dd),7.78(1H,t). |
| 238 | 1 | NMR2:1.26(3H,t),1.45-1.67(4H,m),2.06-2.21(4H,m),2.35(1 H,tt),4.14(2H,q),4.26(1H,tt),5.35(2H,s), 6.61(1H,dd),6.68(1H, dd),7.30-7.41(3H,m),7.43-7.47(2H,m),7.90(1H,t). |
| 239 | 128 | ESP:328. |
| 240 | 127 | ESP:384. |
| 241 | 128 | ESN:326. |
| 242 | 127 | ESP:384. |
| 243 | 2 | ESN:327. |
| 244 | 1 | ESP:419. |
| 245 | 2 | NMR1:1.25-1.97(9H,m),2.26(2H,d).3.59(3H,s),4.75-4.82(1 H,m),7.09-7.16(1H,m),7.60-7.67(1H,m). |
| 246 | 1 | NMR1:1.21-1.95(9H,m),2.25(2H,d),3.59(3H,s),4.77-4.83(1 H,m),5.35(2H,s),7.14-7.20(1H,m), 7.32-7.48(5H,m),7.66-7.73 (1H,m). |
| 247 | 121 | ESP:255. |
| 248 | 2 | ESN:308. |
| 249 | 127 | ESP:400. |
| 250 | 4 | ESP:371. |
| 251 | 3 | ESP:471. |
| 252 | 128 | ESN:327. |
| 253 | 1 | NMR2:1.27(3H,t),1.57(9H,s),1.59-1.80(4H,m),1,94-2.10(4H, m),2.34-2.42(1H,m),4.16(2H,q), 4.48-4.54(1H,m),7.52(2H,d). |
| 254 | 2 | ESP:329. |
| 255 | 1 | ESP:419 |
| 256 | 2 | ESP:318. |
| 257 | 127 | ESP:408. |

(continued)

| Pr | PSy | Data |
|---|---|---|
| 258 | 128 | ESN:327. |
| 259 | 1 | NMR2:1.28(3H,t),1.52-1.67(14H,m),2.07-2.22(3Hm),2.32-2.41(1H,m),4.15(2H,q),4.21-4.30(1H,m), 6.67(1H,dd),7.58(1 H,dd). |
| 260 | 2 | ESN:327. |
| 261 | 1 | ESP:419. |

[Table 143]

| Pr | PSy | Data |
|---|---|---|
| 262 | 121 | NMR1:2.92-3.02(2H,m),3.44-3.53(2H,m),7.21(1H,d),7.85(1 H,d),8.16(2H,s), 9.03-9.12(1H,m). |
| 263 | 24 | EI:238. |
| 264 | 23 | ESP:267. |
| 265 | 21 | NMR1:0.65-0.70(2H,m),0.93-1.00(2H,m),1.86-1.95(1H,m), 6.63(1H,d),7.34(1H,d). |
| 266 | 133 | NMR1:0.72-0.82(2H,m),1.13-1.21(2H,m),2.14-2.23(1H,m), 7.57(1H,s),12.9-13.7(1H,br). |
| 267 | 21 | NMR1:0.62-0.67(2H,m),1.04-1.10(2H,m),2.05-2.13(1H,m), 6.96(1H,d),7.38(1H,d). |
| 268 | 24 | ESN:227. |
| 269 | 20 | ESN:175. |
| 270 | 20 | ESN:179. |
| 271 | 20 | ESN:191. |
| 272 | 19 | ESN:205. |
| 273 | 18 | ESN:219. |
| 274 | 20 | ESN:191. |
| 275 | 20 | ESN:205. |
| 276 | 20 | ESN:245. |
| 277 | 20 | ESP:203. |
| 278 | 20 | NMR1:0.77-0.92(2H,m),1.01-1.14(2H,m),2.03-2.15(1H,m), 6.95(1H,dd),7.54(1H,dd). |
| 279 | 20 | ESN:211. |
| 280 | 20 | ESN:211. |
| 281 | 19 | ESN:225. |
| 282 | 22 | ESP:301. |
| 283 | 20 | ESN:217. |
| 284 | 20 | ESN:195. |
| 285 | 20 | ESN:167. |
| 286 | 135 | ESN:245, |
| 287 | 20 | ESN:211. |
| 288 | 22 | NMR1:1.57(3H,d),5.61(1H,q),6.98(2H,d),7.26(1H,t),7.35(2 H,t),7.41(2H,d),7.79(2H,d). |
| 289 | 1 | NMR2:1.66(3H,d),5.30(2H,s),5.38(2H,q),6.86(2H,d),7.30-7. 42(10H,m),7.93(2H,d). |
| 290 | 22 | NMR1:1.57(3H,d),5.61(1H,q),6.97(2H,d),7.26(1H,t),7.35(2 H,t),7.41(2H,d),7.79(2H,d). |

(continued)

| Pr | PSy | Data |
|---|---|---|
| 291 | | NMR2:1.66(3H,d),5.30(2H,s),5.38(2H,q),6.87(2H,d),7.30-7. 43(10H,m),7.93(2H,d). |

[Table 144]

| Pr | PSy | Data |
|---|---|---|
| 292 | 124 | NMR2:1.42(3H,t),4.43(2H,q),7.78(1H,d),8.03(1H,d),8.17(1 H,s). |
| 293 | 2 | ESN:341. |
| 294 | 1 | ESP:433. |
| 295 | 12 | ESN:277. |
| 296 | 4 | ESP:334. |
| 297 | 9 | ESP:434. |
| 298 | 9 | ESP:547. |
| 299 | 2 | ESN:349. |
| 300 | 125 | ESN:325. |
| 301 | 1 | ESP:369. |
| 302 | 2 | ESN:277. |
| 303 | 2 | ESN:263. |
| 304 | 1 | ESP:355. |
| 305 | 2 | ESN:263. |
| 306 | 1 | ESP:355. |
| 307 | 9 | ESP:389. |
| 308 | 9 | ESP:487. |
| 309 | 2 | ESP:291. |
| 310 | 1 | ESP:381, |
| 311 | 9 | ESP:532. |
| 312 | 2 | FN:334. |
| 313 | 1 | ESP:426. |
| 314 | 9 | ESP:475. |

[Table 145]

| Ex | Syn | Data |
|---|---|---|
| 1 | 1 | ESN : 409. |
| 2 | 2 | ESP : 466. |
| 3 | 3 | ESP : 451. |
| 4 | 4 | ESP : 515. |
| 5 | 5 | ESN : 493. |
| 6 | 6 | ESP : 443. |

(continued)

| Ex | Syn | Data |
|---|---|---|
| 7 | 7 | ESP : 522. |
| 8 | 8 | ESP : 444. |
| 9 | 9 | ESP : 469. |
| 10 | 1 | ESN : 424. |
| 11 | 1 | ESN : 409. |
| 12 | 1 | ESP : 493. |
| 13 | 1 | ESN : 443. |
| 14 | 1 | ESP : 411. |
| 15 | 1 | NMR1 : 1.62-1.86(8H,m),2.32-2.44(1H,m),3.39-3.43(4H,m), 4.59-4.65(1H,m),6.99(2H,d),7.54(2H,d), 7.79(2H,d),7.86(2H, d),8.43-8.48(1H,m),8.66-8.72(1H,m). ESN : 443. |
| 16 | 1 | ESP : 527. |
| 17 | 1 | ESP : 527. |
| 18 | 1 | NMR1 : 1.33(3H,t),1.55-1.70(4H,m),1.75-1.86(4H,m),2.16-2.23(1H,m),3.38-3.42(4H,m),4.06(2H,q), 4.54-4.60(1H,m),6. 94(2H,d),6.99(2H,d),7.82-7.87(4H,m),8.80-8.90(2H,m). ESP : 455. |
| 19 | 1 | ESP : 455. |
| 20 | 1 | ESP : 513. |
| 21 | 1 | ESP : 445. |
| 22 | 1 | ESP : 445. |
| 23 | 1 | ESP : 499. |
| 24 | 1 | ESP : 527. |
| 25 | 1 | ESP : 527. |
| 26 | 1 | ESP : 429. |
| 27 | 1 | ESP : 451. |
| 28 | 1 | ESP : 494. |

[Table 146]

| Ex | Syn | Data |
|---|---|---|
| 29 | 1 | NMR1 : 1.62-1.83(8H,m),2.34-2.41(1H,m),3.40-3.43(4H,m), 4.60-4.65(1H,m),6.85(1H,dd),6.90(1H, dd),7.54(2H,d),7.61(1 H,t),7.86(2H,d),8.16-8.20(1H,m),8.63-8.68(1H,m). ESP : 463. |
| 30 | 1 | ESP : 463. |
| 31 | 2 | ESP : 460. |
| 32 | 5 | ESN : 492. |
| 33 | 3 | ESP : 425. |
| 34 | 3 | ESP : 469. |
| 35 | 3 | ESP : 503. |
| 36 | 3 | ESP : 475. |
| 37 | 3 | ESP : 463. |
| 38 | 1 | ESN : 415. |

(continued)

| Ex | Syn | Data |
|---|---|---|
| 39 | 1 | ESN : 415. |
| 40 | 1 | ESN : 449. |
| 41 | 1 | NMR1 : 1.62-1.85(8H,m),2.34-2.42(1H,m),3.42-3.45(4H,m), 4.60-4.65(1H,m),6.99(2H,d),7.80(2H,d), 7.86(2H,d),8.04(2H, d),8.44-8.49(1H,m),8.82-8.86(1H,m). ESP : 479. |
| 42 | 1 | ESP : 417. |
| 43 | 1 | ESP : 441. |
| 44 | 1 | ESP : 429. |
| 45 | 1 | NMR1 : 1.62-1.85(8H,m),2.34-2.41(1H,m),3.39-3.45(4H,m), 4.60-4.65(1H,m),7.00(2H,d),7.47(2H,d), 7.80(2H,d),7.97(2H, d),8.43-8.47(1H,m),8.70-8.74(1H,m). ESP : 495. |
| 46 | 1 | ESP : 527. |
| 47 | 3 | ESP : 441. |
| 48 | 3 | ESP : 445. |
| 49 | 3 | ESP : 450. |
| 50 | 3 | ESP : 450. |
| 51 | 5 | ESN : 450. |
| 52 | 3 | ESN : 463. |
| 53 | 3 | ESP : 467. |
| 54 | 3 | ESP : 481. |
| 55 | 3 | ESP : 525. |
| 56 | 2 | ESP : 460. |

[Table 147]

| Ex | Syn | Data |
|---|---|---|
| 57 | 2 | ESP : 460. |
| 58 | 3 | ESP : 503. |
| 59 | 3 | ESP : 441. |
| 60 | 3 | ESP : 427. |
| 61 | 3 | ESP : 446. |
| 62 | 3 | ESP : 446. |
| 63 | 3 | ESP : 521. |
| 64 | 1 | NMR1 : 1.73(3H,t),1.62-1.84(8H,m),2.35-2.42(1H,m),3.37-3.42(4H,m),4.18(2H,q),4.60-4.65(1H,m), 6.99(2H,d),7.21(1H, d),7.80(2H,d),7.82(1H,dd),7.93(1H,d),8.42-8.47(1H,m),8.55-8.59(1H,m). ESP : 489. |
| 65 | 1 | ESP : 489. |
| 66 | 1 | ESP : 479. |
| 67 | 1 | NMR1 : 1.62-1.84(8H,m),2.34-241(1H,m),3.39-3.43(4H,m), 4.60-4.65(1H,m),7.00(2H,d),7.74-7.84 (4H,m),8.07(1H,d),8.4 3-8.47(1H,m),8.77-8.82(1H,m). ESP : 479. |
| 68 | 3 | ESP : 451. |

(continued)

| Ex | Syn | Data |
|---|---|---|
| 69 | 3 | ESP : 455. |
| 70 | 3 | ESP : 519. |
| 71 | 3 | ESP : 447. |
| 72 | 3 | ESP : 459. |
| 73 | 3 | ESP : 459. |
| 74 | 3 | ESP : 485. |
| 75 | 3 | ESP : 513. |
| 76 | 3 | ESP : 428. |
| 77 | 3 | ESP : 475. |
| 78 | 3 | ESP : 475. |
| 79 | 1 | NMR1 : 0.68-0.74(2H,m),0.95-1.01(2H,m),1.50-1.70(4H,m), 1.74-1.90(4H,m),1.95(1H,tt),2.05-2.11 (1H,m),3.42-3.48(4H, m),4.51-4.56(1H,m),7.00(2H,d),7.10(2H,d),7.79(2H,d),7.87 (2H,d),9.04-9.28 (2H,m). ESP : 451. |
| 80 | 1 | ESP : 475. |
| 81 | 1 | ESP : 487. |
| 82 | 1 | ESP : 497. |

[Table 148]

| Ex | Syn | Data |
|---|---|---|
| 83 | 1 | NMR1 : 1.54-1.72(4H,m),1.75-1.88(4H,m),2.15-2.23(1H,m), 3.45-3.54(4H,m),4.54-4.59(1H,m),7.01 (2H,d),7.55-7.62(2H, m),7.87(2H,d),7.94-8.01(4H,m),8.51(1H,s),8.91-8.99(1H,m), 9.28-9.38(1H,m). ESP : 461. |
| 84 | 1 | ESN : 459. |
| 85 | 3 | ESP : 446. |
| 86 | 3 | ESP : 446. |
| 87 | 3 | ESP : 446. |
| 88 | 3 | ESP : 446. |
| 89 | 3 | ESP : 561. |
| 90 | 3 | ESP : 553. |
| 91 | 3 | ESP : 453. |
| 92 | 3 | NMR1 : 1.59-1.86(8H,m),2.30-2.45(1H,m),3.22-3.48(4H,m), 4.59-4.68(1H,m),6.99(2H,d),7.25(2H,d), 7.34(1H,t), 7.79(2H, d),7.91(2H,d),8.41-8.48(1H,m),8.60-8.66(1H,m),12.02-12.27 (1H,m). ESP : 477. |
| 93 | 3 | ESP : 467. |
| 94 | 3 | ESP : 511. |
| 95 | 3 | ESP : 455. |
| 96 | 3 | ESP : 451. |
| 97 | 7 | ESP : 551. |
| 98 | 1 | ESP : 446. |

(continued)

| Ex | Syn | Data |
|---|---|---|
| 99 | 1 | ESP : 446. |
| 100 | 3 | ESP : 525. |
| 101 | 3 | ESP : 528. |
| 102 | 3 | ESP : 533. |
| 103 | 1 | NMR1:1.55-1.86(8H,m),2.29(3H,s),2.30-2.42(1H,m),3.20-3. 53(4H,m),4.53-4.64(1H,m),6.74-6.84 (2H,m),7.31(1H,d),7.55 (2H,d),7.87(2H,d),8.15-8.24(1H,m),8.57-8.68(1H,m),12.02-1 2.30(1H,bs). ESP : 459. |
| 104 | 3 | ESP : 495. |
| 105 | 3 | ESP : 463. |

[Table 149]

| Ex | Syn | Data |
|---|---|---|
| 106 | 3 | NMR1:1.59-1.88(8H,m),2.30-2.44(4H,m),3.36-3.47(4H,m), 4.58-4.67(1H,m),7.01(2H,d),7.47-7.55 (1H,m),7.64-7.72(1H, m),7.75-7.88(3H,m),8.40-8.50(1H,m),8.59-8.70(1H,m),12.0 2-12.30(1H,bs). ESP : 459. |
| 107 | 3 | ESP : 425. |
| 108 | 3 | NMR1 : 1.60-1.84(8H,m),2.32-2.42(1H,m),3.38-3.44(4H,m), 4.60-4.64(1H,m),7.00(2H,d),7.77-7.87 (5H,m),8.42-8.44(1H, m),8.80-8.84(1H,m). ESP : 479. |
| 109 | 3 | ESP : 465. |
| 110 | 3 | NMR1 : 1.58-1.86(8H,m),2.30-2.45(1H,m),3.22-3.48(4H,m), 4.58-4.66(1H,m),6.99(2H,d),7.54(1H,d), 7.62(1H,t),7.76-7.83 (3H,d),7.88(1H,d),8.43-8.49(1H,m),8.75-8.81(1H,m),12.02-1 2.27(1H,m). ESP : 495. |
| 111 | 2 | ESP : 510. |
| 112 | 2 | ESP : 494. |
| 113 | 1 | ESP : 459. |
| 114 | 3 | ESN : 505. |
| 115 | 3 | ESP : 515. |
| 116 | 3 | ESP : 509. |
| 117 | 3 | ESP : 455. |
| 118 | 3 | NMR1 : 1.58-1.86(12H,m),2.01-2.16(2H,m),2.30-2.45(1H, m),2.63-2.81(1H,m),3.22-3.48(4H,m),4.00 (2H,d),4.58-4.65(1 H,m),6.94-7.02(4H,m),7.77-7.83(4H,m),8.40-8.48(2H,m),12. 02-12.27(1H,m). ESP : 495. |
| 119 | 3 | NMR1 : 1.35(3H,t),1.61-1.85(8H,m),2.31-2,43(1H,m),3.22-3.48(4H,m),4.16(2H,q),4.59-4.65(1H,m), 6.99(2H,d),7.23(1H, t),7.65-7.71(2H,m),7.79(2H,d),8.42-8.47(1H,m),8.51-8.57(1 H,m),12.01-12.32 (1H,m). ESP : 473. |

[Table 150]

| Ex | Syn | Data |
|---|---|---|
| 120 | 3 | NMR1 : 0.31-0.38(2H,m),0.55-0.61(2H,m),1.19-1.30(1H,m), 1.61-1.85(8H,m),2.31-2.43(1H,m), 3.22-3.45(4H,m),3.95(2H, d),4.59-4.65(1H,m),6.99(2H,d),7.20(1H,d),7.63-7.72(2H,m), 7.79(2H,d), 8.42-8.49(1H,m),8.51-8.59(1H,m),12.01-12.36(1 H,m). ESP : 499. |
| 121 | 3 | NMR1 : 0.31-0.38(2H,m),0.55-0.61(2H,m),1.19-1.30(1H,m), 1.61-1.85(8H,m),2.31-2.43(1H,m), 3.22-3.45(4H,m),3.95(2H, d),4.59-4.65(1H,m),6.99(2H,d),7.18(1H,d),7.76-7.84(3H,m), 7.93(1H,d), 8.43-8.49(1H,m),8.56-8.62(1H,m). ESP : 515. |
| 122 | 3 | ESP : 499. |
| 123 | 3 | ESP : 480. |
| 124 | 3 | ESP : 484. |
| 125 | 1 | NMR1 : 1.62-1.85(8H,m),2.34-2.42(1H,m),3.40-3.44(4H,m), 4.60-4.64(1H,m),6.99(2H,d),7.71(1H,t), 7.77-7.82(3H,m),7.9 2(1H,d),8.43-8.47(1H,m),8.78-8.83(1H,m). ESN : 511. |
| 126 | 1 | NMR1 : 1.62-1.85(8H,m),2.34-2.42(1H,m),3.40-3.44(4H,m), 4.60-4.64(1H,m),6.99(2H,d),7.70(1H, dd),7.79(2H,d),7.93(1 H,dd),8.12(1H,d),8.43-8.48(1H,m),8.80-8.85(1H,m). ESN : 527. |
| 127 | 1 | ESN : 495. |
| 128 | 3 | ESP : 497. |
| 129 | 3 | NMR1 : 1.62-1.83(8H,m),2.31-2.43(1H,m),3.42-3.48(4H,m), 4.64-4.73(1H,m),7.28(1H,t),7.62-7.72 (2H,m),7.93-8.03(2H, m),8.11(1H,s),8.53-8.58(1H,m),8.91-8.96(1H,m),12.11-12.23 (1H,m). ESP : 531. |
| 130 | 4 | ESP : 513. |
| 131 | 3 | ESP : 531. |
| 132 | 3 | NMR1 : 1.62-1.83(8H,m),2.31-2.43(1H,m),3.42-3.48(4H,m), 4.65-4.70(1H,m),7.28(1H,t),7.60-7.72 (3H,m),7.92(1H,dd),8.1 1(1H,d),8.51-8.58(1H,m),8.79-8.86(1H,m),12.07-12.23(1H, m). ESP : 547. |
| 133 | 3 | ESP : 469. |
| 134 | 3 | ESP : 487. |

[Table 151]

| Ex | Syn | Data |
|---|---|---|
| 135 | 3 | NMR1 : 0:73-0.80(2H,m),1.02-1.09(2H,m),1.63-1.87(8H,m), 2.14-2.25(1H,m),2.31-2.42(1H,m), 3.28-3.43(4H,m),4.64-4.7 1(1H,m),7.09(1H,d),7.28(1H,t),7.62-7.72(3H,m),7.88(1H,s), 8.51-8.57(1H, m),8.61-8.68(1H,m),12.05-12.23(1H,m). ESP : 503. |
| 136 | 3 | ESP : 491. |
| 137 | 3 | NMR1 : 1.37(3H,t),1.63-1.86(8H,m),2.31-2.43(1H,m),3.41-3.45(4H,m),4.18(2H,q),4.63-4.71(1H,m), 7.21(1H,d),7.28(1H, t),7.61-7.72(2H,m),7.81(1H,dd),7.93(1H,d),8.51-8.62(2H,m), 12.01-12.37(1H, m). ESP : 507./509. |
| 138 | 3 | NMR1 : 1.62-1.83(8H,m),2.31-2.43(1H,m),3.42-3.48(4H,m), 4.63-4.73(1H,m),7.28(1H,t),7.62-7.85 (4H,m),8.07(1H,s),8.4 9-8.58(1H,m),8.73-8.83(1H,m),12.07-12.24(1H,m). ESP : 497./499. |
| 139 | 3 | ESP : 469. |
| 140 | 3 | ESP : 499. |
| 141 | 3 | ESP : 517. |
| 142 | 3 | NMR1 : 0.33-0.38(2H,m),0.56-0.63(2H,m),1.19-1.31(1H,m), 1.61-1.87(8H,m),2.31-2.43(1H,m), 3.22-3.45(4H,m),3.98(2H, d),4.65-4.70(1H,m),7.19(1H,d),7.28(1H,t),7.61-7.72(2H,m), 7.79(1H,dd), 7.93(1H,d),8.51-8.61(2H,m). ESP : 533. |

(continued)

| Ex | Syn | Data |
|---|---|---|
| 143 | 3 | ESP : 477. |
| 144 | 3 | ESP : 529. |
| 145 | 1 | NMR1 : 1.59-1.85(8H,m),2.27-2.36(1H,m),2.37(3H,s),3.39-3.43(4H,m),4.57-4.63(1H,m),7.00(2H,d), 7.44(1H,d),7.73(1H, d),7.81(2H,d),7.90(1H,s),8.53-8.63(1H,m),8.78-8.88(1H,m). ESP : 459. |
| 146 | 1 | NMR1 : 0.74-0.79(2H,m),1.02-1.09(2H,m),1.62-1.85(8H,m), 2.19(1H,tt),3.33-2.42(1H,m),3.38-3.42 (4H,m),4.60-4.65(1H, m),6.99(2H,d),7.09(1H,d),7.71(1H,d),7.79(2H,d),7.88(1H,s), 8.41-8.46(1H,m), 8.62-8.67(1H,m). ESP : 485. |

[Table 152]

| Ex | Syn | Data |
|---|---|---|
| 147 | 1 | NMR1 : 1.62-1.85(8H,m),2.34-2.42(1H,m),3.41-3.46(4H,m), 4.60-4.65(1H,m),7.00(2H,d),7.80(2H,d), 7.94-8.02(2H,m),8.1 2(1H,s),8.43-8.48(1H,m),8.91-8.95(1H,m). ESP : 513. |
| 148 | 3 | ESP : 534. |
| 149 | 1 | ESP : 475. |
| 150 | 3 | ESP : 518. |
| 151 | 3 | NMR1 : 1.60-1.97(12H,m),2.01-2.16(2H,m),2.31-2.42(1H, m),2.63-2.79(1H,m),3.22-3.48(4H,m),4.10 (2H,d),4.58-4.65(1 H,m),6.99(2H,d),7.22(1H,d),7.76-7.84(3H,m),7.92(1H,d),8.4 1-8.48(1H,m), 8.51-8.60(1H,m),12.02-12.37(1H,m). ESP : 529. |
| 152 | 3 | NMR1 : 0.76-0.81(2H,m),0.99-1.06(2H,m),1.61-1.85(8H,m), 2.04-2.11(1H,m),2.31-2.43(1H,m), 3.22-3.45(4H,m),4.59-4.6 5(1H,m),6.99(2H,d),7.06(1H,t),7.55-7.62(2H,m),7.79(2H,d), 8.41-8.49(1H, m),8.57-8.62(1H,m),12.02-12,27(1H,m). ESP : 469. |
| 153 | 3 | ESP : 469. |
| 154 | 1 | NMR1:1.58-1.84(8H,m),2.29-2.44(1H,m),3.30-3.50(4H,m), 4.59-4,68(1H,m),6.92-7.00(1H,m), 7.03-7.10(1H,m),7.40(1H, d),7.56(2H,d),7.87(2H,d),8.36-8.45(1H,m),8.58-8.66(1H,m). ESN : 477. |
| 155 | 1 | ESN : 477. |
| 156 | 3 | NMR1 : 1.62-1.85(8H,m),2.31-2.45(1H,m),3.42-3.45(4H,m), 4.59-4.65(1H,m),6.82-6.94(2H,m),7.62 (1H,t),7.86(2H,d),8.0 3(2H,d),8.15-8.22(1H,m),8.36-8.53(1H,m),12.00-12.31(1H, m). ESN : 495. |
| 157 | 4 | NMR1 : 1.62-1.85(8H,m),2.31-2.45(1H,m),3.42-3.45(4H,m), 4.59-4.65(1H,m),6.82-6.95(2H,m),7.61 (1H,t),7.82-7.97(3H, m),8.15-8.22(1H,m),8.84-8.90(1H,m),12.00-12.31(1H,m). ESP : 515. |
| 158 | 3 | NMR1 : 1.62-1.83(8H,m),2.31-2.43(1H,m),3.42-3.48(4H,m), 4.59-4.66(1H,m),6.80-6.93(2H,m),7.61 (1H,t),7.93-8.02(3H, m),8.11(1H,s),8.15-8.22(1H,m),8.87-8.93(1H,m). ESP : 531. |
| 159 | 3 | NMR1 : 1.61-1.85(8H,m),2.31-2.45(1H,m),341-3.45(4H,m), 4.59-4.65(1H,m),6.81-6.94(2H,m),7.61 (1H,t),7.71(1H,t),7.80 (1H,d),7.92(1H,dd),8.15-8.21(1H,m),8.72-8.79(1H,m),12.04-12.32(1H,m). ESP : 531. |

[Table 153]

| Ex | Syn | Data |
|---|---|---|
| 160 | 3 | NMR1 : 1.62-1.83(8H,m),2.31-2.43(1H,m),3.42-3.48(4H,m), 4.59-4.66(1H,m),6.81-6.94(2H,m),7.61 (1H,t),7.70(1H,d),7.9 2(1H,dd),8.11(1H,d),8.15-8.22(1H,m),8.87-8.93(1H,m),12.0 3-12.22(1H,m). ESP : 547. |

(continued)

| Ex | Syn | Data |
|---|---|---|
| 161 | 3 | NMR1 : 0.75-0.81(2H,m),0.99-1.06(2H,m),1.61-1.85(8H,m), 2.04-2.13(1H,m),2.31-2.42(1H,m), 3.28-3.45(4H,m),4.59-4.6 7(1H,m),6.83-6.93(2H,m),7.06(1H,t),7.54-7.64(3H,m),8.11-8.20(1H,m), 8.53-8.59(1H,m),12.06-12.25(1H,m). ESP : 487. |
| 162 | 3 | NMR1 : 0.75-0.79(2H,m),1.02-1.09(2H,m),1.61-1.84(8H,m), 2.15-2.25(1H,m),2.31-2.42(1H,m), 3.28-3.45(4H,m),4.60-4.6 7(1H,m),6.83-6.93(2H,m),7.09(1H,d),7.61(1H,t),7.70(1H,d d),7.87(1H,d), 8.13-8.20(1H,m),8.58-8.64(1H,m),12.10-12. (1H,m). ESP : 503. |
| 163 | 3 | NMR1 : 1.36(3H,t),1.61-1.85(8H,m),2.31-2.45(1H,m),3,41-3.45(4H,m),4.16(2H,q),4.58-4.65(1H,m), 6.80-6.95(2H,m),7. 23(1H,t),7.61(1H,d),7.64-7.73(2H,m),8.12-8.21(1H,m),8.46-8.58(1H,m), 12.01-12.27(1H,m). ESP : 491. |
| 164 | 3 | NMR1 : 1.36(3H,t),1.61-1.85(8H,m),2.31-2.45(1H,m),3.41-3.45(4H,m),4.17(2H,q),4.58-4.65(1H,m), 6.82-6.95(2H,m),7. 21(1H,d),7.61(1H,d),7.80(1H,d),7.92(1H,s),8.12-8.21(1H,m), 8.49-8.58(1H,m), 12.07-12.25(1H,m). ESP : 507. |
| 165 | 3 | NMR1 : 1.61-1.85(8H,m),2.31-2.45(1H,m),3.41-3.45(4H,m), 4.58-4.65(1H,m),6.82-6.94(2H,m) 7.61 (1H,d),7.76(1H,d),7.8 1(1H,dd),8.07(1H,d),8.14-8.21(1H,m),8.72-8.78(1H,m),12.0 4-12.25(1H,m). ESP : 497,499. |
| 166 | 3 | ESP : 513. |
| 167 | 3 | NMR1 : 1.62-1.85(8H,m),2.31-2.45(1H,m),3.42-3.45(4H,m), 4.58-4.66(1H,m),6.82-6.94(2H,m),7.24 (2H,d),7.35(1H,d),7.6 1(1H,t),7.90(2H,d),8.13-8.22(1H,m),8.56-8.63(1H,m),12.00-12.35(1H,m). ESP : 495. |

[Table 154]

| Ex | Syn | Data |
|---|---|---|
| 168 | 3 | ESP : 469. |
| 169 | 4 | NMR1 : 1.62-1.85(8H,m),2.31-2.45(1H,m),3.42-3.54(4H,m), 4.60-4.67(1H,m),6.83-6.94(2H,m), 7.56-7.67(3H,m),7.91-8.0 4(4H,m),8.17-8.24(1H,m),8.44(1H,s),8.72-8.77(1H,m),12.00-12.27(1H,br). ESP : 479. |
| 170 | 3 | ESP : 533. |
| 171 | 3 | ESP : 517. |
| 172 | 3 | ESP : 531. |
| 173 | 3 | ESP : 547. |
| 174 | 1 | ESP : 493. |
| 175 | 1 | ESP : 493. |
| 176 | 1 | ESP : 493. |
| 177 | 3 | ESP : 462. |
| 178 | 3 | ESP : 462. |
| 179 | 3 | NMR1 : 1.60-1.97(12H,m),2.01-2.13(2H,m),2.31-2.42(1H, m),2.63-2.79(1H,m),3.22-3.48(4H,m),4.0 (2H,d),4.58-4.65(1 H,m),6.99(2H,d),7.24(1H,d),7.64-7.70(2H,m),7.79(2H,d),8.4 2-8.49(1H,m), 8.51-8.57(1H,m),12.01-12.32(1H,m). ESP : 513. |
| 180 | 3 | ESP : 513, |
| 181 | 3 | ESP : 535. |
| 182 | 3 | ESP : 527. |

(continued)

| Ex | Syn | Data |
|---|---|---|
| 183 | 1 | NMR1 : 1.33(3H,t),1.60-1.84(8H,m),2.30(3H,s),2.32-2.40(1 H,m),3.34-3.41(4H,m),4.07(2H,q), 4.54-4.60(1H,m),6.75-6.8 0(2H,m),6.97(2H,d),7.30(1H,d),7.80(2H,d),8.15-8.20(1H,m), 8.36-8.41(1H, m). ESP : 469. |
| 184 | 1 | NMR1 : 1.37(3H,t),1.60-1.83(8H,m),2.30(3H,s),3.36-3.40(4 H,m),4.18(2H,q),4.54-4.60(1H,m), 6.75-6.81(2H,m),7.21(1H, d),7.30(1H,d),7.81(1H,d),7.92(1H,s),8.16-8.21(1H,m),8.49-8. 54(1H,m). ESP : 503. |
| 185 | 1 | NMR1 : 1.52-1.81(8H,m),2.10-2.20(1H,m),2.30(3H,s),2.37 (3H,s),3.37-3.43(4H,m),4.48-4.53(1H,m), 6.72-6.78(2H,m),7. 31(1H,d),7.50(1H,d),7.69(1H,d),7.85(1H,s),8.29-8.36(1H,m), 8.71-8.77(1H,m). ESP : 473. |

[Table 155]

| Ex | Syn | Data |
|---|---|---|
| 186 | 3 | NMR1 : 0.67-0.78(2H,m),0.95-1.50(2H,m),1.57-1.86(8H,m), 1.88-2.02(1H,m),2.30(3H,s),2.31-2.43 (1H,m),3.26-3.50(4H, m),4.53-4.62(1H,m),6.74-6.82(2H,m),7.15(2H,d),7.26-7.34(1 H,m),7.74(2H,d), 8.15-8.23(1H,m),8.40-8.50(1H,m). ESP : 465. |
| 187 | 3 | NMR1 : 1.56-1.86(8H,m),2.30(3H,s),2.31-2.41(1H,m),3.30-3.52(4H,m),4.52-4.61(1H,m),6.74-6.82 (2H,m),7.28-7.35(1H, m),7.84(2H,d),8.03(2H,d),8.18-8.26(1H,m),8.75-8.83(1H,m). ESP : 493. |
| 188 | 3 | NMR1 : 1.57-1.86(8H,m),2.30(3H,s),2.32-2.43(1H,m),3.30-3.50(4H,m),4.53-4.61(1H,m),6.74-6.83 (2H,m),7.27-7.34(1H, m),7.74-7.86(2H,m),8.05-8.10(1H,m),8.17-8.25(1H,m),8.70-8.80(1H,m). ESP : 493. |
| 189 | 3 | NMR1 : 0.27-0.37(2H,m),0.51-0.62(2H,m),1.14-1.30(1H,m), 1.56-1.87(8H,m),2.30(3H,s),2.32-2.42 (1H,m),3.28-3.48(4H, m),3.83-3.90(2H,m),4.52-4.62(1H,m),6.74-6.83(2H,m),6.96 (2H,d),7.27-7.34 (1H,m),7.78(2H,d),8.15-8.24(1H,m),8.34-8. 43(1H,m). ESP : 495. |
| 190 | 4 | NMR1 : 1.57-1.85(8H,m),230(3H,s),2.31-2.41(1H,m),3.25-3.48(4H,m),4.53-4.61(1H,m),6.74-6.82 (2H,m),7.27-7.34(1H, m),7.46(2H,d),7.95(2H,d),8.16-8.24(1H,m),8.63-8.71(1H,m). ESP : 509. |
| 191 | 3 | NMR1 : 1:20-1.42(2H,m),1.58-1.90(10H,m),1.90-2.11(1H, m),2.31(3H,s),2.32-2.44(1H,m),3.26-3.46 (6H,m),3.84-3.93(4 H,m),4.54-4.62(1H,m),6.74-6.82(2H,m),6.98(2H,d),7.28-7.3 4(1H,m),7.80(2H,d), 8.14-8.23(1H,m),8.36-8.44(1H,m). ESP : 539. |
| 192 | 2 | NMR1 : 1.57-1.86(8H,m),2.24-2.42(4H,m),3.20-3.46(4H,m), 4.53-4.62(1H,m),6.72-6.82(2H,m), 6.90-6.98(1H,m),7.08-7.1 6(1H,m),7.18-7.42(3H,m),8.04-8.20(3H,m). ESP : 474. |
| 193 | 3 | ESP : 527. |
| 194 | 3 | ESP : 499. |
| 195 | 3 | ESP : 509. |
| 196 | 3 | ESP : 497. |
| 197 | 3 | ESP : 497. |
| 198 | 3 | ESP : 527. |
| 199 | 3 | ESP : 527. |

[Table 156]

| Ex | Syn | Data |
|---|---|---|
| 200 | 200 | ESP:475. |

(continued)

| Ex | Syn | Data |
|---|---|---|
| 201 | 201 | ESP:475. |
| 202 | 202 | ESP:447. |
| 203 | 203 | ESP:474. |
| 204 | 204 | ESP:503. |
| 205 | 205 | ESP:518. |
| 206 | 206 | ESP:512. |
| 207 | 207 | ESP:433. |
| 208 | 208 | ESP:447. |
| 209 | 209 | ESN:430. |
| 210 | 210 | ESP:474. |
| 211 | 3 | ESP:464. |
| 212 | 3 | ESP:517. |
| 213 | 3 | ESP:480. |
| 214 | 3 | ESP:521. |
| 215 | 3 | ESP:513, 515. |
| 216 | 3 | ESP:491. |
| 217 | 3 | ESP:461. |
| 218 | 3 | ESP:463, 465. |
| 219 | 3 | ESP: 513, 515. |
| 220 | 3 | ESP:529, 531. |
| 221 | 3 | ESP:477. |
| 222 | 3 | ESP:477. |
| 223 | 3 | ESP:491. |
| 224 | 3 | ESP:527. |
| 225 | 3 | ESP:543. |
| 226 | 4 | ESP:511. |
| 227 | 3 | ESP:527. |
| 228 | 3 | ESP:527. |
| 229 | 3 | ESP:477. |
| 230 | 3 | ESP:489, 491. |
| 231 | 3 | ESP:493, 495. |
| 232 | 3 | ESP:499. |
| 233 | 3 | ESP:465. |
| 234 | 3 | ESP:475. |
| 235 | 3 | ESP:465. |
| 236 | 3 | ESP:469. |

[Table 157]

| Ex | Syn | Data |
|---|---|---|
| 237 | 3 | ESP:443. |
| 238 | 3 | ESP:463. |
| 239 | 3 | ESP:497. |
| 240 | 3 | ESP:439 |
| 241 | 3 | ESP:479, 481. |
| 242 | 3 | ESP:509. |
| 243 | 3 | ESP:487. |
| 244 | 1 | ESN:478. |
| 245 | 1 | ESN:488. |
| 246 | 1 | ESN:450. |
| 247 | 1 | ESN:471. |
| 248 | 1 | ESP:513. |
| 249 | 1 | ESP:477. |
| 250 | 1 | ESN:471. |
| 251 | 3 | ESP:485. |
| 252 | 3 | ESP:499. |
| 253 | 3 | ESP:503. |
| 254 | 3 | ESP:478. |
| 255 | 3 | ESP:548. |
| 256 | 3 | ESP:532. |
| 257 | 3 | ESP:498. |
| 258 | 3 | ESP:483. |
| 259 | 3 | ESP:495. |
| 260 | 2 | ESP:528. |
| 261 | 2 | ESP:474. |
| 262 | 200 | ESP:485. |
| 263 | 1 | ESN:457. |
| 264 | 1 | ESN:457. |
| 265 | 1 | ESP:513. |
| 266 | 3 | ESP:459. |
| 267 | 3 | ESP:483. |
| 268 | 3 | ESP:465. |
| 269 | 4 | ESP:475. |
| 270 | 4 | ESP:495. |
| 271 | 3 | ESP:511. |
| 272 | 3 | ESP:495. |
| 273 | 3 | ESP:503, |

[Table 158]

| Ex | Syn | Data |
|---|---|---|
| 274 | 3 | ESP:503. |
| 275 | 3 | ESP:519, 521. |
| 276 | 3 | ESP:499. |
| 277 | 3 | ESP:469. |
| 278 | 200 | ESP:461. |
| 279 | 3 | ESP:439. |
| 280 | 3 | ESP:453. |
| 281 | 3 | ESP:473. |
| 282 | 1 | ESP:495. |
| 283 | 1 | ESP:489. |
| 284 | 1 | ESP:529. |
| 285 | 1 | ESN:511. |
| 286 | 1 | ESP:523. |
| 287 | 1 | ESP:513. |
| 288 | 1 | ESP:493. |
| 289 | 1 | ESP:485. |
| 290 | 3 | ESP:465. |
| 291 | 3 | ESP:479. |
| 292 | 3 | ESP:483. |
| 293 | 3 | ESP:489. |
| 294 | 3 | ESN:491. |
| 295 | 1 | ESN:460. |
| 296 | 1 | ESP:460. |
| 297 | 1 | ESP:486. |
| 298 | 1 | ESP:457. |
| 299 | 4 | NMR1:1.6-1.84(8H,m),2.32-2.44(1H,m),3.40-3.52(4H,m),4.5 8-4,66(1H,m),6.77-6.85(2H,m), 7.56-7.66(2H,m),7.91-8.05(4H, m),8.45(1H,s),8.66-8.72(2H,m),12-12.3(1H,br). ESP : 497. |
| 300 | 4 | NMR1:1.6-1.94(8H,m),2.32-2.45(1H,m),3.42-3.6(4H,m),4.67-4.76(1H,m),7.10-7.20(1H,m),7.39-7.50 (1H,m),7.55-7.67(2H, m),7.90-8.10(4H,m),8.36-8.43(1H,m),8.45(1H,s),8.70-8.83(1H, m),12.00-12.30 (1H,br). ESP : 497. |
| 301 | 4 | ESN:479. |
| 302 | 4 | ESN:479. |
| 303 | 3 | ESN:485. |
| 304 | 3 | ESN:513. |

[Table 159]

| Ex | Syn | Data |
|---|---|---|
| 305 | 3 | ESN:529. |
| 306 | 3 | ESN:493. |
| 307 | 3 | ESN:485. |
| 308 | 3 | ESN:480. |
| 309 | 3 | ESP:467. |
| 310 | 3 | ESP:495. |
| 311 | 3 | ESP:509. |
| 312 | 3 | ESP:529, 531. |
| 313 | 3 | ESP:546. |
| 314 | 200 | ESP:546. |
| 315 | 3 | ESP:491. |
| 316 | 4 | ESP:501. |
| 317 | 3 | ESP:485. |
| 318 | 3 | ESP:485. |
| 319 | 3 | ESP:485. |
| 320 | 3 | ESP:485. |
| 321 | 3 | ESN:525. |
| 322 | 3 | ESN:492. |
| 323 | 2 | ESN:476. |
| 324 | 5 | ESN:508. |
| 325 | 5 | ESP:508. |
| 326 | 3 | NMR1:1.55-1.89(8H,m),2.29-2.46(1H,m),3.37-3.48(4H,m),4.5 7-4.69(1H,m),6.99(2H,d),7.38-7.52 (2H,d),7.81(2H,d),7.90-8.12 (3H,m),8.43-8.56(1H,m),8.83-8.99(1H,m),12.06-12.26(1H,m). ESP:467. |
| 327 | 3 | ESP:467. |
| 328 | 3 | ESP:481. |
| 329 | 3 | ESP:495. |
| 330 | 3 | ESP:535. |
| 331 | 3 | ESP:519. |
| 332 | 3 | ESP:491. |
| 333 | 3 | ESP:487. |
| 334 | 3 | ESP:457. |
| 335 | 3 | ESP:501. |
| 336 | 3 | ESP:501. |
| 337 | 3 | ESP:501. |
| 338 | 3 | ESP:489, 491. |

[Table 160]

| Ex | Syn | Data |
|---|---|---|
| 339 | 3 | ESP:515, 517. |
| 340 | 3 | ESP:537, 539. |
| 341 | 3 | ESP:569. |
| 342 | 3 | ESP:441. |
| 343 | 3 | ESP:590, 592. |
| 344 | 3 | ESP:530. |
| 345 | 3 | ESP:443. |
| 346 | 3 | ESP:491. |
| 347 | 4 | ESP:475. |
| 348 | 3 | ESP:485. |
| 349 | 3 | ESP:537. |
| 350 | 3 | ESP:475. |
| 351 | 3 | ESP:509. |
| 352 | 3 | ESP:485. |
| 353 | 3 | ESP:547. |
| 354 | 1 | ESP:439. |
| 355 | 1 | ESP:465. |
| 356 | 1 | ESP:453. |
| 357 | 1 | ESP:467. |
| 358 | 1 | ESP:469. |
| 359 | 200 | ESP:545. |
| 360 | 2 | ESN:488. |
| 361 | 3 | ESP:507. |
| 362 | 3 | ESP:491. |
| 363 | 3 | ESP:485. |
| 364 | 3 | ESP:457. |
| 365 | 3 | ESP:475. |
| 366 | 3 | ESP:481. |
| 367 | 3 | ESP:495. |
| 368 | 3 | ESP:479. |
| 369 | 3 | ESP:521. |
| 370 | 3 | ESP:605. |
| 371 | 3 | ESP:464. |
| 372 | 3 | ESN:480, 482. |
| 373 | 3 | ESP:505. |
| 374 | 3 | ESP:519. |
| 375 | 3 | ESP:535. |

[Table 161]

| Ex | Syn | Data |
|---|---|---|
| 376 | 3 | ESP:501. |
| 377 | 3 | ESP:479. |
| 378 | 3 | ESP:519, 521. |
| 379 | 3 | ESN:455. |
| 380 | 3 | ESN:473. |
| 381 | 3 | ESN:473. |
| 382 | 1 | ESN:451. |
| 383 | 1 | ESN:475. |
| 384 | 1 | ESN:475. |
| 385 | 1 | ESN:457. |
| 386 | 1 | ESP:517. |
| 387 | 1 | ESP:535. |
| 388 | 4 | ESP:479. |
| 389 | 3 | ESP:495. |
| 390 | 3 | ESP:507. |
| 391 | 3 | ESP:481. |
| 392 | 4 | ESP:461. |
| 393 | 3 | ESP:501. |
| 394 | 3 | ESP:485. |
| 395 | 3 | ESP:485. |
| 396 | 3 | ESP:535. |
| 397 | 3 | ESP:521. |
| 398 | 3 | ESP:535. |
| 399 | 3 | ESP:539. |
| 400 | 3 | ESP:513. |
| 401 | 3 | ESP:513. |
| 402 | 3 | ESP:509. |
| 403 | 3 | ESP:482. |
| 404 | 1 | ESP:469. |
| 405 | 1 | ESP:551. |
| 406 | 1 | ESP:551. |
| 407 | 1 | ESP:551. |
| 408 | 1 | ESP:531. |
| 409 | 1 | ESP:531. |
| 410 | 3 | ESP:491. |
| 411 | 3 | ESP:509. |
| 412 | 3 | ESP:527. |

[Table 162]

| Ex | Syn | Data |
|---|---|---|
| 413 | 5 | ESP:522. |
| 414 | 5 | ESP:452. |
| 415 | 5 | ESP:480. |
| 416 | 5 | ESP:432. |
| 417 | 5 | ESP:466. |
| 418 | 2 | ESP:444. |
| 419 | 2 | ESP:440. |
| 420 | 2 | ESP:494. |
| 421 | 5 | ESP:466. |
| 422 | 5 | ESP:528. |
| 423 | 5 | ESP:529. |
| 424 | 5 | ESP:529. |
| 425 | 5 | ESP:544. |
| 426 | 5 | ESP:544. |
| 427 | 5 | ESP:544. |
| 428 | 3 | ESP:497. |
| 429 | 3 | ESP:523. |
| 430 | 3 | ESP:493. |
| 431 | 3 | ESP:505. |
| 432 | 3 | ESP:505. |
| 433 | 3 | ESP:531. |
| 434 | 3 | ESP:547. |
| 435 | 3 | ESP:537. |
| 436 | 3 | ESP:537. |
| 437 | 4 | ESP:515. |
| 438 | 4 | ESP:515, 517. |
| 439 | 3 | NMR1: 0.91(3H,d),1.10-1.22(2H,m),1.48-1.71(7H,m),1.74-1.8 8(4H,m),2.11-2.19(1H,m),2.66-2.77 (2H,m),3.36-3.44(4H,m),3. 77-3.84(2H,m),4.51-4.59(1H,m),6.90(2H,d),6.99(2H,d),7.75(2 H,d),7.85 (2H,d),8.71-8.80(1H,m),8.82-8.91(1H,m). ESP:508. |
| 440 | 200 | ESN:458. |
| 441 | 3 | ESP:517. |
| 442 | 3 | ESP:528. |
| 443 | 3 | ESP:571. |
| 444 | 3 | ESP:501. |
| 445 | 3 | ESP:505. |
| 446 | 3 | ESP:517. |

[Table 163]

| Ex | Syn | Data |
|---|---|---|
| 447 | 3 | ESP:523. |
| 448 | 3 | ESP:501. |
| 449 | 3 | ESP:501. |
| 450 | 3 | ESP:501. |
| 451 | 3 | ESP:485. |
| 452 | 3 | ESP:535. |
| 453 | 3 | ESP:481. |
| 454 | 4 | NMR1:1.62-1.88(8H,m),2.34-2.44(1H,m),3.43-3.52(4H,m),4.6 6-4.73(1H.m),7.27(1H,q),7.51(1H,q), 7.56-7.64(2H,m),7.90-8.0 3(4H,m),8.28-8.34(1H,m),8.44(1H,s),8.71-8.77(1H,m),1 1.99-1 2.33(1H, m). ESP:497. |
| 455 | 4 | ESP:515. |
| 456 | 4 | ESP:515, 517. |
| 457 | 3 | ESP:497. |
| 458 | 3 | ESP:497. |
| 459 | 3 | ESP:497. |
| 460 | 3 | ESP:493. |
| 461 | 3 | ESP:487. |
| 462 | 3 | ESP:531. |
| 463 | 3 | ESP:503. |
| 464 | 3 | NM1:1.59-1.87(8H,m),2.34-2.44(1H,m),3.38-3.50(4H,m),4.6 7-4.73(1H,m),7.14(1H,t),7.39-7.49(3H, m),7.91-7.96(1H,m),7.9 9-8.07(2H,m),8.36-8.43(1H,m),8.84-8.90(1H,m),12.06-12.26(1 H,m). ESP: 503. |
| 465 | 3 | NMR1:1.61-1.87(8H,m),2.33-2.44(1H,m),3.37-3.50(4H,m),4.6 5-4.73(1H,m),7.27(1H,q),7.40-7.54 (3H,m),7.92-7.96(1H,m),8.0 0-8.06(2H,m),8.28-8.34(1H,m),8.84-8.90(1H,m),12.06-12.28(1 H,m). ESP:503. |
| 466 | 3 | ESP:485. |
| 467 | 3 | ESP:431. |
| 468 | 3 | ESP:493. |
| 469 | 3 | NMR1:1.60-1.86(8H,m),2.32-2.44(1H,m),3.44-3.54(4Hm),4.5 8-4.66(1H,m),7.00(2H,d),7.55-7.65 (2H,m),7.81(2H,d),7.86-7.9 3(1H,m),8.07-8.15(1H,m),8.42-8.56(2H,m),11.8-12.6(1H,br). ESP:501. |

[Table 164]

| Ex | Syn | Data |
|---|---|---|
| 470 | 3 | NMR1:1.60-1.86(8H,m),2.30-2.44(1H,m),3.38-3.48(4H,m),4.5 6-4.66(1H,m),7.00(2H,d),7.28-7.36 (1H,m),7.81(2H,d),7.91-8.0 1(2H,m),8.05(1H,s),8.44-8.51(1H,m),8.87-8.94(1H,m),11.9-12. 3(1H,br). ESP:485. |
| 471 | 3 | ESP:551. |
| 472 | 3 | ESP:498. |
| 473 | 3 | ESP:451. |

(continued)

| Ex | Syn | Data |
|---|---|---|
| 474 | 3 | ESP:493. |
| 475 | 5 | ESN:484. |
| 476 | 5 | ESN:484. |
| 477 | 5 | ESN:484. |
| 478 | 5 | ESP:542, 544. |
| 479 | 5 | ESP:564, 566. |
| 480 | 5 | ESP:564, 566. |
| 481 | 5 | ESP:564, 566. |
| 482 | 5 | ESP:546, 548. |
| 483 | 5 | ESP:546, 548. |
| 484 | 3 | ESP:503. |
| 485 | 3 | ESP:503. |
| 486 | 3 | NMR1:1.64-1.88(8H,m),2.33-2.43(1H,m),3.40-3.52(4H,m),4.6 5-4.73(1H,m),7.27(1H,q),7.48-7.58 (2H,m),7.80-7.88(2H,m),8.0 9(1H,d),8.27-8.33(1H,m),8.38(1H,d),8.63-8.69(1H,m),12.08-1 2.27(1H, m). ESP:503. |
| 487 | 3 | ESP:485. |
| 488 | 3 | ESP:485. |
| 489 | 3 | ESP:487, 489. |
| 490 | 3 | NMR1:1.64-1.87(8H,m),2.35-2.44(1H,m),3.35-3.50(4H,m),4.6 7-4.75(1H,m),7.14(1H,t),7.18(1H,d), 7.40(1H,t),7.60(1H,d),8.3 4-8.43(1H,m),8.67-8.80(1H,m),12.06-12.26(1H,m). ESP:487, 489. |
| 491 | 3 | ESP:487. |
| 492 | 3 | ESP:469, 471. |
| 493 | 3 | ESP:469, 471. |
| 494 | 1 | ESP:453. |
| 495 | 1 | ESP:521. |
| 496 | 5 | ESN:434. |

[Table 165]

| Ex | Syn | Data |
|---|---|---|
| 497 | 5 | ESP:460. |
| 498 | 5 | ESP:480. |
| 499 | 3 | ESN:478. |
| 500 | 3 | ESN:492. |
| 501 | 5 | ESP:462. |
| 502 | 5 | ESP:488. |
| 503 | 5 | ESN:526. |
| 504 | 5 | ESN:544. |
| 505 | 5 | ESN:452. |

(continued)

| Ex | Syn | Data |
|---|---|---|
| 506 | 5 | ESN:470. |
| 507 | 5 | ESN:484. |
| 508 | 3 | ESP:483. |
| 509 | 3 | ESP:483. |
| 510 | 3 | ESP:483. |
| 511 | 3 | ESP:483. |
| 512 | 3 | ESP:499, 501. |
| 513 | 3 | ESP:499, 501. |
| 514 | 3 | ESP:499, 501. |
| 515 | 3 | ESP:465. |
| 516 | 3 | ESP:537, 539. |
| 517 | 3 | ESP:537, 539. |
| 518 | 3 | ESP:537, 539. |
| 519 | 3 | ESP:521. |
| 520 | 3 | ESP:521. |
| 521 | 3 | ESP:537, 539. |
| 522 | 3 | ESP:537, 539. |
| 523 | 3 | ESP:537, 539. |
| 524 | 3 | ESP:521. |
| 525 | 3 | ESP:521. |
| 526 | 3 | ESP:521. |
| 527 | 3 | ESP:521. |
| 528 | 3 | ESP:519. |
| 529 | 3 | ESP:519. |
| 530 | 3 | ESP:519. |

[Table 166]

| Ex | Syn | Data |
|---|---|---|
| 531 | 200 | NMR1:1.60-1.86(8H,m),2.30-2.44(1H,m),3.38-3.50(4H,m),4.5 8-4.68(1H,m),6.82-6.94(2H,m), 7.31-7.39(1H,m),7.63(1H,t),7.7 7-7.83(1H,m),8.03(1H,s),8.04-8.10(1H,m),8.14-8.22(1H,m),8.8 8-8.96(1H,m),11.9-12.4(1H,br). ESP:503. |
| 532 | 3 | ESP:503. |
| 533 | 3 | ESP:519. |
| 534 | 3 | ESP:519. |
| 535 | 3 | ESP:519. |
| 536 | 3 | ESP:503. |
| 537 | 3 | ESP:503. |

(continued)

| Ex | Syn | Data |
|---|---|---|
| 538 | 3 | ESP:537. |
| 539 | 3 | ESP:537. |
| 540 | 3 | ESP:537. |
| 541 | 3 | ESP:491. |
| 542 | 3 | ESP:526. |
| 543 | 3 | ESP:512. |
| 544 | 3 | ESP:498. |
| 545 | 3 | ESN:542. |
| 546 | 3 | ESN:528. |
| 547 | 3 | ESN:514. |
| 548 | 3 | ESN:482. |
| 549 | 3 | ESP:503. |
| 550 | 3 | NMR1:1.60-1.88(8H,m),2.30-2.44(1H,m),3.39-3.53(4H,m),4.6 6-4.77(1H,m),7.14(1H,t),7.31-7,53 (3H,m),7.83(1H,d),8.15(1H, d),8.37-8.44(1H,m),8.96-9.04(1H,m),11.9-12.3(1H,br). ESP:521. |
| 551 | 3 | ESP:521. |
| 552 | 3 | ESP:521. |
| 553 | 3 | ESP:530. |
| 554 | 3 | NMR1:0.91(3H,d),1.08-1.22(2H,m),1.48-1.87(11H,m),2.34-2. 43(1H,m),2.66-2.79(2H,m),3.36-3.44 (4H,m),3.76-3.86(2H,m), 4.65-4.72(1H,m),6.92(2H,d),7.27(1H,q),7.50(1H,q),7.69(2H,d), 8.22-8.29 (2H,m),12.08-12.28(1H,m). ESP:544. |
| 555 | 3 | ESP:516. |
| 556 | 3 | ESP:503. |
| 557 | 3 | ESP:503. |

[Table 167]

| Ex | Syn | Data |
|---|---|---|
| 558 | 3 | ESP: 521. |
| 559 | 3 | ESP:521. |
| 560 | 3 | ESP:521. |
| 561 | 3 | ESN:514. |
| 562 | 3 | ESN:528. |
| 563 | 3 | ESN:542. |
| 564 | 3 | ESP:503. |
| 565 | 5 | ESP:454. |
| 566 | 1 | ESP:526. |
| 56 | 1 | ESP:512. |
| 568 | 1 | ESP:498. |
| 569 | 1 | ESP:493. |

(continued)

| Ex | Syn | Data |
|---|---|---|
| 570 | 4 | ESP:493. |
| 571 | 4 | NMR1:1.22-1.37(2Hm),1.49-1.67(4H,m),1.71-1.91(3H,m),2.1 4(2H,d),3.40-3.52(4H,m),4.69-4.78 (1H.m),7.25(1H,q),7.51(1H, q),7.56-7.64(2H,m),7.90-8.00(4H,m),8.28-8.33(1H,m),8.44(1H, s), 8.72-8.77(1H,m),11.90-12.08(1H,m). ESP:511. |
| 572 | 3 | ESP: 483. |
| 573 | 3 | ESP:499. |
| 574 | 3 | ESP:499. |
| 575 | 3 | ESP:561. |
| 576 | 3 | ESP:473. |
| 577 | 3 | ESP:465. |
| 578 | 5 | ESN:470. |
| 579 | 5 | ESN:510. |
| 580 | 5 | ESN:556. |
| 581 | 5 | ESN:522. |
| 582 | 3 | ESN:460. |
| 583 | 3 | ESN:461. |
| 584 | 3 | ESN:460. |
| 585 | 3 | ESN:460. |
| 586 | 3 | ESN:466. |
| 587 | 3 | ESN:467. |
| 588 | 3 | ESN:460. |
| 589 | 3 | ESN:507. |
| 590 | 200 | ESP:479. |
| 591 | 200 | ESP:496. |

[Table 168]

| Ex | Syn | Data |
|---|---|---|
| 592 | 200 | ESP:494. |
| 593 | 200 | ESP:496. |
| 594 | 200 | ESP:494. |
| 595 | 200 | ESP:497. |
| 596 | 3 | ESP:496. |
| 597 | 200 | ESP:511. |
| 598 | 3 | ESP:501. |
| 599 | 203 | ESP:488. |
| 600 | 203 | ESP:530. |
| 601 | 203 | ESP:543. |

(continued)

| Ex | Syn | Data |
|---|---|---|
| 602 | 200 | ESP:536, 538. |
| 603 | 200 | ESN:493. |
| 604 | 200 | ESP:536, 538. |
| 605 | 200 | ESN:493. |
| 606 | 200 | ESP:518, 520. |
| 607 | 200 | ESN:475. |
| 608 | 200 | ESP:478. |
| 609 | 200 | ESP:476. |
| 610 | 203 | ESP:536. |
| 611 | 203 | ESP:550. |
| 612 | 3 | ESN:460. |
| 613 | 4 | ESP:497. |
| 614 | 3 | ESP:495, 497. |
| 615 | 3 | ESP:487. |
| 616 | 3 | ESP:530. |
| 617 | 3 | ESN:496. |
| 618 | 200 | ESN:495. |
| 619 | 3 | ESN:457. |
| 620 | 200 | ESP:486. |
| 621 | 3 | ESP:503. |
| 622 | 3 | ESP:537, 539. |
| 623 | 3 | ESP:521. |
| 624 | 3 | ESP:487. |
| 625 | 3 | ESP:495, 497. |
| 626 | 3 | ESP:487. |
| 627 | 3 | ESP:503. |
| 628 | 3 | ESN:449. |

[Table 169]

| Ex | Syn | Data |
|---|---|---|
| 629 | 200 | ESP:497. |
| 630 | 200 | ESP:487, 489. |
| 631 | 200 | ESP:503. |
| 632 | 5 | ESN:478. |
| 633 | 200 | ESP:511. |
| 634 | 200 | ESP:509. |
| 635 | 200 | ESP:501. |

(continued)

| Ex | Syn | Data |
|---|---|---|
| 636 | 200 | ESP:510. |
| 637 | 1 | ESP:460. |
| 638 | 202 | ESP:477. |
| 639 | 200 | ESP:447. |
| 640 | 200 | ESN:443. |
| 641 | 200 | ESP:487. |
| 642 | 200 | ESN:435. |
| 643 | 200 | ESP:447. |
| 644 | 200 | ESP:445. |
| 645 | 200 | ESP:487. |
| 646 | 200 | ESP:437. |
| 647 | 6 | ESP:491. |
| 648 | 201 | ESP:515. |
| 649 | 200 | ESP:459. |
| 650 | 3 | ESN:466. |
| 651 | 1 | ESP:459. |
| 652 | 210 | ESP:500. |

Industrial Applicability

The compound of the formula (I) or a salt thereof has a DRAT1 inhibitory action, and can be therefore used as an agent for preventing and/or treating obesity, type II diabetes mellitus, fatty liver, and diseases associated with these diseases.

## Claims

**1.** A compound of the formula (I) or a salt thereof:

[Chem. 12]

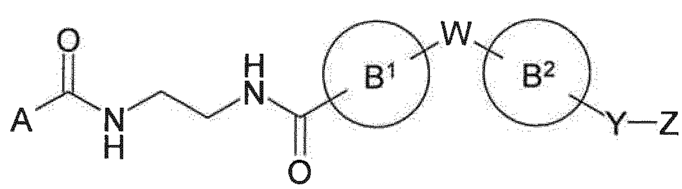

(I)

(wherein A represents aryl which may be substituted, cycloalkyl which may be substituted, an aromatic heterocycle which may be substituted, a non-aromatic heterocycle which may be substituted, or a group represented by the formula (II):

[Chem. 13]

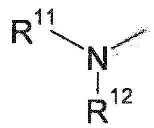

(II)

in which $R^{11}$ and $R^{12}$ are the same as or different from each other, and represent -H, $C_{1-6}$ alkyl, aryl which may be substituted, or $C_{3-8}$ cycloalkyl which may be substituted, provided that $R^{11}$ and $R^{12}$ are not -H at the same time, and

$R^{11}$ and $R^{12}$ may be combined with the nitrogen atom to which they bind to form cyclic amino which may be substituted,

Ring $B^1$ represents phenylene, pyridinediyl, naphthalenediyl, or 1,2,3,4-tetrahydronaphthalenediyl, each of which may be substituted with at least one group selected from the group consisting of -OH, $C_{1-6}$ alkyl which may be substituted with at least one halogen atom, -O-$C_{1-6}$ alkyl which may be substituted with at least one halogen atom, $C_{3-8}$ cycloalkyl, and halogen,

W represents -O-, a bond, -O-$C_{1-6}$ alkylene, -NH-, or $C_{1-6}$ alkylene,

Ring $B^2$ represents cyclohexanediyl, cyclopentanediyl, or a bridged ring, each of which may be substituted with $C_{1-6}$ alkyl, and in the case where W is a bond, it may represent piperidinediyl or 8-azabicyclo[3.2.1]octanediyl,

Y represents a bond, $C_{1-6}$ alkylene, or -O-$C_{1-6}$ alkylene, and

Z represents -$CO_2$H or a biological equivalent thereof; carbamoyl which may be substituted with one or two groups selected from $C_{1-6}$ alkyl (in which the $C_{1-6}$ alkyl may be substituted with amino or carboxyl), phenyl, and benzyl; -CO-(cyclic amino which may be substituted with one or two $C_{1-6}$ alkyl groups); -OH; amino which may be substituted with one or two $C_{1-6}$ alkyl groups; -NH-C(=O)-$C_{1-6}$ alkyl; or -NH-C(=O)-$C_{3-8}$ cycloalkyl).

**2.** The compound or a salt thereof as set forth in claim 1, wherein A is aryl which may be substituted, cycloalkyl which may be substituted, an aromatic heterocycle which may be substituted, a non-aromatic heterocycle which may be substituted, or a group represented by the formula (II), $R^{11}$ and $R^{12}$ are the same as or different from each other, and represent -H, aryl which may be substituted, or $C_{3-8}$ cycloalkyl which may be substituted, provided that $R^{11}$ and $R^{12}$ are not -H at the same time, in which $R^{11}$ and $R^{12}$ may be combined with the nitrogen atom to which they bind to form cyclic amino which may be substituted, Ring $B^1$ represents a group represented by the formula (III):

[Chem. 14]

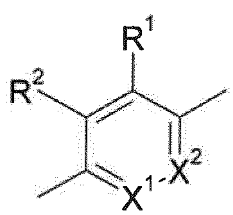

(III)

wherein $X^1$ represents N or $CR^3$, $X^2$ represents N or $CR^4$, $R^1$, $R^2$, $R^3$, and $R^4$ are the same as or different from each other, and represent -H, -OH, $C_{1-6}$ alkyl which may be substituted with at least one halogen atom, -O-$C_{1-6}$ alkyl which may be substituted with at least one halogen atom, $C_{3-8}$ cycloalkyl, or halogen, W is -O- or a bond, Ring $B^2$ represents cyclohexane-1,4-diyl, Y represents a bond or $C_{1-6}$ alkylene, and Z represents -$CO_2$H or a biological equivalent thereof, or -$CONH_2$.

**3.** The compound or a salt thereof as set forth in claim 1, wherein Ring $B^1$ is 1,4-phenylene which may be substituted with at least one halogen atom, W is -O-, Ring $B^2$ is cyclohexane-1,4-diyl, Y is a bond or methylene, and Z is -$CO_2$H

**4.** The compound or a salt thereof as set forth in claim 3, wherein Ring $B^1$ is 1,4-phenylene which may be substituted with one or two fluorine atoms.

**5.** The compound or a salt thereof as set forth in claim 4, wherein Y is a bond.

6. The compound or a salt thereof as set forth in claim 1, which is
cis-4-[4-({2-[(4-cyclopropylbenzoyl)amino]ethyl}carbamoyl)phenoxy]cyclohexanecarboxylic acid,
cis-4-(4-{[2-(2-naphthoylamino)ethyl]carbamoyl}phenoxy)cyclohexanecarboxylic acid,
cis-4-[4-({2-[(4-chloro-3-methylbcnzoyl)amino]ethyl}carbamoyl)phenoxy]cyclohexanecarboxylic acid,
cis-4-(3-fluoro-4-{[2-(2-naphthoylamino)ethyl]carbamoyl}phenoxy)cyclohexanecarboxylic acid,
cis-4-(3,5-difluoro-4- {[2-(2-naphthoylamino)ethyl]carbamoyl}phenoxy)cyclohexanecarboxylic acid,
cis-4-(2,3-difluoro-4-{[2-(2-naphthoylamino)ethyl]carbamoyl}phenoxy)cyclohexanecarboxylic acid,
cis-4-(2,5-difluoro-4-{[2-(2-naphthoylamino)ethyl]carbamoyl}phenoxy)cyclohexanecarboxylic acid,
cis-4-f4-[(2-{[(3-chloro-1-benzothiophen-2-yl)carbonyl]amino}ethyl)carboyl]phenoxy}cyclohexanecarboxylic acid,
cis-4-{4-[(2-{[(5-chlorothiophen-2-yl)carbonyl]amino}ethyl)carbamoyl]-2,3-difluorophenoxy}cyclohexanecarboxylic acid,
cis-4-{3-fluoro-4-[(2-{[(5-fluoro-1-benzothiophen-2-yl)carbonyl]amino}ethyl)carbamoyl]phenoxy}cyclohexanecarboxylic acid, or
[cis-4-(2,5-difluoro-4-{[2-(2-naphthoylamino)ethyl]carbamoyl}phenoxy)cyclohexyl]acetic acid, or
a salt thereof.

7. A pharmaceutical composition comprising the compound or a salt thereof as set forth in claim 1 and a pharmaceutically acceptable excipient.

8. A pharmaceutical composition for preventing or treating obesity, comprising the compound or a salt thereof as set forth in claim 1.

9. Use of the compound or a salt thereof as set forth in claim 1 for the manufacture of a pharmaceutical composition for preventing or treating obesity.

10. The compound or a salt thereof as set forth in claim 1, which is used for preventing or treating obesity.

11. A method for preventing or treating obesity, comprising administering to a subject an effective amount of the compound or a salt thereof as set forth in claim 1.

**INTERNATIONAL SEARCH REPORT**

International application No.
PCT/JP2010/056901

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus, REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2008/011131 A2 (TAKEDA PHARMACEUTICAL CO., LTD.), 24 January 2008 (24.01.2008), & JP 2009-544616 A & EP 2044055 A | 1-10 |
| A | JP 3-77881 A (Yoshitomi Pharmaceutical Industries, Ltd.), 03 April 1991 (03.04.1991), (Family: none) | 1-10 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 May, 2010 (25.05.10) | 08 June, 2010 (08.06.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2010/056901

Continuation of A. CLASSIFICATION OF SUBJECT MATTER
(International Patent Classification (IPC))

*C07C235/46*(2006.01)i, *A61K31/343*(2006.01)i, *A61K31/351*(2006.01)i, *A61K31/357*(2006.01)i, *A61K31/36*(2006.01)i, *A61K31/381*(2006.01)i, *A61K31/40*(2006.01)i, *A61K31/402*(2006.01)i, *A61K31/4035*(2006.01)i, *A61K31/404*(2006.01)i, *A61K31/41*(2006.01)i, *A61K31/415*(2006.01)i, *A61K31/421*(2006.01)i, *A61K31/4245*(2006.01)i, *A61K31/425*(2006.01)i, *A61K31/427*(2006.01)i, *A61K31/428*(2006.01)i, *A61K31/4365*(2006.01)i, *A61K31/439*(2006.01)i, *A61K31/44*(2006.01)i, *A61K31/445*(2006.01)i, *A61K31/451*(2006.01)i, *A61K31/4535*(2006.01)i, *A61K31/4545*(2006.01)i, *A61K31/455*(2006.01)i, *A61K31/47*(2006.01)i, *A61K31/495*(2006.01)i, *A61K31/4985*(2006.01)i, *A61K31/505*(2006.01)i, *A61K31/5375*(2006.01)i, *A61P1/16*(2006.01)i, *A61P3/04*(2006.01)i, *A61P3/10*(2006.01)i, *A61P43/00*(2006.01)i, *C07C235/84*(2006.01)i, *C07C237/34*(2006.01)i, *C07C255/46*(2006.01)i, *C07C275/26*(2006.01)i, *C07C275/28*(2006.01)i, *C07C307/06*(2006.01)i, *C07C311/51*(2006.01)i, *C07C317/44*(2006.01)i, *C07C323/62*(2006.01)i, *C07D207/06*(2006.01)i, *C07D209/08*(2006.01)i, *C07D209/42*(2006.01)i, *C07D209/44*(2006.01)i, *C07D211/16*(2006.01)i, *C07D211/34*(2006.01)i, *C07D211/44*(2006.01)i, *C07D211/60*(2006.01)i, *C07D213/81*(2006.01)i, *C07D213/82*(2006.01)i, *C07D215/48*(2006.01)i, *C07D217/26*(2006.01)i, *C07D231/22*(2006.01)i, *C07D239/42*(2006.01)i, *C07D241/04*(2006.01)i, *C07D257/04*(2006.01)i, *C07D263/32*(2006.01)i, *C07D263/34*(2006.01)i, *C07D263/48*(2006.01)i, *C07D271/06*(2006.01)i, *C07D271/10*(2006.01)i, *C07D275/02*(2006.01)i, *C07D277/20*(2006.01)i, *C07D277/56*(2006.01)i, *C07D277/68*(2006.01)i, *C07D295/14*(2006.01)i, *C07D295/18*(2006.01)i, *C07D295/20*(2006.01)i, *C07D307/79*(2006.01)i, *C07D309/06*(2006.01)i, *C07D317/46*(2006.01)i, *C07D319/18*(2006.01)i, *C07D333/38*(2006.01)i, *C07D333/54*(2006.01)i, *C07D333/68*(2006.01)i, *C07D333/70*(2006.01)i, *C07D451/02*(2006.01)i, *C07D495/04*(2006.01)i

(According to International Patent Classification (IPC) or to both national classification and IPC)

Continuation of B. FIELDS SEARCHED
Minimum documentation searched (International Patent Classification (IPC))

C07C235/46, A61K31/343, A61K31/351, A61K31/357, A61K31/36, A61K31/381, A61K31/40, A61K31/402, A61K31/4035, A61K31/404, A61K31/41, A61K31/415, A61K31/421, A61K31/4245, A61K31/425, A61K31/427, A61K31/428, A61K31/4365, A61K31/439, A61K31/44, A61K31/445, A61K31/451, A61K31/4535, A61K31/4545, A61K31/455, A61K31/47, A61K31/495, A61K31/4985, A61K31/505, A61K31/5375, A61P1/16, A61P3/04, A61P3/10, A61P43/00, C07C235/84, C07C237/34, C07C255/46, C07C275/26, C07C275/28, C07C307/06, C07C311/51, C07C317/44, C07C323/62, C07D207/06, C07D209/08, C07D209/42, C07D209/44, C07D211/16, C07D211/34, C07D211/44, C07D211/60, C07D213/81, C07D213/82, C07D215/48, C07D217/26, C07D231/22, C07D239/42, C07D241/04, C07D257/04, C07D263/32, C07D263/34, C07D263/48, C07D271/06, C07D271/10, C07D275/02, C07D277/20, C07D277/56, C07D277/68, C07D295/14, C07D295/18, C07D295/20, C07D307/79, C07D309/06, C07D317/46, C07D319/18, C07D333/38, C07D333/54, C07D333/68, C07D333/70, C07D451/02, C07D495/04

Minimum documentation searched (classification system followed by classification symbols)

**INTERNATIONAL SEARCH REPORT**

International application No.
PCT/JP2010/056901

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 11
because they relate to subject matter not required to be searched by this Authority, namely:
The claim pertains to a method for treatment of the human body by therapy and thus relates to a subject matter on which the International Searching Authority is not required to carry out a search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*



### Patent documents cited in the description


- WO 2006082952 A
- WO 2008011130 A
- WO 2008011131 A
- WO 2007141517 A
- WO 2007138304 A
- WO 2007138311 A
- WO 2006064189 A
- WO 2009076618 A
- WO 2007115935 A


### Non-patent literature cited in the description


- *Proc. Nat. Acad. Sci.,* 1998, vol. 95, 13018-13023
- *J. Biol. Chem.,* 2001, vol. 276, 38870-38876
- *Nature Genetics,* 2000, vol. 25, 87-90
- *J. Clin. Invest.,* 2002, vol. 109, 1049-1055
- *Arterioscler. Thromb. Vasc. Biol.,* 2005, vol. 25, 482-486
- *chemical Research in Chinese Universities,* 2009, vol. 25 (2), 178-182
- *Prog. Med.,* 1985, vol. 5, 2157-2161
- Pharmaceutical Research and Development. Drug Design. Hirokawa Publishing Company, 1990, vol. 7, 163-198
- **P. G. M. Wuts ; T. W. Greene.** Greene's Protective Groups in Organic Synthesis. 2006
- Greene's Protective Groups in Organic Synthesis. 2006
- *Journal of Medicinal Chemistry,* 1993, vol. 36 (24), 3968-3970